(19) **Europäisches Patentamt / European Patent Office / Office européen des brevets**

(11) **EP 2 315 781 B1**

(12) **FASCICULE DE BREVET EUROPEEN**

(45) Date de publication et mention
de la délivrance du brevet:
**06.05.2015 Bulletin 2015/19**

(21) Numéro de dépôt: **09784478.1**

(22) Date de dépôt: **07.07.2009**

(51) Int Cl.:
*C07K 16/28* [(2006.01)]     *C12N 15/13* [(2006.01)]
*C12N 5/18* [(2006.01)]     *A61K 39/395* [(2006.01)]
*A61P 35/00* [(2006.01)]     *C07K 16/30* [(2006.01)]
*A61K 39/00* [(2006.01)]

(86) Numéro de dépôt international:
**PCT/FR2009/051343**

(87) Numéro de publication internationale:
**WO 2010/004204 (14.01.2010 Gazette 2010/02)**

(54) **ANTAGONISTES SPECIFIQUES DU RECEPTEUR FGF-R4**

FÜR DEN FGF-R4-REZEPTOR SPEZIFISCHE ANTAGONISTEN

FGF-R4 RECEPTOR-SPECIFIC ANTAGONISTS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **08.07.2008 FR 0803888**

(43) Date de publication de la demande:
**04.05.2011 Bulletin 2011/18**

(73) Titulaire: **SANOFI**
**75008 Paris (FR)**

(72) Inventeurs:
• **BAURIN, Nicolas**
**F-75013 Paris (FR)**
• **BERNE, Pierre-François**
**F-75013 Paris (FR)**
• **BLANCHE, Francis**
**F-75013 Paris (FR)**
• **BONO, Françoise**
**F-75013 Paris (FR)**
• **CAMERON, Béatrice**
**F-75013 Paris (FR)**

• **DABDOUBI, Tarik**
**F-75013 Paris (FR)**
• **HERBERT, Corentin**
**F-75013 Paris (FR)**
• **MIKOL, Vincent**
**F-75013 Paris (FR)**
• **REMY, Elisabeth**
**F-75013 Paris (FR)**

(74) Mandataire: **Bouvet, Philippe et al**
**Sanofi**
**Département Brevets**
**54, rue La Boétie**
**75008 Paris (FR)**

(56) Documents cités:
**WO-A-2008/052796     WO-A-2008/052798**

• **RUSNATI M ET AL: "Fibroblast growth factors/ fibroblast growth factor receptors as targets for the development of anti-angiogenesis strategies.", CURRENT PHARMACEUTICAL DESIGN 2007 LNKD- PUBMED:17627537, vol. 13, no. 20, 2007, pages 2025-2044, ISSN: 1873-4286**

EP 2 315 781 B1

**Description**

La présente invention a pour objet des antagonistes spécifiques du récepteur 4 aux FGF (FGF-R4) permettant d'inhiber l'activité de ce récepteur. Ces antagonistes sont en particulier des anticorps dirigés spécifiquement contre le récepteur 4 aux FGF (FGF-R4).

La présente invention a également pour objet l'utilisation thérapeutique de ces antagonistes, en particulier dans le domaine de l'angiogenèse et dans le traitement de certains types de cancer.

Les FGF (Fibroblast Growth Factor) comptent parmi les premières molécules décrites comme étant capables de stimuler la prolifération, la migration et la différenciation des cellules vasculaires *in vitro* et *in vivo.* Une littérature abondante décrit l'induction de l'angiogenèse et la formation de capillaires sanguins *in vitro* et *in vivo* par les FGF. Les FGF sont également impliqués dans l'angiogenèse tumorale en promouvant la formation de vaisseaux sanguins recrutés par la tumeur.

La famille des FGF humains est composée d'au moins 23 membres qui possèdent tous un domaine central conservé de 120 acides aminés. Ils exercent leur activité biologique en interagissant avec leurs récepteurs de haute affinité de type tyrosine-kinase (FGF-R) et des héparanes sulfate protéoglycanes, composants présents sur la plupart des surfaces cellulaires et des matrices extracellulaires (site de liaison de faible affinité) pour former un complexe ternaire. Certains FGF présentent une haute affinité pour plusieurs FGF-R alors que d'autres activent spécifiquement un récepteur ou un isoforme d'un récepteur.

Un ligand spécifique de FGF-R4 a été identifié par Xie et al (Cytokine, 1999, 11:729-35.) Ce ligand, appelé FGF19, est un ligand de haute affinité au FGF-R4 exclusivement et dont la liaison au récepteur est dépendante de l'héparine ou des héparanes sulfatés. Le FGF19 a été identifié chez l'animal adulte, uniquement au niveau des hépatocytes et de l'intestin grêle où il régule la synthèse d'acide biliaire par le foie. Il serait un facteur de croissance intervenant lors du développement embryonnaire et serait impliqué dans le développement du cerveau foetal chez le poisson zèbre et l'homme.

D'autres ligands de FGF-R4 sont décrits comme le FGF1 ou FGF2. Ces ligands activent fortement le FGF-R4 mais ne sont pas spécifiques de FGF-R4 : ils se lient aussi à d'autres FGF-R (Ornitz et al., J. Biol. Chem., 1996, 271:15292-7).

L'activation du récepteur FGF-R4 conduit à plusieurs types de signalisations cellulaires. Parmi celles-ci, la forme la plus classique correspondant à la mise en place d'une voie de signalisation par cascade de phosphorylation à la suite d'une stimulation de FGF-R4 par du FGF. Cette induction conduit à l'autophosphorylation du domaine tyrosine kinase de FGF-R4 et sert à initier une voie de signalisation intracellulaire dépendante des phosphorylations d'autres protéines de signalisation telles que AKT, p44/42, JNK etc... Cette signalisation par phosphorylation varie selon le type cellulaire et selon les co-récepteurs ou les molécules d'adhésion présents à la surface de ces cellules. (Cavallaro et al., Nat. Cell Biol. 3/7, 650-657 (2001) ; Stadler et al., Cell. Signal. 18/6, 783-794. (2006) ; Lin et al., J Biol Chem., 2007, 14:27277-84. (2007)). Un autre mode de signalisation important pour les FGF-R dont FGF-R4 est l'internalisation du récepteur après activation en association avec son ligand. Ce mécanisme n'est pas dépendant de l'activité tyrosine kinase du récepteur mais d'une courte séquence en C-terminale de FGF-R4 (Klingenberg et al., J. Cell Sci., 113/Pt10 :1827-1838 (2000)).

Quatre formes distinctes de FGF-R4 sont décrites dans la littérature. Une forme de pleine taille avec 2 variants polymorphiques à la position 388 à savoir le FGF-R4 Gly388 qui est la forme normale du récepteur et la forme Arg388 qui est décrite dans le cadre de plusieurs tumeurs (Bange et al., Cancer Res. 62/3, 840-847 (2002) ; Spinola et al., J Clin Oncol 23, 7307-7311 (2005) ; Stadler et al., Cell. Signal. 18/6, 783-794. (2006)). Une forme soluble a également été découverte exprimée dans des cellules tumorales mammaires (Takaishi et al., Biochem Biophys Res Commun., 2000, 267:658-62). Une quatrième forme, tronquée dans la partie extracellulaire, a été décrite dans certains adénomes hypophysaires (Ezzat et al., J Clin Invest., 2002, 109:69-78).

Le FGF-R4 est principalement exprimé dans les tissus dérivés de l'endoderme tels que le tractus gastro-intestinal, le pancréas, le foie, les muscles et les glandes surrénales.

Le FGF-R4 est connu dans la littérature comme ayant plusieurs rôles cellulaires, dont les trois principaux sont décrits dans ce qui suit :

Premièrement, ce récepteur est impliqué dans le contrôle de différents processus de différenciation cellulaire *in vitro* et *in vivo* comme la différenciation et la régénération des muscles squelettiques, la différenciation du tissu mésenchymateux , l'ostéogenèse ou encore dans la formation des alvéoles au cours du développement hépatique post-natal.

Deuxièmement, le FGF-R4 est décrit dans le contrôle de l'homéostasie de l'acide biliaire et du cholestérol et interviendrait dans le contrôle de l'adiposité. De plus, la balance entre la production de l'acide biliaire et celle du cholestérol est contrôlée par FGF-R4 *in vitro* et *in vivo.*

Troisièmement, le FGF-R4 est impliqué dans certains phénomènes tumoraux comme le développement des carcinomes hépatocellulaires ou des cancers du colon, dans la prolifération de cellules de fibroadénomes mammaires, de cellules épithéliales de cancers mammaires , comme la motilité des cellules de carcinomes mammaires ou colorectaux L'implication tumorale du FGF-R4 est majoritairement associée à l'apparition du polymorphisme (Gly388Arg) corrélée à l'accélération de la progression tumorale de tumeurs mammaires et colorectales (Bange et al., Cancer Res. 62/3, 840-847.2002), prostatiques (Wang et al., Clin. Cancer Res.10/18, 6169-6178, 2004) ou hépatiques (Nicholes et al., Am. J. PAthol.160/6, 2295-307, 2002). Ce polymorphisme est aussi associé à un mauvais pronostic dans le cadre de sarcomes (Morimoto et al., Cancer 98/10, 2245-2250, 2003), d'adénocarcinomes pulmonaires (Spinola et al., J Clin Oncol 23, 7307-7311, 2005) ou de sarcomes squameux (da Costa Andrade et al., Exp Mol Pathol 82, 53-7, 2007). La surexpression de FGF-R4 est aussi décrite dans certaines lignées de cancers pancréatiques (Shah et al., Oncogene 21/54, 8251-61, 2002) et corrèle avec la malignité d'astrocytomes (Yamada et al., Neurol. Res. 24/3, 244-8, 2002). De plus, l'utilisation d'un anticorps monoclonal anti-FGF19 dans des modèles *in vivo* de xénogreffes de tumeurs de colon ou dans des modèles de carcinomes hépatocellulaires, montre que l'inactivation du FGF19 et donc le blocage de l'activation de FGF-R4 peut être bénéfique dans le traitement de cancer du colon ou du foie.

Il est admis dans la littérature que les couples FGF/FGF-R1 et FGF/FGF-R2 participent à la formation des nouveaux vaisseaux sanguins dans un contexte normal ou pathologique. Cependant, l'implication potentielle de FGF-R4 dans le contrôle de ce phénomène cellulaire n'a jamais été étudiée. Il a, en effet, jusqu'à présent été supposé que l'activation de l'angiogenèse est médiée par FGF-R1 et/ou FGF-R2 (Presta et al, Cytokine Growth Factor Rev., 2005, 16:159-78).

Des exemples d'antagonistes de FGF-R4 sont décrits dans la littérature, notamment: des petites molécules, mais elles ne ciblent pas le FGF-R4 de manière spécifique, entraînant ainsi des effets indésirables. Ainsi des petites molécules chimiques inhibitrices du domaine tyrosine-kinase qui inhibent plusieurs FGF-R ainsi que d'autres récepteur tyrosine-kinase ont été décrites par Thompson et al (Thompson et al J Med Chem., 2000, 43:4200-11.). Des petites molécules chimiques inhibitrices des FGF-R par association à leur partie extracellulaire ont aussi été décrites dans la demande WO20077080325.

Des anticorps ont aussi été étudiés, comme les anticorps anti-FGFR1 et/ou anti-FGFR4 décrits dans les demandes internationales WO2005/066211 ou par Chen et al (Hybridoma 24/3, 152-159, 2005). La demande WO2005/037235 décrit des anticorps antagonistes des FGF-R pour le traitement de l'obésité et du diabète D'autre part, des anticorps anti-FGF-R4 agonistes sont décrits dans la demande WO03/063893.

Plus récemment un anticorps spécifique du FGFR4, nommé 10F10, a été décrit dans les demandes WO2008/052796 et WO2008/052798 ; celui-ci est capable d'induire l'apoptose de cellules cancéreuses d'origine rénale et de cellules neuronales.

La présente invention a pour objet un antagoniste du récepteur FGF-R4, caractérisé en ce qu'il se lie spécifiquement à celui-ci.

De manière avantageuse, ledit antagoniste est un anticorps spécifique du récepteur FGF-R4.

Dans un mode de réalisation, l'antagoniste objet de l'invention se lie au domaine D2-D3 du récepteur FGF-R4. Dans un mode de réalisation avantageux, l'antagoniste objet de l'invention se lie au domaine D2 du récepteur FGF-R4. Dans un mode encore plus avantageux, l'antagoniste se lie à la séquence SEQ ID NO 70.

De manière avantageuse l'antagoniste spécifique du récepteur FGF-R4 a un $K_D$ vis à vis du récepteur FGF-R4 déterminé par la technique Biacore inférieur à 10E-8 M, inférieur à 5 x 10E-9M, inférieur à 2 x 10E-9M ou inférieur à 1 x 10E-9M.

Dans un mode de réalisation avantageux l'antagoniste spécifique du récepteur FGF-R4 est actif à la fois contre FGF-R4 humain et FGF-R4 murin.

Dans un autre mode de réalisation avantageux l'antagoniste spécifique du récepteur FGF-R4 est actif à la fois contre FGF-R4 humain, FGF-R4 murin et FGF-R4 de rat.

Dans un mode de réalisation avantageux, l'anticorps antagoniste spécifique du récepteur FGF-R4 comprend au moins un CDR présentant une séquence identique à SEQ ID NO : 9, 10, 11, 12, 13, 14, 73, 74, 75, 78, 79, 80, 83, 84, 85, 88, 89, 90, 93, 94, 95, 98, 99, 100, 103, 104, 105, 108, 109 ou 110 ou au moins un CDR dont la séquence diffère de un à deux acides aminés par rapport aux séquences SEQ ID NO 9, 10, 11, 12, 13, 14, 73, 74, 75, 78, 79, 80, 83, 84, 85, 88, 89, 90, 93, 94, 95, 98, 99, 100, 103, 104, 105, 108, 109 ou 110, pour autant que l'anticorps garde sa spécificité de liaison.

Dans un mode de réalisation particulièrement avantageux, l'anticorps antagoniste spécifique du récepteur FGF-R4 comprend les CDR de séquence SEQ ID NO : 9, 10, 11, 12, 13, 14, 73, 74, 75, 78, 79, 80, 83, 84, 85, 88, 89, 90, 93, 94, 95, 98, 99, 100, 103, 104, 105, 108, 109 ou 110 ou des CDR dont les séquences diffèrent de un à deux acides aminés par rapport - respectivement - aux séquences sus-citées, pour autant que cela ne modifie pas la spécificité de liaison de l'anticorps au récepteur FGF-R4.

Dans un mode de réalisation avantageux, les anticorps de l'invention comprennent au moins une chaîne lourde et au moins une chaîne légère, ladite chaîne lourde comprenant trois séquences CDR ayant des séquences en acides aminés sélectionnées dans le groupe consistant en SEQ ID NO : 9, 10 et 11 ou 73, 74 et 75, 83, 84 et 85, ou 93, 94 et 95, ou 103, 104 et 105 ladite chaîne légère comprenant trois séquences CDR ayant des séquences en acides aminés sélectionnées dans le groupe consistant en SEQ ID NO : 12, 13 et 14 ou 78, 79 et 80, ou 88, 89 et 90, ou 98, 99 et 100, ou 108, 109 et 110.

Dans un mode de réalisation avantageux les parties variables de la chaîne lourde de l'anticorps antagoniste spécifique du récepteur FGF-R4 comprennent une séquence présentant au moins 80%, 90%, 95% ou 99% d'identité avec la séquence SEQ ID NO : 6, 77, 87, 97 ou 107.

Dans un mode de réalisation avantageux les parties variables de la chaîne légère de l'anticorps antagoniste spécifique du récepteur FGF-R4 comprennent une séquence présentant au moins 80%, 90%, 95% ou 99% d'identité avec la séquence SEQ ID NO : 8, 72, 82, 92 ou 102.

La présente invention a également pour objet un anticorps antagoniste spécifique du récepteur FGF-R4 comprenant une chaîne lourde comprenant une partie variable codée par une séquence nucléotidique présentant une identité d'au moins 80%, 90%, 95% ou 99% avec la séquence SEQ ID NO : 5, 76, 86, 96 ou 106.

La présente invention a également pour objet un anticorps antagoniste spécifique du récepteur FGF-R4 comprenant une chaîne légère comprenant une partie variable codée par une séquence nucléotidique présentant une identité d'au moins 80%, 90%, 95% ou 99% avec la séquence SEQ ID NO 7, 71, 81, 91 ou 101.

La présente invention a également pour objet un anticorps antagoniste spécifique du récepteur FGF-R4 comprenant une chaîne lourde comprenant une partie variable de séquence polypeptidique SEQ ID NO : 6, 77, 87, 97 ou 107.

La présente invention a également pour objet un anticorps antagoniste spécifique du récepteur FGF-R4 comprenant une chaîne légère comprenant une partie variable de séquence polypeptidique SEQ ID NO : 8, 72, 82, 92 ou 102.

La présente invention a également pour objet un anticorps antagoniste spécifique du récepteur FGF-R4 comprenant des séquences codées par les séquences nucléotidiques SEQ ID NO : 5 et 7 ou 71 et 76, ou 81 et 86, ou 91 et 96, ou 101 et 106.

La présente invention a également pour objet un anticorps antagoniste spécifique du récepteur FGF-R4 comprenant les séquences polypeptidiques SEQ ID NO : 6 et 8, ou 72 et 77, ou 82 et 87, ou 92 et 97 ou 102 et 107.

Dans un mode de réalisation avantageux l'anticorps antagoniste spécifique du récepteur FGF-R4 comprend des séquences identiques au moins à 80%, 90%, 95% ou 99% aux SEQ ID NO : 2 et/ou SEQ ID NO : 4 ; ou SEQ ID NO : 72 et/ou SEQ ID NO : 77 ; ou SEQ ID NO : 82 et/ou SEQ ID NO : 87 ; ou SEQ ID NO : 92 et/ou SEQ ID NO : 97 ; ou SEQ ID NO : 102 et/ou SEQ ID NO : 107.

Dans un mode de réalisation particulièrement avantageux, l'anticorps antagoniste spécifique du récepteur FGF-R4 comprend une chaîne lourde codée par une séquence nucléotidique identique au moins à 80%, 90%, 95% ou 99% à la séquence SEQ ID NO 1.

La présente invention a également pour objet un anticorps antagoniste du récepteur FGF-R4 comprenant une chaîne lourde de séquence polypeptidique identique au moins à 80%, 90%, 95% ou 99% à la séquence SEQ ID NO 2.

La présente invention a également pour objet un anticorps antagoniste spécifique du récepteur FGF-R4 comprenant une chaîne légère codée par une séquence nucléotidique identique au moins à 80%, 90%, 95% ou 99% à la séquence SEQ ID NO 3.

La présente invention a également pour objet un anticorps antagoniste spécifique du récepteur FGF-R4 comprenant une chaîne légère de séquence polypeptidique identique au moins à 80%, 90%, 95% ou 99% à la séquence SEQ ID NO 4.

La présente invention a également pour objet un anticorps antagoniste spécifique du récepteur FGF-R4 comprenant les séquences codées par les séquences nucléotidiques SEQ ID NO : 1 et 3.

Dans un mode encore plus avantageux, l'anticorps antagoniste spécifique de FGF-R4 comprend une chaîne lourde comprenant la séquence SEQ ID NO 2 et une chaîne légère comprenant la séquence SEQ ID NO 4.

L'anticorps composé d'une chaîne lourde de séquence SEQ ID NO 2 et d'une séquence légère SEQ ID NO 4 sera appelé 40-12 dans la suite de la demande.

Dans un mode de réalisation de l'invention les anticorps spécifiques de FGF-R4 sont actifs à la fois contre FGF-R4 humain, FGF-R4 murin et FGF-R4 de rat.

Dans un mode de réalisation avantageux, l'antagoniste spécifique du récepteur FGF-R4 induit une inhibition des voies cellulaires AKT/p38.

Dans un mode de réalisation avantageux, l'antagoniste spécifique du récepteur FGF-R4 induit une inhibition des voies cellulaires Erk1/2.

Dans un mode de réalisation avantageux, l'antagoniste spécifique du récepteur FGF-R4 induit une inhibition des voies signalisation cellulaire contrôlées par FGF-.R4

Dans un autre mode de réalisation avantageux l'antagoniste spécifique du récepteur FGF-R4 induit une inhibition de la prolifération cellulaire tumorale.

Dans encore un autre mode de réalisation avantageux antagoniste spécifique du récepteur FGF-R4 induit une inhibition de l'angiogenèse.

Dans un autre mode de réalisation particulièrement avantageux, l'antagoniste spécifique du récepteur FGF-R4 présente une affinité pour FGF-R4 10 fois supérieure à son affinité pour les autres récepteurs à FGF.

Dans un mode de réalisation particulièrement avantageux, l'anticorps selon l'invention est un anticorps humanisé antagoniste spécifique du récepteur FGF-R4.

Dans un mode de réalisation l'anticorps humanisé antagoniste spécifique du récepteur FGF-R4 comprend une chaîne légère dont la partie variable est codée par une séquence nucléotidique identique au moins à 80%, 90%, 95% ou 99% à la séquence SEQ ID NO 29 ou à la séquence SEQ ID NO 31.

Dans un autre mode de réalisation l'anticorps humanisé antagoniste spécifique du récepteur FGF-R4 comprend une chaîne légère dont la partie variable est identique au moins à 80%, 90%, 95% ou 99% à la séquence SEQ ID NO 30 ou à la séquence SEQ ID NO 32.

Dans un autre mode de réalisation l'anticorps humanisé antagoniste spécifique du récepteur FGF-R4 comprend une chaîne légère dont la partie variable est codée par une séquence identique à la séquence nucléotidique SEQ ID NO 29 ou à la séquence SEQ ID NO: 31.

La présente invention a également pour objet un anticorps humanisé antagoniste spécifique du récepteur FGF-R4 comprenant une chaîne lourde dont la partie variable est codée par une séquence identique au moins à 80%, 90%, 95% ou 99% à la séquence SEQ ID NO 33, à la séquence SEQ ID NO 35 ou à la séquence SEQ ID NO 37.

La présente invention a également pour objet un anticorps humanisé antagoniste spécifique du récepteur FGF-R4 comprenant une chaîne lourde dont la partie variable est identique au moins à 80%, 90%, 95% ou 99% à la séquence SEQ ID NO 34, à la séquence SEQ ID NO 36 ou à la séquence SEQ ID NO 38.

La présente invention a également pour objet un anticorps humanisé antagoniste spécifique du récepteur FGF-R4 comprenant une chaîne lourde codée par une séquence nucléotidique SEQ ID NO 33 et/ou SEQ ID NO : 35 et/ou SEQ ID NO : 37. La présente invention a également pour objet un anticorps humanisé antagoniste spécifique du récepteur FGF-R4 dont les séquences humanisées de séquence SEQ ID NO : 30 ou 32 sont utilisées en combinaison avec les séquences humanisées de séquence SEQ ID NO : 34, 36 ou 38.

Dans un autre mode de réalisation, l'anticorps antagoniste spécifique du récepteur FGF-R4 comprend les CDR de séquence SEQ ID NO : 73, 74, 75, 78, 79 et 80 ou des CDR dont les séquences diffèrent de un à deux acides aminés par rapport - respectivement - aux séquences suscitées, pour autant que cela ne modifie pas la spécificité de liaison de l'anticorps au récepteur FGF-R4.

Dans un autre mode de réalisation, l'anticorps antagoniste spécifique du récepteur FGF-R4 comprend les CDR de séquence SEQ ID NO : 83, 84, 85, 88, 89 et 90 ou des CDR dont les séquences diffèrent de un à deux acides aminés par rapport - respectivement - aux séquences suscitées; pour autant que cela ne modifie pas la spécificité de liaison de l'anticorps au récepteur FGF-R4.

Dans un autre mode de réalisation, l'anticorps antagoniste spécifique du récepteur FGF-R4 comprend les CDR de séquence SEQ ID NO : 93, 94, 95, 98, 99 et 100 ou des CDR dont les séquences diffèrent de un à deux acides aminés par rapport - respectivement - aux séquences suscitées, pour autant que cela ne modifie pas la spécificité de liaison de l'anticorps au récepteur FGF-R4.

Dans un autre mode de réalisation, l'anticorps antagoniste spécifique du récepteur FGF-R4 comprend les CDR de séquence SEQ ID NO : 103, 104, 105, 108, 109 et 110 ou des CDR dont les séquences diffèrent de un à deux acides aminés par rapport - respectivement - aux séquences suscitées, pour autant que cela ne modifie pas la spécificité de liaison de l'anticorps au récepteur FGF-R4.

Dans un mode de mise en oeuvre préféré de l'invention, l'anticorps antagoniste spécifique du récepteur FGF-R4 comprend les CDR de séquence SEQ ID NO : 83, 84, 85, 88, 89 et 90.

Dans un mode de réalisation avantageux, l'anticorps antagoniste spécifique du récepteur FGF-R4 est un anticorps humain dont les parties variables de la chaîne lourde comprennent une séquence nucléotidique présentant au moins 80%, 90%, 95% ou 99% d'identité avec la séquence SEQ ID NO : 76, 86, 96 ou 106.

Dans un mode de réalisation avantageux, l'anticorps antagoniste spécifique du récepteur FGF-R4 est un anticorps humain dont les parties variables de la chaîne légère comprennent une séquence nucléotidique présentant au moins 80%, 90%, 95% ou 99% d'identité avec la séquence SEQ ID NO : 71, 81, 91 ou 101.

La présente invention a également pour objet un anticorps humain antagoniste spécifique du récepteur FGF-R4 comprenant une chaîne lourde comprenant une partie variable codée par une séquence protéique présentant une identité d'au moins 80%, 90%, 95% ou 99% avec la séquence SEQ ID NO : 77, 87, 97 ou 107.

La présente invention a également pour objet un anticorps humain antagoniste spécifique du récepteur FGF-R4 comprenant une chaîne légère comprenant une partie variable codée par une séquence protéique présentant une identité d'au moins 80%, 90%, 95% ou 99% avec la séquence SEQ ID NO : 72, 82, 92 ou 102.

La présente invention a également pour objet un anticorps humain antagoniste spécifique du récepteur FGF-R4 comprenant des séquences codées par les séquences nucléotidiques SEQ ID NO : 71 et 76 ou les séquences nucléotidiques SEQ ID NO : 81 et 86 ou les séquences nucléotidiques SEQ ID NO : 91 et 96 ou les séquences nucléotidiques SEQ ID NO : 101 et 106.

La présente invention a également pour objet un anticorps humain antagoniste spécifique du récepteur FGF-R4 comprenant les séquences polypeptidiques SEQ ID NO: 72 et 77 ou les séquences polypeptidiques SEQ ID NO : 82 et 87 ou les séquences polypeptidiques SEQ ID NO : 92 et 97 ou les séquences polypeptidiques SEQ ID NO : 102 et 107.

Dans un mode de réalisation avantageux, l'anticorps antagoniste spécifique du récepteur FGF-R4 comprend des séquences identiques au moins à 80%, 90%, 95% ou 99% aux SEQ ID NO : 72 et/ou SEQ ID NO: 77.

Dans un autre mode de réalisation avantageux, l'anticorps humain antagoniste spécifique du récepteur FGF-R4 comprend des séquences identiques au moins à 80%, 90%, 95% ou 99% aux SEQ ID NO : 82 et/ou SEQ ID NO : 87.

Dans un autre mode de réalisation avantageux, l'anticorps humain antagoniste spécifique du récepteur FGF-R4 comprend des séquences identiques au moins à 80%, 90%, 95% ou 99% aux SEQ ID NO : 92 et/ou SEQ ID NO : 97.

Dans un autre mode de réalisation avantageux, l'anticorps humain antagoniste spécifique du récepteur FGF-R4 comprend des séquences identiques au moins à 80%, 90%, 95% ou 99% aux SEQ ID NO : 102 et/ou SEQ ID NO : 107.

Dans un mode de réalisation préféré, la présente invention a pour objet un anticorps antagoniste humain spécifique du récepteur FGF-R4 comprenant une chaîne légère codée par une séquence nucléotidique identique au moins à 80%, 90%, 95% ou 99% à la séquence SEQ ID NO 82 et comprenant une chaîne légère de séquence polypeptidique identique au moins à 80%, 90%, 95% ou 99% à la séquence SEQ ID NO 87.

De manière encore plus préférée, la présente invention a pour objet un anticorps humain antagoniste spécifique du récepteur FGF-R4 comprenant les séquences codées par les séquences nucléotidiques SEQ ID NO : 82 et 87.

L'anticorps composé d'une chaîne lourde de séquence SEQ ID NO 77 et d'une séquence légère SEQ ID NO 72 sera appelé clone 8 dans la suite de la demande. L'anticorps composé d'une chaîne lourde de séquence SEQ ID NO 87 et d'une séquence légère SEQ ID NO 82 sera appelé clone 31 dans la suite de la demande. L'anticorps composé d'une chaîne lourde de séquence SEQ ID NO 97 et d'une séquence légère SEQ ID NO 92 sera appelé clone 33 dans la suite de la demande. L'anticorps composé d'une chaîne lourde de séquence SEQ ID NO 107 et d'une séquence légère SEQ ID NO 102 sera appelé clone 36 dans la suite de la demande.

Le champ de la présente invention ne se limite pas aux anticorps comprenant ces séquences. En effet, tous les anticorps qui se lient de manière spécifique à FGF-R4 en ayant une action antagoniste sur ce récepteur, rentrent dans le champ de la présente invention.

La présente invention a également pour objet un anticorps antagoniste spécifique du récepteur FGF-R4 conjugué à un agent cytotoxique.

La présente invention a pour objet l'utilisation d'un antagoniste spécifique du récepteur FGF-R4 dans le traitement des maladies associées à l'angiogenèse.

La présente invention a pour objet l'utilisation d'un antagoniste spécifique du récepteur FGF-R4 dans le traitement d'un cancer.

La présente invention a pour objet l'utilisation d'un antagoniste spécifique du récepteur FGF-R4 dans le traitement des hépatocarcinomes ou de tout autre type de cancer hépatique.

La présente invention a pour objet l'utilisation d'un antagoniste spécifique du récepteur FGF-R4 dans le traitement du cancer du pancréas.

La présente invention a pour objet un anticorps spécifique du récepteur FGF-R4 utile à la fois dans le traitement des maladies associées à l'angiogenèse et dans le traitement des hépatocarcinomes ou de tout autre type de cancer hépatique.

La présente invention a pour objet un anticorps spécifique du récepteur FGF-R4 utile à la fois dans le traitement des maladies associées à l'angiogenèse, dans le traitement des hépatocarcinomes ou de tout autre type de cancer hépatique, dans le traitement du cancer du pancréas, des organes du tractus gastro-intestinal ou tout autre organe exprimant FGF-R4.La présente invention a pour objet un anticorps antagoniste spécifique du récepteur FGF-R4 pour son utilisation dans le traitement des maladies associées à une angiogenèse pathologique.

La présente invention a pour objet un anticorps selon la revendication 1 pour son utilisation pour inhiber la croissance tumorale simultanément à l'inhibition de l'angiogenèse.

La présente invention a pour objet un anticorps selon l'une des revendications 1 et 2 pour son utilisation dans le traitement des hépatocarcinomes ou de tout autre type de cancer hépatique.

La présente invention a pour objet un anticorps selon l'une des revendications 1 à 3 pour son utilisation dans le traitement du cancer du pancréas.

La présente invention a pour objet un anticorps pour son utilisation selon l'une des revendications 1 à 4, se liant au domaine D2-D3 du récepteur FGF-R4.

La présente invention a pour objet un anticorps pour son utilisation selon l'une des revendications 1 à 5, se liant au domaine D2 du récepteur FGF-R4.

La présente invention a pour objet un anticorps pour son utilisation selon l'une des revendications 1 à 6, ayant un $K_D$ vis à vis du récepteur FGF-R4 déterminé par la technique surface Plasmon Resonance (Biacore) inférieur à 10E-8 M.

La présente invention a pour objet un anticorps pour son utilisation selon l'une des revendications 1 à 7, qui est actif à la fois contre FGF-R4 humain et FGF-R4 murin.

La présente invention a pour objet un anticorps antagoniste spécifique du récepteur FGF-R4 comprenant les CDR de séquence SEQ ID NO : 9, 10, 11, 12, 13 et 14 ; ou 73, 74, 75, 78, 79 et 80 ; ou 83, 84, 85, 88, 89 et 90 ; ou 93, 94, 95, 98, 99 et 100; ou 103, 104, 105, 108, 109 et 110, où l'un des CDR peut différer de un à deux acides aminés par rapport à l'une au moins des séquences citées ci-dessus, pour autant que l'anticorps garde sa spécificité de liaison pour son utilisation dans le traitement des maladies associées à une angiogenèse pathologique.

La présente invention a pour objet un anticorps antagoniste spécifique du récepteur FGF-R4 dont la partie variable de sa chaîne lourde comprend une séquence nucléotidique présentant au moins 80% d'identité avec la séquence SEQ ID NO : 5, 76, 86, 96 ou 106 et dont la partie variable de sa chaîne légère comprend une séquence nucléotidique présentant au moins 80% d'identité avec la séquence SEQ ID NO : 7, 71, 81, 91 ou 101, pour autant que l'anticorps garde sa spécificité de liaison pour son utilisation dans le traitement des maladies associées à une angiogenèse pathologique.

La présente invention a pour objet un anticorps antagoniste spécifique du récepteur FGF-R4 dont la séquence comprend les séquences polypeptidiques SEQ ID NO : 2 et 4 ; ou 6 et 8 ; ou 72 et 77 ; ou 82 et 87 ; ou 92 et 97 ; ou 102 et 107 ou des séquences correspondant à ces séquences ayant au moins 80% d'identité avec elles, pour autant que l'anticorps garde sa spécificité de liaison pour son utilisation dans le traitement des maladies associées à une angiogenèse pathologique.

La présente invention a pour objet un anticorps spécifique du récepteur FGF-R4 pour son utilisation selon l'une quelconque des revendications 1 à 11, caractérisé en ce qu'il est un anticorps humanisé.La présente invention a pour objet un anticorps pour son utilisation selon la revendication 12, caractérisé en ce qu'il comprend une chaîne variable légère présentant au moins 80% d'identité avec l'une des séquences polypeptidiques SEQ ID NO 30 ou 32 et une chaîne variable lourde présentant au moins 80% d'identité avec une séquence SEQ ID NO 34, 36 ou 38.

La présente invention a pour objet un anticorps pour son utilisation selon l'une des revendications 1 à 13, caractérisé en ce qu'il est un anticorps conjugué à un agent cytotoxique.

La présente invention a pour objet une composition pharmaceutique comprenant un antagoniste spécifique du récepteur FGF-R4 et des excipients.

La présente invention a pour objet une méthode de traitement d'un cancer comprenant l'administration au patient d'un anticorps antagoniste spécifique du récepteur FGF-R4.

La présente invention a pour objet une méthode de traitement d'une maladie associée à une augmentation pathologique de l'angiogenèse comprenant l'administration au patient d'un anticorps antagoniste spécifique du récepteur FGF-R4.

La présente invention a pour objet un procédé de sélection d'un anticorps monoclonal antagoniste spécifique du précepteur FGF-R4, comprenant les étapes suivantes :

    a. immunisation chez la souris
    b. prélèvement de nodules lymphatiques chez les souris, et
    c. criblage des surnageants d'hybridomes

La présente invention a pour objet une lignée cellulaire produisant des anticorps antagonistes spécifiques du récepteur FGF-R4.

La présente invention a pour objet un procédé de production d'un anticorps antagoniste spécifique du récepteur FGF-R4 comprenant la mise en culture d'une lignée cellulaire produisant des anticorps antagonistes du récepteur FGF-R4.

La présente invention a pour objet un médicament comprenant un anticorps antagoniste spécifique du récepteur FGF-R4.

La présente invention a également pour objet un polynucléotide codant pour un polypeptide spécifique du FGFR4 sélectionné dans le groupe constitué des SEQ ID NOS : 2, 4, 6, 8, 9, 10, 11, 12, 13, 14, 30, 32, 34, 36, 38, 72, 73, 74, 75, 77, 78, 79, 80, 82, 83, 84, 85, 87, 88, 89, 90, 92, 93, 94, 95, 97, 98, 99, 100, 102, 103, 104, 105, 107, 108, 109, 110 et des séquences identique au moins à 80%, 90%, 95% ou 99% à l'une de ces séquences.

La présente invention a pour objet un polynucléotide codant pour un polypeptide antagoniste spécifique du récepteur FGF-R4 dont les séquences présentent au moins 80% d'identité avec l'une des séquences suivantes : SEQ ID NOS 2 et 4; ou SEQ ID NOS 6 est 8; ou SEQ ID NOS 9, 10, 11, 12, 13 et 14; ou SEQ ID NOS 30 ou 32 et 34 ou 36 ou 38; ou SEQ ID NOS 72 et 77; ou SEQ ID NOS 73, 74, 75, 78, 79 et 80 ; ou SEQ ID NOS 82 et 87; ou SEQ ID NOS 83, 84, 85, 88, 89 et 90; ou SEQ ID NOS 92 et 97 ; ou SEQ ID NOS 93, 94, 95, 98, 99 et 100; ou SEQ ID NOS 102 et 107; ou SEQ ID NOS 103, 104, 105, 108, 109 et 110, pour autant que le polypeptide qu'il code garde la spécificité de liaison, pour son utilisation dans le traitement des maladies associées à une angiogenèse pathologique.

La présente invention a pour objet un polynucléotide caractérisé en ce qu'il présente une séquence présentant au moins 80% d'identité avec l'une des séquences SEQ ID NOS 1 et 3 ; ou SEQ ID NOS 5 et 7; SEQ ID NOS 29 ou 31, et 33, 35 ou 37 ; ou SEQ ID NOS 71 et 76 ; ou SEQ ID NOS 81 et 86 ; ou SEQ ID NOS 91 et 96 ; ou SEQ ID NOS 101 et 106, pour autant que le polypeptide qu'il code garde la spécificité de liaison antagoniste au récepteur FGF-R4, pour son utilisation dans le traitement des maladies associées à une angiogenèse pathologique.

La présente invention a pour objet un vecteur recombinant comprenant un polynucléotide tel que décrit ci dessus ou codant pour un polypeptide tel que décrit ci dessus.

Afin de permettre l'expression de chaînes lourdes et/ou chaînes légères de l'anticorps antagoniste du récepteur FGF-R4, objet de l'invention, les polynucléotides codant pour lesdites chaînes sont insérés dans des vecteurs d'expression. Ces vecteurs d'expression peuvent être des plasmides, des YACs, des cosmides, des rétrovirus, des épisomes dérivés d'EBV, et tous les vecteurs que l'homme du métier peut juger appropriés à l'expression desdites chaînes.

La présente invention a pour objet une cellule hôte comprenant un vecteur recombinant tel que décrit ci-dessus.

Description détaillée de l'invention

La présente invention a pour objet l'utilisation d'anticorps antagonistes dirigés spécifiquement contre FGF-R4 (sans réaction croisée avec FGF-R1, R2 ou R3) pour l'inhibition de l'angiogenèse et de la croissance tumorale.

De façon inattendue, les inventeurs ont montré que le FGF-R4 joue un rôle actif et spécifique dans le contrôle de l'angiogenèse.

Cette fonction du FGF-R4 n'avait jamais été montrée ou proposée auparavant. Par conséquent, ce récepteur peut être utilisé eh tant que cible pour traiter les pathologies présentant un dysfonctionnement angiogéhique. Les ligands du FGF-R4 capables de moduler son activité sont donc des agents thérapeutiques potentiels: pour de nombreuses pathologies liées:à l'angiogenèse.

La présente invention peut donc être utilisée dans le traitement de toutes les pathologies impliquant une dérégulation de l'angiogenése et nécessitant une inhibition de celle-ci. Les pathologies visées peuvent être le cancer, avec l'utilisation des antagonistes selon l'invention comme inhibiteurs de l'angiogenèse tumorale, ou les pathologies pour lesquelles une dérégulation de l'angiogenèse est décrite comme : la dégénérescence maculaire liée à l'âge ou DMLA, les maladies inflammatoires comme l'arthrite rhumatoïde, l'ostéoarthrite, les colites, les ulcères ou toute maladie inflammatoire des intestins, l'athérosclérose ou encore dans le traitement de l'obésité. Dans un mode encore plus avantageux, l'anticorps se lie à un épitope compris dans le domaine comprenant les acides aminés 144 à 365 du récepteur au FGF-R4.

Dans un mode encore plus avantageux, l'anticorps se lie à un épitope compris dans le domaine D2 du récepteur au FGF-R4, cet épitope correspondant aux acides aminés 145 à 242 décrits dans la séquence SEQ ID NO 70.

Un anticorps, aussi appelé immunoglobuline, est composé de deux chaînes lourdes identiques (« VH ») et de deux chaînes légères identiques (« VL ») qui sont liées par un pont disulfure. Chaque chaîne contient une région constante et une région variable. Chaque région variable comprend trois segments appelés « régions déterminant la complémentarité » (« CDRs ») ou « régions hypervariables », qui sont principalement responsables de la liaison à l'épitope d'un antigène.

Le terme « VH » fait référence aux régions variables d'une chaîne lourde d'immunoglobuline d'un anticorps, incluant les chaînes lourdes d'un fragment Fv, scFv, dsFv, Fab, Fab' ou F(ab)'.

Le terme « VL » fait référence aux régions variables d'une chaîne légère d'immunoglobuline d'un anticorps, incluant les chaînes légères d'un fragment Fv, scFv, dsFv, Fab, Fab' ou F(ab)'.

Par « fragment d'anticorps » on entend toute partie de celui-ci : Fab (Fragment antigen binding), Fv, scFv (single chain Fv), Fc (Fragment cristallisable). De préférence, ces fragments fonctionnels seront des fragments de type Fv, scFv, Fab, F(ab') 2, Fab', scFv-Fc ou diabodies, qui possèdent généralement la même spécificité de fixation que l'anticorps monoclonal, chimérique ou humanisé dont ils sont issus. Selon la présente invention, des fragments d'anticorps de l'invention peuvent être obtenus à partir des anticorps monoclonaux, chimériques ou humanisés, par des méthodes telles que la digestion par des enzymes, comme la pepsine ou la papaïne et/ou par clivage des ponts disulfures par réduction chimique.

Par « régions CDR ou CDRs », on entend désigner les régions hypervariables des chaînes lourdes et légères des immunoglobulines comme définies par Kabat et al. (Kabat et al., Sequences of proteins of immunological interest, 5t'' Ed., U.S. Department of Health and Human Services, NIH, 1991, and later editions). Il existe 3 CDRs de chaîne lourde et 3 CDRs de chaîne légère. Le terme CDR ou CDRs est utilisé ici pour désigner suivant les cas, l'une de ces régions ou plusieurs, voire l'ensemble, de ces régions qui contiennent la majorité des résidus d'acides aminés responsables de la liaison affine de l'anticorps pour l'antigène ou l'épitope qu'il reconnaît. Les régions les plus conservées des domaines variables sont appelés régions ou séquences FR pour « framework » ou régions « charpentes ».

Dans un autre mode de réalisation de l'invention, l'antagoniste spécifique de FGF-R4 est un anticorps chimérique.

Par « anticorps chimérique » on entend un anticorps dans lequel la région constante, ou une portion de celle-ci, est altérée, remplacée ou échangée, telle que la région variable est liée à une région constante d'une espèce différente, ou appartient à une autre classe ou sous-classe d'anticorps.

On entend également par « anticorps chimérique » un anticorps dans lequel la région variable, ou une portion de celle-ci, est altérée, remplacée ou échangée, telle que la région constante est liée à une région variable d'une espèce différente, ou appartient à une autre classe ou sous-classe d'anticorps.

Les méthodes de production d'anticorps chimériques sont connues de l'homme du métier. Voir par exemple, Morrison, 1985, Science, 229 :1202 ; Oi et al., 1986, Bio Techniques, 4 :214 ; Gillies et al., 1989, J. Immunol. Methods, 125 :191-202 ; U.S. Pat. Nos. 5,807,715 ; 4,816,567 ; and 4,816,397.

Ces versions chimériques de l'anticorps peuvent consister en la fusion des parties variables VL et VH aux parties constantes de Ckappa et CH (IgG1) d'origine humaine afin de générer un anticorps monoclonal chimère.

La partie CH (IgG1) peut aussi être modifiée par des mutations ponctuelles afin d'augmenter l'affinité du fragment Fc pour le récepteur FcyRIIIa et par là même augmenter les fonctions effectrices de l'anticorps (Lazar et al., 2006, Proc. Natl. Acad. Sci. USA 103 : 4005-4010 ; Stavenhagen et al., 2007, Cancer Res. 67 : 8882-8890).

La présente invention inclut les versions humanisées des anticorps.

Par « anticorps humanisé » on entend un anticorps qui contient principalement des séquences immunoglobuline humaines. Ce terme fait généralement référence à une immunoglobuline non humaine qui a été modifiées par incorporation de séquences humaines ou de résidus trouvés dans des séquences humaines.

En général, les anticorps humanisés comprennent un ou typiquement deux domaines variables dans lesquels tout ou partie des régions CDR correspondent à des parties issues de la séquence parente non humaine et dans lesquels tout ou partie des régions FR sont ceux issus d'une séquence immunoglobuline humaine L'anticorps humanisé peut alors comprendre au moins une portion d'une région constante d'immunoglobuline (Fc), en particulier celle de l'immunoglobuline humaine de référence choisie.

On cherche ainsi à obtenir un anticorps qui soit immunogène a minima chez un humain. Ainsi il est possible qu'un ou deux acides aminés d'un ou plusieurs CDRs soient modifiés par un acide aminé moins immunogène pour l'hôte humain, ceci sans réduire substantiellement la spécificité de liaison de l'anticorps au FGF-R4,. De même, les résidus des régions charpentes peuvent ne pas être humains et il est possible qu'ils ne soient pas modifiés car ils ne contribuent pas au potentiel immunogénique de l'anticorps.

Il existe plusieurs méthodes d'humanisation connues de l'homme du métier pour modifier un anticorps parent non-humain en un anticorps moins immunogène pour l'humain. Une identité globale des séquences avec un anticorps humain n'est pas forcément nécessaire. En effet l'identité globale de séquence n'est pas nécessairement un indicateur prédictif d'une immunogénicité réduite et la modification d'un nombre limité de résidus peut conduire à des anticorps humanisés présentant un potentiel immunogénique très atténué chez l'homme (Molecular Immunology (2007) 44, 1986-1998).

Diverses méthodes sont par exemple l'inclusion de CDRs (grafting) (EPO 0 239 400; WO 91/09967; et U.S. Pat. Nos. 5,530,101 et 5,585,089), le resurfaçage (EPO 0 592 106; EPO 0 519 596; Padlan, 1991, Molec Imm 28(4/5):489-498; Studnicka et al., 1994, Prot Eng 7(6):805-814; et Roguska et al., 1994, PNAS 91:969-973) ou encore le mélange des chaînes (U.S. Pat. No. 5,565,332).

La présente invention concerne en particulier des anticorps humanisés dont les parties variables sont modifiées selon la technologie suivante:

Les chaînes légères et lourdes les plus similaires aux chaînes correspondantes de l'anticorps murin anti-FGF-R4 40-12 sont identifiées par comparaison à la Protein Data Bank (H.M. Berman, J. Westbrook, Z. Feng, G. Gilliland, T.N. Bhat, H. Weissig, I.N. Shindyalov, P.E. Bourne. Nucleic Acids Research, 2000, 28:235-242). L'alignement de séquences utilise l'algorithme BLAST (J Mol Biol. 1990 Oct 215:403-410). Il s'agit des structures tridimensionnelles répondant aux codes PDB 1NDM & 1 ETZ qui ont été respectivement utilisées pour construire les modèles par homologie des chaînes légères et lourdes des domaines variables. Ces modèles tridimensionnels sont ensuite affinés en minimisant leur énergie potentielle avec la procédure standard implémentée dans le logiciel MOE (Molecular Operating Environment, Chemical Computing Group, Quebec, Canada). Une simulation de dynamique moléculaire (DM) est ensuite réalisée à partir de ces modèles tridimensionnels minimisés de l'anticorps avec le logiciel Amber (D.A. Case, T.E. Cheatham, III, T. Darden, H. Gohlke, R. Luo, K.M. Merz, Jr., A. Onufriev, C. Simmerling, B. Wang and R. Woods. J. Computat. Chem. 2005, 26:1668-1688). Cette simulation a lieu avec des contraintes harmoniques appliquées aux atomes du squelette protéique à une température de 500K pendant une durée de 1,1 nanoseconde dans un modèle de solvant implicite tel qu'implémenté dans la méthode généralisée de Born (Gallicchio & Levy, J Comput Chem 2004, 25:479-499). Dix conformations diverses sont ainsi extraites de cette première simulation à raison d'une conformation tridimensionnelle toutes les cent picosecondes pendant la dernière nanoseconde de la simulation. Ces dix conformations diverses sont ensuite chacune soumises à une simulation de dynamique moléculaire, sans contraintes sur le squelette protéique, à une température de 27 °C pendant 2,3 nanosecondes dans un modèle de solvant implicite tel qu'implémenté dans la méthode généralisé de Born. Les liaisons impliquant un atome hydrogène sont contraintes avec l'algorithme SHAKE (Barth. et al., J Comp Chem, 1995, 16:1192-1209), le pas est de 1 femto seconde, et la simulation a lieu selon l'équation de Langevin à volume constant et une température constante de 27°C. Pour chacune des dix simulations de dynamique moléculaire, les dernières deux mille conformations, extraites à la fréquence d'une par picoseconde, sont ensuite utilisées pour quantifier, pour chaque acide-aminé de l'anticorps à humaniser, la déviation des positions des atomes par rapport à une conformation moyenne, dite médoïde, de l'acide aminé. L'ensemble de l'analyse décrite ci-après est réalisée avec le langage de script Scientific Vector Language (SVL) du logiciel MOE. La conformation médoïde de l'acide aminé est la conformation issue de la dynamique moléculaire qui est la plus proche de la conformation moyenne calculée à partir de la position des atomes de toutes les conformations de l'acide aminé. La distance utilisée pour détecter la conformation médoïde est l'écart quadratique moyen des distances scalaires entre les atomes de deux conformations de l'acide aminé. De manière similaire, la déviation des positions des atomes d'une conformation d'un acide-aminé par rapport à la conformation médoïde est quantifiée en calculant l'écart quadratique moyen des distances scalaires entre les atomes de l'acide aminé d'une conformation de la simulation et les mêmes atomes de la conformation médoïde. Par la suite, c'est en comparant l'écart quadratique moyen des positions des atomes d'un acide amine donné (i) moyenné sur l'ensemble des dix simulations de dynamique moléculaire ($F_i$), à l'écart quadratique moyen des positions de tous les acides-aminés de l'anticorps moyenné sur l'ensemble des dix simulations de dynamique moléculaire ($F_m$) que l'on décide si l'acide-aminé est assez flexible pour être considéré comme pouvant potentiellement interagir avec les récepteurs de cellules T et provoquer l'activation du système immunitaire. Un acide-aminé i est considéré comme flexible s'il présente un score de flexibilité $Z_i$ au-dessus de 0,15 tel que défini ci-après : $Z_i = (F_i - F_m)/F_m$. 45 acides-aminés sont ainsi identifiés comme flexible dans le domaine variable de l'anticorps, à l'exclusion de la région déterminant la complémentarité à l'antigène (RDC) et de son environnement immédiat. On définit l'environnement immédiat de la RDC comme tout acide-aminé dont le carbone alpha se situe à une distance de 5 angströms (A) ou moins d'un carbone alpha de la RDC.

Les mouvements des 60 acide-aminés les plus flexibles de l'anticorps, pendant les 20 nanosecondes (10x2ns) de simulation, sont ensuite comparés aux mouvements des acide-aminés correspondants de 49 modèles par homologie de séquences germinales humaines d'anticorps, pour chacun desquels les dix simulations de dynamique moléculaire (10x2ns) ont été réalisées selon le même mode opératoire. Les 60 acide-aminés les plus flexibles excluent la région déterminant la complémentarité à l'antigène (RDC) et son environnement immédiat. Les 49 modèles humains de séquences germinales d'anticorps ont été construits en combinant systématiquement les 7 chaînes légères humaines les plus fréquentes (vk1, vk2, vk3, vk4, vlambda1, vlambda2, vlambda3) et les 7 chaînes lourdes humaines les plus fréquentes (vh1a, vh1b, vh2, vh3, vh4, vh5, vh6) (Nucleic Acids Research, 2005, Vol. 33, Database issue D593-D597).

La similarité de l'anticorps à humaniser aux 49 modèles de séquences humaines germinales est quantifiée en échantillonnant les positions de certains atomes des 60 acides-aminés flexibles d'un anticorps, au cours des dix dynamiques moléculaires, au moyen d'une même grille cubique tridimensionnelle de résolution 1Å. On parle de similarité quadridimensionnelle. La grille tridimensionnelle utilisée comporte 445740 points et elle est initialisée à partir de la structure tridimensionnelle de l'anticorps humain répondant au code PDB 8FAB. La structure 8FAB est aussi utilisée pour positionner toutes les conformations d'un anticorps à échantillonner dans la grille tridimensionnelle. On superpose pour ce faire la conformation médoïde de la dynamique moléculaire de l'anticorps sur la structure 8FAB. Cette superposition consiste en l'alignement des moments d'inertie des deux conformations, suivi de l'optimisation des distances scalaires entre les atomes de carbone alpha des deux conformations. Toutes les autres conformations de la dynamique moléculaire de l'anticorps sont superposées sur la conformation médoïde selon la même méthode. Deux types d'échantillonnage sont réalisés résultant en deux similarités (similarité électrostatique et similarité lipophile), pour un couple d'anticorps à comparer, que l'on additionne pour obtenir la similarité totale. Le premier échantillonnage, électrostatique, considère tous les atomes de la chaîne latérale de l'acide-aminé. La valeur en un point x de la grille se calcule en appliquant aux atomes de la chaîne latérale de l'acide aminés une fonction gaussienne tridimensionnelle f(x) que l'on pondère avec la charge électrostatique de l'atome telle que décrite dans le champ de force Amber99 (Cieplak, J., et al.; J. Comp. Chem. 2001, 22 :1048-1057). La fonction f(x) est appliquée selon les 3 axes de coordonnées cartésiennes et répond à la formule : $f(x) = \left(s\sqrt{2\pi}\right)^{-3} \times \exp\left(\dfrac{-(x-u)^2}{2s^2}\right)$, avec x et u respectivement coordonnées cartésiennes d'un point x de la grille et d'un atome échantillonné, s= r/1,6 (r = rayon de covalence de l'atome). L'échantillonnage est répété pour toutes les conformations de l'acide aminé et les résultats obtenus sont moyennés en tout point x de la grille tridimensionnelle. Le deuxième échantillonnage, lipophile, considère uniquement les atomes lipophiles de la chaîne latérale de l'acide aminé. La valeur en un point x de la grille se calcule à l'aide de la même fonction gaussienne f(x) sans pondération. Les deux ensembles de conformations des dynamiques moléculaires des deux anticorps que l'on compare sont ainsi échantillonnés par la même grille tridimensionnelle. On mesure la similarité électrostatique (sim-elec), entre 2 anticorps a et b, à l'aide de la formule suivante: $sim-elec = \dfrac{\sum\limits_{i=1}^{445740}\left(\left|x_i^a+x_i^b\right|-\left|x_i^a-x_i^b\right|\right)}{\sum\limits_{i=1}^{445740}\left(\left|x_i^a+x_i^b\right|\right)}$ . La similarité lipophile est calculée avec la même formule sur les données issues de l'échantillonnage lipophile décrit ci-dessus.

Le modèle de séquences germinales humaines vlambda2-vh2 montre ainsi la plus forte similarité quadridimensionnelle (similarité totale = 58 %) de ces 60 acide-aminés flexibles par rapport aux acide-aminés flexibles de l'anticorps murin 40-12. Le modèle de séquence germinale humaine vlambda2-vh2 a donc été utilisé pour humaniser l'anticorps à humaniser en se focalisant sur les 45 acide-aminés flexibles. Pour déterminer les mutations à opérer, la structure tridimensionnelle du modèle de l'anticorps murin 40-12 est superposée à celle du modèle issu des séquences germinales montrant la plus forte similarité en optimisant les positions des carbones alpha des acides aminés. Les acides aminés identifiés comme flexibles sont mutés par les acides aminés correspondant dans la séquence du modèle montrant la plus forte similarité.

Les motifs de séquences indésirables considérés sont les suivants: Aspartate-Proline (liaison peptidique labile en milieu acide), Asparagine-X-Serine/Thréonine (site de glycosylation, X=tout acide-aminé sauf Proline), Aspartate-Glycine/Serine/Thréonine (formation potentielle de succinimide/iso-aspartate dans les zones flexibles), Asparagine-Glycine/Histidine/Serine/Alanine/Cystéine (sites exposés de déamidation), Methionine (oxydation dans les zones exposées). Les séquences humanisées ainsi obtenues sont finalement comparées, au moyen de l'algorithme BLAST de comparaison de séquence, aux séquences de la base de données IEDB (http://www.immuneepitope.org/ The immune epitope database and analysis resource: from vision to blueprint. PLoS Biol. 2005 Mar;3(3):e91) pour s'assurer que les séquences ne contiennent pas d'épitopes connus pour être reconnu par les cellules lymphocytes B et T. Si la séquence contient des résidus qui présentent des séquences indésirables, elles sont alors aussi modifiées. Si la séquence composite contient un épitope connu répertorié dans l'IEDB, une autre structure de référence germinale montrant une forte similarité est alors utilisée comme modèle.

De manière plus avantageuse, l'anticorps selon l'invention comprend des séquences présentant au moins 80%, 90%, 95% ou 99% d'identité avec la séquence SEQ ID NO 30 ou la séquence SEQ ID NO 32 sont utilisées, en combinaison avec les séquences présentant au moins 80%, 90%, 95% ou 99% d'identité avec la séquence SEQ ID NO 34, séquence SEQ ID NO 36 ou la séquence SEQ ID NO 38.

Dans un mode de réalisation, l'anticorps selon l'invention comprend des chaînes variables légères de séquence SEQ ID NO 30 et des chaînes variables lourdes de séquence SEQ ID NO 34.

Dans un autre mode de réalisation, l'anticorps comprend des chaînes variables légères de séquence SEQ ID NO 32 et des chaînes variables lourdes de séquence SEQ ID NO 38.

Dans un autre mode de réalisation, l'anticorps comprend des chaînes variables légères de séquence SEQ ID NO 30 et des chaînes variables lourdes de séquence SEQ ID NO 36.

Dans un autre mode de réalisation, l'anticorps comprend des chaînes variables légères de séquence SEQ ID NO 32 et des chaînes variables lourdes de séquence SEQ ID NO 34.

Dans un autre mode de réalisation l'anticorps comprend des chaînes variables légères de séquences SEQ ID NO 32 et des chaînes variables lourdes de séquence SEQ ID NO 36.

Dans un autre mode de réalisation l'anticorps comprend des chaînes variables légères de séquences SEQ ID NO 30 et des chaînes variables lourdes de séquence SEQ ID NO 38.

La présente invention a également pour objet des anticorps humains antagonistes spécifiques du FGF-R4. De tels anticorps peuvent être obtenus par phage display selon des méthodes connus de l'homme du métier (McCafferty J. *et al,* 1990 ; Hoogenboom, HR *et al,* 2005). D'autres technologies sont disponibles pour la préparation d'anticorps humains telles que la technologie XenoMouse décrite dans le brevet U.S. 5,939,598.

Dans un mode de réalisation particulier, l'anticorps antagoniste spécifique du récepteur FGF-R4 est un anticorps humain dont les parties variables de la chaîne lourde comprennent une séquence présentant au moins 80%, 90%, 95% ou 99% d'identité avec la séquence SEQ ID NO : 76, 86, 96 ou 106.

Dans un autre mode de réalisation, l'anticorps antagoniste spécifique du récepteur FGF-R4 est un anticorps humain dont les parties variables de la chaîne légère comprennent une séquence présentant au moins 80%, 90%, 95% ou 99% d'identité avec la séquence SEQ ID NO : 71, 81, 91 ou 101.

La présente invention a également pour objet un anticorps humain antagoniste spécifique du récepteur FGF-R4 comprenant une chaîne lourde comprenant une partie variable codée par une séquence nucléotidique présentant une identité d'au moins 80%, 90%, 95% ou 99% avec la séquence SEQ ID NO 77, 87, 97 ou 107.

La présente invention a également pour objet un anticorps humain antagoniste spécifique du récepteur FGF-R4 comprenant une chaîne légère comprenant une partie variable codée par une séquence nucléotidique présentant une identité d'au moins 80%, 90%, 95% ou 99% avec la séquence SEQ ID NO 72, 82, 92 ou 102.

La présente invention a également pour objet un anticorps humain antagoniste spécifique du récepteur FGF-R4 comprenant des séquences codées par les séquences nucléotidiques SEQ ID NO : 71 et 76 ou les séquences nucléotidiques SEQ ID NO : 81 et 86 ou les séquences nucléotidiques SEQ ID NO : 91 et 96 ou les séquences nucléotidiques SEQ ID NO : 101 et 106.

La présente invention a également pour objet un anticorps humain antagoniste spécifique du récepteur FGF-R4 comprenant les séquences polypeptidiques SEQ ID NO: 72 et 77 ou les séquences polypeptidiques SEQ ID NO: 82 et 87 ou les séquences polypeptidiques SEQ ID NO : 92 et 97 ou les séquences polypeptidiques SEQ ID NO: 102 et 107.

De manière particulièrement préférée, un anticorps humain antagoniste spécifique du récepteur FGF-R4 comprenant les séquences polypeptidiques SEQ ID NO : 82 et 87. Les motifs de séquences indésirables considérés sont les suivants: Aspartate-Proline (liaison peptidique labile en milieu acide), Asparagine-X-Serine/Thréonine (site de glycosylation, X=tout acide-aminé sauf Proline), Aspartate-Glycine/Serine[Thréonine (formation potentielle de succinimide/iso-aspartate dans les zones flexibles), Asparagine-Glycine/Histidine/Serine/Alanine/Cystéine (sites exposés de déamidation), Methionine (oxydation dans les zones exposées). Les séquences humanisées ainsi obtenues sont finalement comparées, au moyen de l'algorithme BLAST de comparaison de séquence, aux séquences de la base de données IEDB (http://www.immuneepitope.org/ The immune epitope database and analysis resource: from vision to blueprint. PLoS Biol. 2005 Mar;3(3):e91) pour s'assurer que les séquences ne contiennent pas d'épitopes connus pour être reconnu par les cellules lymphocytes B et T. Si la séquence contient des résidus qui présentent des séquences indésirables, elles sont alors aussi modifiées. Si la séquence composite contient un épitope connu répertorié dans l'IEDB, une autre structure de référence germinale montrant une forte similarité est alors utilisée comme modèle.

De manière plus avantageuse, l'anticorps antagoniste spécifique du récepteur FGFR4 selon l'invention comprend des séquences présentant au moins 80%, 90%, 95% ou 99% d'identité avec la séquence SEQ ID NO 30 ou la séquence SEQ ID NO 32 sont utilisées, en combinaison avec les séquences présentant au moins 80%, 90%, 95% ou 99% d'identité avec la séquence SEQ ID NO 34, séquence SEQ ID NO 36 ou la séquence SEQ ID NO 38.

Dans un mode de réalisation, l'anticorps antagoniste spécifique du récepteur FGFR4 selon l'invention comprend des chaînes variables légères de séquence SEQ ID NO 30 et des chaînes variables lourdes de séquence SEQ ID NO 34.

Dans un autre mode de réalisation, l'anticorps antagoniste spécifique du récepteur FGFR4 comprend des chaînes variables légères de séquence SEQ ID NO 32 et des chaînes variables lourdes de séquence SEQ ID NO 38.

Dans un autre mode de réalisation, l'anticorps antagoniste spécifique du récepteur FGFR4 comprend des chaînes variables légères de séquence SEQ ID NO 30 et des chaînes variables lourdes de séquence SEQ ID NO 36.

Dans un autre mode de réalisation, l'anticorps antagoniste spécifique du récepteur FGFR4 comprend des chaînes variables légères de séquence SEQ ID NO 32 et des chaînes variables lourdes de séquence SEQ ID NO 34.

Dans un autre mode de réalisation l'anticorps antagoniste spécifique du récepteur FGFR4 comprend des chaînes variables légères de séquences SEQ ID NO 32 et des chaînes variables lourdes de séquence SEQ ID NO 36.

Dans un autre mode de réalisation l'anticorps antagoniste spécifique du récepteur FGFR4 comprend des chaînes variables légères de séquences SEQ ID NO 30 et des chaînes variables lourdes de séquence SEQ ID NO 38.

La présente invention a également pour objet des anticorps humains antagonistes spécifiques du FGF-R4. De tels anticorps peuvent être obtenus par phage display selon des méthodes connus de l'homme du métier (McCafferty J. *et al,* 1990 ; Hoogenboom, HR *et al,* 2005). D'autres technologies sont disponibles pour la préparation d'anticorps humains telles que la technologie XenoMouse décrite dans le brevet U.S. 5,939,598.

Dans un mode de réalisation particulier, l'anticorps antagoniste spécifique du récepteur FGF-R4 est un anticorps humain dont les parties variables de la chaîne lourde comprennent une séquence présentant au moins 80%, 90%, 95% ou 99% d'identité avec la séquence SEQ ID NO : 76, 86, 96 ou 106.

Dans un autre mode de réalisation, l'anticorps antagoniste spécifique du récepteur FGF-R4 est un anticorps humain dont les parties variables de la chaîne légère comprennent une séquence présentant au moins 80%, 90%, 95% ou 99% d'identité avec la séquence SEQ ID NO : 71, 81, 91 ou 101.

La présente invention a également pour objet un anticorps humain antagoniste spécifique du récepteur FGF-R4 comprenant une chaîne lourde comprenant une partie variable codée par une séquence nucléotidique présentant une identité d'au moins 80%, 90%, 95% ou 99% avec la séquence SEQ ID NO 77, 87, 97 ou 107.

La présente invention a également pour objet un anticorps humain antagoniste spécifique du récepteur FGF-R4 comprenant une chaîne légère comprenant une partie variable codée par une séquence nucléotidique présentant une identité d'au moins 80%, 90%, 95% ou 99% avec la séquence SEQ ID NO 72, 82, 92 ou 102.

La présente invention a également pour objet un anticorps humain antagoniste spécifique du récepteur FGF-R4 comprenant des séquences codées par les séquences nucléotidiques SEQ ID NO : 71 et 76 ou les séquences nucléotidiques SEQ ID NO : 81 et 86 ou les séquences nucléotidiques SEQ ID NO : 91 et 96 ou les séquences nucléotidiques SEQ ID NO : 101 et 106.

La présente invention a également pour objet un anticorps humain antagoniste spécifique du récepteur FGF-R4 comprenant les séquences polypeptidiques SEQ ID NO : 72 et 77 ou les séquences polypeptidiques SEQ ID NO :

82 et 87 ou les séquences polypeptidiques SEQ ID NO : 92 et 97 ou les séquences polypeptidiques SEQ ID NO : 102 et 107.

De manière particulièrement préférée, un anticorps humain antagoniste spécifique du récepteur FGF-R4 comprenant les séquences polypeptidiques SEQ ID NO : 82 est 87.

Les séquences en acides aminés ainsi modifiées peuvent aussi être modifiées par des modifications post-traductionnelles au cours de la production dans la cellule mammifère. En particulier l'emploi de lignées stables déficientes dans la biosynthèse du fucose peut permettre de produire des anticorps monoclonaux dont le N-glycan du Fc (position N297) est dépourvu partiellement ou totalement de fucose et permet d'augmenter l'effet effecteur ADCC (Kanda et al., 2006, Biotechnol. Bioeng. 94 :680-688 et Ripka et al., 1986 Arch Biochem Bioph 249 : 533-545).

Dans un autre mode de réalisation de l'invention, l'anticorps antagoniste spécifique de FGF-R4 est un anticorps conjugué.

Les anticorps peuvent être conjugués à un agent cytotoxique. Le terme « agent cytotoxique » désigne ici une substance qui réduit ou bloque la fonction, ou la croissance, des cellules, ou cause la destruction des cellules.

Dans un mode de réalisation, l'anticorps ou un fragment de liaison de celui-ci peut-être conjugué à un médicament, tel qu'un maytansinoïde, pour former un « pro-médicament» ayant une cytotoxicité envers les cellules exprimant l'antigène.

L'agent cytotoxique de la présente invention peut-être tout composé qui résulte en la mort d'une cellule, ou induit la mort d'une cellule, ou diminue de diverses façons la viabilité des cellules. Les agents cytotoxiques préférés incluent, par exemple, des

**Figure 2B :** Carte du plasmide pXL4613 permettant l'expression de hFGFR4-Streptag. (SEQ ID NO 69).

**Figure 3 :** Carte du plasmide pXL4615 permettant l'expression de hFGFR4(D2,D3)-Histag. (SEQ ID NO 42)

**Figure 4 :** Carte du plasmide pXL4621 permettant l'expression de mFGFR4-Histag. (SEQ ID NO 44)

**Figure 5 :** Carte du plasmide pXL4328 permettant l'expression de hFGFR1-Fc (de séquence SEQ ID NO 46)

**Figure 6 :** Carte du plasmide pXL4327 permettant l'expression de hFGFR2-Fc (de séquence SEQ ID NO 48)

**Figure 7 :** Des plaques ELISA sont recouvertes par les 4 récepteurs humains aux FGF. La capacité des anticorps anti-FGFR4 40-12 et 64-12 à reconnaître ces différents FGF-Rs est mesurée par dosage ELISA. Le clone 40-12 (histogramme noir) est spécifique de FGF-R4. Le clone 64-12 (histogramme gris) reconnaît en plus très faiblement le FGF-R3.

**Figures 8A et 8B :** L'anticorps antagoniste actif 40-12 et son contrôle inactif 64-12 n'ont pas d'effet propre sur l'angiogenèse basale. Par contre, à la dose de 30 µg/ml (200 nM environ), l'anticorps monoclonal anti-FGFR4 40-12 est capable d'inhiber l'angiogenèse de cellules HUVEC induite par le FGF2 alors que l'anticorps contrôle 64-12 n'en est pas capable (Fig 8A).

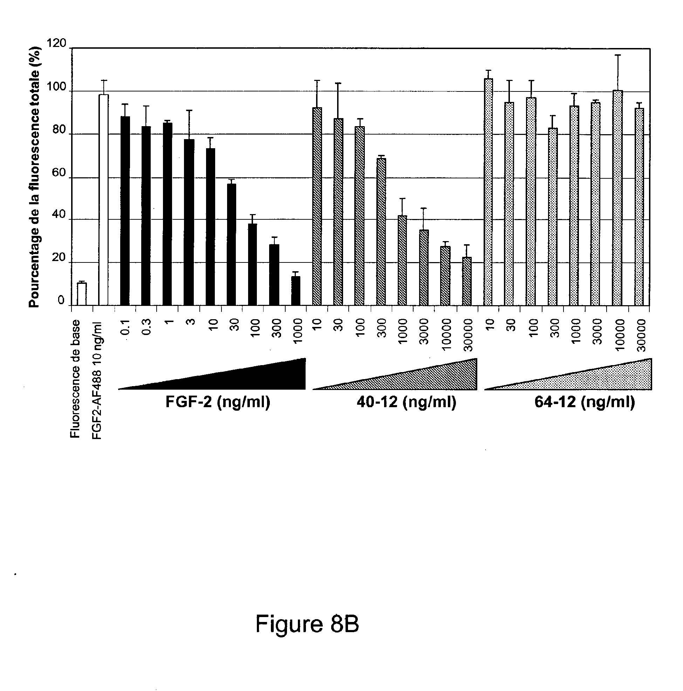

Du FGF2 marqué à l'aide d'un AlexaFluor® 488 nm est capable de se lier au FGF-R4 exprimé par des cellules 300-19 (histogramme blanc). Cette interaction peut être dissociée par du FGF2 non marqué (histogrammes noirs) ou par l'anticorps anti-FGFR4 40-12 (histogrammes gris foncés) alors que l'anticorps témoins 64-12 n'en n'est pas capable (histogrammes gris clairs) (Fig 8B).

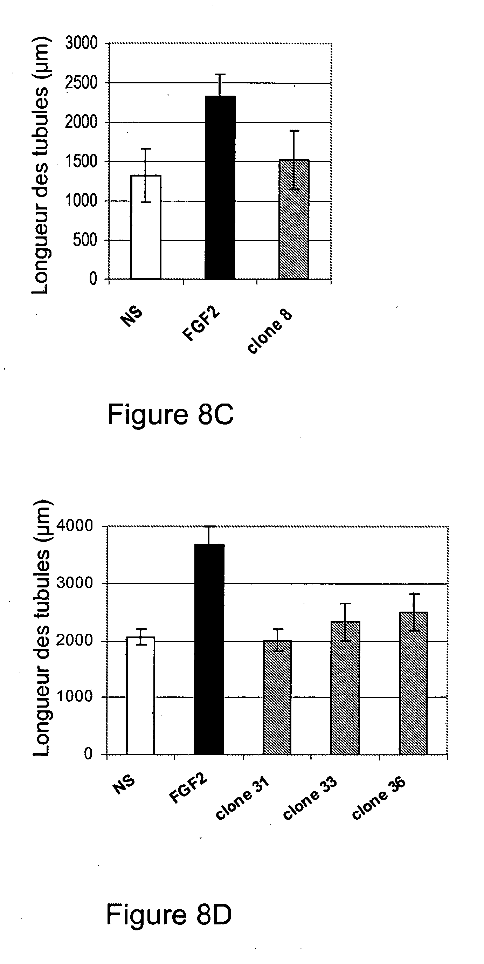

**Figure 8C et 8D :** Effet des anticorps anti-FGFR4 issus des clones 8, 31, 33 et 36 à 10µg/ml sur l'angiogenèse *in vitro* induite par 3 ng/ml de FGF-2. Les clones 8 (Fig 8C) et 31, 33 et 36 (Fig 8D) inhibent l'angiogenèse de cellules HUVEC induite par le FGF-2.

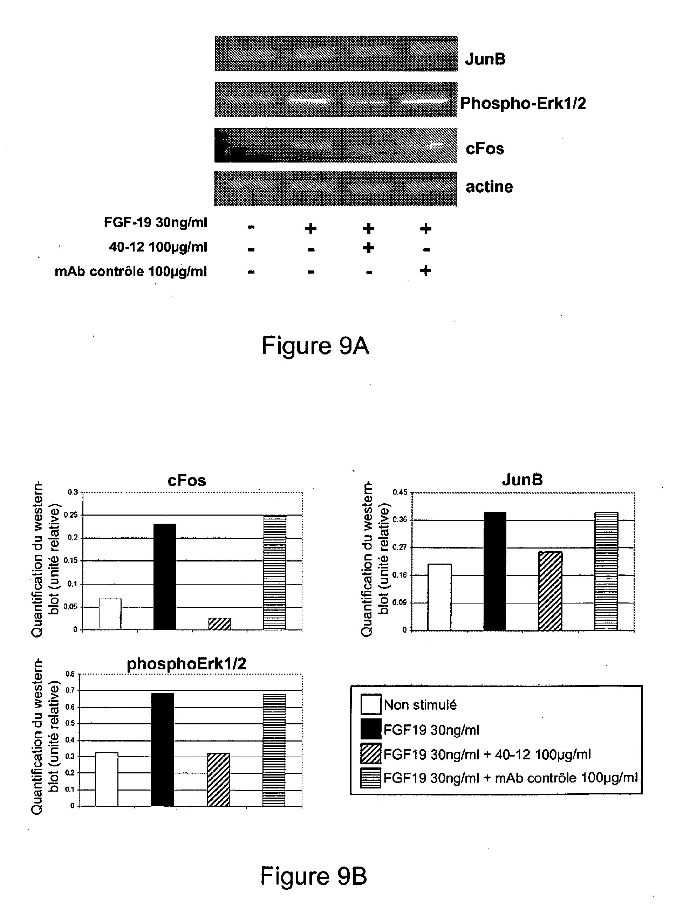

**Figures 9A et 9B :** Des cellules d'hépatocarcinomes humains Hep3b sont stimulées par du FGF19 à 30 ng/ml. Cette stimulation induit une signalisation cellulaire spécifique de FGF-R4 aboutissant à la synthèse des protéines cFos et JunB et à la phosphorylation de Erk1/2 observé par Western-blot (Fig 9A). Chaque bande est alors quantifiée. Cette quantification est représentée sous forme d'histogramme (Fig 9B). L'anticorps 40-12 à 100 µg/ml inhibe totalement l'induction de la signalisation cellulaire spécifique de FGF-R4 alors que l'anticorps contrôle n'a aucun effet. En effet, l'anticorps 40-12 bloque complètement la synthèse de JunB et de cFos ainsi que la phosphorylation de Erk1/2 induite par le FGF19 après une simulation de 3h.

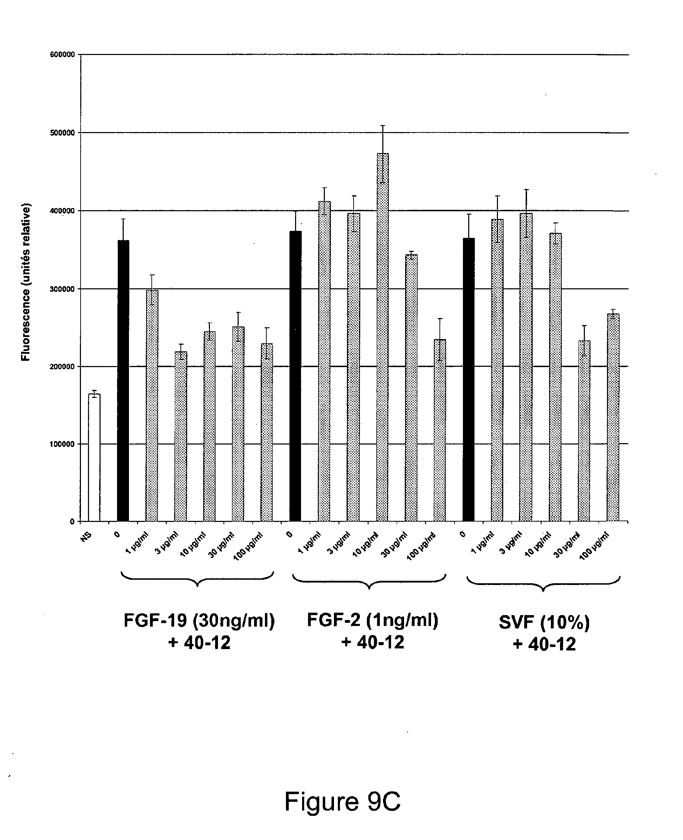

**Figure 9C :** L'effet inhibiteur de l'anticorps anti-FGFR4 40-12 sur la phosphorylation de Erk1/2 induite dans les cellules Hep3b par le FGF-19 (30 ng/ml) est confirmée à l'aide de l'ELISA anti-phosphoERk1/2. L'anticorps 40-12 est aussi capable d'inhiber la phosphorylation de Erk1/2 dans les cellules Hep3b stimulées par du FGF-2 (1 ng/ml) ou par du sérum (SVF) à 10%.

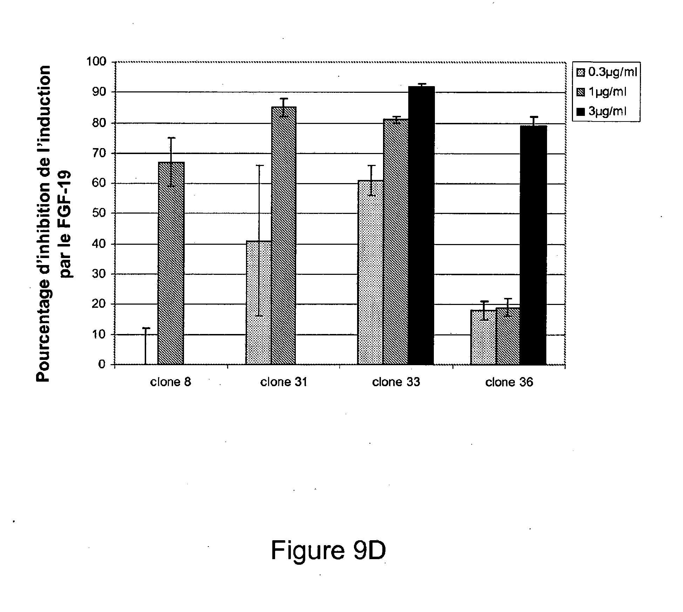

**Figure 9D :** Pourcentage d'inhibition de la phosphorylation de Erk1/2 induite par le FGF-19 (30ng/ml) obtenue par ELISA sur cellules Hep3b à l'aide des anticorps issus des clones 8, 31, 33 et 36.

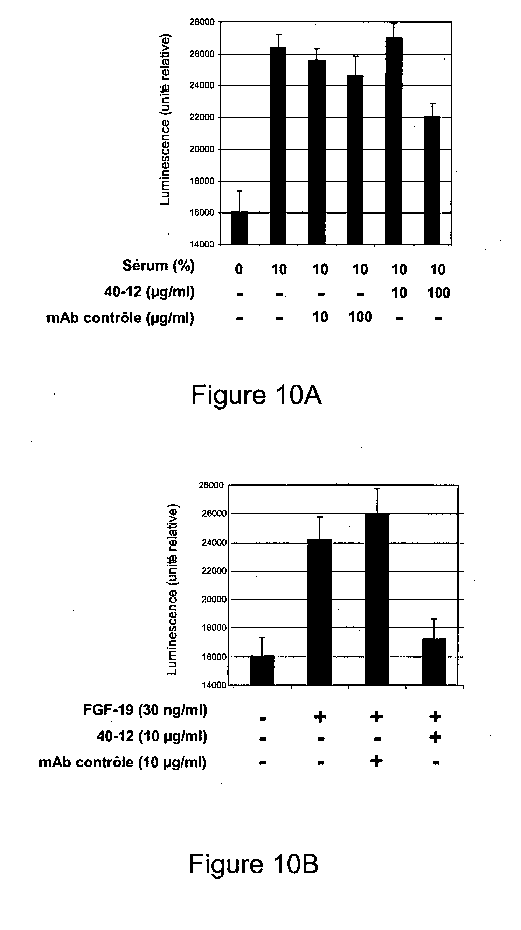

**Figures 10A et 10B :** La prolifération de cellules Hep3b peut être stimulée par l'ajout de sérum (Fig 10A) ou de FGF19 (Fig 10B). L'induction par le sérum est partiellement inhibée par l'anticorps 40-12 à 100 μg/ml alors que l'anticorps contrôle n'a pas d'effet (Fig 10A). La prolifération induite par le FGF19 est totalement bloquée par 10 μg/ml d'anticorps anti-FGFR4 40-12 alors qu'à cette même dose, l'anticorps contrôle n'en n'est pas capable (Fig 10B).

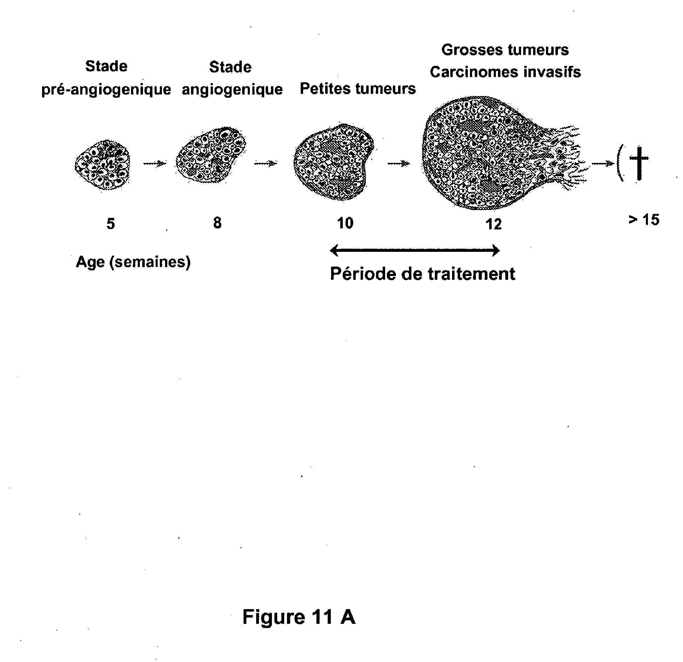

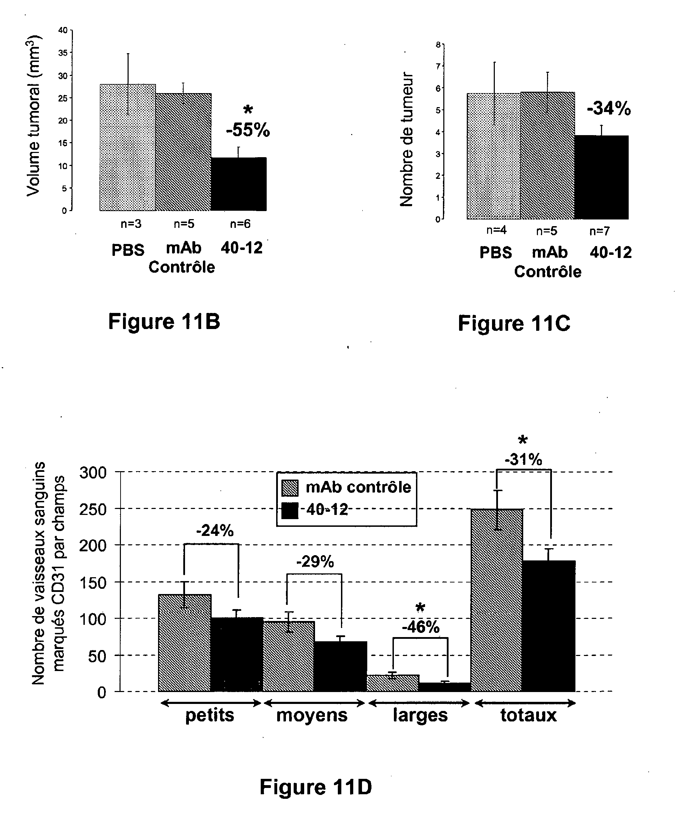

**Figures 11 A à 11D:** L'anticorps anti-FGFR4 40-12 est capable de réduire le développement de tumeurs pancréatiques dans un modèle murin RipTag en inhibant l'angiogenèse tumorale : Le modèle Rip1-Tag2 est un modèle murin où des souris transgéniques expriment l'antigène T du SV40 dans les cellules β des ilots du pancréas produisant l'insuline (Hanahan D. Nature, 1985, 9-15:115-22.). Cet antigène T est exprimé au cours du développement embryonnaire du pancréas jusqu'à 4 à 5 semaines de vie, sans effet apparent. Une partie des ilots pancréatiques exprimant l'antigène T progresse alors pendant les 5 semaines suivantes vers la formation d'ilots angiogéniques associés à une activation de la vasculature puis vers le développement de petites tumeurs de type adénome. Quelques semaines plus tard quelques adénomes se développent pour former des carcinomes invasifs (Fig.11A). Ces souris Rip1-Tag2 sont traitées par voie sous-cutanée une fois par semaine entre les semaines 10 et 13 à la dose de 25 mg/kg à l'aide de l'anticorps 40-12 ou de l'anticorps contrôle. Après 13 semaines, les souris sont sacrifiées. Le volume tumoral ainsi que le nombre de tumeurs par pancréas et la densité vasculaire sont déterminés. Le traitement par l'anticorps anti-FGFR4 40-12 permet de réduire significativement le volume tumoral de 55% alors que l'anticorps témoins ne présente aucun effet (Fig. 11B). L'anticorps 40-12 permet également de réduire de 34% le nombre de tumeurs par pancréas en comparaison du témoin (Fig. 11 C). Cette réduction du volume tumoral est accompagnée par une réduction de la densité vasculaire, que ce soit au niveau du nombre de vaisseaux petits, moyens ou larges (Fig. 11 D).

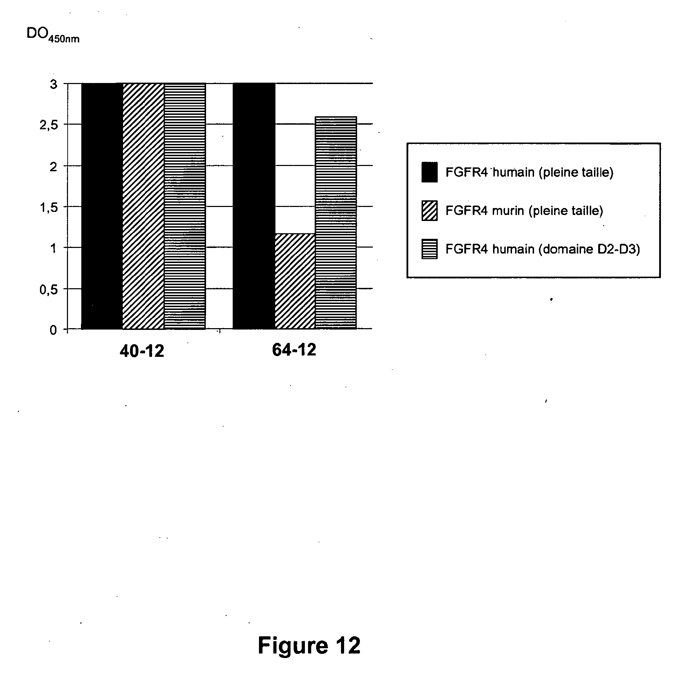

**Figure 12 :** La capacité des anticorps 40-12 et 64-12 à reconnaître le domaine extracellulaire complet du FGF-R4 humain ou murin ainsi que le domaine extracellulaire du FGF-R4 amputé de son domaine D1 est mesuré par ELISA. Le clone 40-12 est capable de se lier aussi bien avec les 3 constructions de FGF-R4 alors que le clone 64-12 reconnaît moins bien la forme murine du FGF-R4.

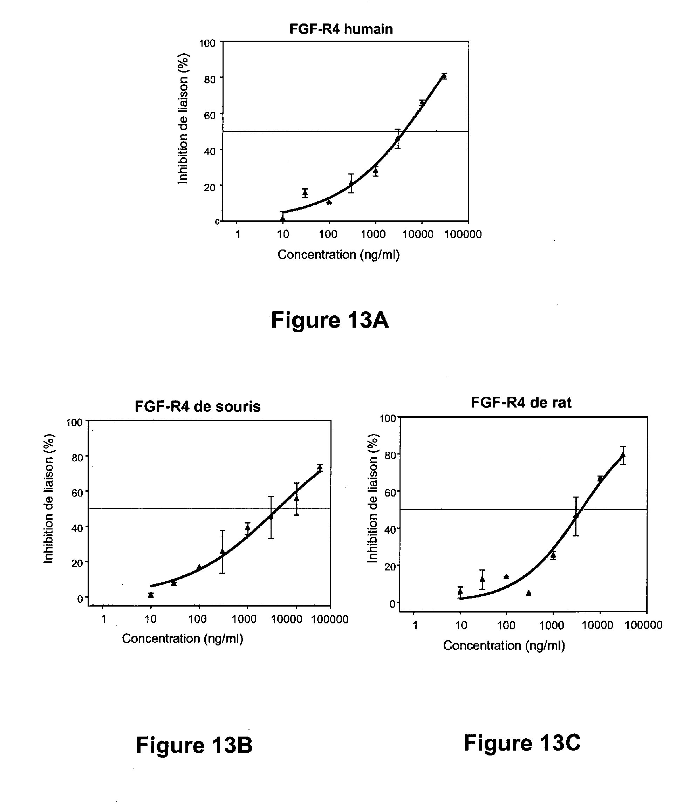

**Figure 13A à 13C :** L'effet antagoniste de l'anticorps anti-FGFR4 clone 40-12 sur la liaison FGF2/FGF-R4 est mesuré par des expériences de compétition de liaison avec du FGF2 marqué à l'aide d'un AlexaFluor® 488 nm. Le clone 40-12 est capable de bloquer la liaison du FGF2 humain (Fig. 13A), murin (Fig. 13B) et de rat (Fig. 13C) sur des cellules 300-19 murines transfectées à l'aide d'ADNc codant pour les formes humaines, murines ou de rat du récepteur FGF-R4, et ce, avec la même efficacité (3500, 4110 et 3940 ng/ml soit 23, 27 et 26 nM pour les complexes humains, murins ou de rat respectivement).

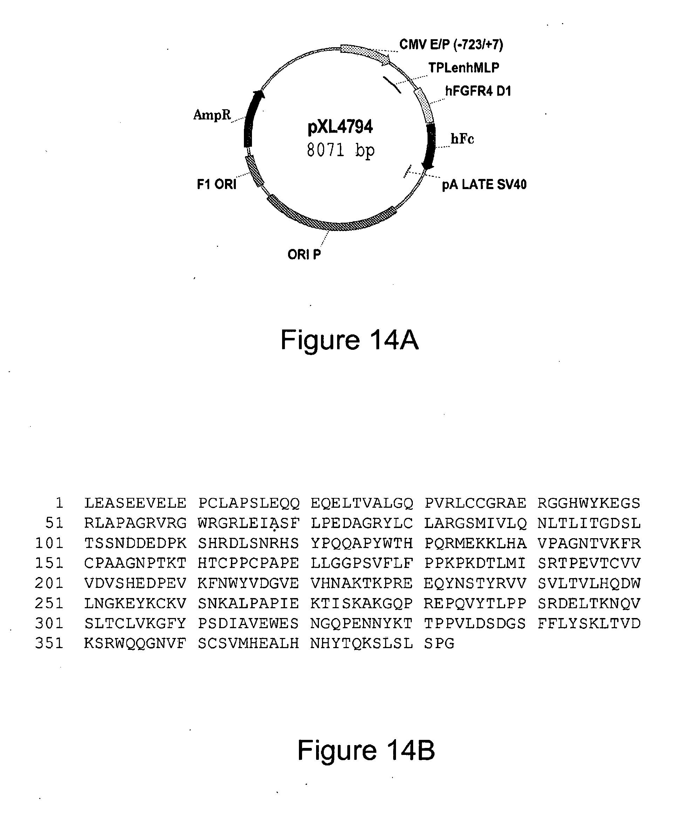

Figure 14A : Carte du plasmide pXL4794 permettant l'expression de hFGFR4_D1 : Fc

Figure 14B : Séquence en acides aminés de la protéine hFGFR4_D1 : Fc sécrétée dans la lignée HEK293 transfectée avec le plasmide pXL4794

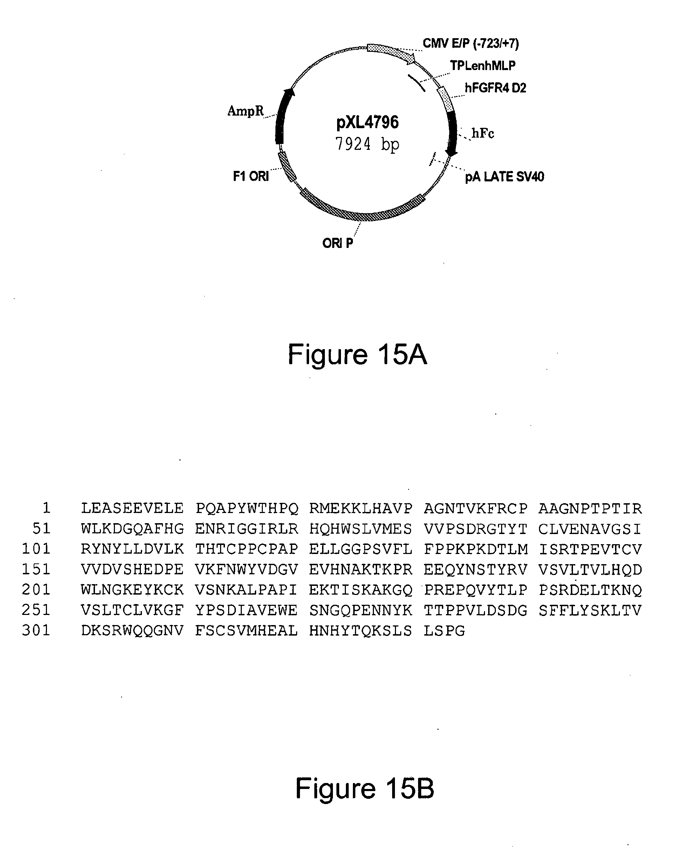

Figure 15A : Carte du plasmide pXL4796 permettant l'expression de hFGFR4_D2 : Fc

Figure 15B : Séquence en acides aminés de la protéine hFGFR4_D2 : Fc sécrétée dans la lignée HEK293 transfectée avec le plasmide pXL4796

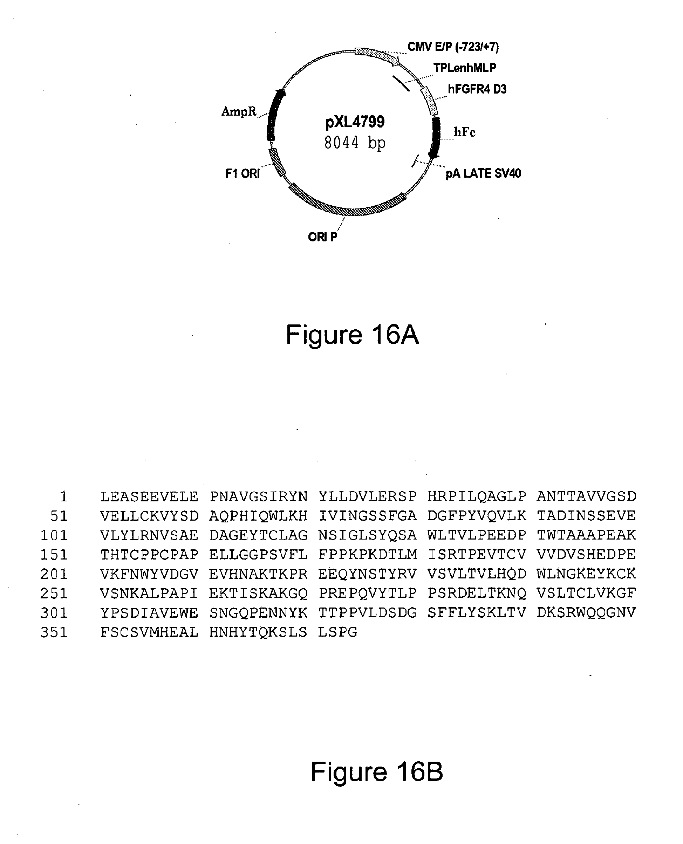

Figure 16A : Carte du plasmide pXL4799 permettant l'expression de hFGFR4_D3 : Fc.

Figure 16B : Séquence en acides aminés de la protéine hFGFR4_D3 : Fc sécrétée dans la lignée HEK293 transfectée avec le plasmide pXL4799

**EXEMPLES :**

**Exemple 1 : Mise en évidence du rôle de FGF-R4 dans le contrôle de l'angiogenèse**

Afin de démontrer le rôle de FGF-R4 dans le contrôle de l'angiogenèse, des expériences d'angiogenèse *in vitro* ont été réalisées avec des cellules endothéliales humaines de type HUVEC stimulées avec plusieurs FGF. Le FGF2, ligand activateur de la plupart des récepteurs aux FGF. Le FGF19, ligand activateur spécifique de FGF-R4 et le FGF4 ligand non activateur de FGF-R4.

Pour ce faire, des gels ont été préparés en distribuant dans chaque puits de chamberslide (Biocoat Cellware collagen, Type I, 8-well culturesides : Becton dickinson 354630), 160$\mu$l de matrigel dilué au 1/6 (Growth factor reduced Matrigel : Becton dickinson 356230) dans du collagène (rat Tail collagene, type I : Becton dickinson 354236). Les gels sont maintenus pendant 1 heure à 37°C pour permettre leur polymérisation. Ensuite, les cellules endothéliales veineuses humaines (HUVEC réf : C-12200 - Promocell) ont été ensemencées à $15.10^3$ cellules/puits dans 400 $\mu$l de milieu EBM (Clonetics C3121) + 2 % FBS + hEGF 10$\mu$g/ml. Ce protocole peut être adapté à des plaques 96 puits : 60$\mu$l par puits de plaques 96 puits (Biocoat collagenl cellware, Becton Dickinson 354407). La matrice est préparée en mélangeant 1/3 de matrigel, 1mg/ml final de collagène, NaOH (0.026x le volume de collagène en $\mu$l), PBS 1x, le volume est ensuite ajusté par de l'eau. Les cellules endothéliales sont stimulées avec 1 ng/ml de FGF2 (R&D, 133-FB-025) ou 10 ng/ml de FGF4 (R&D, 235-F4-025) ou de FGF19 (R&D, 969-FG-025) pendant 24h à 37°c en présence de 5 % de $CO_2$. Après 24 heures, la longueur du réseau de microtubules formés est mesurée à l'aide d'un système d'analyse d'images assisté par ordinateur (Imagenia Biocom, Courtaboeuf, France) et la longueur totale des pseudo-tubules dans chaque puits est déterminée. La moyenne de la longueur totale du réseau de microcapillaire est calculée en $\mu$m pour chaque condition correspondant à la moyenne sur 6 réplicats.

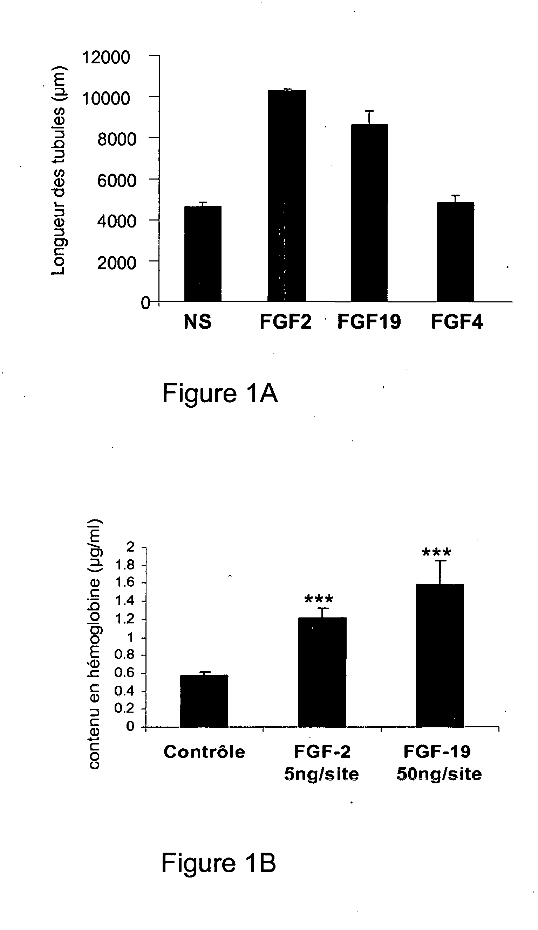

La stimulation par du FGF2 ou du FGF19 permet l'induction de la formation de nouveaux tubules alors que le FGF4 n'a pas d'effet (Fig. 1A). Ces résultats montrent que l'activation spécifique de FGF-R4 permet d'induire la formation de néo-vaisseaux et donc de conclure que FGF-R4 contrôle l'angiogenèse *in vitro.*

La corrélation *in vivo* avec les données *in vitro* a été obtenue à l'aide du modèle d'induction de l'angiogenèse *in vivo* dans des implants de cellulose chez la souris. Ce modèle est une adaptation du modèle décrit par Andrade et al. (Microvascular Research, 1997, 54, 253-61).

Les animaux, souris blanches BALB/c J consanguines sont anesthésiées avec un mélange Xylazine (Rompun®, 10mg/kg)/ Kétamine (Imalgène 1000, 100mg/kg) par voie intra péritonéale. Le dos de l'animal est rasé et désinfecté à l'Héxomédine®. Une poche d'air est créée en sous cutané sur le dos de la souris par injection de 5 ml d'air stérile. Une incision d'environ 2 cm, sur le haut du dos de l'animal est réalisée afin d'introduire un implant de cellulose stérile (disque de 1 cm de diamètre, 2 mm d'épaisseur, Cellspon® réf 0501) imprégné avec 50 $\mu$l de solution stérile contenant la protéine ou le produit à tester. L'incision est ensuite suturée et nettoyée à l'Héxomédine®. Les jours suivants la pose de l'implant, les souris ont reçu dans l'implant la protéine ou le produit par une injection à travers la peau (50 $\mu$l/implant/jour) sous anesthésie gazeuse (Isoflurane à 5 % (Aerrane®, Baxter). Sept jours après la pose de l'éponge, les souris sont sacrifiées par une dose létale de Pentobarbital sodique (CEVA santé animale), administrée par voie intra-péritonéale. La peau est ensuite découpée, environ 1 cm autour de l'éponge en évitant la cicatrice afin de dégager la peau et l'éponge. L'éponge est ensuite découpée en plusieurs morceaux et placée dans un tube Ribolyser® contenant 1 ml de tampon de lyse (Cell Death Detection ELISA, Roche). Les tubes sont agités 4 fois consécutives, durant 20 sec, force 4, au broyeur cellulaire (FastPrep® FP 120). Les tubes sont ensuite centrifugés 10 min à 2000 g à 20°C et les surnageants sont congelés à -20°C en attendant le dosage Hémoglobine.

Le jour du dosage, les tubes sont à nouveau centrifugés après décongélation et la concentration d'hémoglobine est mesurée avec le réactif de Drabkin (Sigma, volume à volume) par lecture au spectrophotomètre à 405 nm contre une gamme étalon d'hémoglobine bovine (Sigma). La concentration d'hémoglobine dans chaque échantillon est exprimée en mg/ml d'après la régression polynomiale réalisée à partir de la gamme. Les résultats sont exprimés en valeur moyenne ($\pm$ sem) pour chaque groupe. Les différences entre les groupes sont testées avec une ANOVA suivie d'un test de Dunnett sur la racine carrée des valeurs.

Dans ce modèle, le FGF19 à 50 ng par site et 5 ré-injections est capable d'induire significativement la colonisation de l'éponge par des vaisseaux matures nouvellement formés avec la même efficacité que le FGF2 à 5 ng par site. La présence de vaisseaux sanguins fonctionnels dans l'éponge est mise en évidence par la présence d'hémoglobine (Fig. 1 B). Ces résultats montrent que l'activation spécifique de FGF-R4 permet le recrutement de vaisseaux sanguins fonctionnels indiquant que FGF-R4 contrôle également l'angiogenèse *in vivo.*

**Exemple 2 : Description des protéines FGFR utilisées comme immunogène et antigène.**

Des récepteurs aux facteurs de croissance FGFR et en particulier leurs domaines extracellulaires des FGF-R1, FGF-R2, FGF-R3, FGF-R4 sont fusionnés à un tag (Histag) ou au domaine Fc d'immunoglobuline.

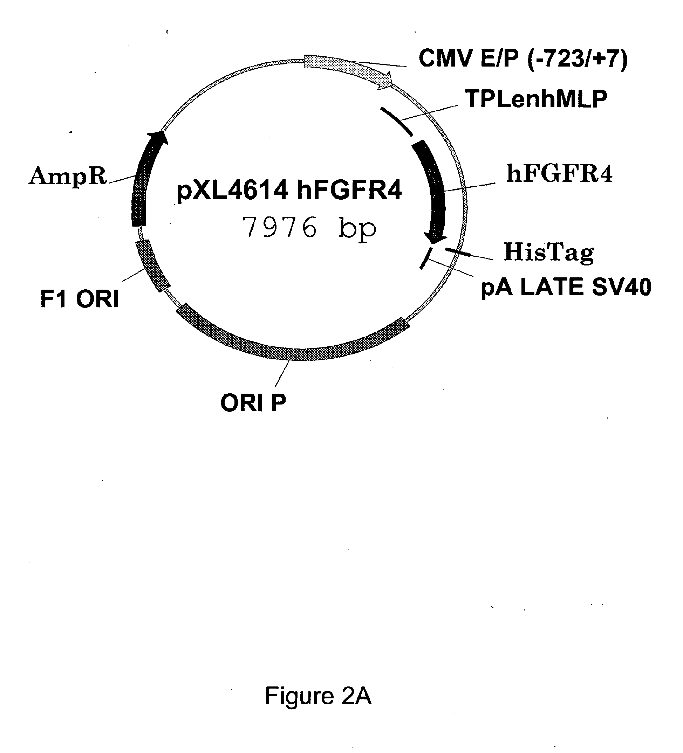

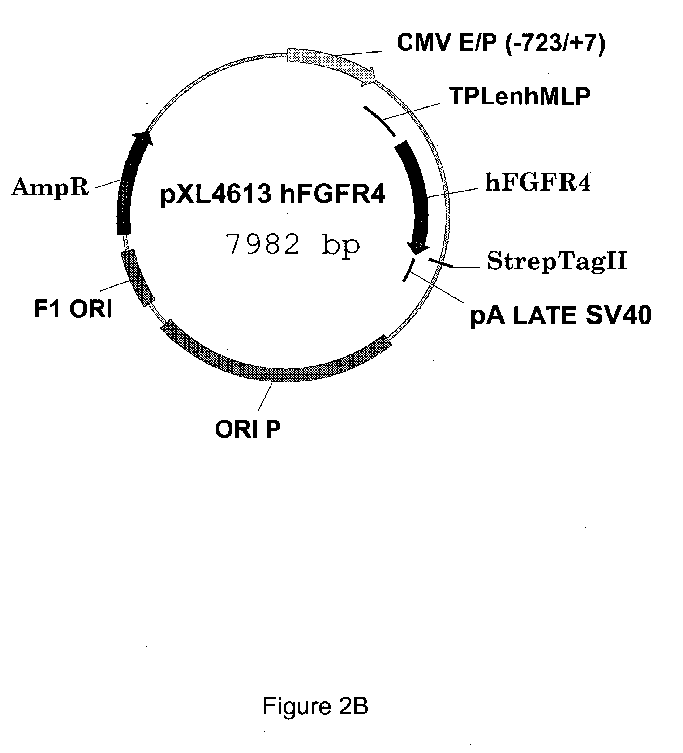

Le cDNA codant pour le domaine extracellulaire de FGF-R4 humain, correspond à la protéine décrite dans SwissProt FGF-R4_HUMAN position 1-365 avec la mutation L136P. Il a été cloné dans le vecteur d'expression eucaryote pXL4614 représenté sur la figure 2 afin d'exprimer une protéine contenant un Histag en position C-terminale dans le domaine extracellulaire.

Les protéines nommées hFGFR4-Histag, de séquence SEQ ID NO 40 ont été produites par transfection transitoire dans la lignée HEK293 EBNA (Invitrogen) à l'aide du plasmide pXL4614 et des plasmides accessoires pXL4544 et pXL4551 permettant l'expression de deux enzymes de glycosylation des N-glycans, i.e. la α-2,3-sialyltransférase et la β-1,4-galactosyltransférase comme cela a été décrit dans la demande WO2008/065543.

La protéine hFGFR4-Histag exprimée dans le surnageant de culture des cellules HEK293 EBNA a été purifiée par chromatographie sur colonne sépharose Ni-chelating (Amersham Biosciences; réf. 17-0575-01) et élution en tampon imidazole puis formulée en tampon PBS (Invitrogen ; réf. 14190-094). L'analyse de la composition en monosaccharides et la quantification des acides sialiques des N-glycans comme décrit par Saddic et al. 2002. (Methods Mol. Biol. 194:23-36 and Anumula et al.1998. Glycobiology 8:685-694) ont permis de démontrer que la protéine était très largement sialylée (91 %). Par conséquent la protéine avait toutes les caractéristiques pour avoir des propriétés pharmacocinétiques suffisantes.

De façon comparable la protéine hFGFR4-Streptag (SEQ ID NO 69) a été purifiée par chromatographie sur colonne Strep-Tactin Superflow (IBA ; réf. 2-1206) et élution en tampon desthiobiotine puis formulée en tampon PBS.

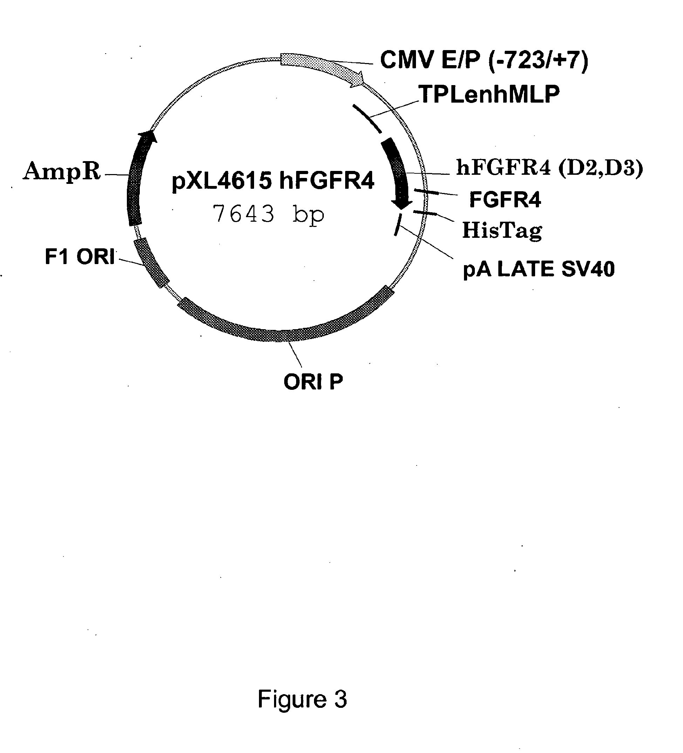

La protéine hFGFR4(D2,D3)-Histag correspond à la séquence SEQ ID NO 42. Le cDNA a été cloné dans le plasmide d'expression eucaryote pXL4615 représenté sur la figure 3 et la protéine a été produite et purifiée dans des conditions comparables à la protéine hFGFR4-Histag.

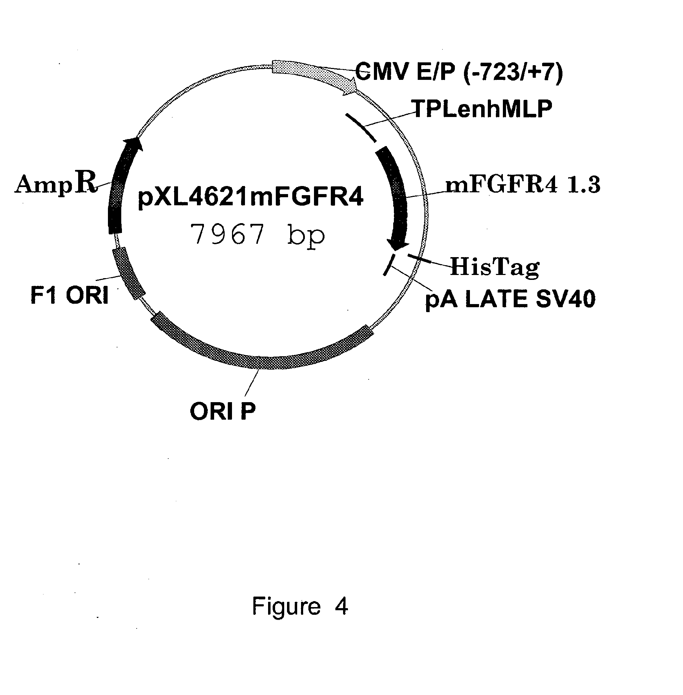

Le cDNA codant pour la protéine mFGFR4-Histag (SEQ ID NO 43) a été cloné dans le plasmide d'expression eucaryote pXL4621 représenté sur la figure 4 et la protéine a été produite et purifiée dans des conditions comparables à la protéine hFGFR4-Histag.

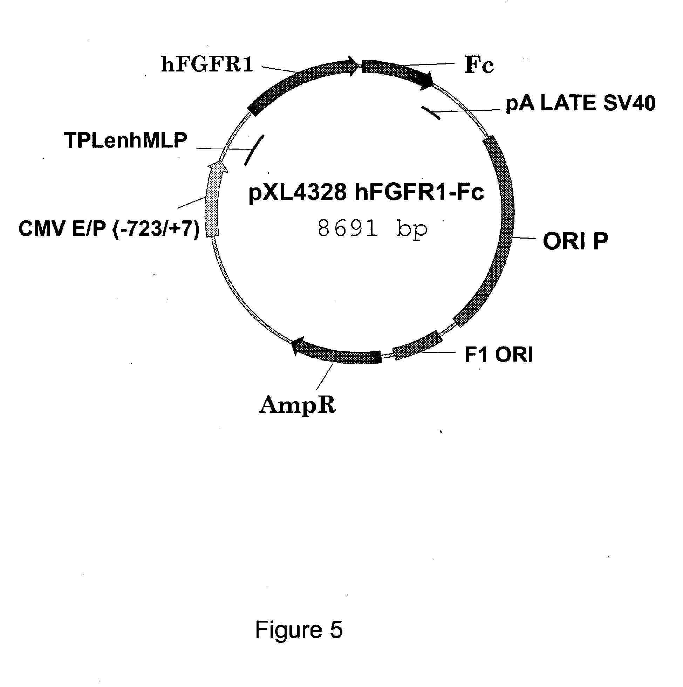

La protéine hFGFR1-Fc, (SEQ ID NO 46) contient le domaine extracellulaire de FGF-R1 IIIc humain fusionné en C-terminal au domaine Fc de l'IgG1 humain. Le cDNA a été cloné dans le plasmide d'expression eucaryote pXL4728 représenté sur la figure 5 et la protéine a été produite dans des conditions comparables à la protéine hFGFR4-Histag puis purifiée par chromatographie sur colonne d'affinité Sépharose protéine G (Amersham Biosciences) et élution en tampon 100 mM glycine/HCl pH 2,7 puis formulée en tampon PBS.

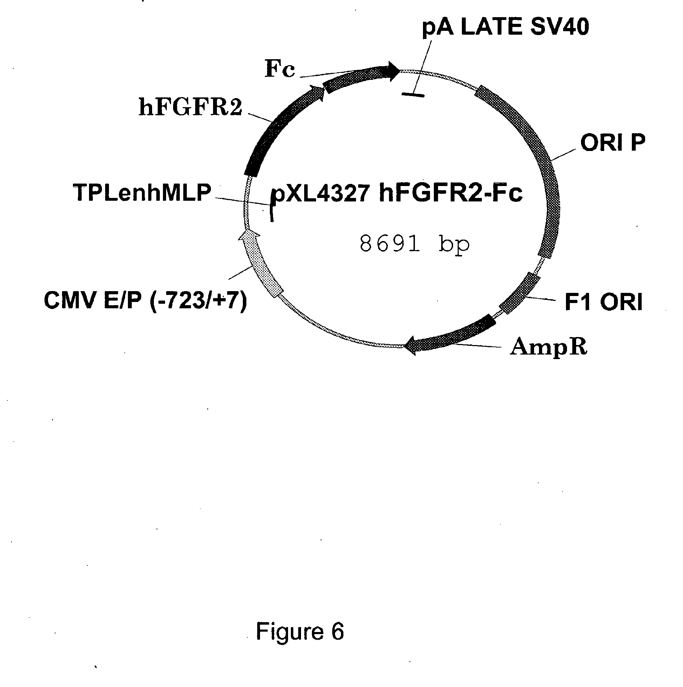

La protéine hFGFR2-Fc, de séquence SEQ ID NO 48, contient le domaine extracellulaire de FGF-R2 IIIc humain fusionné en C-terminal au domaine Fc de l'IgG1 humain. Le cDNA a été cloné dans le plasmide d'expression eucaryote pXL4327 représentée sur la figure 6 et la protéine a été produite puis purifiée dans des conditions comparables à la protéine hFGFR1-Fc.

La protéine hFGFR3-Fc est une protéine fusionnant le domaine extracellulaire de FGF-R3 IIIc humain au domaine Fc de l'IgG1 humain a été obtenue de R&D System (réf : 760-FR).

La protéine hFGFR4-Fc est une protéine fusionnant le domaine extracellulaire de FGF-R4 humain au domaine Fc de l'IgG1 humain a été obtenue de R&D Systems (réf : 685-FR).

Différents sous-domaines de la partie extracellulaire de protéine hFGFR4 ont été fusionnés au domaine Fc de l'IgG1 humain. Le sous-domaine D1 est contenu dans la construction SABVA4794 (SEQ ID NO 112 et Fig 14A et 14B). Le sous-domaine D2 est contenu dans la construction SABVA4796 (SEQ ID NO 114 t Fig 15A et 15B). Le sous-domaine D3 est contenu dans la construction SABVA4799 (SEQ ID NO 116 et Fig 16A et 16B). Ces trois sous-domaines s'étendent respectivement des positions 1 à 179 pour SABVA4794, 1 à 32 plus 145 à 242 pour le SABVA4796 et 1 à 32 plus 228 à 360 pour le SABVA4799 (positions décrites dans SwissProt FGF-R4_HUMAN). Ces protéines ont été produites à partir des plasmides pXL4794 (séquence codante SEQ ID NO 111), pXL4796 (séquence codante SEQ ID NO 113) et pXL4799 (séquence codante SEQ ID NO 115) dans des conditions comparables à la protéine FGFR1-Fc .

**Exemple 3 : Génération et criblage des anticorps monoclonaux anti-FGF-R4**

A - Anticorps obtenus par immunisation

Les anticorps monoclonaux ont été obtenus par immunisation avec l'immunogène hFGFR4-Histag chez cinq souris BALB/cJ (Charles River), âgées de 6 à 8 semaines immunisées avec chacune un total de 24 μg de hFGFR4-Histag par la méthode RIMMS décrite par Kilpatrick et al. (1997. Hybridoma 16 : 381389) et le protocole de fusion décrit dans ClonaCell™- HY Hybridoma Cloning Kit (StemCell Technologies ; réf 03800).

Deux jours après la dernière injection, les souris ont été sacrifiées et les nodules lymphatiques ont été fusionnés avec les cellules de myélome P3X63-AG8.653 (ATCC, CRL-1580) dans un rapport 5:1 en présence de Polyéthylène Glycol (ClonaCellTM-HY réf. 03806). La suspension cellulaire a été répartie aseptiquement dans des boites de Petri incubées à 37°C en présence de 5 % $CO_2$. Les colonies apparues après 12 jours d'incubation ont été isolées et mises en culture dans du milieu E (ClonaCellTM-HY; ref. 03805) dans des boites 96 puits.

Le criblage primaire d'anticorps monoclonaux obtenus par immunisation avec hFGFR4-Histag a été réalisé par dosage ELISA en utilisant comme antigène de capture hFGFR4 -Streptag. L'antigène de capture a été fixé sur des boîtes Immulon-4 enzyme-linked (VWR Scientific Inc. Swedesboro, NJ). Les surnageants de culture d'hybridomes ont ensuite été ajoutés puis détectés grâce à l'anticorps de lapin anti-souris IgG conjugué à la péroxidase (Sigma ; réf. A9044-dilution au 1:50000). La révélation a été réalisée avec le substrat TMB-H2O2 (Interchim ; réf UP664780) et les mesures avec le lecteur de plaques à 450 nm. Parmi les 444 hybridomes testés, 129 étaient positifs par dosage ELISA avec l'antigène hFGFR4-Streptag dont 120 hybridomes étaient aussi positifs avec la protéine dimère hFGFR4-Fc.

Un criblage secondaire a été réalisé pour sélectionner uniquement les anticorps spécifiques au FGF-R4 par dosage ELISA en utilisant comme antigène de capture la protéine hFGFR4-Streptag, puis avec les protéines hFGFR1-Fc, hFGFR2-Fc et hFGFR3-Fc décrites dans l'exemple 2. L'antigène de capture a été fixé sur des boîtes Immulon-4 enzyme-linked (VWR Scientific Inc. Swedesboro, NJ). Les surnageants de culture d'hybridomes ont ensuite été ajoutés puis détectés grâce à l'anticorps de lapin anti-souris IgG conjugué à la péroxidase (Sigma ; réf. A9044-dilution au 1:50000). La révélation a été réalisée avec le substrat TMB-H2O2 (Interchim ; réf UP664780) et les mesures avec le lecteur de plaques à 450 nm.

Parmi les 129 hybridomes testés positifs par dosage ELISA avec l'antigène, 84 hybridomes étaient positifs avec hFGFR4-Streptag et n'avaient pas d'affinité ni pour hFGFR1-Fc, ni pour hFGFR2-Fc ni pour hFGFR3-Fc. En fonction de leur croissance et de leur morphologie, 39 hybridomes ont été conservés. Leur isotype a été déterminé à l'aide du kit SEROTEC (réf. MMT1) ; 95 % étaient des IgG1.

Un criblage tertiaire a été réalisé sur un test de prolifération de cellules modifiées Baf/3, induite par FGF2 afin de caractériser l'inhibition par les anticorps anti-FGFR4. Les hybridomes murins exprimant les anticorps anti-FGFR4 antagonistes ont été clonés par dilution-limite. A partir des cellules d'hybridomes cultivées en phase exponentielle, la séquence codante (cDNA) a été déterminée après extraction de l'ARNm à l'aide du kit Oligotex (Qiagen ; réf 72022) ; obtention et amplification du cDNA par la méthode RACE-RT avec le kit Gene Racer, la reverse transcriptase SuperScript III (Invitrogen ; réf L1500) et les amorces (primers) décrites dans le tableau 2 ci-dessous ; amplification des fragments cDNA à l'aide de la polymérase Phusion (Finnzymes ; réf. F-530S), les amorces (primers) et les conditions de température décrites dans le tableau 2. Les fragments amplifiés contenant les parties codantes pour VH (partie variable de la chaîne lourde HC) ou VL (partie variable de la chaîne légère LC) ont été clonés dans le vecteur pGEM-T Easy de Promega ; réf A137A), les inserts des plasmides obtenus ont été séquencés, de sorte que la séquence codante de chaque domaine variable ait été analysée dans les sens 5'-3' et 3'-5' sur au moins 6 plasmides correspondant aux anticorps anti-FGFR4 40-12 et anti-FGFR4 64-12. Les analyses de séquence, contigs et alignements ont été réalisées à l'aide du logiciel disponible sur Vector NTI (Invitrogen).

Les plasmides contenant les séquences consensus codant pour les parties variables des anticorps anti-FGFR4 ont été conservés. Le plasmide pXL4691 contient la séquence codant pour la VH de séquence SEQ ID NO 5 de l'anticorps anti-FGFR4 40-12, et le plasmide pXL4693 contient la séquence codant pour la VH de séquence SEQ ID NO 19 de l'anticorps anti-FGFR4 64-12 comme présenté dans le tableau 2 ci-dessous.

Le plasmide pXL4690 contient la séquence nucléotidique SEQ ID NO 7 codant pour la VL de séquence SEQ ID NO 8 de l'anticorps anti-FGFR4 40-12, et le plasmide pXL4692 contient la séquence nucléotidique SEQ ID NO 21 codant pour la VL de séquence SEQ ID NO 22 de l'anticorps anti-FGFR4 64-12 comme présenté dans le tableau 2 ci-dessous.

Tableau 2 - Conditions opératoires et analyse des réactions de transcriptase reverse et PCR -Identification des plasmides pXL4690 à pXL4693.

| Anti-FGFR4 | 40-12 | 64-12 |
|---|---|---|
| Réaction RT Température Primers | 55°C SEQ ID NO 64 (HC) SEQ ID NO 65 (LC) | 55°C SEQ ID NO 64 (HC) SEQ ID NO 66 & SEQ ID NO 67 LC) |
| Primer 5'-GeneRacer (SEQ ID NO 63) | 5'-CGACTGGAGCACGAGG ACACTGA-3' | |
| Primer 3'- interne au hinge murin (SEQ ID NO 64) | 5'-TATGCAAGGCTTACAAC CACA-3' | |
| Primer 3'- interne au Cκ murin (SEQ ID NO 65) | 5'-CTCATTCCTGTT GAAGCTCTT GAC-3' | |

(suite)

| Anti-FGFR4 | 40-12 | 64-12 |
|---|---|---|
| Primers 3'- internes à Cλ murin (SEQ ID NO 66 et SEQ ID NO 67) | | 5'- ACACTCAGCACGGG ACAAACTCTTCTC-3' 5'- ACACTCTGCAGGAG ACAGACTCTTTTC-3' |
| PCR de HC: Température Primers | 55°C SEQ ID NO 63 - SEQ ID NO 64 | 55°C SEQ ID NO 63 - SEQ ID NO 64 |
| Séquence des produits PCR clonés (VH) | 9 clones ont la même séquence VH et une séquence CH1 identique à celle du mCH1- | 6 clones ont la même séquence VH et une séquence CH1 identique à celle du mCH1 |
| Plasmide contenant VH | pXL4691 | pXL4693 |
| PCR de LC : Température Primers | 55°C SEQ ID NO 63 - SEQ ID NO 65 | 55°C SEQ ID NO 63 - SEQ ID NO 66 & 67 |
| Séquence des produits PCR clonés (VL) | 11 clones ont la même séquence VL et une séquence Cκ identique à mCκ | 6 clones ont la même séquence VL et une séquence Cλ identique à mCÀ |
| Plasmide contenant VL | pXL4690 | pXL4692 |

Les séquences en acides aminés des parties variables légères et lourdes respectivement des anticorps anti-FGFR4 64-12 et anti-FGFR4 40-12 sont différentes. Les numéros des séquences utilisées, obtenues et déduites sont indiqués dans le tableau 7.

Les anticorps 40-12 et 64-12 ont été produits en fiasques T500. Le surnageant de culture a été récolté après 7 jours. Les anticorps anti-FGFR4 ont été purifiés par affinité sur protéine G puis dialysés contre du PBS filtrés stériles et conservés à 4°C.

Les anticorps antagonistes purifiés ont un $K_D$ de 6.5 x $10^{-9}$ M (anti-FGFR4 40-12) et 5.75 $10^{-8}$ M (anti-FGFR4 64-12).

B - Anticorps sélectionné à l'aide de la méthode phage display

Le criblage primaire d'anticorps monoclonaux obtenus par phage display avec hFGFR4-Histag a été réalisé par dosage ELISA en utilisant comme antigène de capture hFGFR4-Histag. L'antigène de capture a été fixé sur des boîtes Immulon-2 enzyme-linked (VWR Scientific Inc. Swedesboro, NJ). Les surnageants de culture d'E. coli infectées par les phages ont ensuite été ajoutés puis détectés grâce à l'anticorps de souris antiM13 conjugué à la péroxidase (GE Healthcare ; réf. 27-9421-01, dilution au 1:5000). La révélation a été réalisée avec le substrat TMB-H2O2 (Interchim ; réf UP664780) et les mesures de densité optique (O.D.) effectuées à 450 nm.

Un criblage secondaire a été réalisé pour sélectionner uniquement les anticorps spécifiques au FGF-R4 par dosage ELISA en utilisant comme antigène de capture la protéine hFGFR4-Histag, puis avec les protéines hFGFR1-Fc, hFGFR2-Fc et hFGFR3-Fc décrites dans l'exemple 2. L'antigène de capture a été fixé sur des boîtes Immulon-2 enzyme-linked (VWR Scientific Inc. Swedesboro, NJ). Les surnageants de culture d'E. coli infectées par les phages ont ensuite été ajoutés puis détectés grâce à l'anticorps de souris antiM13 conjugué à la péroxidase (GE Healthcare ; réf. 27-9421-01, dilution au 1:5000). La révélation a été réalisée avec le substrat TMB-H2O2 (Interchim ; réf UP664780) et les mesures de densité optique (O.D.) effectuées à 450 nm.

Les clones spécifiques au FGF-R4 sélectionnés ont été séquencés et reclonés dans un vecteur d'expression pour transfection transitoire de cellules HEK293.

Pour cela, dans une première étape, les régions codant le Fab, c'est à dire la chaîne légère de l'anticorps, un site de liaison de ribosome bactérien, et la région variable de la chaîne lourde de l'anticorps, sont extraites du phagemide par restriction et insérées dans un plasmide d'expression d'IgG pour cellules de mammifères, en aval d'une séquence signal d'anticorps eucaryote et en amont de la région constante d'une chaîne lourde d'IgG1 humaine. Dans une seconde étape, la région contenant le site de liaison du ribosome bactérien et le peptide signal bactérien de la chaîne lourde est échangée contre une séquence IRES et une séquence signal eucaryote. Les IgG sont exprimées par transfection transitoire de cellules HEK293. Ce processus est décrit en détail dans T. Jostock et al., Journal of Immunological Methods 289 (2004) 65-80 Un exemple de séquence de région constante d'IgG1 humaine qui peut être utilisée dans la présente invention est la séquence SEQ ID NO 117.

Un criblage tertiaire a été réalisé sur un test de prolifération de cellules modifiées Baf/3, induite par FGF2 et décrite dans l'exemple 4 afin de caractériser l'inhibition par les anticorps anti-FGFR4 en utilisant les surnageants de cultures de cellules HEK293 transfectées transitoirement par les vecteurs d'expression des anticorps. Ce criblage a permis d'identifier les anticorps des clones 8, 31, 33 et 36. Les séquences correspondantes sont décrites dans le tableau 7.

**Exemple 4: Etablissement de la lignée clonale murine BaF/3 FGF-R4-hMpl et protocole de prolifération cellulaire**

La partie extracellulaire et transmembranaire du FGF-R4 a été clonée en fusion traductionnelle avec le domaine intracellulaire du hMpl dans un vecteur pEF6/V5-His A muté afin d'obtenir la présence en 5' du tag HA devant le récepteur chimère FGF-R4-hMpl.

Construction du pEF6A muté

Le vecteur pEF6/V5-His A (Invitrogen, référence V961-20) a été amélioré afin d'intégrer le MCS (multi Clonage Sites) associé au tag HA placé sous le peptide signal de l'IgGk du pDisplay (Invitrogen, référence V660-20). Pour se faire, le MCS associé au tag HA et à son peptide signal ont été amplifiés par PCR entre les amorces sens de séquence SEQ ID NO : 51 et reverse de séquence SEQ ID NO : 52 permettant d'insérer les sites de restriction Kpnl en 5' et Xbal en 3'. Le fragment PCR a été digéré par les enzymes Kpnl et Xbal puis cloné dans le vecteur pEF6/V5-His A ouverts par les mêmes enzymes. Enfin, le premier site BamHI du MCS a été remplacé par le site BsrGI en digérant vecteur nouvellement formé par les enzymes Kpnl et Spel et en insérant entre ces sites les amorces de séquences SEQ ID NO : 53 et SEQ ID NO : 54 hybridées entre elles et contenant le site enzymatique BsrGI. Le vecteur obtenu a été nommé : pEF6mut-HA.

Construction du vecteur pEF6mut-HA-FGF-R4-hMpl

Le domaine intracellulaire du Mpl a été amplifié dans un vecteur pEF6/V5-His TOPO (Invitrogen, référence K9610-20) entre les amorces sens de séquence SEQ ID NO : 55 permettant l'insertion du site de digestion Sacl et reverse de séquence SEQ ID NO : 56 appelée communément revBGH. Les fragments PCR générés ont ensuite été digérés par les enzymes Sacl et Notl.

Le domaine extracellulaire et transmembranaire du FGF-R4 a été amplifié à l'aide du couple d'amorces (sens : séquence SEQ ID NO : 57 ; reverse : séquence SEQ ID NO : 58). Ces amorces permettent l'insertion des sites enzymatiques BamHI en 5' et Sacl en 3'. Le fragment PCR obtenu a alors été digéré par les enzymes BamHI et Sacl.

Les amplifications d'ADN codant pour le hMpl et pour le FGF-R4 ont ensuite été clonées simultanément dans le vecteur pEF6mut-HA ouvert BamHI - Notl. La construction qui en résulte a été nommée « pEF6mut-HA FGF-R4αIIIc-hMpl2 ». Cette construction a ensuite été améliorée par mutagenèse dirigée afin de changer le domaine transmembranaire du FGF-R4 (séquence protéique SEQ ID NO : 59) pour la séquence SEQ ID NO: 60. Pour se faire, le kit "Quick-Change® Site-directed mutagenesis kit" (Clontech, référence 200518) a été utilisé avec les amorces sens de séquence SEQ ID NO : 61 et reverse de séquence SEQ ID NO : 62. La nouvelle construction chimère obtenue a été appelée pEF6mut-HA FGF-R4αIIIcmut-hMpl2.

Création d'une lignée stable par transfection de la lignée murine BaF/3 avec la construction FGF- R4αIIIcmut-hMpl2:

La construction « pEF6mut-HA FGF-R4αIIIcmut-hMpl2 » a été introduite de façon stable par électroporation dans le génome des cellules murines BaF/3. La lignée obtenue a été sélectionnée en présence de FGF2 à 20 ng/ml (R&D, référence 234-FSE-025) et d'héparine à 100 ng/ml (Sigma, référence H3149). La lignée transfectée et sélectionnée est alors de type clonal.

Protocole de prolifération cellulaire avec la lignée cellulaire BaF/3 FGFR4-hMpl :

Les cellules BaF/3 FGFR4-hMpl ont été cultivées et entretenues dans du milieu RPMI 1640 (Invitrogen ; réf. 32404-014) complet (10 % SVF (Hyclone; réf. SH30070.03), Glutamine à 2mM, MEM Non Essential Amino Acid 1x (Gibco, réf 11140-035), MEM Sodium Pyruvate 1 x (Gibco, réf 11360-039)), supplémenté en FGF2 à 20ng/ml (R&D System, ref 234-FSE) et héparine à 3ng/ml (Sigma, ref H3149).. La veille, les cellules sont ensemencées à 0.4 10$^6$ cellules/ml dans du milieu RPMI 1640 complet supplémenté en FGF2 à 20ng/ml et en héparine à 3ng/ml. Le lendemain, 50µl de suspension cellulaire BaF/3 FGFR4-hMpl dans du milieu RPMI 1640 complet supplémenté en FGF2 à 20ng/ml et en héparine à 3ng/ml à 0.2 $10^6$ cellules/ml ont été dispensés en plaque 96 puits (Porvair, réf 214006) suivi de 50µl de surnageant d'hybridome contenant l'anticorps à tester. Les plaques ont ensuite été placées à 37°C, 5 % CO$_2$ pendant 24 à 30 h. Pour la lecture de la prolifération cellulaire, la quantité d'ATP a été quantifiée par l'ajout de 100 µl de Cell Titer Glo Luminesent Cell Viability Assay (Promega, ref G7571) et la luminescence a été lue à l'aide d'un luminomètre.

Les clones présentant dans ce test un signal 50 % plus faible que celui du milieu RPMI 1640 complet contenant les additifs FGF2 à 20 ng/ml et héparine à 3 ng/ml ont été retenus.

Lorsque les anticorps anti-FGFR4, spécifiques de FGF-R4 ont été incubés avec les cellules Baf/3-FGFR4-hMpl et avec les additifs suivants FGF2 à 20 ng/ml et l'héparine à 3 ng/ml, un effet antagoniste sur la prolifération cellulaire a été observé. Parmi les 39 hybridomes testés, 14 sont capable d'inhiber la prolifération cellulaire, induite par le FGF, des cellules Baf/3-FGFR4-hMpl en présence de FGF2.

Il a été montré par ELISA (Fig 12) que les anticorps anti-FGFR4 antagonistes 40-12 et 64-12 étaient affins pour les protéines murines mFGFR4 et humaines hFGFR4 (D2, D3).

Enfin un dernier criblage a été réalisé par Surface Plasmon Resonance (BIAcore 2000) pour déterminer les constantes d'affinité des anticorps anti-FGFR4. L'interaction entre la protéine FGF-R4 et l'anticorps anti-FGFR4 présent dans le surnageant de culture de l'hybridome a été analysée après avoir fixé l'anticorps anti-FGFR4 à un anticorps anti-Fc lui-même fixé sur la chips CM. Les mesures de cinétiques sont réalisées selon le protocole de Canziani et al 2004. Anal. Biochem. 325 : 301-307.

Deux anticorps parmi ceux ayant des constantes d'affinité de $10^{-8}$ à $10^{-9}$ M et des vitesses de dissociation de $10^{-3}$ à $10^{-5}$ s$^{-1}$ ont été sélectionnés. Les caractéristiques de ces anticorps sont décrites dans le tableau 1 ci-dessous.

La méthode de référence utilisée pour la détermination du $K_D$ est le Surface Plasmon Resonance (BIAcore).

Tableau 1A : Constantes d'affinité et d'association/dissociation des anticorps anti-FGFR4 antagonistes purifiés 40-12 et 64-12

| Anti-FGFR4 | $k_{on}$ (M$^{-1}$.s$^{-1}$) | $k_{off}$ (s$^{-1}$) | $K_D$ (M) |
|---|---|---|---|
| 40-12 | 1.94E+05 | 1.26E-03 | 6.50E-09 |
| 64-12 | 1.05E+04 | 6.02E-04 | 5.75E-08 |

Tableau 1B : Paramètres cinétiques de liaison des anticorps anti-FGFR4 8, 31, 33 et 36 mesurés par Surface Plasmon Resonance (BIAcore 3000) :

| | sur h-FGFR4-Histag | | | sur m-FGFR4-Histag | | |
|---|---|---|---|---|---|---|
| | Kon (M$^{-1}$s$^{-1}$) | Koff (s$^{-1}$) | **KD (M)** | Kon (M$^{-1}$s$^{-1}$) | Koff (s$^{-1}$) | **KD (M)** |
| **Clone 8** | 8,93E+05 | **2,92E-04** | **3,27E-10** | 1,20E+06 | **1,36E-04** | **1,15E-10** |
| **Clone 31** | 6,48E+05 | **9,80E-04** | **1,52E-09** | 9,14E+05 | **1,80E-03** | **1,97E-09** |
| **Clone 33** | 8,05E+05 | **6,17E-04** | **7,71E-10** | 1,01E+06 | **7,04E-04** | **6,92E-10** |
| **Clone 36** | 2,44E+05 | **6,31E-04** | **2,62E-09** | 3,30E+05 | **7,52E-04** | **2,28E-09** |

**Exemple 5 : Spécificité des anticorps anti-FGFR4**

A- Spécificité de l'anticorps anti-FGFR4 40-12 pour le FGF-R4

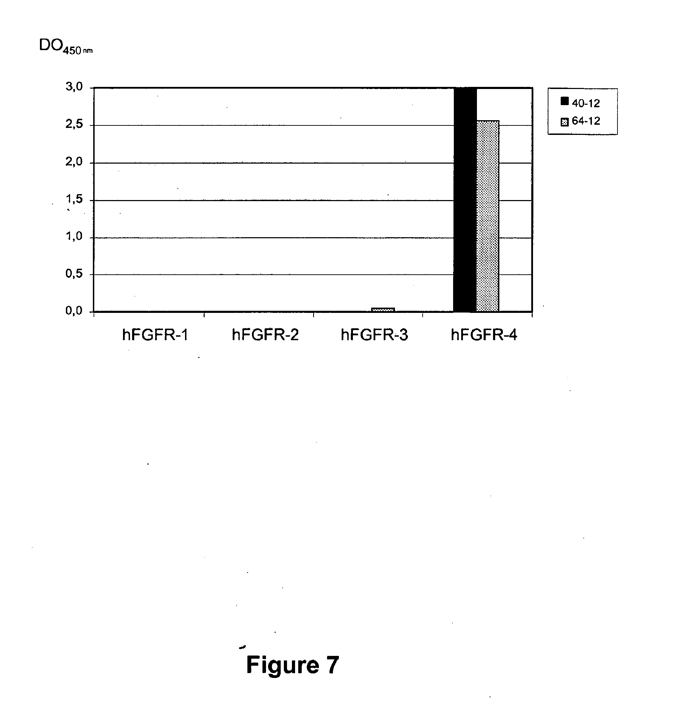

La spécificité de chaque anticorps est établie par ELISA selon le protocole décrit dans l'exemple 4. De cette façon, on observe la capacité de chaque anticorps à se lier à chaque FGF-R. Cette expérience montre clairement que l'anticorps 40-12 ne reconnaît que FGF-R4 et est donc spécifique de FGF-R4. L'anticorps 64-12 est capable de se lier principalement à FGF-R4 mais également faiblement à FGF-R3 (Fig. 7).

B - Spécificité des anticorps anti-FGFR4 8, 31, 33 et 36 pour le FGF-R4

La spécificité de chaque anticorps est établie par ELISA selon le protocole suivant : des suspensions de phages présentant à leur surface les anticorps au format Fab sont générés par infection de bactéries *E. coli*. Les antigènes de capture ont été fixés sur des boîtes Immulon-2 enzyme-linked (VWR Scientific Inc. Swedesboro, NJ). Les suspensions de phages ont ensuite été ajoutés puis détectés grâce à l'anticorps de souris anti-phage M13 conjugué à la péroxidase (GE Healthcare, réf. 27-9421-01, dilution au 1:5000). La révélation a été réalisée avec le substrat TMB-H2O2 (Interchim ; réf UP664780) et les mesures de densité optique (O.D.) effectuées à 450 nm. Le tableau 2 résume les résultats obtenus:

De cette façon, on observe la capacité de chaque anticorps à se lier à chaque FGF-R. Cette expérience montre clairement que les anticorps 8, 31, 33, 36 ne reconnaissent que FGF-R4 et sont donc spécifiques de FGF-R4.

Tableau 2 : Spécificité des anticorps anti-FGF-R4 issus des clones 8, 31, 33 et 36 établie par ELISA (Signal = O.D., 450 nm)

|  | h-FGFR4(D2,D3)-histag | h-FGFR1-Fc | h-FGFR2-Fc | h-FGFR3-Fc |
|---|---|---|---|---|
| Clone 8 | 2,55 | 0,05 | 0,00 | 0,00 |
| Clone 31 | 2,52 | 0,00 | 0,00 | 0,01 |
| Clone 33 | 2,73 | 0,00 | 0,00 | -0,01 |
| Clone 36 | 1,87 | -0,01 | -0,01 | -0,02 |

**Exemple 6 : Effet antagoniste de l'anticorps 40-12 et des anticorps issus des clones 8, 31, 33, 36 sur l'angiogenèse (*in vitro*)**

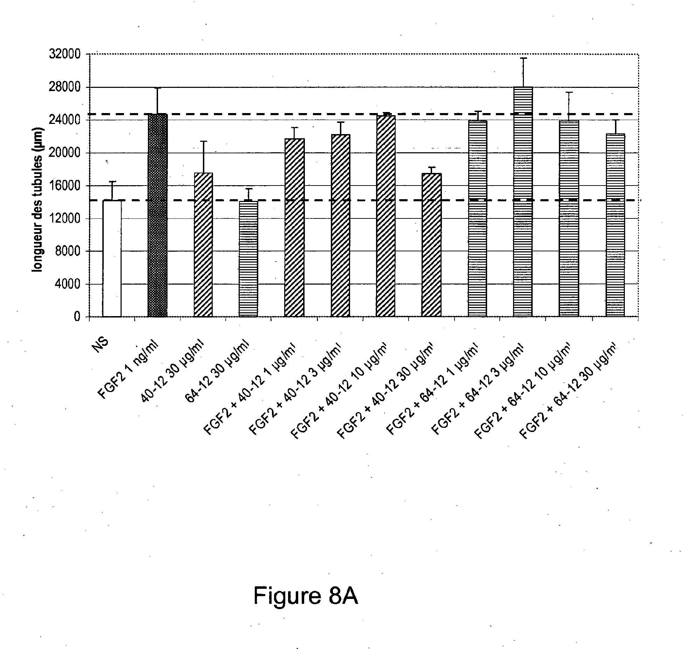

Afin de déterminer l'activité biologique de l'anticorps monoclonal 40-12 anti-FGFR4 au cours de l'angiogenèse de cellules endothéliales humaines, des expériences d'angiogenèse *in vitro* ont été réalisées à l'aide de cellules HUVEC stimulées par du FGF2 en présence de l'anticorps 40-12 ou de l'anticorps témoins en doses croissantes de 1 à 30 μg/ml. (Fig 8A et 8B)

Dans ce cadre, l'anticorps monoclonal anti-FGFR4 antagoniste actif 40-12 est capable d'inhiber l'angiogenèse de cellules HUVEC induite par le FGF2, et ce, à la dose de 30 μg/ml ou 200 nM alors que l'anticorps témoin 64-12 n'a aucun effet. De plus, l'anticorps 40-12 n'a aucun effet propre sur l'angiogenèse basale.

Ces résultats indiquent qu'un anticorps antagoniste spécifique de FGF-R4 est capable d'inhiber l'angiogenèse.

Comme pour l'anticorps 40-12, les anticorps anti-FGFR4 des clones 8, 31, 33 et 36 issus du phage display ont été évalués quant à leur capacité d'inhiber l'angiogenèse de cellules endothéliales humaines de type HUVEC induite par le FGF-2. Ces 4 anticorps bloquent la stimulation de l'angiogenèse *in vitro* obtenue avec le FGF-2, et ce à la dose de 10 μg/ml (Fig. 8C et 8D).

**Exemple 7 : Effet antagoniste de l'anticorps 40-12 et des anticorps issus des clones 8, 31, 33, 36 sur des cellules d'hépatocarcinomes humains (*in vitro*)**

Afin de déterminer l'effet anti-tumoral de l'anticorps monoclonal anti-FGFR4 antagoniste 40-12, des expériences ont été réalisées sur des cellules d'hépatocarcinome humain Hep3b dont la prolifération et les voies de signalisation y aboutissant sont dépendantes du couple ligand-récepteur : FGF19/FGF-R4.

Premièrement, l'étude des voies de signalisation dépendantes de FGF-R4 et aboutissant à la prolifération des cellules Hep3b a été entreprise par Western-blot. Cette signalisation cellulaire passe par la néo-synthèse des protéines cFos et JunB ainsi que par la phosphorylation de Erk1/2 (Lin et al., J Biol Chem., 2007, 14:27277-84.). Pour ce faire, $5.10^5$ cellules sont ensemencées en boîte 35 mm de diamètre dans 2 ml de milieu complet (DMEM, 10 % SVF, 2 mM glutamine). 24 h après, les cellules sont mises en carence 24 h dans 1.8 ml de milieu dépourvu de sérum. Les cellules sont ensuite stimulées pendant 3 h par 200 μl de FGF19 concentré 10 fois en absence ou en présence d'anticorps anti-FGFR4 contrôle ou 40-12. Le milieu est ensuite retiré, les cellules lavées une fois par du PBS froid et lysées sur boîte pendant 30 min à 4°C par 75 μl de tampon RIPA supplémenté en inhibiter de protéase. L'extrait protéique total est ensuite centrifugé 10 min à 4 °C à 13000 rpm et le surnageant analysé par la technique de Western blot. Les membranes sont ensuite incubées 2 h à température ambiante dans du TBS, tween 0.05 %, 5 % lait puis l'anticorps primaire anti-cFos (Cell Signaling Technology, réf 2250), anti-JunB (Cell Signaling Technology, réf 3746) et anti-phospsoErk1/2 (Cell Signaling Technology, réf 4377) sont ajoutés au 1/1000ème et incubés une nuit à 4°C sous agitation lente. La membrane est rincée trois fois par du TBS, tween 0.05 % et l'anticorps secondaire couplé à la HRP est incubé 4 h à 4°C dilué au 1/2000ème dans du TBS, tween 0.05 %, 5 % lait. Les résultats de Western-blot sont alors quantifiés à l'aide d'un Chemigenius (Syngene). L'intensité des bandes obtenues avec les différents anticorps sont pondérées par l'intensité des bandes obtenues avec l'anticorps anti-actine directement couplé à la HRP et utilisé au 1/3000ème (Santa Cruz Biotechnology, réf Sc-8432-HRP).

Le FGF19 à 30 ng/ml induit la synthèse des protéines JunB et cFos ainsi que la phosphorylation de Erk1/2 dans les cellules Hep3b. Cette néo-synthèse de protéine et la phosphorylation de Erk sont inhibées complètement par l'anticorps anti-FGFR4 40-12 à 100 μg/ml alors que l'anticorps contrôle n'a aucun effet inhibiteur. Ces effets observés sur les membranes de Western-blot (Fig. 9A) sont quantifiés et les intensités des bandes de chaque membrane sont représentées sous forme de graphe (Fig. 9B).

Deuxièmement, des expériences de prolifération cellulaire proprement dites ont été menées. 5000 cellules sont ensemencées en plaque 96 puits dans 100 μl de milieu DMEM, 10 % SVF, 2 mM glutamine. 24 h après, les cellules sont déprivées dans un milieu de culture sans sérum pendant 24 h. Les cellules Hep3b sont ensuite stimulées 72 h par 100 μl de milieu sans sérum supplémenté par 10 ng/ml de FGF19 (production interne à Sanofi-Aventis R&D) ou par 10 % sérum en absence ou en présence d'anticorps témoins ou d'anticorps monoclonal anti-FGFR4 antagoniste 40-12. Après 3 jours, la prolifération cellulaire est quantifiée à l'aide du kit CellTiter Glo (Promega, France).

De ces expériences, il en ressort que le sérum ainsi que le FGF19 sont capables de stimuler la prolifération des Hep3b. L'anticorps anti-FGFR4 antagoniste 40-12 inhibe partiellement à 100 μg/ml cette prolifération induite au sérum alors que l'anticorps témoin ne présente pas d'activité inhibitrice (Fig. 10A). De plus, l'anticorps 40-12 à 10 μg/ml bloque complètement la prolifération induite par le FGF19 (Fig. 10B). L'anticorps témoin n'a pas d'effet.

Ceci démontre que l'anti-FGFR4 antagoniste objet de l'invention peut être utilisé comme agent thérapeutique anti-tumoral dans la cadre de tumeurs dépendantes du FGF19 ou du FGF-R4 et que cet anticorps serait particulièrement efficace dans le traitement des hépatocarcinomes.

Afin de simplifier l'étude de l'effet des anticorps anti-FGFR4 sur les cellules Hep3b, un test ELISA a été mis au point pour la détection de la phosphorylation de Erk1/2 suite à la stimulation de ces cellules par du FGF-19 (en corrélation avec les expériences décrites dans l'exemple 8), du FGF-2 ou du sérum de veau foetal.

Pour ce faire, 50 000 cellules Hep3b sont ensemencées en plaque 96 puits noires fond transparent (COSTAR, ref 3603) dans 100μl de milieu DMEM, 10% SVF, 2mM glutamine. Après 24h, les cellules sont mises en carence pendant 24h dans du milieu DMEM, 2mM glutamine sans SVF. Le milieu est ensuite aspiré et remplacé par 100μl de milieu de carence pré-équilibré à 37°C contenant le FGF ou le SVF, ainsi que les anticorps évalués aux différentes doses. Les cellules sont incubées 3h à 37°C, 5% $CO_2$. Le milieu de stimulation est alors aspirer, les puits sont rincés par du PBS à 4°C et les cellules sont fixées par ajout de 200μl de PFA (paraformaldéhyde) 4% dans du PBS pendant 15min à température ambiante. Le PFA est retiré et les cellules lavées trois fois par 200μl de PBS. Le marquage par des anticorps pour la détection de phospho-Erk1/2 directement sur les cellules Hep3b commence par la saturation des sites non spécifiques avec 100μl de tampon de saturation (21.25ml de PBS, 1.25ml de sérum non immun de chèvre à 10% (Zymed, ref 50-062Z), 75μl de triton X100) pendant 2h. Le tampon de saturation est remplacé par 50μl d'anticorps primaire anti-phospho Erk1/2 (Cell Signaling Technology, ref 4377) dilué au 1/100ème dans du tampon PBS, 1% BSA, 0.3% triton X100. L'anticorps primaire est incubé avec les cellules une nuit à 4°C. Il est ensuite rincé par 3 lavages de 200μl de PBS et révélé à l'aide d'un anticorps secondaire anti-rabbit couplé à l'AlexaFluor 488 (Molecular Probes, ref A11008) dilué au 1/5000ème dans du tampon PBS, 1% BSA, 0.3% triton X100 pendant 4h. L'anticorps secondaire est ensuite rincé par 3 lavages de 200μl de PBS et 100μl de PBS est alors ajouté à chaque puits. La fluorescence est lue pardessus à l'aide d'un lecteur de plaque de type EnVision 2103 Multilabel Reader (Perkin Elmer) en utilisant le filtre FITC.

Cette technique permet de confirmer que le FGF-19 à 30ng/ml induit la phospshorylation de Erk1/2 dans les cellules Hep3b et que le 40-12 est capable de bloquer cette stimulation dès la dose de 3 μg/ml (Fig. 9C). Le FGF-2 ainsi que le sérum sont également capable d'induire le système. Dans ces 2 derniers, l'anticorps 40-12 inhibe l'effet du FGF-2 et du sérum à des doses plus élevées (30-100μg/ml ; Fig. 9C).

La détection de phospho-Erk1/2 par ELISA à aussi permis de montrer que des anticorps anti-FGFR4 actifs dans le modèle de l'angiogenèse *in vitro* sont également capables de bloquer entre 70 et 95% de la phosphorylation de Erk1/2 induite par le FGF-19 à la dose de 3μg/ml (Fig. 9D).

De manière avantageuse, les anticorps de la présente invention ont un effet antagoniste à la fois sur l'angiogenèse pathologique associée au développement de la tumeur et sur la croissance tumorale hépatique proprement dit, en particulier sur un modèle d'hépatocarcinome.

**Exemple 8 : Effet antagoniste de l'anticorps 40-12 dans le modèle murin de cancer pancréatique**

Pour ce modèle pharmacologique, des souris femelles Rip1-Tag2 (Charles River Laboratory, France) de fond génétique C57Bl/6J sont utilisées. Les animaux ont à partir de la semaine 9 après naissance de l'eau de boisson supplémentée par 5% de saccharose. Les souris sont traitées de la semaine 10 à la semaine 12.5 dans un protocole de traitement en intervention, une fois par semaine par une injection par voie sous-cutanée des anticorps anti-FGFR4 40-12 ou contrôle à la dose de 25 mg/kg (Fig. 14A). Ce protocole est approuvé par le « Comité expérimentation Animale (Animal Care and Use Committee) » de Sanofi-Aventis Recherche. Nos installations zootechniques , l'attention prêtée aux animaux ainsi que les protocoles de traitement sont en accord avec les principes imposés par la convention européenne sur la protection des animaux Vertébrés et euthanasiés après la période de traitement ou lorsque la charge tumorale et/ou les effets secondaires oblige leur retrait de l'étude. Pour la mesure de la charge tumorale, les animaux sont euthanasiés à la fin de l'expérience et les tumeurs sont micro-disséquées à partir de pancréas fraichement excisés. Le volume tumoral en mm$^3$ est mesuré à l'aide d'un pied à coulisse en appliquant la formule [volume = 0.52 x (largeur)$^2$ x (longueur)] afin de se rapprocher du volume d'un sphéroïde. La charge tumorale par souris est calculée par le cumul du volume des tumeurs de chaque souris.

Pour l'analyse histochimique de la densité vasculaire, les animaux sont anesthésiés, les pancréas collectés, fixés une nuit dans de l'accustain® (Sigma) puis enchâssés dans de la paraffine. Des coupes de 5 $\mu$m d'épaisseur sont préparées pour chaque échantillon. Les cellules endothéliales sont détectées par l'incubation des coupes avec de la trypsine (Zymed, ref 00-3003) à 37°C pendant 10 min, puis avec un anticorps anti-CD31 de souris fait chez le rat et dilué au 1/50$^{ème}$ (BD Pharmingen). Pour révéler les régions marquées par l'anticorps, les coupes sont incubées avec un anticorps anti-rat couplé à la biotine pendant 30 min, puis avec de la streptavidine couplé à la HRP pendant également 30 min (Vectastain® ABC kit, Vector), et enfin avec du DAB pendant 5 min (Vector, ref SK4100). Les coupes sont alors marquées avec de l'hématoxylin diluée au 1/10$^{ème}$ (Dako, S-3309). Les prises de vue sont réalisées à l'ide d'une caméra montée sur un microscope (Nikon, E-800) à un grossissement total de 200 fois. Les images sont analysées par un logiciel (Visiolab, Biocom). Les vaisseaux sanguins dans la tumeur sont comptés et classés en fonction de leur surface : les petits vaisseaux entre 5 et 20 $\mu$m$^2$, les moyens entre 21 et 100 $\mu$m$^2$ et les gros à partir de 101 $\mu$m$^2$. Deux porte-objets par pancréas sont analysés afin de déterminer la densité vasculaire correspondant au nombre total d'éléments marqués par champ.

Dans ce modèle, le traitement sous-cutané à l'aide de l'anticorps anti-FGFR4 40-12 à 25 mg/kg une fois par semaine entre les dixième et douzième semaines, permet de réduire la charge tumorale de 55% et de façon significative (Fig11B) et à tendance à réduire de 34% le nombre de tumeurs par pancréas (Fig. 11 C) alors que le traitement contrôle n'a aucun effet. Cette inhibition du développement tumoral grâce à l'anticorps anti-FGFR4 40-12 est accompagnée par une réduction significative de 31% de la densité vasculaire totale (Fig. 11D) correspondant à une réduction observée du nombre de vaisseaux sanguins dans l'ensemble des groupes de taille de vaisseaux marqués à l'aide d'un anticorps anti-CD31 (Fig. 11 D).

Ces résultats *in vivo* montrent clairement qu'un anticorps anti-FGFR4 antagoniste est capable d'inhiber le recrutement et la formation des vaisseaux sanguins dans la tumeur. Cette inhibition de la vascularisation tumorale s'accompagne d'une réduction du nombre de tumeurs par pancréas et du volume tumoral total.

De manière avantageuse, les anticorps de la présente invention ont un effet antagoniste à la fois sur l'angiogenèse pathologique et sur la croissance tumorale hépatique (sur un modèle d'hépatocarcinome) et pancréatique.

**Exemple 9 : Réactivité croisée de l'anticorps envers les FGF-R4 humain, murin et de rat.**

Dans un premier temps, des expériences de compétition de liaison ont été réalisées. Pour ce faire, du FGF2 a été marqué au niveau des 2 cystéines libres à l'aide d'un AlexaFluor® 488 nm C-5 maléimide (Molecular Probes, A10254) selon les recommandations du fournisseur. Ce FGF2-AF488 à 10 ng/ml est capable de se lier sur les FGF-R4 humains exprimés à la surface de cellules 300-19 transfectées (Fig. 8). Cette liaison est spécifique puisque l'ajout en excès de FGF2 non marqué permet de déplacer l'interaction FGF2/FGF-R4 (Fig. 8A). La même expérience a été réalisée en rajoutant des doses croissantes de l'anticorps 40-12 ou de l'anticorps témoins 64-12. Seul l'anticorps antagoniste 40-12 est apte à déplacer la liaison FGF2-AF488/FGF-R4, et ce, avec une IC$_{50}$ de 3500 ng/ml soit 23 nM montrant que l'effet antagoniste de cet anticorps serait dû à sa capacité de déplacer la liaison FGF/FGF-R4.

Dans un second temps et dans le but de déterminer l'aptitude du clone 40-12 à bloquer la liaison FGF/FGF-R4 chez d'autres espèces que l'Homme, une expérience de dissociation a été réalisée en utilisant les couples FGF2/FGF-R4 de souris, de rat et humain en référence. Pour ce faire, du FGF2 murin (R&D, réf 3139-FB-025) ou de rat (R&D, réf 3339-FB-025) a été marqué de la même façon que le FGF2 humain par un AlexaFluor 488. Les expériences de dissociation ont été réalisées en utilisant les lignées 300-19 transfectées avec les récepteurs FGF-R4 de souris ou de rat. Les résultats montrent que l'anticorps anti-FGFR4 antagoniste 40-12 est capable de dissocier la liaison FGF2/FGF-R4 dans un système souris ou rat avec la même efficacité que dans le système humain. En effet, les IC$_{50}$ sont de 3500, 4110 et 3940 ng/ml soit 23, 27 et 26 nM pour les complexes FGF2/FGF-R4 humain, murin ou de rat respectivement (Fig. 13A, 13B et 13C respectivement). Cette capacité à se lier au FGF-R4 de rongeurs a été vérifié par ELISA. L'anticorps 40-12 se lie aussi bien au FGF-R4 humain et murin (Fig. 12).

Ces résultats indiquent que l'anticorps anti-FGFR4 40-12 pourra être utilisé dans des modèles pharmacologiques sur rongeurs (au moins souris et rat) et que les résultats obtenus devraient être prédictifs de l'efficacité chez l'Homme.

De même, des études menées sur les anticorps issus des clones 8, 31, 33 et 36 montrent que ces anticorps reconnaissent à la fois le FGF-R4 humain et le FGF-R4 murin (Tableau 4 ci-dessous)

Tableau 4 : Paramètres cinétiques de liaison des anticorps anti-FGFR4 8, 31, 33 et 36 mesurés par Surface Plasmon Resonance (BIAcore 3000) :

| | sur h-FGFR4-Histag | | | sur m-FGFR4-Histag | | |
|---|---|---|---|---|---|---|
| | Kon (M$^{-1}$s$^{-1}$) | Koff (s$^{-1}$) | **KD (M)** | Kon (M$^{-1}$s$^{-1}$) | Koff (s$^{-1}$) | **KD (M)** |
| **Clone 8** | 8,93E+05 | 2,92E-04 | **3,27E-10** | 1,20E+06 | 1,36E-04 | **1,15E-10** |
| **Clone 31** | 6,48E+05 | 9,80E-04 | **1,52E-09** | 9,14E+05 | 1,80E-03 | **1,97E-09** |
| **Clone 33** | 8,05E+05 | 6,17E-04 | **7,71E-10** | 1,01E+06 | 7,04E-04 | **6,92E-10** |
| **Clone 36** | 2,44E+05 | 6,31E-04 | **2,62E-09** | 3,30E+05 | 7,52E-04 | **2,28E-09** |

**Example 10 : Détermination de l'épitope reconnu par les anticorps anti-FGF-R4**

A- Détermination de l'épitope reconnu par l'anticorps anti-FGFR4 40-12

Un criblage a été réalisé pour déterminer le domaine spécifique du FGFR4 reconnu par l'anticorps 40-12 par dosage ELISA. Grâce à l'utilisation d'une forme délétée du domaine D1 de FGF-R4 en ELISA, il a été établi que l'anticorps 40-12 interagissait au niveau des domaines D2-D3 de FGF-R4 (Fig. 12).

Un second criblage a été réalisé par dosage ELISA, en utilisant comme antigène de capture les constructs contenant soit le domaine D1 (SABVA4794) ou le domaine D2 (SABVA4796) ou le domaine D3 (SABVA4799) de protéine hFGFR4 comme décrits dans l'exemple 2. L'antigène de capture a été fixé sur des boîtes Immulon-4 enzyme-linked (VWR Scientific Inc. Swedesboro, NJ). L'hybridome 40-12 a ensuite été ajouté puis détecté grâce à l'anticorps de lapin anti-souris IgG conjugué à la péroxidase (Sigma ; réf. A9044-dilution au 1:50000). La révélation a été réalisée avec le substrat TMB-H2O2 (Interchim ; réf UP664780) et les mesures de densité optique (O.D.) effectuées à 450 nm. Le tableau 5 résume les résultats obtenus:

Tableau 5 : Mesure de la liaison de l'anticorps 40-12 aux différents sous-domaines de FGF-R4

| Domaine étudié | D1 SABVA4794 | D2 SABVA4796 | D3 SABVA4799 | D1-D3 FGFR4 -Fc |
|---|---|---|---|---|
| Signal (O.D., 450 nm) | 0.185 | 3.000 | 0.105 | 3.000 |

L'anticorps anti-FGFR4 reconnaît donc le domaine D2 de la partie extracellulaire de la protéine FGFR4.

D'autre part, la protéine FGFR4-Fc dénaturée avec du SDS n'est pas reconnue par l'anticorps 40-12 en analyse par Western Blot, ce qui indique que l'épitope ciblé par le 40-12 sur le domaine D2 de FGFR4 est de type conformationel.

B- Détermination de l'épitope reconnu par les anticorps anti-FGFR4 8. 31, 33, 36.

Un criblage a été réalisé pour déterminer le domaine spécifique du FGFR4 reconnu par les anticorps 8, 31, 33, 36 par dosage ELISA en utilisant comme antigène de capture les constructs contenant soit les domaines D2 et D3 (SEQ ID NO 42) soit la protéine hFGFR4-Histag (SABVA4614, SEQ ID NO 40). L'antigène de capture a été fixé sur des boîtes Immulon-4 enzyme-linked (VWR Scientific Inc. Swedesboro, NJ). Des surnageants de cultures de HEK293 transfectées transitoirement par des plasmides permettant la sécrétion des anticorps 8, 31, 33, 36 ont ensuite été ajoutés puis détectés grâce à l'anticorps de lapin anti-humain IgG conjugué à la péroxidase (DakoCytomation, réf. P0214-dilution au 1:5000). La révélation a été réalisée avec le substrat TMB-H2O2 (Interchim ; réf UP664780) et les mesures de densité optique (O.D.) effectuées à 450 nm. Le tableau 6 ci-dessous résume les résultats obtenus:

Tableau 6 : Liaison des anticorps issus des clones 8, 31, 33 et 36 au FGF-R4 entier ou au sous-domaine D2-D3 (signal OD à 450nm)

| | hFGFR4-Histag | hFGFR4(D2,D3)-Histag |
|---|---|---|
| Clone 8 | 3,898 | 3,860 |
| Clone 31 | 3,859 | 3,741 |
| Clone 33 | 3,752 | 3,621 |
| Clone 36 | 3,751 | 3,616 |

Les anticorps 8, 31, 33 et 36 reconnaissent donc le domaine D2-D3 de la partie extracellulaire de la protéine FGFR4.

Tableau 7 : séquences utilisées, obtenues et déduites

| | Séquences nucléotidiques | Séquences protéiques |
|---|---|---|
| **Anticorps 40-12** | | |
| VH + CH | SEQ ID NO 1 | SEQ ID NO 2 |
| VL + CL | SEQ ID NO 3 | SEQ ID NO 4 |
| VH | SEQ ID NO 5 | SEQ ID NO 6 |
| VL | SEQ ID NO 7 | SEQ ID NO 8 |
| CDR VH | | SEQ ID NO 9 ; 10 ; 11 |
| CDR VL | | SEQ ID NO 12 ; 13 ; 14 |
| **Anticorps 64-12** | | |
| VH + CH | SEQ ID NO 15 | SEQ ID NO 16 |
| VL + CL | SEQ ID NO 17 | SEQ ID NO 18 |
| VH | SEQ ID NO 19 | SEQ ID NO 20 |
| VL | SEQ ID NO 21 | SEQ ID NO 22 |
| CDR VH | | SEQ ID NO 23; 24; 25 |
| CDR VL | | SEO ID NO 26 ; 27 ; 28 |
| **Séquences humanisées** | | |
| Chaîne légère 1 | SEQ ID NO 29 | SEQ ID NO 30 |
| Chaîne légère 2 | SEQ ID NO 31 | SEQ ID NO 32 |
| Chaîne lourde 1 | SEQ ID NO 33 | SEQ ID NO 34 |
| Chaîne lourde 2 | SEQ ID NO 35 | SEQ ID NO 36 |
| Chaîne lourde 3 | SEQ ID NO 37 | SEQ ID NO 38 |
| **Constructions** | | |
| hFGFR4-Histag | SEQ ID NO 39 | SEQ ID NO 40 |
| hFGFR4-Streptag | SEQ ID NO 68 | SEQ ID NO 69 |
| hFGFR4(D2D3)-Histag | SEQ ID NO 41 | SEQ ID NO 42 |

(suite)

| Constructions | | |
|---|---|---|
| mFGFR4-Histag | SEQ ID NO 43 | SEQ ID NO 44 |
| hFGFR1-Fc | SEQ ID NO 45 | SEQ ID NO 46 |
| hFGFR2-Fc | SEQ ID NO 47 | SEQ ID NO 48 |
| rFGFR4 | SEQ ID NO 49 | SEQ ID NO 50 |
| Séquences amorces pour établissement lignée clonale (exemple 4) | SEQ ID NO 51 ; 52 ; 53 ; 54 ; 55 ; 56 ; 58 SEQ ID NO 61 ; 62 | |
| Domaine transmembranaire FGFR4 | | SEQ ID NO 59 ; 60 |
| Oligonucléotides amorces tableau 1 | SEQ ID NO 63; 64; 65 ; 66 ; 67 | |
| Epitope dans domaine D2 | | SEQ ID NO 70 |
| Anticorps clone 8 | | |
| Chaîne légère entière | SEQ ID NO 71 | SEQ ID NO 72 |
| Chaîne légère CDR | | SEQ ID NO 73 ; 74 ; 75 |
| Chaîne lourde entière | SEQ ID NO 76 | SEQ ID NO 77 |
| Chaîne lourde CDR | | SEQ ID NO 78 ; 79; 80 |
| Anticorps clone 31 | | |
| Chaîne légère entière | SEQ ID NO 81 | SEQ ID NO 82 |
| Chaîne légère CDR | | SEQ ID NO 83 ; 84 ; 85 |
| Chaîne lourde entière | SEQ ID NO 86 | SEQ ID NO 87 |
| Chaîne lourde CDR | | SEQ ID NO 88 ; 89 ; 90 |
| Anticorps clone 33 | | |
| Chaîne légère entière | SEQ ID NO 91 | SEQ ID NO 92 |
| Chaîne légère CDR | | SEQ ID NO 93 ; 94 ; 95 |
| Chaîne lourde entière | SEQ ID NO 96 | SEQ ID NO 97 |
| Chaîne lourde CDR | | SEQ ID NO 98 ; 99 ; 100 |
| Anticorps clone 36 | | |
| Chaîne légère entière | SEQ ID NO 101 | SEQ ID NO 102 |

(suite)

| Anticorps clone 36 | | |
|---|---|---|
| Chaîne légère CDR | | SEQ ID NO 103; 104; 105 |
| Chaîne lourde entière | SEQ ID NO 106 | SEQ ID NO 107 |
| Chaîne lourde CDR | | SEQ ID NO 108; 109; 110 |
| **Sous-domaines de la partie extracellulaire de hFGFR4 fusionnés au domaine Fc de l'IgG1** | | |
| fGFGR4_D1::Fc | SEQ ID NO 111 | SEQ ID NO 112 |
| fGFGR4_D2::Fc | SEQ ID NO 113 | SEQ ID NO 114 |
| fGFGR4_D3::Fc | SEQ ID NO 115 | SEQ ID NO 116 |
| **Séquence de région constante d'IgG1 humaine** | | SEQ ID NO 117 |

## Revendications

1.  Anticorps antagoniste spécifique du récepteur Fibroblast Growt Factor-Receptor 4 (FGF-R4) pour son utilisation dans le traitement des maladies associées à une angiogenèse pathologique.

2.  Anticorps selon la revendication 1 pour son utilisation pour inhiber la croissance tumorale simultanément à l'inhibition de l'angiogenèse.

3. Anticorps selon l'une des revendications 1 et 2 pour son utilisation dans le traitement des hépatocarcinomes ou de tout autre type de cancer hépatique.

4. Anticorps selon l'une des revendications 1 à 3 pour son utilisation dans le traitement du cancer du pancréas.

5. Anticorps pour son utilisation selon l'une des revendications 1 à 4, se liant au domaine D2-D3 du récepteur FGF-R4.

6. Anticorps pour son utilisation selon l'une des revendications 1 à 5, se liant au domaine D2 du récepteur FGF-R4.

7. Anticorps pour son utilisation selon l'une des revendications 1 à 6, ayant un $K_D$ vis à vis du récepteur FGF-R4 déterminé par la technique Surface Plasmon Resonance (Biacore) inférieur à 10E-8 M.

8. Anticorps pour son utilisation selon l'une des revendications 1 à 7, qui est actif à la fois contre FGF-R4 humain et FGF-R4 murin.

9. Anticorps antagoniste spécifique du récepteur FGF-R4 comprenant les CDR de séquence SEQ ID NO : 9, 10, 11, 12, 13 et 14 ; ou 73, 74, 75, 78, 79 et 80; ou 83, 84, 85, 88, 89 et 90 ; ou 93, 94, 95, 98, 99 et 100 ; ou 103, 104, 105, 108, 109 et 110, où l'un des CDR peut différer de un à deux acides aminés par rapport à l'une au moins des séquences citées ci-dessus, pour autant que l'anticorps garde sa spécificité de liaison pour son utilisation dans le traitement des maladies associées à une angiogenèse pathologique.

10. Anticorps antagoniste spécifique du récepteur FGF-R4 dont la partie variable de sa chaîne lourde comprend une séquence nucléotidique présentant au moins 80% d'identité avec la séquence SEQ ID NO : 5, 76, 86, 96 ou 106 et dont la partie variable de sa chaîne légère comprend une séquence nucléotidique présentant au moins 80% d'identité avec la séquence SEQ ID NO: 7, 71, 81, 91 ou 101, pour autant que l'anticorps garde sa spécificité de liaison pour son utilisation dans le traitement des maladies associées à une angiogenèse pathologique.

11. Anticorps antagoniste spécifique du récepteur FGF-R4 dont la séquence comprend les séquences polypeptidiques SEQ ID NO : 2 et 4 ; ou 6 et 8 ; ou 72 et 77 ; ou 82 et 87 ; ou 92 et 97 ; ou 102 et 107 ou des séquences correspondant à ces séquences ayant au moins 80% d'identité avec elles, pour autant que l'anticorps garde sa spécificité de liaison pour son utilisation dans le traitement des maladies associées à une angiogenèse pathologique.

12. Anticorps spécifique du récepteur FGF-R4 pour son utilisation selon l'une quelconque des revendications 1 à 11, **caractérisé en ce qu'**il est un anticorps humanisé.

13. Anticorps pour son utilisation selon la revendication 12, **caractérisé en ce qu'**il comprend une chaîne variable légère présentant au moins 80% d'identité avec l'une des séquences polypeptidiques SEQ ID NO 30 ou 32 et une chaîne variable lourde présentant au moins 80% d'identité avec une séquence SEQ ID NO 34, 36 ou 38.

14. Anticorps pour son utilisation selon l'une des revendications 1 à 13, **caractérisé en ce qu'**il est un anticorps conjugué à un agent cytotoxique.

15. Anticorps selon l'une des revendications 9 à 14 pour son utilisation pour inhiber la prolifération cellulaire tumorale dans le traitement des hépatocarcinomes ou de tout autre type de cancer hépatique et dans le traitement des cancers pancréatiques.

16. Composition pharmaceutique comprenant un anticorps selon l'une quelconque des revendications 1 à 15 et des excipients pour son utilisation dans le traitement des maladies associées à une angiogenèse pathologique.

17. Médicament comprenant un anticorps selon l'une quelconque des revendications 1 à 16 pour son utilisation dans le traitement des maladies associées à une angiogenèse pathologique.

18. Polynucléotide codant pour un polypeptide antagoniste spécifique du récepteur FGF-R4 dont les séquences présentent au moins 80% d'identité avec l'une des séquences suivantes: SEQ ID NOS 2 et 4; ou SEQ ID NOS 6 et 8; ou SEQ ID NOS 9, 10, 11, 12, 13 et 14; ou SEQ ID NOS 30 ou 32 et 34 ou 36 ou 38; ou SEQ ID NOS 72 et 77; ou SEQ ID NOS 73, 74, 75, 78, 79 et 80 ; ou SEQ ID NOS 82 et 87; ou SEQ ID NOS 83, 84, 85, 88, 89 et 90; ou SEQ ID NOS 92 et 97 ; ou SEQ ID NOS 93, 94, 95, 98, 99 et 100; ou SEQ ID NOS 102 et 107; ou SEQ ID NOS 103, 104, 105, 108, 109 et 110, pour autant que le polypeptide qu'il code garde la spécificité de liaison, pour son utilisation dans le traitement des maladies associées à une angiogenèse pathologique.

19. Polynucléotide **caractérisé en ce qu'**il présente une séquence présentant au moins 80% d'identité avec l'une des séquences SEQ ID NOS 1 et 3 ; ou SEQ ID NOS 5 et 7; SEQ ID NOS 29 ou 31, et 33, 35 ou 37 ; ou SEQ ID NOS 71 et 76 ; ou SEQ ID NOS 81 et 86 ; ou SEQ ID NOS 91 et 96 ; ou SEQ ID NOS 101 et 106, pour autant que le polypeptide qu'il code garde la spécificité de liaison antagoniste au récepteur FGF-R4, pour son utilisation dans le traitement des maladies associées à une angiogenèse pathologique.

**Patentansprüche**

1. Antagonistischer Antikörper, spezifisch für den Fibroblasten-Wachstumsfaktor-Rezeptor 4 (FGF-R4), für die Verwendung zur Behandlung von Krankheiten in Verbindung mit einer pathologischen Angiogenese.

2. Antikörper nach Anspruch 1 für die Verwendung zur Hemmung des Tumorwachstums gleichzeitig mit der Hemmung der Angiogenese.

3. Antikörper nach einem der Ansprüche 1 und 2 für die Verwendung zur Behandlung von Hepatokarzinomen oder jedem anderen Typ des Leberkrebses.

4. Antikörper nach einem der Ansprüche 1 bis 3 für die Verwendung zur Behandlung von Bauchspeicheldrüsenkrebs.

5. Antikörper für die Verwendung nach einem der Ansprüche 1 bis 4, der an die Domäne D2-D3 des Rezeptors FGF-R4 bindet.

6. Antikörper für die Verwendung nach einem der Ansprüche 1 bis 5, der an die Domäne D2 des Rezeptors FGF-R4 bindet.

7. Antikörper für die Verwendung nach einem der Ansprüche 1 bis 6 mit einer $K_D$ in Bezug auf den Rezeptor FGF-R4, bestimmt mit der Oberflächen-Plasmon-Resonanz-Technik, von weniger als 10E-8 M.

8. Antikörper für die Verwendung nach einem der Ansprüche 1 bis 7, der sowohl gegen humanen FGF-R4 als auch gegen murinen FGF-R4 aktiv ist.

9. Antagonistischer Antikörper, spezifisch für den Rezeptor FGF-R4, umfassend die CDR der Sequenz SEQ ID NO: 9, 10, 11, 12, 13 und 14; oder 73, 74, 75, 78, 79 und 80; oder 83, 84, 85, 88, 89 und 90; oder 93, 94, 95, 98, 99 und 100; oder 103, 104, 105, 108, 109 und 110, wobei sich eine der CDR um eine bis zwei Aminosäuren von mindestens einer der vorstehend genannten Sequenzen unterscheiden kann, soweit der Antikörper seine Bindungsspezifität beibehält, für die Verwendung zur Behandlung von Krankheiten in Verbindung mit einer pathologischen Angiogenese.

10. Antagonistischer Antikörper, spezifisch für den Rezeptor FGF-R4, dessen variabler Teil seiner schweren Kette eine Nukleotidsequenz umfasst, die mindestens 80% Identität mit der Sequenz SEQ ID NO: 5, 76, 86, 96 oder 106 aufweist, und dessen variabler Teil seiner leichten Kette eine Nukleotidsequenz umfasst, die mindestens 80% Identität mit der Sequenz SEQ ID NO: 7, 71, 81, 91 oder 101 aufweist, soweit der Antikörper seine Bindungsspezifität beibehält, für die Verwendung zur Behandlung von Krankheiten in Verbindung mit einer pathologischen Angiogenese.

11. Antagonistischer Antikörper, spezifisch für den Rezeptor FGF-R4, dessen Sequenz die Polypeptidsequenzen SEQ ID NO: 2 und 4; oder 6 und 8; oder 72 und 77; oder 82 und 87; oder 92 und 97; oder 102 und 107 oder diesen Sequenzen entsprechende Sequenzen mit mindestens 80% Identität mit diesen umfasst, soweit der Antikörper seine Bindungsspezifität beibehält, für die Verwendung zur Behandlung von Krankheiten in Verbindung mit einer pathologischen Angiogenese.

12. Antikörper, spezifisch für den Rezeptor FGF-R4, für die Verwendung nach einem der Ansprüche 1 bis 11, **dadurch gekennzeichnet, dass** es sich um einen humanisierten Antikörper handelt.

13. Antikörper für die Verwendung nach Anspruch 12, **dadurch gekennzeichnet, dass** er eine variable leichte Kette, die mindestens 80% Identität mit einer der Polypeptidsequenzen SEQ ID NO: 30 oder 32 aufweist, und eine variable schwere Kette umfasst, die mindestens 80% Identität mit einer Sequenz SEQ ID NO: 34, 36 oder 38 aufweist.

14. Antikörper für die Verwendung nach einem der Ansprüche 1 bis 13, **dadurch gekennzeichnet, dass** es sich um einen an ein zytotoxisches Agens konjugierten Antikörper handelt.

15. Antikörper nach einem der Ansprüche 9 bis 14 für die Verwendung zur Hemmung der Tumorzellproliferation bei der Behandlung von Hepatokarzinomen oder jedem anderen Typ des Leberkrebses und der Behandlung von Bauchspeicheldrüsenkrebserkrankungen.

16. Pharmazeutische Zusammensetzung, umfassend einen Antikörper nach einem der Ansprüche 1 bis 15 und Exzipienten, für die Verwendung zur Behandlung von Krankheiten in Verbindung mit einer pathologischen Angiogenese.

17. Arzneimittel, umfassend einen Antikörper nach einem der Ansprüche 1 bis 16, für die Verwendung zur Behandlung von Krankheiten in Verbindung mit einer pathologischen Angiogenese.

18. Polynukleotid, das ein für den Rezeptor FGF-R4 spezifisches antagonistisches Polypeptid codiert, dessen Sequenzen mindestens 80% Identität mit einer der folgenden Sequenzen aufweisen: SEQ ID NOS: 2 und 4; oder SEQ ID NOS: 6 und 8; oder SEQ ID NOS: 9, 10, 11, 12, 13 und 14; oder SEQ ID NOS: 30 oder 32 und 34 oder 36 oder 38; oder SEQ ID NOS: 72 und 77; oder SEQ ID NOS: 73, 74, 75, 78, 79 und 80; oder SEQ ID NOS: 82 und 87; oder SEQ ID NOS: 83, 84, 85, 88, 89 und 90; oder SEQ ID NOS: 92 und 97; oder SEQ ID NOS: 93, 94, 95, 98, 99 und 100; oder SEQ ID NOS: 102 und 107; oder SEQ ID NOS: 103, 104, 105, 108, 109 und 110, soweit das Polypeptid, das es codiert, die Bindungsspezifität beibehält, für die Verwendung zur Behandlung von Krankheiten in Verbindung mit einer pathologischen Angiogenese.

19. Polynukleotid, **dadurch gekennzeichnet, dass** es eine Sequenz mit mindestens 80% Identität mit einer der Sequenzen SEQ ID NOS: 1 und 3; oder SEQ ID NOS: 5 und 7; SEQ ID NOS: 29 oder 31 und 33, 35 oder 37; oder SEQ ID NOS: 71 und 76; oder SEQ ID NOS: 81 und 86; oder SEQ ID NOS: 91 und 96; oder SEQ ID NOS: 101 und 106 aufweist, soweit das Polypeptid, das es codiert, die antagonistische Bindungsspezifität an den Rezeptor FGF-R4 beibehält, für die Verwendung zur Behandlung von Krankheiten in Verbindung mit einer pathologischen Angiogenese.

**Claims**

1. Fibroblast growth factor-receptor 4 (FGF-R4)-specific antagonist antibody for use in the treatment of diseases associated with a pathological angiogenesis.

2. Antibody according to Claim 1, for use in inhibiting tumour growth simultaneously with the inhibition of angiogenesis.

3. Antibody according to either of Claims 1 and 2, for use in the treatment of hepatocarcinomas or of any other type of hepatic cancer.

4. Antibody according to one of Claims 1 to 3, for use in the treatment of pancreatic cancer.

5. Antibody for use according to one of Claims 1 to 4, which binds to the D2-D3 domain of the FGF-R4 receptor.

6. Antibody for use according to one of Claims 1 to 5, which binds to the D2 domain of the FGF-R4 receptor.

7. Antibody for use according to one of Claims 1 to 6, which has a $K_D$ with respect to the FGF-R4 receptor, determined by means of the Surface Plasmon Resonance (Biacore) technique, of less than $10^{-8}$ M.

8. Antibody for use according to one of Claims 1 to 7, which is active both against human FGF-R4 and against murine FGF-R4.

9. FGF-R4 receptor-specific antagonist antibody comprising the CDRs of sequence SEQ ID NOs: 9, 10, 11, 12, 13 and 14; or 73, 74, 75, 78, 79 and 80; or 83, 84, 85, 88, 89 and 90; or 93, 94, 95, 98, 99 and 100; or 103, 104, 105, 108, 109 and 110, where one of the CDRs may differ by one or two amino acids compared with at least one of the sequences mentioned above, provided that the antibody keeps its binding specificity, for use in the treatment of diseases associated with a pathological angiogenesis.

10. FGF-R4 receptor-specific antagonist antibody of which the variable region of its heavy chain comprises a nucleotide sequence having at least 80% identity with the sequence SEQ ID NO: 5, 76, 86, 96 or 106 and of which the variable region of its light chain comprises a nucleotide sequence having at least 80% identity with the sequence SEQ ID NO: 7, 71, 81, 91 or 101, provided that the antibody keeps its binding specificity, for use in the treatment of diseases associated with a pathological angiogenesis.

11. FGF-R4 receptor-specific antagonist antibody, the sequence of which comprises the polypeptide sequences SEQ ID NOs: 2 and 4; or 6 and 8; or 72 and 77; or 82 and 87; or 92 and 97; or 102 and 107 or sequences, corresponding to these sequences, having at least 80% identity therewith, provided that the antibody keeps its binding specificity, for use in the treatment of diseases associated with a pathological angiogenesis.

12. FGF-R4 receptor-specific antibody for use according to any one of Claims 1 to 11, **characterized in that** it is a humanized antibody.

13. Antibody for use according to Claim 12, **characterized in that** it comprises a variable light chain having at least 80% identity with one of the polypeptide sequences SEQ ID NO: 30 or 32 and a variable heavy chain having at least 80% identity with a sequence SEQ ID NO: 34, 36 or 38.

14. Antibody for use according to one of Claims 1 to 13, **characterized in that** it is an antibody which is conjugated to a cytotoxic agent.

15. Antibody according to one of Claims 9 to 14, for use in inhibiting tumour cell proliferation in the treatment of hepatocarcinomas or of any other type of hepatic cancer, and in the treatment of pancreatic cancer.

16. Pharmaceutical composition comprising an antibody according to any one of Claims 1 to 15 and excipients, for use in the treatment of diseases associated with a pathological angiogenesis.

17. Medicament comprising an antibody according to any one of Claims 1 to 16, for use in the treatment of diseases associated with a pathological angiogenesis.

18. Polynucleotide encoding an FGF-R4 receptor-specific antagonist polypeptide, the sequences of which have at least 80% identity with one of the following sequences: SEQ ID NOs: 2 and 4; or SEQ ID NOs: 6 and 8; or SEQ ID NOs: 9, 10, 11, 12, 13 and 14; or SEQ ID NOs: 30 or 32 and 34 or 36 or 38; or SEQ ID NOs: 72 and 77; or SEQ ID NOs: 73, 74, 75, 78, 79 and 80; or SEQ ID NOs: 82 and 87; or SEQ ID NOs: 83, 84, 85, 88, 89 and 90; or SEQ ID NOs: 92 and 97; or SEQ ID NOs: 93, 94, 95, 98, 99 and 100; or SEQ ID NOs: 102 and 107; or SEQ ID NOs: 103, 104, 105, 108, 109 and 110, provided that the polypeptide which it encodes keeps the binding specificity, for use in the treatment of diseases associated with a pathological angiogenesis.

19. Polynucleotide **characterized in that** it has a sequence having at least 80% identity with one of the sequences SEQ ID NOs: 1 and 3; or SEQ ID NOs: 5 and 7; SEQ ID NOs: 29 or 31, and 33, 35 or 37; or SEQ ID NOs: 71 and 76; or SEQ ID NOs: 81 and 86; or SEQ ID NOs: 91 and 96; or SEQ ID NOs: 101 and 106, provided that the polypeptide that it encodes keeps the specificity of antagonistic binding to the FGF-R4 receptor, for use in the treatment of diseases associated with a pathological angiogenesis.

Figure 1A

Figure 1B

Figure 2A

Figure 2B

Figure 3

Figure 4

hFGFR1
Fc
pA LATE SV40
TPLenhMLP
ORI P
CMV E/P (-723/+7)
F1 ORI
AmpR

pXL4328 hFGFR1-Fc
8691 bp

Figure 5

Figure 6

Figure 7

Figure 8A

Figure 8B

Figure 8C

Figure 8D

Figure 9A

Figure 9B

Figure 9C

Figure 9D

| Sérum (%) | 0 | 10 | 10 | 10 | 10 | 10 |
|---|---|---|---|---|---|---|
| 40-12 (µg/ml) | - | - | - | - | 10 | 100 |
| mAb contrôle (µg/ml) | - | - | 10 | 100 | - | - |

Figure 10A

| FGF-19 (30 ng/ml) | - | + | + | + |
|---|---|---|---|---|
| 40-12 (10 µg/ml) | - | - | - | + |
| mAb contrôle (10 µg/ml) | - | - | + | - |

Figure 10B

Stade pré-angiogenique — Stade angiogenique — Petites tumeurs — Grosses tumeurs Carcinomes invasifs

5    8    10    12    > 15

Age (semaines)

Période de traitement

**Figure 11 A**

**Figure 11B**

**Figure 11C**

**Figure 11D**

**Figure 12**

FGF-R4 humain

Figure 13A

FGF-R4 de souris

Figure 13B

FGF-R4 de rat

Figure 13C

## Figure 14A

```
  1   LEASEEVELE PCLAPSLEQQ EQELTVALGQ PVRLCCGRAE RGGHWYKEGS
 51   RLAPAGRVRG WRGRLEIASF LPEDAGRYLC LARGSMIVLQ NLTLITGDSL
101   TSSNDDEDPK SHRDLSNRHS YPQQAPYWTH PQRMEKKLHA VPAGNTVKFR
151   CPAAGNPTKT HTCPPCPAPE LLGGPSVFLF PPKPKDTLMI SRTPEVTCVV
201   VDVSHEDPEV KFNWYVDGVE VHNAKTKPRE EQYNSTYRVV SVLTVLHQDW
251   LNGKEYKCKV SNKALPAPIE KTISKAKGQP REPQVYTLPP SRDELTKNQV
301   SLTCLVKGFY PSDIAVEWES NGQPENNYKT TPPVLDSDGS FFLYSKLTVD
351   KSRWQQGNVF SCSVMHEALH NHYTQKSLSL SPG
```

## Figure 14B

Figure 15A

```
  1    LEASEEVELE  PQAPYWTHPQ  RMEKKLHAVP  AGNTVKFRCP  AAGNPTPTIR
 51    WLKDGQAFHG  ENRIGGIRLR  HQHWSLVMES  VVPSDRGTYT  CLVENAVGSI
101    RYNYLLDVLK  THTCPPCPAP  ELLGGPSVFL  FPPKPKDTLM  ISRTPEVTCV
151    VVDVSHEDPE  VKFNWYVDGV  EVHNAKTKPR  EEQYNSTYRV  VSVLTVLHQD
201    WLNGKEYKCK  VSNKALPAPI  EKTISKAKGQ  PREPQVYTLP  PSRDELTKNQ
251    VSLTCLVKGF  YPSDIAVEWE  SNGQPENNYK  TTPPVLDSDG  SFFLYSKLTV
301    DKSRWQQGNV  FSCSVMHEAL  HNHYTQKSLS  LSPG
```

Figure 15B

CMV E/P (-723/+7)

TPLenhMLP

hFGFR4 D3

AmpR

**pXL4799**
**8044 bp**

hFc

F1 ORI

pA LATE SV40

ORI P

# Figure 16A

```
  1   LEASEEVELE  PNAVGSIRYN  YLLDVLERSP  HRPILQAGLP  ANTTAVVGSD
 51   VELLCKVYSD  AQPHIQWLKH  IVINGSSFGA  DGFPYVQVLK  TADINSSEVE
101   VLYLRNVSAE  DAGEYTCLAG  NSIGLSYQSA  WLTVLPEEDP  TWTAAAPEAK
151   THTCPPCPAP  ELLGGPSVFL  FPPKPKDTLM  ISRTPEVTCV  VVDVSHEDPE
201   VKFNWYVDGV  EVHNAKTKPR  EEQYNSTYRV  VSVLTVLHQD  WLNGKEYKCK
251   VSNKALPAPI  EKTISKAKGQ  PREPQVYTLP  PSRDELTKNQ  VSLTCLVKGF
301   YPSDIAVEWE  SNGQPENNYK  TTPPVLDSDG  SFFLYSKLTV  DKSRWQQGNV
351   FSCSVMHEAL  HNHYTQKSLS  LSPG
```

# Figure 16B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 20077080325 A
- WO 2005066211 A
- WO 2005037235 A
- WO 03063893 A
- WO 2008052796 A
- WO 2008052798 A
- US 5807715 A
- US 4816567 A
- US 4816397 A
- EP O0239400 A

- WO 9109967 A
- US 5530101 A
- US 5585089 A
- EP O0592106 A
- EP O0519596 A
- US 5565332 A
- US 5939598 A
- WO 2008065543 A
- FR 2008072 W

**Littérature non-brevet citée dans la description**

- **XIE et al.** *Cytokine,* 1999, vol. 11, 729-35
- **ORNITZ et al.** *J. Biol. Chem.,* 1996, vol. 271, 15292-7
- **CAVALLARO et al.** *Nat. Cell Biol.,* 2001, vol. 3 (7), 650-657
- **STADLER et al.** *Cell. Signal.,* 2006, vol. 18 (6), 783-794
- **LIN et al.** *J Biol Chem.,* 2007, vol. 14, 27277-84
- **KLINGENBERG et al.** *J. Cell Sci.,* 2000, vol. 113, 1827-1838
- **BANGE et al.** *Cancer Res.,* 2002, vol. 62 (3), 840-847
- **SPINOLA et al.** *J Clin Oncol,* 2005, vol. 23, 7307-7311
- **TAKAISHI et al.** *Biochem Biophys Res Commun.,* 2000, vol. 267, 658-62
- **EZZAT et al.** *J Clin Invest.,* 2002, vol. 109, 69-78
- **WANG et al.** *Clin. Cancer Res.,* 2004, vol. 10 (18), 6169-6178
- **NICHOLES et al.** *Am. J. PAthol.,* 2002, vol. 160 (6), 2295-307
- **MORIMOTO et al.** *Cancer,* 2003, vol. 98 (10), 2245-2250
- **SPINOLA et al.** *J Clin Onco,* 2005, vol. 23, 7307-7311
- **DA COSTA ANDRADE et al.** *Exp Mol Pathol,* 2007, vol. 82, 53-7
- **SHAH et al.** *Oncogene,* 2002, vol. 21 (54), 8251-61
- **YAMADA et al.** *Neurol. Res.,* 2002, vol. 24 (3), 244-8
- **PRESTA et al.** *Cytokine Growth Factor Rev.,* 2005, vol. 16, 159-78

- **THOMPSON et al.** *J Med Chem.,* 2000, vol. 43, 4200-11
- **CHEN et al.** *Hybridoma,* 2005, vol. 24 (3), 152-159
- **KABAT et al.** Sequences of proteins of immunological interest. U.S. Department of Health and Human Services, NIH, 1991
- **MORRISON.** *Science,* 1985, vol. 229, 1202
- **OI et al.** *Bio Techniques,* 1986, vol. 4, 214
- **GILLIES et al.** *J. Immunol. Methods,* 1999, vol. 125, 191-202
- **LAZAR et al.** *Proc. Natl. Acad. Sci. USA,* 2006, vol. 103, 4005-4010
- **STAVENHAGEN et al.** *Cancer Res.,* 2007, vol. 67, 8882-8890
- *Molecular Immunology,* 2007, vol. 44, 1986-1998
- **PADLAN.** *Molec Imm,* 1991, vol. 28 (4/5), 489-498
- **STUDNICKA et al.** *Prot Eng,* 1994, vol. 7 (6), 805-814
- **ROGUSKA et al.** *PNAS,* 1994, vol. 91, 969-973
- **H.M. BERMAN ; J. WESTBROOK ; Z. FENG ; G. GILLILAND ; T.N. BHAT ; H. WEISSIG ; I.N. SHINDYALOV ; P.E. BOURNE.** *Nucleic Acids Research,* 2000, vol. 28, 235-242
- *J Mol Biol.,* 21 Octobre 1990, vol. 5, 403-410
- **D.A. CASE ; T.E. CHEATHAM, III ; T. DARDEN ; H. GOHLKE ; R. LUO ; K.M. MERZ, JR. ; A. ONUFRIEV ; C. SIMMERLING ; B. WANG ; R. WOODS.** *J. Computat. Chem.,* 2005, vol. 26, 1668-1688
- **GALLICCHIO ; LEVY.** *J Comput Chem,* 2004, vol. 25, 479-499

- **BARTH. et al.** *J Comp Chem,* 1995, vol. 16, 1192-1209
- *Nucleic Acids Research,* 2005, vol. 33, D593-D597
- **CIEPLAK, J. et al.** *J. Comp. Chem.,* 2001, vol. 22, 1048-1057
- *PLoS Biol.,* Mars 2005, vol. 3 (3), e91
- The immune epitope database and analysis resource: from vision to blueprint. *PLoS Biol.,* Mars 2005, vol. 3 (3), e91, http://www.immuneepitope.org
- **KANDA et al.** *Biotechnol. Bioeng.,* 2006, vol. 94, 680-688
- **RIPKA et al.** *Arch Biochem Bioph,* 1986, vol. 249, 533-545
- **HANAHAN D.** *Nature,* 1985, vol. 9-15, 115-22
- **ANDRADE et al.** *Microvascular Research,* 1997, vol. 54, 253-61
- **SADDIC et al.** *Methods Mol. Biol.,* 2002, vol. 194, 23-36
- **ANUMULA et al.** *Glycobiology,* 1998, vol. 8, 685-694
- **KILPATRICK et al.** *Hybridoma,* 1997, vol. 16, 381389
- **T. JOSTOCK et al.** *Journal of Immunological Methods,* 2004, vol. 289, 65-80
- **CANZIANI et al.** *Anal. Biochem.,* 2004, vol. 325, 301-307

SEQUENCE LISTING

<110> SANOFI-AVENTIS

<120> Antagonistes spécifiques du récepteur FGF-R4

<130> FR2008-072 PCT

<160> 117

<170> PatentIn version 3.3

<210> 1
<211> 1389
<212> DNA
<213> Mus sp.

<220>
<221> CDS
<222> (1)..(1389)

<400> 1

```
atg aac agg ctt act tcc tca ttg ctg ctg ctg att gtc cct gca tat      48
Met Asn Arg Leu Thr Ser Ser Leu Leu Leu Leu Ile Val Pro Ala Tyr
1               5                   10                  15

gtc ctg tcc cag gtt act ctg aaa gag tct ggc cct ggg ata ttg cag      96
Val Leu Ser Gln Val Thr Leu Lys Glu Ser Gly Pro Gly Ile Leu Gln
                20                  25                  30

ccc tcc cag acc ctc agt ctg act tgt tct ttc tct ggg ttt tca ctg     144
Pro Ser Gln Thr Leu Ser Leu Thr Cys Ser Phe Ser Gly Phe Ser Leu
            35                  40                  45

agc act tct ggt atg ggt gtg agc tgg att cgt cag cct tca gga aag     192
Ser Thr Ser Gly Met Gly Val Ser Trp Ile Arg Gln Pro Ser Gly Lys
        50                  55                  60

ggt ctg gag tgg ctg gca cac att tac tgg gat gat gac aag cgc tat     240
Gly Leu Glu Trp Leu Ala His Ile Tyr Trp Asp Asp Asp Lys Arg Tyr
65                  70                  75                  80

aac cca tcc ctg aag agc cgg ctc aca atc tcc aag gat acc tcc agc     288
Asn Pro Ser Leu Lys Ser Arg Leu Thr Ile Ser Lys Asp Thr Ser Ser
                85                  90                  95

aac cag gta ttc ctc aag atc acc agt gtg gac act gca gat act gcc     336
Asn Gln Val Phe Leu Lys Ile Thr Ser Val Asp Thr Ala Asp Thr Ala
                100                 105                 110

aca tac tac tgt gct cga gat tac tac gct agt agc ttt gac tac tgg     384
Thr Tyr Tyr Cys Ala Arg Asp Tyr Tyr Ala Ser Ser Phe Asp Tyr Trp
            115                 120                 125

ggc caa ggc acc act ctc aca gtc tcc tca gcc aaa acg aca ccc cca     432
Gly Gln Gly Thr Thr Leu Thr Val Ser Ser Ala Lys Thr Thr Pro Pro
        130                 135                 140

tct gtc tat cca ctg gcc cct gga tct gct gcc caa act aac tcc atg     480
Ser Val Tyr Pro Leu Ala Pro Gly Ser Ala Ala Gln Thr Asn Ser Met
```

|     | 145 | | | | 150 | | | | 155 | | | | 160 | |
|-----|-----|-----|-----|-----|-----|-----|-----|-----|-----|-----|-----|-----|-----|-----|

gtg acc ctg gga tgc ctg gtc aag ggc tat ttc cct gag cca gtg aca    528
Val Thr Leu Gly Cys Leu Val Lys Gly Tyr Phe Pro Glu Pro Val Thr
                    165              170            175

gtg acc tgg aac tct gga tcc ctg tcc agc ggt gtg cac acc ttc cca    576
Val Thr Trp Asn Ser Gly Ser Leu Ser Ser Gly Val His Thr Phe Pro
             180              185           190

gct gtc ctg cag tct gac ctc tac act ctg agc agc tca gtg act gtc    624
Ala Val Leu Gln Ser Asp Leu Tyr Thr Leu Ser Ser Ser Val Thr Val
           195              200           205

ccc tcc agc acc tgg ccc agc gag acc gtc acc tgc aac gtt gcc cac    672
Pro Ser Ser Thr Trp Pro Ser Glu Thr Val Thr Cys Asn Val Ala His
         210              215           220

ccg gcc agc agc acc aag gtg gac aag aaa att gtg ccc agg gat tgt    720
Pro Ala Ser Ser Thr Lys Val Asp Lys Lys Ile Val Pro Arg Asp Cys
225              230              235           240

ggt tgt aag cct tgc ata tgt aca gtc cca gaa gta tca tct gtc ttc    768
Gly Cys Lys Pro Cys Ile Cys Thr Val Pro Glu Val Ser Ser Val Phe
             245              250           255

atc ttc ccc cca aag ccc aag gat gtg ctc acc att act ctg act cct    816
Ile Phe Pro Pro Lys Pro Lys Asp Val Leu Thr Ile Thr Leu Thr Pro
             260              265           270

aag gtc acg tgt gtt gtg gta gac atc agc aag gat gat ccc gag gtc    864
Lys Val Thr Cys Val Val Val Asp Ile Ser Lys Asp Asp Pro Glu Val
           275              280           285

cag ttc agc tgg ttt gta gat gat gtg gag gtg cac aca gct cag acg    912
Gln Phe Ser Trp Phe Val Asp Asp Val Glu Val His Thr Ala Gln Thr
         290              295           300

caa ccc cgg gag gag cag ttc aac agc act ttc cgc tca gtc agt gaa    960
Gln Pro Arg Glu Glu Gln Phe Asn Ser Thr Phe Arg Ser Val Ser Glu
305              310              315           320

ctt ccc atc atg cac cag gac tgg ctc aat ggc aag gag ttc aaa tgc   1008
Leu Pro Ile Met His Gln Asp Trp Leu Asn Gly Lys Glu Phe Lys Cys
             325              330           335

agg gtc aac agt gca gct ttc cct gcc ccc atc gag aaa acc atc tcc   1056
Arg Val Asn Ser Ala Ala Phe Pro Ala Pro Ile Glu Lys Thr Ile Ser
             340              345           350

aaa acc aaa ggc aga ccg aag gct cca cag gtg tac acc att cca cct   1104
Lys Thr Lys Gly Arg Pro Lys Ala Pro Gln Val Tyr Thr Ile Pro Pro
             355              360           365

ccc aag gag cag atg gcc aag gat aaa gtc agt ctg acc tgc atg ata   1152
Pro Lys Glu Gln Met Ala Lys Asp Lys Val Ser Leu Thr Cys Met Ile
         370              375           380

aca gac ttc ttc cct gaa gac att act gtg gag tgg cag tgg aat ggg   1200
Thr Asp Phe Phe Pro Glu Asp Ile Thr Val Glu Trp Gln Trp Asn Gly
385              390              395           400

```
cag cca gcg gag aac tac aag aac act cag ccc atc atg gac aca gat     1248
Gln Pro Ala Glu Asn Tyr Lys Asn Thr Gln Pro Ile Met Asp Thr Asp
            405                 410                 415

ggc tct tac ttc gtc tac agc aag ctc aat gtg cag aag agc aac tgg     1296
Gly Ser Tyr Phe Val Tyr Ser Lys Leu Asn Val Gln Lys Ser Asn Trp
            420                 425                 430

gag gca gga aat act ttc acc tgc tct gtg tta cat gag ggc ctg cac     1344
Glu Ala Gly Asn Thr Phe Thr Cys Ser Val Leu His Glu Gly Leu His
            435                 440                 445

aac cac cat act gag aag agc ctc tcc cac tct cct ggt aag tga         1389
Asn His His Thr Glu Lys Ser Leu Ser His Ser Pro Gly Lys
            450                 455                 460
```

<210> 2
<211> 462
<212> PRT
<213> Mus sp.

<400> 2

```
Met Asn Arg Leu Thr Ser Ser Leu Leu Leu Ile Val Pro Ala Tyr
1               5                   10              15

Val Leu Ser Gln Val Thr Leu Lys Glu Ser Gly Pro Gly Ile Leu Gln
            20                  25                  30

Pro Ser Gln Thr Leu Ser Leu Thr Cys Ser Phe Ser Gly Phe Ser Leu
            35                  40                  45

Ser Thr Ser Gly Met Gly Val Ser Trp Ile Arg Gln Pro Ser Gly Lys
            50                  55                  60

Gly Leu Glu Trp Leu Ala His Ile Tyr Trp Asp Asp Asp Lys Arg Tyr
65                  70                  75                  80

Asn Pro Ser Leu Lys Ser Arg Leu Thr Ile Ser Lys Asp Thr Ser Ser
                85                  90                  95

Asn Gln Val Phe Leu Lys Ile Thr Ser Val Asp Thr Ala Asp Thr Ala
            100                 105                 110

Thr Tyr Tyr Cys Ala Arg Asp Tyr Tyr Ala Ser Ser Phe Asp Tyr Trp
            115                 120                 125

Gly Gln Gly Thr Thr Leu Thr Val Ser Ser Ala Lys Thr Thr Pro Pro
            130                 135                 140
```

```
Ser Val Tyr Pro Leu Ala Pro Gly Ser Ala Ala Gln Thr Asn Ser Met
145             150             155             160

Val Thr Leu Gly Cys Leu Val Lys Gly Tyr Phe Pro Glu Pro Val Thr
                165             170             175

Val Thr Trp Asn Ser Gly Ser Leu Ser Ser Gly Val His Thr Phe Pro
            180             185             190

Ala Val Leu Gln Ser Asp Leu Tyr Thr Leu Ser Ser Ser Val Thr Val
        195             200             205

Pro Ser Ser Thr Trp Pro Ser Glu Thr Val Thr Cys Asn Val Ala His
    210             215             220

Pro Ala Ser Ser Thr Lys Val Asp Lys Lys Ile Val Pro Arg Asp Cys
225             230             235             240

Gly Cys Lys Pro Cys Ile Cys Thr Val Pro Glu Val Ser Ser Val Phe
            245             250             255

Ile Phe Pro Pro Lys Pro Lys Asp Val Leu Thr Ile Thr Leu Thr Pro
            260             265             270

Lys Val Thr Cys Val Val Val Asp Ile Ser Lys Asp Asp Pro Glu Val
        275             280             285

Gln Phe Ser Trp Phe Val Asp Asp Val Glu Val His Thr Ala Gln Thr
    290             295             300

Gln Pro Arg Glu Glu Gln Phe Asn Ser Thr Phe Arg Ser Val Ser Glu
305             310             315             320

Leu Pro Ile Met His Gln Asp Trp Leu Asn Gly Lys Glu Phe Lys Cys
            325             330             335

Arg Val Asn Ser Ala Ala Phe Pro Ala Pro Ile Glu Lys Thr Ile Ser
            340             345             350

Lys Thr Lys Gly Arg Pro Lys Ala Pro Gln Val Tyr Thr Ile Pro Pro
        355             360             365

Pro Lys Glu Gln Met Ala Lys Asp Lys Val Ser Leu Thr Cys Met Ile
    370             375             380

Thr Asp Phe Phe Pro Glu Asp Ile Thr Val Glu Trp Gln Trp Asn Gly
```

385 390 395 400

Gln Pro Ala Glu Asn Tyr Lys Asn Thr Gln Pro Ile Met Asp Thr Asp
405 410 415

Gly Ser Tyr Phe Val Tyr Ser Lys Leu Asn Val Gln Lys Ser Asn Trp
420 425 430

Glu Ala Gly Asn Thr Phe Thr Cys Ser Val Leu His Glu Gly Leu His
435 440 445

Asn His His Thr Glu Lys Ser Leu Ser His Ser Pro Gly Lys
450 455 460

<210> 3
<211> 705
<212> DNA
<213> Mus sp.

<220>
<221> CDS
<222> (1)..(705)

<400> 3

```
atg gtt ttc aca cct cag att ctt gga ctt atg ctt ttc tgg att tca          48
Met Val Phe Thr Pro Gln Ile Leu Gly Leu Met Leu Phe Trp Ile Ser
1               5                   10                  15

gcc tcc aga ggt gat att gtg cta act cag tct cca gcc acc ctg tct          96
Ala Ser Arg Gly Asp Ile Val Leu Thr Gln Ser Pro Ala Thr Leu Ser
                20                  25                  30

gtg act cca gga gat aga gtc agt ctt tcc tgc agg gcc agt caa agt         144
Val Thr Pro Gly Asp Arg Val Ser Leu Ser Cys Arg Ala Ser Gln Ser
            35                  40                  45

att agc aac tac cta cac tgg tat caa caa aaa tca cat gag tct cca         192
Ile Ser Asn Tyr Leu His Trp Tyr Gln Gln Lys Ser His Glu Ser Pro
        50                  55                  60

agg ctt ctc atc aag tat gct tcc cag tcc atc tct ggg atc ccc tcc         240
Arg Leu Leu Ile Lys Tyr Ala Ser Gln Ser Ile Ser Gly Ile Pro Ser
65                  70                  75                  80

agg ttc agt ggc agt gga tca ggg aca gat ttc att ctc agt ttc aac         288
Arg Phe Ser Gly Ser Gly Ser Gly Thr Asp Phe Ile Leu Ser Phe Asn
                85                  90                  95

agt gtg gag act gaa gat ttt gga atg tat ttc tgt caa cag agt aac         336
Ser Val Glu Thr Glu Asp Phe Gly Met Tyr Phe Cys Gln Gln Ser Asn
                100                 105                 110

agc tgg cct ttc acg ttc ggc tcg ggg aca aaa ttg gaa ata aaa cgg         384
Ser Trp Pro Phe Thr Phe Gly Ser Gly Thr Lys Leu Glu Ile Lys Arg
        115                 120                 125

gct gat gct gca cca act gta tcc atc ttc cca cca tcc agt gag cag         432
Ala Asp Ala Ala Pro Thr Val Ser Ile Phe Pro Pro Ser Ser Glu Gln
        130                 135                 140

tta aca tct gga ggt gcc tca gtc gtg tgc ttc ttg aac aac ttc tac         480
Leu Thr Ser Gly Gly Ala Ser Val Val Cys Phe Leu Asn Asn Phe Tyr
145                 150                 155                 160

ccc aaa gac atc aat gtc aag tgg aag att gat ggc agt gaa cga caa         528
Pro Lys Asp Ile Asn Val Lys Trp Lys Ile Asp Gly Ser Glu Arg Gln
                165                 170                 175

aat ggc gtc ctg aac agt tgg act gat cag gac agc aaa gac agc acc         576
Asn Gly Val Leu Asn Ser Trp Thr Asp Gln Asp Ser Lys Asp Ser Thr
            180                 185                 190

tac agc atg agc agc acc ctc acg ttg acc aag gac gag tat gaa cga         624
Tyr Ser Met Ser Ser Thr Leu Thr Leu Thr Lys Asp Glu Tyr Glu Arg
        195                 200                 205

cat aac agc tat acc tgt gag gcc act cac aag aca tca act tca ccc         672
His Asn Ser Tyr Thr Cys Glu Ala Thr His Lys Thr Ser Thr Ser Pro
        210                 215                 220

att gtc aag agc ttc aac agg aat gag tgt taa                             705
Ile Val Lys Ser Phe Asn Arg Asn Glu Cys
225                 230
```

<210> 4
<211> 234
<212> PRT
<213> Mus sp.

<400> 4

```
Met Val Phe Thr Pro Gln Ile Leu Gly Leu Met Leu Phe Trp Ile Ser
1               5                   10                  15

Ala Ser Arg Gly Asp Ile Val Leu Thr Gln Ser Pro Ala Thr Leu Ser
            20                  25                  30

Val Thr Pro Gly Asp Arg Val Ser Leu Ser Cys Arg Ala Ser Gln Ser
        35                  40                  45

Ile Ser Asn Tyr Leu His Trp Tyr Gln Gln Lys Ser His Glu Ser Pro
    50                  55                  60

Arg Leu Leu Ile Lys Tyr Ala Ser Gln Ser Ile Ser Gly Ile Pro Ser
65                  70                  75                  80

Arg Phe Ser Gly Ser Gly Ser Gly Thr Asp Phe Ile Leu Ser Phe Asn
                85                  90                  95
```

```
Ser Val Glu Thr Glu Asp Phe Gly Met Tyr Phe Cys Gln Gln Ser Asn
            100                 105                 110

Ser Trp Pro Phe Thr Phe Gly Ser Gly Thr Lys Leu Glu Ile Lys Arg
            115                 120                 125

Ala Asp Ala Ala Pro Thr Val Ser Ile Phe Pro Pro Ser Ser Glu Gln
            130                 135                 140

Leu Thr Ser Gly Gly Ala Ser Val Val Cys Phe Leu Asn Asn Phe Tyr
145             150                 155                 160

Pro Lys Asp Ile Asn Val Lys Trp Lys Ile Asp Gly Ser Glu Arg Gln
                165                 170                 175

Asn Gly Val Leu Asn Ser Trp Thr Asp Gln Asp Ser Lys Asp Ser Thr
            180                 185                 190

Tyr Ser Met Ser Ser Thr Leu Thr Leu Thr Lys Asp Glu Tyr Glu Arg
            195                 200                 205

His Asn Ser Tyr Thr Cys Glu Ala Thr His Lys Thr Ser Thr Ser Pro
            210                 215                 220

Ile Val Lys Ser Phe Asn Arg Asn Glu Cys
225                 230
```

<210> 5
<211> 442
<212> DNA
<213> Mus sp.

<220>
<221> CDS
<222> (29)..(442)

<400> 5

```
tgaaggagta gaaaactagt gtgcagat atg aac agg ctt act tcc tca ttg          52
                                   Met Asn Arg Leu Thr Ser Ser Leu
                                   1               5

ctg ctg ctg att gtc cct gca tat gtc ctg tcc cag gtt act ctg aaa        100
Leu Leu Leu Ile Val Pro Ala Tyr Val Leu Ser Gln Val Thr Leu Lys
        10              15              20

gag tct ggc cct ggg ata ttg cag ccc tcc cag acc ctc agt ctg act       148
Glu Ser Gly Pro Gly Ile Leu Gln Pro Ser Gln Thr Leu Ser Leu Thr
25              30              35                          40

tgt tct ttc tct ggg ttt tca ctg agc act tct ggt atg ggt gtg agc       196
Cys Ser Phe Ser Gly Phe Ser Leu Ser Thr Ser Gly Met Gly Val Ser
                45              50                  55

tgg att cgt cag cct tca gga aag ggt ctg gag tgg ctg gca cac att       244
Trp Ile Arg Gln Pro Ser Gly Lys Gly Leu Glu Trp Leu Ala His Ile
                60              65              70

tac tgg gat gat gac aag cgc tat aac cca tcc ctg aag agc cgg ctc       292
Tyr Trp Asp Asp Asp Lys Arg Tyr Asn Pro Ser Leu Lys Ser Arg Leu
            75              80              85

aca atc tcc aag gat acc tcc agc aac cag gta ttc ctc aag atc acc       340
Thr Ile Ser Lys Asp Thr Ser Ser Asn Gln Val Phe Leu Lys Ile Thr
        90              95              100

agt gtg gac act gca gat act gcc aca tac tac tgt gct cga gat tac       388
Ser Val Asp Thr Ala Asp Thr Ala Thr Tyr Tyr Cys Ala Arg Asp Tyr
105             110             115             120

tac gct agt agc ttt gac tac tgg ggc caa ggc acc act ctc aca gtc       436
Tyr Ala Ser Ser Phe Asp Tyr Trp Gly Gln Gly Thr Thr Leu Thr Val
                125             130             135

tcc tca                                                                442
Ser Ser
```

<210> 6
<211> 138
<212> PRT
<213> Mus sp.

<400> 6

```
Met Asn Arg Leu Thr Ser Ser Leu Leu Leu Leu Ile Val Pro Ala Tyr
1               5               10              15

Val Leu Ser Gln Val Thr Leu Lys Glu Ser Gly Pro Gly Ile Leu Gln
            20              25              30

Pro Ser Gln Thr Leu Ser Leu Thr Cys Ser Phe Ser Gly Phe Ser Leu
            35              40              45

Ser Thr Ser Gly Met Gly Val Ser Trp Ile Arg Gln Pro Ser Gly Lys
        50              55              60

Gly Leu Glu Trp Leu Ala His Ile Tyr Trp Asp Asp Asp Lys Arg Tyr
65              70              75              80

Asn Pro Ser Leu Lys Ser Arg Leu Thr Ile Ser Lys Asp Thr Ser Ser
                85              90              95

Asn Gln Val Phe Leu Lys Ile Thr Ser Val Asp Thr Ala Asp Thr Ala
            100             105             110

Thr Tyr Tyr Cys Ala Arg Asp Tyr Tyr Ala Ser Ser Phe Asp Tyr Trp
        115             120             125

Gly Gln Gly Thr Thr Leu Thr Val Ser Ser
        130             135
```

<210> 7
<211> 401
<212> DNA
<213> Mus sp.

<220>
<221> CDS
<222> (18)..(401)

<400> 7

```
tcagggaaag ctcgaag atg gtt ttc aca cct cag att ctt gga ctt atg          50
                    Met Val Phe Thr Pro Gln Ile Leu Gly Leu Met
                    1               5                   10

ctt ttc tgg att tca gcc tcc aga ggt gat att gtg cta act cag tct         98
Leu Phe Trp Ile Ser Ala Ser Arg Gly Asp Ile Val Leu Thr Gln Ser
            15                  20                  25

cca gcc acc ctg tct gtg act cca gga gat aga gtc agt ctt tcc tgc         146
Pro Ala Thr Leu Ser Val Thr Pro Gly Asp Arg Val Ser Leu Ser Cys
        30                  35                  40

agg gcc agt caa agt att agc aac tac cta cac tgg tat caa caa aaa         194
Arg Ala Ser Gln Ser Ile Ser Asn Tyr Leu His Trp Tyr Gln Gln Lys
    45                  50                  55

tca cat gag tct cca agg ctt ctc atc aag tat gct tcc cag tcc atc         242
Ser His Glu Ser Pro Arg Leu Leu Ile Lys Tyr Ala Ser Gln Ser Ile
60                  65                  70                  75

tct ggg atc ccc tcc agg ttc agt ggc agt gga tca ggg aca gat ttc        290
Ser Gly Ile Pro Ser Arg Phe Ser Gly Ser Gly Ser Gly Thr Asp Phe
                80                  85                  90

att ctc agt ttc aac agt gtg gag act gaa gat ttt gga atg tat ttc        338
Ile Leu Ser Phe Asn Ser Val Glu Thr Glu Asp Phe Gly Met Tyr Phe
                95                  100                 105

tgt caa cag agt aac agc tgg cct ttc acg ttc ggc tcg ggg aca aaa        386
Cys Gln Gln Ser Asn Ser Trp Pro Phe Thr Phe Gly Ser Gly Thr Lys
            110                 115                 120

ttg gaa ata aaa cgg                                                     401
Leu Glu Ile Lys Arg
    125
```

<210> 8
<211> 128
<212> PRT
<213> Mus sp.

<400> 8

```
Met Val Phe Thr Pro Gln Ile Leu Gly Leu Met Leu Phe Trp Ile Ser
1               5                   10                  15

Ala Ser Arg Gly Asp Ile Val Leu Thr Gln Ser Pro Ala Thr Leu Ser
            20                  25                  30

Val Thr Pro Gly Asp Arg Val Ser Leu Ser Cys Arg Ala Ser Gln Ser
            35                  40                  45

Ile Ser Asn Tyr Leu His Trp Tyr Gln Gln Lys Ser His Glu Ser Pro
        50                  55                  60

Arg Leu Leu Ile Lys Tyr Ala Ser Gln Ser Ile Ser Gly Ile Pro Ser
65                  70                  75                  80

Arg Phe Ser Gly Ser Gly Ser Gly Thr Asp Phe Ile Leu Ser Phe Asn
                85                  90                  95

Ser Val Glu Thr Glu Asp Phe Gly Met Tyr Phe Cys Gln Gln Ser Asn
            100                 105                 110

Ser Trp Pro Phe Thr Phe Gly Ser Gly Thr Lys Leu Glu Ile Lys Arg
            115                 120                 125
```

<210> 9
<211> 7
<212> PRT
<213> Mus sp.

<400> 9

```
Thr Ser Gly Met Gly Val Ser
1               5
```

<210> 10
<211> 16
<212> PRT
<213> Mus sp.

<400> 10

```
His Ile Tyr Trp Asp Asp Asp Lys Arg Tyr Asn Pro Ser Leu Lys Ser
1               5                   10                  15
```

<210> 11
<211> 9
<212> PRT
<213> Mus sp.

<400> 11

Asp Tyr Tyr Ala Ser Ser Phe Asp Tyr
1               5

<210> 12
<211> 11
<212> PRT
<213> Mus sp.

<400> 12

Arg Ala Ser Gln Ser Ile Ser Asn Tyr Leu His
1               5                   10

<210> 13
<211> 7
<212> PRT
<213> Mus sp.

<400> 13

Tyr Ala Ser Gln Ser Ile Ser
1               5

<210> 14
<211> 9
<212> PRT
<213> Mus sp.

<400> 14

Gln Gln Ser Asn Ser Trp Pro Phe Thr
1               5

<210> 15
<211> 1395
<212> DNA
<213> Mus sp.

<220>
<221> CDS
<222> (1)..(1395)

<400> 15

```
atg gac tcc agg ctc aat tta gtt ttc ctt gtc ctt att tta aaa ggt     48
Met Asp Ser Arg Leu Asn Leu Val Phe Leu Val Leu Ile Leu Lys Gly
1               5                   10                  15

gtc cag tgt gat gtg cag ctg gtg gag tct ggg gga ggc tta gtg cag     96
Val Gln Cys Asp Val Gln Leu Val Glu Ser Gly Gly Gly Leu Val Gln
                20                  25                  30

cct gga ggg tcc cgg aaa ctc tcc tgt gca gcc tct gga ttc act ttc    144
Pro Gly Gly Ser Arg Lys Leu Ser Cys Ala Ala Ser Gly Phe Thr Phe
```

```
                35                      40                      45              .

        agt aac ttt gga atg cac tgg gtt cgt cag gct cca gag aag gga ctg        192
        Ser Asn Phe Gly Met His Trp Val Arg Gln Ala Pro Glu Lys Gly Leu
            50                      55                      60

        gag tgg gtc gca tac att agt agt ggc agt agt acc atc tac tat gca        240
        Glu Trp Val Ala Tyr Ile Ser Ser Gly Ser Ser Thr Ile Tyr Tyr Ala
        65                      70                      75                      80

        gac aca gtg aag ggc cga ttc acc atc tcc aga gac aat ccc aag aac        288
        Asp Thr Val Lys Gly Arg Phe Thr Ile Ser Arg Asp Asn Pro Lys Asn
                            85                      90                      95

        acc ctg ttc ctg caa atg acc agt cta agg tct gag gac acg gcc atg        336
        Thr Leu Phe Leu Gln Met Thr Ser Leu Arg Ser Glu Asp Thr Ala Met
                        100                     105                     110

        tat tac tgt gca aga tat gat tac gac gat gat tac tat gct atg gaa        384
        Tyr Tyr Cys Ala Arg Tyr Asp Tyr Asp Asp Asp Tyr Tyr Ala Met Glu
                        115                     120                     125

        tac tgg ggt caa gga acc tca gtc acc gtc tcc tca gcc aaa acg aca        432
        Tyr Trp Gly Gln Gly Thr Ser Val Thr Val Ser Ser Ala Lys Thr Thr
                130                     135                     140

        ccc cca tct gtc tat cca ctg gcc cct gga tct gct gcc caa act aac        480
        Pro Pro Ser Val Tyr Pro Leu Ala Pro Gly Ser Ala Ala Gln Thr Asn
        145                     150                     155                     160

        tcc atg gtg acc ctg gga tgc ctg gtc aag ggc tat ttc cct gag cca        528
        Ser Met Val Thr Leu Gly Cys Leu Val Lys Gly Tyr Phe Pro Glu Pro
                        165                     170                     175

        gtg aca gtg acc tgg aac tct gga tcc ctg tcc agc ggt gtg cac acc        576
        Val Thr Val Thr Trp Asn Ser Gly Ser Leu Ser Ser Gly Val His Thr
                        180                     185                     190

        ttc cca gct gtc ctg cag tct gac ctc tac act ctg agc agc tca gtg        624
        Phe Pro Ala Val Leu Gln Ser Asp Leu Tyr Thr Leu Ser Ser Ser Val
                        195                     200                     205

        act gtc ccc tcc agc acc tgg ccc agc gag acc gtc acc tgc aac gtt        672
        Thr Val Pro Ser Ser Thr Trp Pro Ser Glu Thr Val Thr Cys Asn Val
                210                     215                     220

        gcc cac ccg gcc agc agc acc aag gtg gac aag aaa att gtg ccc agg        720
        Ala His Pro Ala Ser Ser Thr Lys Val Asp Lys Lys Ile Val Pro Arg
        225                     230                     235                     240

        gat tgt ggt tgt aag cct tgc ata tgt aca gtc cca gaa gta tca tct        768
        Asp Cys Gly Cys Lys Pro Cys Ile Cys Thr Val Pro Glu Val Ser Ser
                        245                     250                     255

        gtc ttc atc ttc ccc cca aag ccc aag gat gtg ctc acc att act ctg        816
        Val Phe Ile Phe Pro Pro Lys Pro Lys Asp Val Leu Thr Ile Thr Leu
                        260                     265                     270

        act cct aag gtc acg tgt gtt gtg gta gac atc agc aag gat gat ccc        864
        Thr Pro Lys Val Thr Cys Val Val Val Asp Ile Ser Lys Asp Asp Pro
                275                     280                     285
```

```
gag gtc cag ttc agc tgg ttt gta gat gat gtg gag gtg cac aca gct        912
Glu Val Gln Phe Ser Trp Phe Val Asp Asp Val Glu Val His Thr Ala
    290             295             300

cag acg caa ccc cgg gag gag cag ttc aac agc act ttc cgc tca gtc        960
Gln Thr Gln Pro Arg Glu Glu Gln Phe Asn Ser Thr Phe Arg Ser Val
305             310             315             320

agt gaa ctt ccc atc atg cac cag gac tgg ctc aat ggc aag gag ttc       1008
Ser Glu Leu Pro Ile Met His Gln Asp Trp Leu Asn Gly Lys Glu Phe
            325             330             335

aaa tgc agg gtc aac agt gca gct ttc cct gcc ccc atc gag aaa acc       1056
Lys Cys Arg Val Asn Ser Ala Ala Phe Pro Ala Pro Ile Glu Lys Thr
            340             345             350

atc tcc aaa acc aaa ggc aga ccg aag gct cca cag gtg tac acc att       1104
Ile Ser Lys Thr Lys Gly Arg Pro Lys Ala Pro Gln Val Tyr Thr Ile
            355             360             365

cca cct ccc aag gag cag atg gcc aag gat aaa gtc agt ctg acc tgc       1152
Pro Pro Pro Lys Glu Gln Met Ala Lys Asp Lys Val Ser Leu Thr Cys
370             375             380

atg ata aca gac ttc ttc cct gaa gac att act gtg gag tgg cag tgg       1200
Met Ile Thr Asp Phe Phe Pro Glu Asp Ile Thr Val Glu Trp Gln Trp
385             390             395             400

aat ggg cag cca gcg gag aac tac aag aac act cag ccc atc atg gac       1248
Asn Gly Gln Pro Ala Glu Asn Tyr Lys Asn Thr Gln Pro Ile Met Asp
            405             410             415

aca gat ggc tct tac ttc gtc tac agc aag ctc aat gtg cag aag agc       1296
Thr Asp Gly Ser Tyr Phe Val Tyr Ser Lys Leu Asn Val Gln Lys Ser
            420             425             430

aac tgg gag gca gga aat act ttc acc tgc tct gtg tta cat gag ggc       1344
Asn Trp Glu Ala Gly Asn Thr Phe Thr Cys Ser Val Leu His Glu Gly
            435             440             445

ctg cac aac cac cat act gag aag agc ctc tcc cac tct cct ggt aag       1392
Leu His Asn His His Thr Glu Lys Ser Leu Ser His Ser Pro Gly Lys
    450             455             460

tga                                                                    1395
```

<210> 16
<211> 464
<212> PRT
<213> Mus sp.

<400> 16

```
Met Asp Ser Arg Leu Asn Leu Val Phe Leu Val Leu Ile Leu Lys Gly
1               5               10              15

Val Gln Cys Asp Val Gln Leu Val Glu Ser Gly Gly Gly Leu Val Gln
            20              25              30
```

42

```
Pro Gly Gly Ser Arg Lys Leu Ser Cys Ala Ala Ser Gly Phe Thr Phe
        35                  40                  45

Ser Asn Phe Gly Met His Trp Val Arg Gln Ala Pro Glu Lys Gly Leu
    50                  55                  60

Glu Trp Val Ala Tyr Ile Ser Ser Gly Ser Ser Thr Ile Tyr Tyr Ala
65                  70                  75                      80

Asp Thr Val Lys Gly Arg Phe Thr Ile Ser Arg Asp Asn Pro Lys Asn
                85                  90                  95

Thr Leu Phe Leu Gln Met Thr Ser Leu Arg Ser Glu Asp Thr Ala Met
            100                 105                 110

Tyr Tyr Cys Ala Arg Tyr Asp Tyr Asp Asp Asp Tyr Tyr Ala Met Glu
        115                 120                 125

Tyr Trp Gly Gln Gly Thr Ser Val Thr Val Ser Ser Ala Lys Thr Thr
    130                 135                 140

Pro Pro Ser Val Tyr Pro Leu Ala Pro Gly Ser Ala Ala Gln Thr Asn
145                 150                 155                 160

Ser Met Val Thr Leu Gly Cys Leu Val Lys Gly Tyr Phe Pro Glu Pro
            165                 170                 175

Val Thr Val Thr Trp Asn Ser Gly Ser Leu Ser Ser Gly Val His Thr
            180                 185                 190

Phe Pro Ala Val Leu Gln Ser Asp Leu Tyr Thr Leu Ser Ser Ser Val
        195                 200                 205

Thr Val Pro Ser Ser Thr Trp Pro Ser Glu Thr Val Thr Cys Asn Val
    210                 215                 220

Ala His Pro Ala Ser Ser Thr Lys Val Asp Lys Lys Ile Val Pro Arg
225                 230                 235                 240

Asp Cys Gly Cys Lys Pro Cys Ile Cys Thr Val Pro Glu Val Ser Ser
            245                 250                 255

Val Phe Ile Phe Pro Pro Lys Pro Lys Asp Val Leu Thr Ile Thr Leu
            260                 265                 270
```

```
Thr Pro Lys Val Thr Cys Val Val Val Asp Ile Ser Lys Asp Asp Pro
        275             280             285

Glu Val Gln Phe Ser Trp Phe Val Asp Asp Val Glu Val His Thr Ala
    290             295             300

Gln Thr Gln Pro Arg Glu Glu Gln Phe Asn Ser Thr Phe Arg Ser Val
305             310             315             320

Ser Glu Leu Pro Ile Met His Gln Asp Trp Leu Asn Gly Lys Glu Phe
            325             330             335

Lys Cys Arg Val Asn Ser Ala Ala Phe Pro Ala Pro Ile Glu Lys Thr
            340             345             350

Ile Ser Lys Thr Lys Gly Arg Pro Lys Ala Pro Gln Val Tyr Thr Ile
    355             360             365

Pro Pro Pro Lys Glu Gln Met Ala Lys Asp Lys Val Ser Leu Thr Cys
    370             375             380

Met Ile Thr Asp Phe Phe Pro Glu Asp Ile Thr Val Glu Trp Gln Trp
385             390             395             400

Asn Gly Gln Pro Ala Glu Asn Tyr Lys Asn Thr Gln Pro Ile Met Asp
            405             410             415

Thr Asp Gly Ser Tyr Phe Val Tyr Ser Lys Leu Asn Val Gln Lys Ser
            420             425             430

Asn Trp Glu Ala Gly Asn Thr Phe Thr Cys Ser Val Leu His Glu Gly
            435             440             445

Leu His Asn His His Thr Glu Lys Ser Leu Ser His Ser Pro Gly Lys
    450             455             460
```

<210> 17
<211> 705
<212> DNA
<213> Mus sp.

<220>
<221> CDS
<222> (1)..(705)

<400> 17

atg gcc tgg att tca ctt ata ctc tct ctc ctg .gct ctc agc tca ggg        48

```
Met Ala Trp Ile Ser Leu Ile Leu Ser Leu Leu Ala Leu Ser Ser Gly
1               5               10              15

gcc att tcc cag gct gtt gtg act cag gaa tct gca ctc acc aca tca      96
Ala Ile Ser Gln Ala Val Val Thr Gln Glu Ser Ala Leu Thr Thr Ser
                20                  25                  30

cct ggt gaa aca gtc aca ctc act tgt cgc tca agt act ggg gct gtt     144
Pro Gly Glu Thr Val Thr Leu Thr Cys Arg Ser Ser Thr Gly Ala Val
            35                  40                  45

aca act agt aac tat gcc aac tgg gtc caa gaa aaa cca gat cat tta     192
Thr Thr Ser Asn Tyr Ala Asn Trp Val Gln Glu Lys Pro Asp His Leu
        50                  55                  60

ttc act ggt cta ata ggt ggt acc aac aac cga gct cca ggt gtt cct     240
Phe Thr Gly Leu Ile Gly Gly Thr Asn Asn Arg Ala Pro Gly Val Pro
65                  70                  75                  80

gcc aga ttc tca ggc tcc ctg att gga gac aag gct gcc ctc acc atc     288
Ala Arg Phe Ser Gly Ser Leu Ile Gly Asp Lys Ala Ala Leu Thr Ile
                85                  90                  95

aca ggg gca cag act gag gat gag gca ata tat ttc tgt gct cta tgg     336
Thr Gly Ala Gln Thr Glu Asp Glu Ala Ile Tyr Phe Cys Ala Leu Trp
            100                 105                 110

tac agc aac cat tgg gtg ttc ggt gga gga acc aaa ctg act gtc cta     384
Tyr Ser Asn His Trp Val Phe Gly Gly Gly Thr Lys Leu Thr Val Leu
        115                 120                 125

ggc cag ccc aag tct tcg cca tca gtc acc ctg ttt cca cct tcc tct     432
Gly Gln Pro Lys Ser Ser Pro Ser Val Thr Leu Phe Pro Pro Ser Ser
        130                 135                 140

gaa gag ctc gag act aac aag gcc aca ctg gtg tgt acg atc act gat     480
Glu Glu Leu Glu Thr Asn Lys Ala Thr Leu Val Cys Thr Ile Thr Asp
145                 150                 155                 160

ttc tac cca ggt gtg gtg aca gtg gac tgg aag gta gat ggt acc cct     528
Phe Tyr Pro Gly Val Val Thr Val Asp Trp Lys Val Asp Gly Thr Pro
                165                 170                 175

gtc act cag ggt atg gag aca acc cag cct tcc aaa cag agc aac aac     576
Val Thr Gln Gly Met Glu Thr Thr Gln Pro Ser Lys Gln Ser Asn Asn
            180                 185                 190

aag tac atg gct agc agc tac ctg acc ctg aca gca aga gca tgg gaa     624
Lys Tyr Met Ala Ser Ser Tyr Leu Thr Leu Thr Ala Arg Ala Trp Glu
        195                 200                 205

agg cat agc agt tac agc tgc cag gtc act cat gaa ggt cac act gtg     672
Arg His Ser Ser Tyr Ser Cys Gln Val Thr His Glu Gly His Thr Val
        210                 215                 220

gag aag agt ttg tcc cgt gct gac tgt tcc tag                         705
Glu Lys Ser Leu Ser Arg Ala Asp Cys Ser
225                 230
```

<210> 18

46

<211> 234
<212> PRT
<213> Mus sp.

<400> 18

```
Met Ala Trp Ile Ser Leu Ile Leu Ser Leu Leu Ala Leu Ser Ser Gly
1               5                   10                  15

Ala Ile Ser Gln Ala Val Val Thr Gln Glu Ser Ala Leu Thr Thr Ser
            20                  25                  30

Pro Gly Glu Thr Val Thr Leu Thr Cys Arg Ser Ser Thr Gly Ala Val
            35                  40                  45

Thr Thr Ser Asn Tyr Ala Asn Trp Val Gln Glu Lys Pro Asp His Leu
        50                  55                  60

Phe Thr Gly Leu Ile Gly Gly Thr Asn Asn Arg Ala Pro Gly Val Pro
65                  70                  75                  80

Ala Arg Phe Ser Gly Ser Leu Ile Gly Asp Lys Ala Ala Leu Thr Ile
                85                  90                  95

Thr Gly Ala Gln Thr Glu Asp Glu Ala Ile Tyr Phe Cys Ala Leu Trp
            100                 105                 110

Tyr Ser Asn His Trp Val Phe Gly Gly Gly Thr Lys Leu Thr Val Leu
        115                 120                 125

Gly Gln Pro Lys Ser Ser Pro Ser Val Thr Leu Phe Pro Pro Ser Ser
    130                 135                 140

Glu Glu Leu Glu Thr Asn Lys Ala Thr Leu Val Cys Thr Ile Thr Asp
145                 150                 155                 160

Phe Tyr Pro Gly Val Val Thr Val Asp Trp Lys Val Asp Gly Thr Pro
                165                 170                 175

Val Thr Gln Gly Met Glu Thr Thr Gln Pro Ser Lys Gln Ser Asn Asn
            180                 185                 190

Lys Tyr Met Ala Ser Ser Tyr Leu Thr Leu Thr Ala Arg Ala Trp Glu
        195                 200                 205

Arg His Ser Ser Tyr Ser Cys Gln Val Thr His Glu Gly His Thr Val
    210                 215                 220
```

```
                    Glu Lys Ser Leu Ser Arg Ala Asp Cys Ser
                    225                 230
```

```
<210> 19
<211> 420
<212> DNA
<213> Mus sp.

<220>
<221> CDS
<222> (1)..(420)

<400> 19
```

```
atg gac tcc agg ctc aat tta gtt ttc ctt gtc ctt att tta aaa ggt     48
Met Asp Ser Arg Leu Asn Leu Val Phe Leu Val Leu Ile Leu Lys Gly
1               5                   10                  15

gtc cag tgt gat gtg cag ctg gtg gag tct ggg gga ggc tta gtg cag     96
Val Gln Cys Asp Val Gln Leu Val Glu Ser Gly Gly Gly Leu Val Gln
                20                  25                  30

cct gga ggg tcc cgg aaa ctc tcc tgt gca gcc tct gga ttc act ttc    144
Pro Gly Gly Ser Arg Lys Leu Ser Cys Ala Ala Ser Gly Phe Thr Phe
            35                  40                  45

agt aac ttt gga atg cac tgg gtt cgt cag gct cca gag aag gga ctg    192
Ser Asn Phe Gly Met His Trp Val Arg Gln Ala Pro Glu Lys Gly Leu
        50                  55                  60

gag tgg gtc gca tac att agt agt ggc agt agt acc atc tac tat gca    240
Glu Trp Val Ala Tyr Ile Ser Ser Gly Ser Ser Thr Ile Tyr Tyr Ala
65                  70                  75                  80

gac aca gtg aag ggc cga ttc acc atc tcc aga gac aat ccc aag aac    288
Asp Thr Val Lys Gly Arg Phe Thr Ile Ser Arg Asp Asn Pro Lys Asn
                    85                  90                  95

acc ctg ttc ctg caa atg acc agt cta agg tct gag gac acg gcc atg    336
Thr Leu Phe Leu Gln Met Thr Ser Leu Arg Ser Glu Asp Thr Ala Met
            100                 105                 110

tat tac tgt gca aga tat gat tac gac gat gat tac tat gct atg gaa    384
Tyr Tyr Cys Ala Arg Tyr Asp Tyr Asp Asp Asp Tyr Tyr Ala Met Glu
        115                 120                 125

tac tgg ggt caa gga acc tca gtc acc gtc tcc tca                    420
Tyr Trp Gly Gln Gly Thr Ser Val Thr Val Ser Ser
    130                 135                 140
```

```
<210> 20
<211> 140
<212> PRT
<213> Mus sp.

<400> 20
```

```
Met Asp Ser Arg Leu Asn Leu Val Phe Leu Val Leu Ile Leu Lys Gly

1                   5                   10                  15


Val Gln Cys Asp Val Gln Leu Val Glu Ser Gly Gly Gly Leu Val Gln
             20                  25                  30


Pro Gly Gly Ser Arg Lys Leu Ser Cys Ala Ala Ser Gly Phe Thr Phe
             35                  40                  45


Ser Asn Phe Gly Met His Trp Val Arg Gln Ala Pro Glu Lys Gly Leu
         50              55              60


Glu Trp Val Ala Tyr Ile Ser Ser Gly Ser Ser Thr Ile Tyr Tyr Ala
65              70                  75                  80


Asp Thr Val Lys Gly Arg Phe Thr Ile Ser Arg Asp Asn Pro Lys Asn
                 85                  90                  95


Thr Leu Phe Leu Gln Met Thr Ser Leu Arg Ser Glu Asp Thr Ala Met
             100                 105                 110


Tyr Tyr Cys Ala Arg Tyr Asp Tyr Asp Asp Asp Tyr Tyr Ala Met Glu
         115                 120                 125


Tyr Trp Gly Gln Gly Thr Ser Val Thr Val Ser Ser
         130                 135                 140
```

<210> 21
<211> 412
<212> DNA
<213> Mus sp.

<220>
<221> CDS
<222> (29)..(12)

<400> 21

```
tgaaaagaat agacctggtt tgtgaatt atg gcc tgg att tca ctt ata ctc          52
                                 Met Ala Trp Ile Ser Leu Ile Leu
                                 1                   5

tct ctc ctg gct ctc agc tca ggg gcc att tcc cag gct gtt gtg act        100
Ser Leu Leu Ala Leu Ser Ser Gly Ala Ile Ser Gln Ala Val Val Thr
    10                  15                  20

cag gaa tct gca ctc acc aca tca cct ggt gaa aca gtc aca ctc act        148
Gln Glu Ser Ala Leu Thr Thr Ser Pro Gly Glu Thr Val Thr Leu Thr
25                  30                  35                  40

tgt cgc tca agt act ggg gct gtt aca act agt aac tat gcc aac tgg        196
Cys Arg Ser Ser Thr Gly Ala Val Thr Thr Ser Asn Tyr Ala Asn Trp
                45                  50                  55

gtc caa gaa aaa cca gat cat tta ttc act ggt cta ata ggt ggt acc        244
Val Gln Glu Lys Pro Asp His Leu Phe Thr Gly Leu Ile Gly Gly Thr
                60                  65                  70

aac aac cga gct cca ggt gtt cct gcc aga ttc tca ggc tcc ctg att        292
Asn Asn Arg Ala Pro Gly Val Pro Ala Arg Phe Ser Gly Ser Leu Ile
            75                  80                  85

gga gac aag gct gcc ctc acc atc aca ggg gca cag act gag gat gag        340
Gly Asp Lys Ala Ala Leu Thr Ile Thr Gly Ala Gln Thr Glu Asp Glu
        90                  95                  100

gca ata tat ttc tgt gct cta tgg tac agc aac cat tgg gtg ttc ggt        388
Ala Ile Tyr Phe Cys Ala Leu Trp Tyr Ser Asn His Trp Val Phe Gly
105                 110                 115                 120

gga gga acc aaa ctg act gtc cta                                         412
Gly Gly Thr Lys Leu Thr Val Leu
                125
```

<210> 22
<211> 128
<212> PRT
<213> Mus sp.

<400> 22

```
        Met Ala Trp Ile Ser Leu Ile Leu Ser Leu Leu Ala Leu Ser Ser Gly
        1               5                   10                  15


        Ala Ile Ser Gln Ala Val Val Thr Gln Glu Ser Ala Leu Thr Thr Ser
                        20                  25                  30


        Pro Gly Glu Thr Val Thr Leu Thr Cys Arg Ser Ser Thr Gly Ala Val
                        35                  40                  45


        Thr Thr Ser Asn Tyr Ala Asn Trp Val Gln Glu Lys Pro Asp His Leu
                50                  55                  60


        Phe Thr Gly Leu Ile Gly Gly Thr Asn Asn Arg Ala Pro Gly Val Pro
        65                  70                  75                  80


        Ala Arg Phe Ser Gly Ser Leu Ile Gly Asp Lys Ala Ala Leu Thr Ile
                        85                  90                  95


        Thr Gly Ala Gln Thr Glu Asp Glu Ala Ile Tyr Phe Cys Ala Leu Trp
                        100                 105                 110


        Tyr Ser Asn His Trp Val Phe Gly Gly Gly Thr Lys Leu Thr Val Leu
                115                 120                 125
```

<210> 23
<211> 5
<212> PRT
<213> Mus sp.

<400> 23

```
                        Asn Phe Gly Met His
                        1               5
```

<210> 24
<211> 17
<212> PRT
<213> Mus sp.

<400> 24

```
        Tyr Ile Ser Ser Gly Ser Ser Thr Ile Tyr Tyr Ala Asp Thr Val Lys
        1               5                   10                  15


        Gly
```

<210> 25
<211> 12
<212> PRT

<213> Mus sp.

<400> 25

```
                    Tyr Asp Tyr Asp Asp Asp Tyr Tyr Ala Met Glu Tyr
                    1               5                   10
```

<210> 26
<211> 14
<212> PRT
<213> Mus sp.

<400> 26

```
              Arg Ser Ser Thr Gly Ala Val Thr Thr Ser Asn Tyr Ala Asn
              1               5                   10
```

<210> 27
<211> 7
<212> PRT
<213> Mus sp.

<400> 27

```
                        Gly Thr Asn Asn Arg Ala Pro
                        1               5
```

<210> 28
<211> 9
<212> PRT
<213> Mus sp.

<400> 28

```
                    Ala Leu Trp Tyr Ser Asn His Trp Val
                    1               5
```

<210> 29
<211> 321
<212> DNA
<213> Artificial

<220>
<223> Séquence humanisée

<220>
<221> CDS
<222> (1)..(321)

<400> 29

```
cag tct gcc ctg act cag tcc cca gca act ctc tct gtg tct ccc ggc        48
Gln Ser Ala Leu Thr Gln Ser Pro Ala Thr Leu Ser Val Ser Pro Gly
1               5                   10                  15

caa tct gtg agc ctg tcc tgt cgg gca agc cag tct att tcc aat tat        96
Gln Ser Val Ser Leu Ser Cys Arg Ala Ser Gln Ser Ile Ser Asn Tyr
            20                  25              .   30

ctg cac tgg tat cag caa cac cca ggc gag agc cca cgg ctc ctg atc       144
Leu His Trp Tyr Gln Gln His Pro Gly Glu Ser Pro Arg Leu Leu Ile
        35                  40                  45

aaa tac gcc tct caa tcc att tct ggc tcc aac aga cgc ttc tct ggc       192
Lys Tyr Ala Ser Gln Ser Ile Ser Gly Ser Asn Arg Arg Phe Ser Gly
    50              .   55                  60

agc ggc tcc ggc acc gat ttc att ctc agc ttt aat agc gtt cag gca       240
Ser Gly Ser Gly Thr Asp Phe Ile Leu Ser Phe Asn Ser Val Gln Ala
65                  70                  75                  80

gag gat ttt ggg atg tac ttc tgt caa cag agc aac tct tgg cct ttt       288
Glu Asp Phe Gly Met Tyr Phe Cys Gln Gln Ser Asn Ser Trp Pro Phe
                85                  90                  95          .

acc ttc gga agc ggg act aaa ctg acc gtt ctg                           321
Thr Phe Gly Ser Gly Thr Lys Leu Thr Val Leu
            100                 105
```

<210> 30
<211> 107
<212> PRT
<213> Artificial

<220>
<223> Synthetic Construct

<400> 30

```
Gln Ser Ala Leu Thr Gln Ser Pro Ala Thr Leu Ser Val Ser Pro Gly
1               5               10              15

Gln Ser Val Ser Leu Ser Cys Arg Ala Ser Gln Ser Ile Ser Asn Tyr
        20              25              30

Leu His Trp Tyr Gln Gln His Pro Gly Glu Ser Pro Arg Leu Leu Ile
        35              40              45

Lys Tyr Ala Ser Gln Ser Ile Ser Gly Ser Asn Arg Arg Phe Ser Gly
    50              55              60

Ser Gly Ser Gly Thr Asp Phe Ile Leu Ser Phe Asn Ser Val Gln Ala
65              70              75              80

Glu Asp Phe Gly Met Tyr Phe Cys Gln Gln Ser Asn Ser Trp Pro Phe
            85              90              95

Thr Phe Gly Ser Gly Thr Lys Leu Thr Val Leu
            100             105
```

<210> 31
<211> 321
<212> DNA
<213> Artificial

<220>
<223> Séquence humanisée

<220>
<221> CDS
<222> (1)..(321)

<400> 31

```
caa tct gca ctg act caa tct cca gca aca ctg tcc gtt tct cct gga    48
Gln Ser Ala Leu Thr Gln Ser Pro Ala Thr Leu Ser Val Ser Pro Gly
1               5                   10                  15

caa tcc gtg agc ctc agc tgt agg gca tcc cag tcc att agc aac tat    96
Gln Ser Val Ser Leu Ser Cys Arg Ala Ser Gln Ser Ile Ser Asn Tyr
                20                  25                  30

ctc cac tgg tac cag cag cac cca gga gaa agc cct cgc ctg ctg ata   144
Leu His Trp Tyr Gln Gln His Pro Gly Glu Ser Pro Arg Leu Leu Ile
            35                  40                  45

aaa tac gca agc cag tct att agc ggc agc aac cgg cgc ttc agc ggg   192
Lys Tyr Ala Ser Gln Ser Ile Ser Gly Ser Asn Arg Arg Phe Ser Gly
        50                  55                  60

tcc ggc tcc ggg aca gac ttt atc ctg tct ttt agc agc gtg caa gcc   240
Ser Gly Ser Gly Thr Asp Phe Ile Leu Ser Phe Ser Ser Val Gln Ala

65                  70                  75                  80

gaa gac ttt ggc atg tac ttc tgc cag cag tct aac aat tgg cca ttc   288
Glu Asp Phe Gly Met Tyr Phe Cys Gln Gln Ser Asn Asn Trp Pro Phe
                85                  90                  95

acc ttc gga tcc ggc aca aag ctg acc gtg ctg                        321
Thr Phe Gly Ser Gly Thr Lys Leu Thr Val Leu
            100                 105
```

<210> 32
<211> 107
<212> PRT
<213> Artificial

<220>
<223> Synthetic Construct

<400> 32

```
Gln Ser Ala Leu Thr Gln Ser Pro Ala Thr Leu Ser Val Ser Pro Gly
1               5               10              15

Gln Ser Val Ser Leu Ser Cys Arg Ala Ser Gln Ser Ile Ser Asn Tyr
            20              25              30

Leu His Trp Tyr Gln Gln His Pro Gly Glu Ser Pro Arg Leu Leu Ile
            35              40              45

Lys Tyr Ala Ser Gln Ser Ile Ser Gly Ser Asn Arg Arg Phe Ser Gly
        50              55              60

Ser Gly Ser Gly Thr Asp Phe Ile Leu Ser Phe Ser Ser Val Gln Ala
65              70              75              80

Glu Asp Phe Gly Met Tyr Phe Cys Gln Gln Ser Asn Asn Trp Pro Phe
                85              90              95

Thr Phe Gly Ser Gly Thr Lys Leu Thr Val Leu
            100             105
```

<210> 33
<211> 342
<212> DNA
<213> Artificial

<220>
<223> Séquence humanisée

<220>
<221> CDS
<222> (1)..(342)

<400> 33

```
cag gtt act ctg aaa gaa tct ggc cca act ctg gtt aag cct aca cag      48
Gln Val Thr Leu Lys Glu Ser Gly Pro Thr Leu Val Lys Pro Thr Gln
1               5               10              15

acc ctg tcc ctc acc tgt tct ttc tcc ggc ttt agc ctg agc aca agc      96
Thr Leu Ser Leu Thr Cys Ser Phe Ser Gly Phe Ser Leu Ser Thr Ser
            20              25              30

gga atg ggc gtc agc tgg atc aga caa cca ccc ggc aag ggc ctg gag     144
Gly Met Gly Val Ser Trp Ile Arg Gln Pro Pro Gly Lys Gly Leu Glu
        35              40              45

tgg ctg gcc cac atc tat tgg gat gat gat aag agg tat aac cct tcc     192
Trp Leu Ala His Ile Tyr Trp Asp Asp Asp Lys Arg Tyr Asn Pro Ser
    50              55              60

ctg aaa tcc aga ctc aca att tcc aag gac acc tcc agc aac cag gtc     240
Leu Lys Ser Arg Leu Thr Ile Ser Lys Asp Thr Ser Ser Asn Gln Val
65              70              75              80

ttc ctg aag ata acc tct gtt gat cca gtg gac acc gca act tac tac     288
Phe Leu Lys Ile Thr Ser Val Asp Pro Val Asp Thr Ala Thr Tyr Tyr
            85              90              95

tgc gcc cgc gac tac tat gcc agc tct ttt gac tac tgg ggg cag ggc     336
Cys Ala Arg Asp Tyr Tyr Ala Ser Ser Phe Asp Tyr Trp Gly Gln Gly
        100             105             110

aca ctc                                                              342
Thr Leu
```

<210> 34
<211> 114
<212> PRT
<213> Artificial

<220>
<223> Synthetic Construct

<400> 34

```
Gln Val Thr Leu Lys Glu Ser Gly Pro Thr Leu Val Lys Pro Thr Gln
1               5               10              15

Thr Leu Ser Leu Thr Cys Ser Phe Ser Gly Phe Ser Leu Ser Thr Ser
            20              25              30

Gly Met Gly Val Ser Trp Ile Arg Gln Pro Pro Gly Lys Gly Leu Glu
        35              40              45

Trp Leu Ala His Ile Tyr Trp Asp Asp Asp Lys Arg Tyr Asn Pro Ser
    50              55              60
```

```
    Leu Lys Ser Arg Leu Thr Ile Ser Lys Asp Thr Ser Ser Asn Gln Val
    65              70              75                  80


    Phe Leu Lys Ile Thr Ser Val Asp Pro Val Asp Thr Ala Thr Tyr Tyr
                    85                  90                  95
                                        .

    Cys Ala Arg Asp Tyr Tyr Ala Ser Ser Phe Asp Tyr Trp Gly Gln Gly
                100                 105                 110


    Thr Leu
```

<210> 35
<211> 342
<212> DNA
<213> Artificial

<220>
<223> Séquence humanisée

<220>
<221> CDS
<222> (1)..(342)

<400> 35

```
    cag gtg act ctc aaa gaa tct ggc cct aca ctg gtg aaa ccc act cag        48
    Gln Val Thr Leu Lys Glu Ser Gly Pro Thr Leu Val Lys Pro Thr Gln
    1               5                   10                  15

    aca ctc agc ctg acc tgc tcc ttc agc ggc ttc tct ctg agc act tcc        96
    Thr Leu Ser Leu Thr Cys Ser Phe Ser Gly Phe Ser Leu Ser Thr Ser
                20                  25                  30

    gga ctc gga gtg agc tgg att cgc caa cct cct ggg aaa ggc ctg gag       144
    Gly Leu Gly Val Ser Trp Ile Arg Gln Pro Pro Gly Lys Gly Leu Glu
            35                  40                  45

    tgg ctg gca cac ata tac tgg gat gac gac aaa cgc tat aac cct agc       192
    Trp Leu Ala His Ile Tyr Trp Asp Asp Asp Lys Arg Tyr Asn Pro Ser
        50                  55                  60

    ctg aag tcc aga ctc aca atc tcc aaa gat aca tcc agc aat cag gtg       240
    Leu Lys Ser Arg Leu Thr Ile Ser Lys Asp Thr Ser Ser Asn Gln Val
    65                  70                  75                  80

    ttt ctg aag atc acc tcc gtc gat cca gtc gac acc gcc act tac tat       288
    Phe Leu Lys Ile Thr Ser Val Asp Pro Val Asp Thr Ala Thr Tyr Tyr
                    85                  90                  95

    tgt gct cgc gat tac tat gcc agc tcc ttc gac tac tgg gga cag gga       336
    Cys Ala Arg Asp Tyr Tyr Ala Ser Ser Phe Asp Tyr Trp Gly Gln Gly
                100                 105                 110

    aca ctg                                                               342
    Thr Leu
```

<210> 36
<211> 114
<212> PRT
<213> Artificial

<220>
<223> Synthetic Construct

<400> 36

```
Gln Val Thr Leu Lys Glu Ser Gly Pro Thr Leu Val Lys Pro Thr Gln
1               5                   10                  15


Thr Leu Ser Leu Thr Cys Ser Phe Ser Gly Phe Ser Leu Ser Thr Ser
                20                  25                  30


Gly Leu Gly Val Ser Trp Ile Arg Gln Pro Pro Gly Lys Gly Leu Glu
                35                  40                  45


Trp Leu Ala His Ile Tyr Trp Asp Asp Asp Lys Arg Tyr Asn Pro Ser
        50                  55                  60


Leu Lys Ser Arg Leu Thr Ile Ser Lys Asp Thr Ser Ser Asn Gln Val
65                  70                  75                  80


Phe Leu Lys Ile Thr Ser Val Asp Pro Val Asp Thr Ala Thr Tyr Tyr
                85                  90                  95


Cys Ala Arg Asp Tyr Tyr Ala Ser Ser Phe Asp Tyr Trp Gly Gln Gly
                100                 105                 110


Thr Leu
```

<210> 37
<211> 342
<212> DNA
<213> Artificial

<220>
<223> Séquence humanisée

<220>
<221> CDS
<222> (1)..(342)

<400> 37

```
cag gtc aca ctc aaa gaa tct gga cca aca ctc gtt aaa cca act caa      48
Gln Val Thr Leu Lys Glu Ser Gly Pro Thr Leu Val Lys Pro Thr Gln
1               5                   10                  15
```

```
aca ctc agc ctg act tgc agc ttt tct ggc ttc agc ctc tcc act agc        96
Thr Leu Ser Leu Thr Cys Ser Phe Ser Gly Phe Ser Leu Ser Thr Ser
            20              25                  30

ggc ctc ggc gtg tcc tgg atc cgg cag ccc cct gga aaa ggc ctg gag       144
Gly Leu Gly Val Ser Trp Ile Arg Gln Pro Pro Gly Lys Gly Leu Glu
        35              40                  45

tgg ctg gcc cac atc tat tgg gac gat gac aag cgg tac aat cca agc       192
Trp Leu Ala His Ile Tyr Trp Asp Asp Asp Lys Arg Tyr Asn Pro Ser
    50              55                  60

ctg aaa tct cgg ctg acc ata tct aag gat act tct tcc aat cag gtg       240
Leu Lys Ser Arg Leu Thr Ile Ser Lys Asp Thr Ser Ser Asn Gln Val
65              70                  75                  80

ttc ctg aag atc aca tct gtg gct cct gtc gac act gcc act tat tat       288
Phe Leu Lys Ile Thr Ser Val Ala Pro Val Asp Thr Ala Thr Tyr Tyr
                85                  90                  95

tgc gca agg gat tac tac gca tcc tcc ttc gat tac tgg ggc cag gga       336
Cys Ala Arg Asp Tyr Tyr Ala Ser Ser Phe Asp Tyr Trp Gly Gln Gly
            100                 105                 110

act ctg                                                                342
Thr Leu
```

<210> 38
<211> 114
<212> PRT
<213> Artificial

<220>
<223> Synthetic Construct

<400> 38

```
Gln Val Thr Leu Lys Glu Ser Gly Pro Thr Leu Val Lys Pro Thr Gln
1               5                   10                  15

Thr Leu Ser Leu Thr Cys Ser Phe Ser Gly Phe Ser Leu Ser Thr Ser
            20              25                  30

Gly Leu Gly Val Ser Trp Ile Arg Gln Pro Pro Gly Lys Gly Leu Glu
        35              40                  45

Trp Leu Ala His Ile Tyr Trp Asp Asp Asp Lys Arg Tyr Asn Pro Ser
    50              55                  60

Leu Lys Ser Arg Leu Thr Ile Ser Lys Asp Thr Ser Ser Asn Gln Val
65              70                  75                  80

Phe Leu Lys Ile Thr Ser Val Ala Pro Val Asp Thr Ala Thr Tyr Tyr
```

|  |  |  |  |  |  |  |  | 85 |  |  |  |  |  | 90 |  |  |  |  |  | 95 |

Cys Ala Arg Asp Tyr Tyr Ala Ser Ser Phe Asp Tyr Trp Gly Gln Gly
100 105 110

Thr Leu

<210> 39
<211> 1059
<212> DNA
<213> Homo sapiens

<220>
<221> CDS
<222> (1)..(1059)

<400> 39

```
ctg gag gcc tct gag gaa gtg gag ctt gag ccc tgc ctg gct ccc agc     48
Leu Glu Ala Ser Glu Glu Val Glu Leu Glu Pro Cys Leu Ala Pro Ser
1               5                   10                  15

ctg gag cag caa gag cag gag ctg aca gta gcc ctt ggg cag cct gtg     96
Leu Glu Gln Gln Glu Gln Glu Leu Thr Val Ala Leu Gly Gln Pro Val
            20                  25                  30

cgg ctg tgc tgt ggg cgg gct gag cgt ggt ggc cac tgg tac aag gag    144
Arg Leu Cys Cys Gly Arg Ala Glu Arg Gly Gly His Trp Tyr Lys Glu
        35                  40                  45

ggc agt cgc ctg gca cct gct ggc cgt gta cgg ggc tgg agg ggc cgc    192
Gly Ser Arg Leu Ala Pro Ala Gly Arg Val Arg Gly Trp Arg Gly Arg
        50                  55                  60

cta gag att gcc agc ttc cta cct gag gat gct ggc cgc tac ctc tgc    240
Leu Glu Ile Ala Ser Phe Leu Pro Glu Asp Ala Gly Arg Tyr Leu Cys
65                  70                  75                  80

ctg gca cga ggc tcc atg atc gtc ctg cag aat ctc acc ttg att aca    288
Leu Ala Arg Gly Ser Met Ile Val Leu Gln Asn Leu Thr Leu Ile Thr
                85                  90                  95

ggt gac tcc ttg acc tcc agc aac gat gat gag gac ccc aag tcc cat    336
Gly Asp Ser Leu Thr Ser Ser Asn Asp Asp Glu Asp Pro Lys Ser His
                100                 105                 110

agg gac ctc tcg aat agg cac agt tac ccc cag caa gca ccc tac tgg    384
Arg Asp Leu Ser Asn Arg His Ser Tyr Pro Gln Gln Ala Pro Tyr Trp
            115                 120                 125

aca cac ccc cag cgc atg gag aag aaa ctg cat gca gta cct gcg ggg    432
Thr His Pro Gln Arg Met Glu Lys Lys Leu His Ala Val Pro Ala Gly
            130                 135                 140

aac acc gtc aag ttc cgc tgt cca gct gca ggc aac ccc acg ccc acc    480
Asn Thr Val Lys Phe Arg Cys Pro Ala Ala Gly Asn Pro Thr Pro Thr
145                 150                 155                 160
```

```
atc cgc tgg ctt aag gat gga cag gcc ttt cat ggg gag aac cgc att        528
Ile Arg Trp Leu Lys Asp Gly Gln Ala Phe His Gly Glu Asn Arg Ile
            165                 170                 175

gga ggc att cgg ctg cgc cat cag cac tgg agt ctc gtg atg gag agc        576
Gly Gly Ile Arg Leu Arg His Gln His Trp Ser Leu Val Met Glu Ser
            180                 185                 190

gtg gtg ccc tcg gac cgc ggc aca tac acc tgc ctg gta gag aac gct        624
Val Val Pro Ser Asp Arg Gly Thr Tyr Thr Cys Leu Val Glu Asn Ala
            195                 200                 205

gtg ggc agc atc cgt tat aac tac ctg cta gat gtg ctg gag cgg tcc        672
Val Gly Ser Ile Arg Tyr Asn Tyr Leu Leu Asp Val Leu Glu Arg Ser
            210                 215                 220

ccg cac cgg ccc atc ctg cag gcc ggg ctc ccg gcc aac acc aca gcc        720
Pro His Arg Pro Ile Leu Gln Ala Gly Leu Pro Ala Asn Thr Thr Ala
225                 230                 235                 240

gtg gtg ggc agc gac gtg gag ctg ctg tgc aag gtg tac agc gat gcc        768
Val Val Gly Ser Asp Val Glu Leu Leu Cys Lys Val Tyr Ser Asp Ala
            245                 250                 255

cag ccc cac atc cag tgg ctg aag cac atc gtc atc aac ggc agc agc        816
Gln Pro His Ile Gln Trp Leu Lys His Ile Val Ile Asn Gly Ser Ser
            260                 265                 270

ttc gga gcc gac ggt ttc ccc tat gtg caa gtc cta aag act gca gac        864
Phe Gly Ala Asp Gly Phe Pro Tyr Val Gln Val Leu Lys Thr Ala Asp
            275                 280                 285

atc aat agc tca gag gtg gag gtc ctg tac ctg cgg aac gtg tca gcc        912
Ile Asn Ser Ser Glu Val Glu Val Leu Tyr Leu Arg Asn Val Ser Ala
            290                 295                 300

gag gac gca ggc gag tac acc tgc ctc gca ggc aat tcc atc ggc ctc        960
Glu Asp Ala Gly Glu Tyr Thr Cys Leu Ala Gly Asn Ser Ile Gly Leu
305                 310                 315                 320

tcc tac cag tct gcc tgg ctc acg gtg ctg cca gag gag gac ccc aca       1008
Ser Tyr Gln Ser Ala Trp Leu Thr Val Leu Pro Glu Glu Asp Pro Thr
            325                 330                 335

tgg acc gca gca gcg ccc gag gcc gct agc cat cac cat cat cat cac       1056
Trp Thr Ala Ala Ala Pro Glu Ala Ala Ser His His His His His His
            340                 345                 350

tga                                                                    1059
```

<210> 40
<211> 352
<212> PRT
<213> Homo sapiens

<400> 40

Leu Glu Ala Ser Glu Glu Val Glu Leu Glu Pro Cys Leu Ala Pro Ser
1                   5                   10                  15

Leu Glu Gln Gln Glu Gln Glu Leu Thr Val Ala Leu Gly Gln Pro Val
            20                  25              30

Arg Leu Cys Cys Gly Arg Ala Glu Arg Gly Gly His Trp Tyr Lys Glu
        35                  40                  45

Gly Ser Arg Leu Ala Pro Ala Gly Arg Val Arg Gly Trp Arg Gly Arg
    50                  55                  60

Leu Glu Ile Ala Ser Phe Leu Pro Glu Asp Ala Gly Arg Tyr Leu Cys
65                  70                  75                  80

Leu Ala Arg Gly Ser Met Ile Val Leu Gln Asn Leu Thr Leu Ile Thr
                85                  90                  95

Gly Asp Ser Leu Thr Ser Ser Asn Asp Asp Glu Asp Pro Lys Ser His
            100                 105                 110

Arg Asp Leu Ser Asn Arg His Ser Tyr Pro Gln Gln Ala Pro Tyr Trp
        115                 120                 125

Thr His Pro Gln Arg Met Glu Lys Lys Leu His Ala Val Pro Ala Gly
    130                 135                 140

Asn Thr Val Lys Phe Arg Cys Pro Ala Ala Gly Asn Pro Thr Pro Thr
145                 150                 155                 160

Ile Arg Trp Leu Lys Asp Gly Gln Ala Phe His Gly Glu Asn Arg Ile
                165                 170                 175

Gly Gly Ile Arg Leu Arg His Gln His Trp Ser Leu Val Met Glu Ser
            180                 185                 190

Val Val Pro Ser Asp Arg Gly Thr Tyr Thr Cys Leu Val Glu Asn Ala
        195                 200                 205

Val Gly Ser Ile Arg Tyr Asn Tyr Leu Leu Asp Val Leu Glu Arg Ser
    210                 215                 220

Pro His Arg Pro Ile Leu Gln Ala Gly Leu Pro Ala Asn Thr Thr Ala
225                 230                 235                 240

Val Val Gly Ser Asp Val Glu Leu Leu Cys Lys Val Tyr Ser Asp Ala
            245                 250                 255

```
Gln Pro His Ile Gln Trp Leu Lys His Ile Val Ile Asn Gly Ser Ser
            260             265             270

Phe Gly Ala Asp Gly Phe Pro Tyr Val Gln Val Leu Lys Thr Ala Asp
            275             280             285

Ile Asn Ser Ser Glu Val Glu Val Leu Tyr Leu Arg Asn Val Ser Ala
            290             295             300

Glu Asp Ala Gly Glu Tyr Thr Cys Leu Ala Gly Asn Ser Ile Gly Leu
305             310             315             320

Ser Tyr Gln Ser Ala Trp Leu Thr Val Leu Pro Glu Glu Asp Pro Thr
            325             330             335

Trp Thr Ala Ala Ala Pro Glu Ala Ala Ser His His His His His His
            340             345             350
```

<210> 41
<211> 726
<212> DNA
<213> Homo sapiens

<220>
<221> CDS
<222> (1)..(726)

<400> 41

```
ctg gag gcc tct gag gaa gtg gag ctt gag ccc tgc caa gca ccc tac      48
Leu Glu Ala Ser Glu Glu Val Glu Leu Glu Pro Cys Gln Ala Pro Tyr
1               5               10                  15

tgg aca cac ccc cag cgc atg gag aag aaa ctg cat gca gta cct gcg      96
Trp Thr His Pro Gln Arg Met Glu Lys Lys Leu His Ala Val Pro Ala
            20                  25                  30

ggg aac acc gtc aag ttc cgc tgt cca gct gca ggc aac ccc acg ccc     144
Gly Asn Thr Val Lys Phe Arg Cys Pro Ala Ala Gly Asn Pro Thr Pro
            35                  40                  45

acc atc cgc tgg ctt aag gat gga cag gcc ttt cat ggg gag aac cgc     192
Thr Ile Arg Trp Leu Lys Asp Gly Gln Ala Phe His Gly Glu Asn Arg
        50                  55                  60

att gga ggc att cgg ctg cgc cat cag cac tgg agt ctc gtg atg gag     240
Ile Gly Gly Ile Arg Leu Arg His Gln His Trp Ser Leu Val Met Glu
65                  70                  75                  80

agc gtg gtg ccc tcg gac cgc ggc aca tac acc tgc ctg gta gag aac     288
Ser Val Val Pro Ser Asp Arg Gly Thr Tyr Thr Cys Leu Val Glu Asn
                85                  90                  95

gct gtg ggc agc atc cgt tat aac tac ctg cta gat gtg ctg gag cgg     336
```

```
      Ala Val Gly Ser Ile Arg Tyr Asn Tyr Leu Leu Asp Val Leu Glu Arg
              100                 105             110

tcc ccg cac cgg ccc atc ctg cag gcc ggg ctc ccg gcc aac acc aca      384
Ser Pro His Arg Pro Ile Leu Gln Ala Gly Leu Pro Ala Asn Thr Thr
        115                 120             125

gcc gtg gtg ggc agc gac gtg gag ctg ctg tgc aag gtg tac agc gat      432
Ala Val Val Gly Ser Asp Val Glu Leu Leu Cys Lys Val Tyr Ser Asp
    130                 135             140

gcc cag ccc cac atc cag tgg ctg aag cac atc gtc atc aac ggc agc      480
Ala Gln Pro His Ile Gln Trp Leu Lys His Ile Val Ile Asn Gly Ser
145                 150             155             160

agc ttc gga gcc gac ggt ttc ccc tat gtg caa gtc cta aag act gca      528
Ser Phe Gly Ala Asp Gly Phe Pro Tyr Val Gln Val Leu Lys Thr Ala
                165             170             175

gac atc aat agc tca gag gtg gag gtc ctg tac ctg cgg aac gtg tca      576
Asp Ile Asn Ser Ser Glu Val Glu Val Leu Tyr Leu Arg Asn Val Ser
                180             185             190

gcc gag gac gca ggc gag tac acc tgc ctc gca ggc aat tcc atc ggc      624
Ala Glu Asp Ala Gly Glu Tyr Thr Cys Leu Ala Gly Asn Ser Ile Gly
        195                 200             205

ctc tcc tac cag tct gcc tgg ctc acg gtg ctg cca gag gag gac ccc      672
Leu Ser Tyr Gln Ser Ala Trp Leu Thr Val Leu Pro Glu Glu Asp Pro
        210                 215             220

aca tgg acc gca gca gcg ccc gag gcc gct agc cat cac cat cat cat      720
Thr Trp Thr Ala Ala Ala Pro Glu Ala Ala Ser His His His His His
225                 230             235             240

cac tga                                                              726
His
```

<210> 42
<211> 241
<212> PRT
<213> Homo sapiens

<400> 42

```
Leu Glu Ala Ser Glu Glu Val Glu Leu Glu Pro Cys Gln Ala Pro Tyr
1               5               10              15

Trp Thr His Pro Gln Arg Met Glu Lys Lys Leu His Ala Val Pro Ala
            20              25              30

Gly Asn Thr Val Lys Phe Arg Cys Pro Ala Ala Gly Asn Pro Thr Pro
            35              40              45

Thr Ile Arg Trp Leu Lys Asp Gly Gln Ala Phe His Gly Glu Asn Arg
            50              55              60
```

```
Ile Gly Gly Ile Arg Leu Arg His Gln His Trp Ser Leu Val Met Glu
65              70              75                      80

Ser Val Val Pro Ser Asp Arg Gly Thr Tyr Thr Cys Leu Val Glu Asn
                85              90                  95

Ala Val Gly Ser Ile Arg Tyr Asn Tyr Leu Leu Asp Val Leu Glu Arg
            100             105             110

Ser Pro His Arg Pro Ile Leu Gln Ala Gly Leu Pro Ala Asn Thr Thr
        115             120             125

Ala Val Val Gly Ser Asp Val Glu Leu Leu Cys Lys Val Tyr Ser Asp
    130             135             140

Ala Gln Pro His Ile Gln Trp Leu Lys His Ile Val Ile Asn Gly Ser
145             150             155             160

Ser Phe Gly Ala Asp Gly Phe Pro Tyr Val Gln Val Leu Lys Thr Ala
            165             170             175

Asp Ile Asn Ser Ser Glu Val Glu Val Leu Tyr Leu Arg Asn Val Ser
        180             185             190

Ala Glu Asp Ala Gly Glu Tyr Thr Cys Leu Ala Gly Asn Ser Ile Gly
    195             200             205

Leu Ser Tyr Gln Ser Ala Trp Leu Thr Val Leu Pro Glu Glu Asp Pro
    210             215             220

Thr Trp Thr Ala Ala Ala Pro Glu Ala Ala Ser His His His His His
225             230             235             240

His
```

<210> 43
<211> 1059
<212> DNA
<213> Mus sp.

<220>
<221> CDS
<222> (1)..(1059)

<400> 43

```
ctt gag gcc tct gag gaa atg gag cag gag ccc tgc cta gcc cca atc        48
Leu Glu Ala Ser Glu Glu Met Glu Gln Glu Pro Cys Leu Ala Pro Ile
1               5                   10                  15

ctg gag cag caa gag cag gtg ttg acg gtg gcc ctg ggg cag cct gtg        96
Leu Glu Gln Gln Glu Gln Val Leu Thr Val Ala Leu Gly Gln Pro Val
                20                  25                  30

agg ctg tgc tgt ggg cgc acc gag cgt ggt cgt cac tgg tac aaa gag       144
Arg Leu Cys Cys Gly Arg Thr Glu Arg Gly Arg His Trp Tyr Lys Glu
            35                  40                  45

ggc agc cgc cta gca tct gct ggg cga gta cgg ggt tgg aga ggc cgc       192
Gly Ser Arg Leu Ala Ser Ala Gly Arg Val Arg Gly Trp Arg Gly Arg
        50                  55                  60

ctg gag atc gcc agc ttc ctt cct gag gat gct ggc cga tac ctc tgc       240
Leu Glu Ile Ala Ser Phe Leu Pro Glu Asp Ala Gly Arg Tyr Leu Cys
65                  70                  75                  80

ctg gcc cgt ggc tcc atg acc gtc gta cac aat ctt acg ttg ctt atg       288
Leu Ala Arg Gly Ser Met Thr Val Val His Asn Leu Thr Leu Leu Met
                85                  90                  95

gat gac tcc tta acc tcc atc agt aat gat gaa gac ccc aag aca ctc       336
Asp Asp Ser Leu Thr Ser Ile Ser Asn Asp Glu Asp Pro Lys Thr Leu
            100                 105                 110

agc agc tcc tcg agt ggt cat gtc tac cca cag caa gca ccc tac tgg       384
Ser Ser Ser Ser Ser Gly His Val Tyr Pro Gln Gln Ala Pro Tyr Trp
            115                 120                 125

aca cac ccc caa cgc atg gag aag aaa ctg cat gca gtg cct gcc ggg       432
Thr His Pro Gln Arg Met Glu Lys Lys Leu His Ala Val Pro Ala Gly
        130                 135                 140

aat act gtc aaa ttc cgc tgt cca gct gca ggg aac ccc atg cct acc       480
Asn Thr Val Lys Phe Arg Cys Pro Ala Ala Gly Asn Pro Met Pro Thr
145                 150                 155                 160

atc cac tgg ctc aag gat gga cag gcc ttc cac ggg gag aat cgt att       528
Ile His Trp Leu Lys Asp Gly Gln Ala Phe His Gly Glu Asn Arg Ile
                165                 170                 175

gga ggc att cgg ctg cgc cac caa cac tgg agc ctg gtg atg gaa agt       576
Gly Gly Ile Arg Leu Arg His Gln His Trp Ser Leu Val Met Glu Ser
            180                 185                 190

gtg gta ccc tcg gac cgt ggc aca tac aca tgc ctt gtg gag aac tct       624
Val Val Pro Ser Asp Arg Gly Thr Tyr Thr Cys Leu Val Glu Asn Ser
            195                 200                 205

ctg ggt agc att cgc tac agc tat ctc ctg gat gtg ctg gag cgg tcc       672
Leu Gly Ser Ile Arg Tyr Ser Tyr Leu Leu Asp Val Leu Glu Arg Ser
            210                 215                 220

ccg cac cgg ccc atc ctg cag gcg ggg ctc cca gcc aac acc aca gct       720
Pro His Arg Pro Ile Leu Gln Ala Gly Leu Pro Ala Asn Thr Thr Ala
225                 230                 235                 240

gtg gtg ggc agc gac gtg gag cta ctc tgc aag gtg tac agc gac gcc       768
```

```
Val Val Gly Ser Asp Val Glu Leu Leu Cys Lys Val Tyr Ser Asp Ala
            245             250             255

cag ccc cac ata cag tgg ctg aaa cac gtc gtc atc aac ggc agc agc    816
Gln Pro His Ile Gln Trp Leu Lys His Val Val Ile Asn Gly Ser Ser
            260             265             270

ttc ggc gcc gac ggt ttc ccc tac gta caa gtc ctg aag aca aca gac    864
Phe Gly Ala Asp Gly Phe Pro Tyr Val Gln Val Leu Lys Thr Thr Asp
            275             280             285

atc aat agc tcg gag gta gag gtc ttg tat ctg agg aac gtg tcc gct    912
Ile Asn Ser Ser Glu Val Glu Val Leu Tyr Leu Arg Asn Val Ser Ala
            290             295             300

gag gat gca gga gag tat acc tgt ctg gcg ggc aac tcc atc ggc ctt    960
Glu Asp Ala Gly Glu Tyr Thr Cys Leu Ala Gly Asn Ser Ile Gly Leu
305                 310             315             320

tcc tac cag tca gcg tgg ctc acg gtg ctg cca gag gaa gac ctc acg   1008
Ser Tyr Gln Ser Ala Trp Leu Thr Val Leu Pro Glu Glu Asp Leu Thr
            325             330             335

tgg aca aca gca acc cct gag gcc gct agc cat cac cat cat cat cac   1056
Trp Thr Thr Ala Thr Pro Glu Ala Ala Ser His His His His His His
            340             345             350

tga                                                               1059
```

<210> 44
<211> 352
<212> PRT
<213> Mus sp.

<400> 44

```
Leu Glu Ala Ser Glu Glu Met Glu Gln Glu Pro Cys Leu Ala Pro Ile
1               5               10              15

Leu Glu Gln Gln Glu Gln Val Leu Thr Val Ala Leu Gly Gln Pro Val
            20              25              30

Arg Leu Cys Cys Gly Arg Thr Glu Arg Gly Arg His Trp Tyr Lys Glu
            35              40              45

Gly Ser Arg Leu Ala Ser Ala Gly Arg Val Arg Gly Trp Arg Gly Arg
            50              55              60

Leu Glu Ile Ala Ser Phe Leu Pro Glu Asp Ala Gly Arg Tyr Leu Cys
65              70              75              80

Leu Ala Arg Gly Ser Met Thr Val Val His Asn Leu Thr Leu Leu Met
            85              90              95
```

```
Asp Asp Ser Leu Thr Ser Ile Ser Asn Asp Glu Asp Pro Lys Thr Leu
            100                 105             110

Ser Ser Ser Ser Ser Gly His Val Tyr Pro Gln Gln Ala Pro Tyr Trp
            115                 120             125

Thr His Pro Gln Arg Met Glu Lys Lys Leu His Ala Val Pro Ala Gly
            130                 135             140

Asn Thr Val Lys Phe Arg Cys Pro Ala Ala Gly Asn Pro Met Pro Thr
145                 150             155                 160

Ile His Trp Leu Lys Asp Gly Gln Ala Phe His Gly Glu Asn Arg Ile
                165             170             175

Gly Gly Ile Arg Leu Arg His Gln His Trp Ser Leu Val Met Glu Ser
            180             185             190

Val Val Pro Ser Asp Arg Gly Thr Tyr Thr Cys Leu Val Glu Asn Ser
            195             200             205

Leu Gly Ser Ile Arg Tyr Ser Tyr Leu Leu Asp Val Leu Glu Arg Ser
    210                 215             220

Pro His Arg Pro Ile Leu Gln Ala Gly Leu Pro Ala Asn Thr Thr Ala
225                 230             235                 240

Val Val Gly Ser Asp Val Glu Leu Leu Cys Lys Val Tyr Ser Asp Ala
                245             250             255

Gln Pro His Ile Gln Trp Leu Lys His Val Val Ile Asn Gly Ser Ser
            260             265             270

Phe Gly Ala Asp Gly Phe Pro Tyr Val Gln Val Leu Lys Thr Thr Asp
            275             280             285

Ile Asn Ser Ser Glu Val Glu Val Leu Tyr Leu Arg Asn Val Ser Ala
    290                 295             300

Glu Asp Ala Gly Glu Tyr Thr Cys Leu Ala Gly Asn Ser Ile Gly Leu
305                 310             315                 320

Ser Tyr Gln Ser Ala Trp Leu Thr Val Leu Pro Glu Glu Asp Leu Thr
            325             330             335

Trp Thr Thr Ala Thr Pro Glu Ala Ala Ser His His His His His His
```

340                     345                     350

<210> 45
<211> 1773
<212> DNA
<213> Homo sapiens

<220>
<221> CDS
<222> (1)..(1773)

<400> 45

```
agg ccg tcc ccg acc ttg cct gaa caa gcc cag ccc tgg gga gcc cct          48
Arg Pro Ser Pro Thr Leu Pro Glu Gln Ala Gln Pro Trp Gly Ala Pro
1               5                   10                  15

gtg gaa gtg gag tcc ttc ctg gtc cac ccc ggt gac ctg ctg cag ctt          96
Val Glu Val Glu Ser Phe Leu Val His Pro Gly Asp Leu Leu Gln Leu
            20                  25                  30

cgc tgt cgg ctg cgg gac gat gtg cag agc atc aac tgg ctg cgg gac         144
Arg Cys Arg Leu Arg Asp Asp Val Gln Ser Ile Asn Trp Leu Arg Asp
        35                  40                  45

ggg gtg cag ctg gcg gaa agc aac cgc acc cgc atc aca ggg gag gag         192
Gly Val Gln Leu Ala Glu Ser Asn Arg Thr Arg Ile Thr Gly Glu Glu
    50                  55                  60

gtg gag gtg cag gac tcc gtg ccc gca gac tcc ggc ctc tat gct tgc         240
Val Glu Val Gln Asp Ser Val Pro Ala Asp Ser Gly Leu Tyr Ala Cys
65                  70                  75                  80

gta acc agc agc ccc tcg ggc agt gac acc acc tac ttc tcc gtc aat         288
Val Thr Ser Ser Pro Ser Gly Ser Asp Thr Thr Tyr Phe Ser Val Asn
                85                  90                  95

gtt tca gat gct ctc ccc tcc tcg gag gat gat gat gat gat gat gac         336
Val Ser Asp Ala Leu Pro Ser Ser Glu Asp Asp Asp Asp Asp Asp Asp
            100                 105                 110

tcc tct tca gag gag aaa gaa aca gat aac acc aaa cca aac cgt atg         384
Ser Ser Ser Glu Glu Lys Glu Thr Asp Asn Thr Lys Pro Asn Arg Met
            115                 120                 125

ccc gta gct cca tat tgg aca tcc cca gaa aag atg gaa aag aaa ttg         432
Pro Val Ala Pro Tyr Trp Thr Ser Pro Glu Lys Met Glu Lys Lys Leu
    130                 135                 140

cat gca gtg ccg gct gcc aag aca gtg aag ttc aaa tgc cct tcc agt         480
His Ala Val Pro Ala Ala Lys Thr Val Lys Phe Lys Cys Pro Ser Ser
145                 150                 155                 160

ggg acc cca aac ccc aca ctg cgc tgg ttg aaa aat ggc aaa gaa ttc         528
Gly Thr Pro Asn Pro Thr Leu Arg Trp Leu Lys Asn Gly Lys Glu Phe
                165                 170                 175

aaa cct gac cac aga att gga ggc tac aag gtc cgt tat gcc acc tgg         576
Lys Pro Asp His Arg Ile Gly Gly Tyr Lys Val Arg Tyr Ala Thr Trp
                180                 185                 190
```

73

```
agc atc ata atg gac tct gtg gtg ccc tct gac aag ggc aac tac acc        624
Ser Ile Ile Met Asp Ser Val Val Pro Ser Asp Lys Gly Asn Tyr Thr
        195                 200                 205

tgc att gtg gag aat gag tac ggc agc atc aac cac aca tac cag ctg        672
Cys Ile Val Glu Asn Glu Tyr Gly Ser Ile Asn His Thr Tyr Gln Leu
        210                 215                 220

gat gtc gtg gag cgg tcc cct cac cgg ccc atc ctg caa gca ggg ttg        720
Asp Val Val Glu Arg Ser Pro His Arg Pro Ile Leu Gln Ala Gly Leu
225                 230                 235                 240

ccc gcc aac aaa aca gtg gcc ctg ggt agc aac gtg gag ttc atg tgt        768
Pro Ala Asn Lys Thr Val Ala Leu Gly Ser Asn Val Glu Phe Met Cys
                245                 250                 255

aag gtg tac agt gac ccg cag ccg cac atc cag tgg cta aag cac atc        816
Lys Val Tyr Ser Asp Pro Gln Pro His Ile Gln Trp Leu Lys His Ile
                260                 265                 270

gag gtg aat ggg agc aag att ggc cca gac aac ctg cct tat gtc cag        864
Glu Val Asn Gly Ser Lys Ile Gly Pro Asp Asn Leu Pro Tyr Val Gln
                275                 280                 285

atc ttg aag act gct gga gtt aat acc acc gac aaa gag atg gag gtg        912
Ile Leu Lys Thr Ala Gly Val Asn Thr Thr Asp Lys Glu Met Glu Val
        290                 295                 300

ctt cac tta aga aat gtc tcc ttt gag gac gca ggg gag tat acg tgc        960
Leu His Leu Arg Asn Val Ser Phe Glu Asp Ala Gly Glu Tyr Thr Cys
305                 310                 315                 320

ttg gcg ggt aac tct atc gga ctc tcc cat cac tct gca tgg ttg acc       1008
Leu Ala Gly Asn Ser Ile Gly Leu Ser His His Ser Ala Trp Leu Thr
                325                 330                 335

gtt ctg gaa gcc ctg gaa gag agg ccg gca gtg atg acc tcg ccc ctg       1056
Val Leu Glu Ala Leu Glu Glu Arg Pro Ala Val Met Thr Ser Pro Leu
                340                 345                 350

tac ctg gag ata gcg cta gag ccc aaa tct tgt gac aaa act cac aca       1104
Tyr Leu Glu Ile Ala Leu Glu Pro Lys Ser Cys Asp Lys Thr His Thr
        355                 360                 365

tgc cca ccg tgc cca gca cct gaa ctc ctg ggg gga ccg tca gtc ttc       1152
Cys Pro Pro Cys Pro Ala Pro Glu Leu Leu Gly Gly Pro Ser Val Phe
        370                 375                 380

ctc ttc ccc cca aaa ccc aag gac acc ctc atg atc tcc cgg acc cct       1200
Leu Phe Pro Pro Lys Pro Lys Asp Thr Leu Met Ile Ser Arg Thr Pro
385                 390                 395                 400

gag gtc aca tgc gtg gtg gtg gac gtg agc cac gaa gac cct gag gtc       1248
Glu Val Thr Cys Val Val Val Asp Val Ser His Glu Asp Pro Glu Val
                405                 410                 415

aag ttc aac tgg tac gtg gac ggc gtg gag gtg cat aat gcc aag aca       1296
Lys Phe Asn Trp Tyr Val Asp Gly Val Glu Val His Asn Ala Lys Thr
                420                 425                 430
```

74

```
aag ccg cgg gag gag cag tac aac agc acg tac cgt gtg gtc agc gtc    1344
Lys Pro Arg Glu Glu Gln Tyr Asn Ser Thr Tyr Arg Val Val Ser Val
        435             440             445

ctc acc gtc ctg cac cag gac tgg ctg aat ggc aag gag tac aag tgc    1392
Leu Thr Val Leu His Gln Asp Trp Leu Asn Gly Lys Glu Tyr Lys Cys
    450             455             460

aag gtc tcc aac aaa gcc ctc cca gcc ccc atc gag aaa acc atc tcc    1440
Lys Val Ser Asn Lys Ala Leu Pro Ala Pro Ile Glu Lys Thr Ile Ser
465             470             475             480

aaa gcc aaa ggg cag ccc cga gaa cca cag gtg tac acc ctg ccc cca    1488
Lys Ala Lys Gly Gln Pro Arg Glu Pro Gln Val Tyr Thr Leu Pro Pro
            485             490             495

tcc cgg gat gag ctg acc aag aac cag gtc agc ctg acc tgc ctg gtc    1536
Ser Arg Asp Glu Leu Thr Lys Asn Gln Val Ser Leu Thr Cys Leu Val
        500             505             510

aaa ggc ttc tat ccc agc gac atc gcc gtg gag tgg gag agc aat ggg    1584
Lys Gly Phe Tyr Pro Ser Asp Ile Ala Val Glu Trp Glu Ser Asn Gly
        515             520             525

cag ccg gag aac aac tac aag acc acg cct ccc gtg ctg gac tcc gac    1632
Gln Pro Glu Asn Asn Tyr Lys Thr Thr Pro Pro Val Leu Asp Ser Asp
        530             535             540

ggc tcc ttc ttc ctc tac agc aag ctc acc gtg gac aag agc agg tgg    1680
Gly Ser Phe Phe Leu Tyr Ser Lys Leu Thr Val Asp Lys Ser Arg Trp
545             550             555             560

cag cag ggg aac gtc ttc tca tgc tcc gtg atg cat gag gct ctg cac    1728
Gln Gln Gly Asn Val Phe Ser Cys Ser Val Met His Glu Ala Leu His
            565             570             575

aac cac tac acg cag aag agc ctc tcc ctg tct ccg ggt aaa tga       1773
Asn His Tyr Thr Gln Lys Ser Leu Ser Leu Ser Pro Gly Lys
            580             585             590
```

<210> 46
<211> 590
<212> PRT
<213> Homo sapiens

<400> 46

```
Arg Pro Ser Pro Thr Leu Pro Glu Gln Ala Gln Pro Trp Gly Ala Pro
1               5                   10                  15

Val Glu Val Glu Ser Phe Leu Val His Pro Gly Asp Leu Leu Gln Leu
            20                  25                  30

Arg Cys Arg Leu Arg Asp Asp Val Gln Ser Ile Asn Trp Leu Arg Asp
        35                  40                  45

Gly Val Gln Leu Ala Glu Ser Asn Arg Thr Arg Ile Thr Gly Glu Glu
```

|  | 50 |  |  |  |  | 55 |  |  |  |  | 60 |  |  |  |
|---|---|---|---|---|---|---|---|---|---|---|---|---|---|---|

Val Glu Val Gln Asp Ser Val Pro Ala Asp Ser Gly Leu Tyr Ala Cys
65                70                75                80

Val Thr Ser Ser Pro Ser Gly Ser Asp Thr Thr Tyr Phe Ser Val Asn
          85                90                95

Val Ser Asp Ala Leu Pro Ser Ser Glu Asp Asp Asp Asp Asp Asp Asp
          100               105               110

Ser Ser Ser Glu Glu Lys Glu Thr Asp Asn Thr Lys Pro Asn Arg Met
          115               120               125

Pro Val Ala Pro Tyr Trp Thr Ser Pro Glu Lys Met Glu Lys Lys Leu
          130               135               140

His Ala Val Pro Ala Ala Lys Thr Val Lys Phe Lys Cys Pro Ser Ser
145               150               155               160

Gly Thr Pro Asn Pro Thr Leu Arg Trp Leu Lys Asn Gly Lys Glu Phe
          165               170               175

Lys Pro Asp His Arg Ile Gly Gly Tyr Lys Val Arg Tyr Ala Thr Trp
          180               185               190

Ser Ile Ile Met Asp Ser Val Val Pro Ser Asp Lys Gly Asn Tyr Thr
          195               200               205

Cys Ile Val Glu Asn Glu Tyr Gly Ser Ile Asn His Thr Tyr Gln Leu
          210               215               220

Asp Val Val Glu Arg Ser Pro His Arg Pro Ile Leu Gln Ala Gly Leu
225               230               235               240

Pro Ala Asn Lys Thr Val Ala Leu Gly Ser Asn Val Glu Phe Met Cys
          245               250               255

Lys Val Tyr Ser Asp Pro Gln Pro His Ile Gln Trp Leu Lys His Ile
          260               265               270

Glu Val Asn Gly Ser Lys Ile Gly Pro Asp Asn Leu Pro Tyr Val Gln
          275               280               285

Ile Leu Lys Thr Ala Gly Val Asn Thr Thr Asp Lys Glu Met Glu Val
          290               295               300

Leu His Leu Arg Asn Val Ser Phe Glu Asp Ala Gly Glu Tyr Thr Cys
305                 310                 315                 320

Leu Ala Gly Asn Ser Ile Gly Leu Ser His His Ser Ala Trp Leu Thr
                325                 330                 335

Val Leu Glu Ala Leu Glu Glu Arg Pro Ala Val Met Thr Ser Pro Leu
                340                 345                 350

Tyr Leu Glu Ile Ala Leu Glu Pro Lys Ser Cys Asp Lys Thr His Thr
                355                 360                 365

Cys Pro Pro Cys Pro Ala Pro Glu Leu Leu Gly Gly Pro Ser Val Phe
    370                 375                 380

Leu Phe Pro Pro Lys Pro Lys Asp Thr Leu Met Ile Ser Arg Thr Pro
385                 390                 395                 400

Glu Val Thr Cys Val Val Val Asp Val Ser His Glu Asp Pro Glu Val
                405                 410                 415

Lys Phe Asn Trp Tyr Val Asp Gly Val Glu Val His Asn Ala Lys Thr
                420                 425                 430

Lys Pro Arg Glu Glu Gln Tyr Asn Ser Thr Tyr Arg Val Val Ser Val
    435                 440                 445

Leu Thr Val Leu His Gln Asp Trp Leu Asn Gly Lys Glu Tyr Lys Cys
    450                 455                 460

Lys Val Ser Asn Lys Ala Leu Pro Ala Pro Ile Glu Lys Thr Ile Ser
465                 470                 475                 480

Lys Ala Lys Gly Gln Pro Arg Glu Pro Gln Val Tyr Thr Leu Pro Pro
                485                 490                 495

Ser Arg Asp Glu Leu Thr Lys Asn Gln Val Ser Leu Thr Cys Leu Val
                500                 505                 510

Lys Gly Phe Tyr Pro Ser Asp Ile Ala Val Glu Trp Glu Ser Asn Gly
    515                 520                 525

Gln Pro Glu Asn Asn Tyr Lys Thr Thr Pro Pro Val Leu Asp Ser Asp
    530                 535                 540

```
        Gly Ser Phe Phe Leu Tyr Ser Lys Leu Thr Val Asp Lys Ser Arg Trp
        545                 550                 555                 560


        Gln Gln Gly Asn Val Phe Ser Cys Ser Val Met His Glu Ala Leu His
                        565                 570                 575


        Asn His Tyr Thr Gln Lys Ser Leu Ser Leu Ser Pro Gly Lys
                        580                 585                 590
```

<210> 47
<211> 1773
<212> DNA
<213> Homo sapiens

<220>
<221> CDS
<222> (1)..(1773)

<400> 47

```
        cgg ccc tcc ttc agt tta gtt gag gat acc aca tta gag cca gaa gag      48
        Arg Pro Ser Phe Ser Leu Val Glu Asp Thr Thr Leu Glu Pro Glu Glu
        1               5                   10                  15

        cca cca acc aaa tac caa atc tct caa cca gaa gtg tac gtg gct gca      96
        Pro Pro Thr Lys Tyr Gln Ile Ser Gln Pro Glu Val Tyr Val Ala Ala
                        20                  25                  30

        cca ggg gag tcg cta gag gtg cgc tgc ctg ttg aaa gat gcc gcc gtg     144
        Pro Gly Glu Ser Leu Glu Val Arg Cys Leu Leu Lys Asp Ala Ala Val
                35                  40                  45

        atc agt tgg act aag gat ggg gtg cac ttg ggg ccc aac aat agg aca     192
        Ile Ser Trp Thr Lys Asp Gly Val His Leu Gly Pro Asn Asn Arg Thr
                50                  55                  60

        gtg ctt att ggg gag tac ttg cag ata aag ggc gcc acg cct aga gac     240
        Val Leu Ile Gly Glu Tyr Leu Gln Ile Lys Gly Ala Thr Pro Arg Asp
        65                  70                  75                  80

        tcc ggc ctc tat gct tgt act gcc agt agg act gta gac agt gaa act     288
        Ser Gly Leu Tyr Ala Cys Thr Ala Ser Arg Thr Val Asp Ser Glu Thr
                        85                  90                  95

        tgg tac ttc atg gtg aat gtc aca gat gcc atc tca tcc gga gat gat     336
        Trp Tyr Phe Met Val Asn Val Thr Asp Ala Ile Ser Ser Gly Asp Asp
                        100                 105                 110

        gag gat gac acc gat ggt gcg gaa gat ttt gtc agt gag aac agt aac     384
        Glu Asp Asp Thr Asp Gly Ala Glu Asp Phe Val Ser Glu Asn Ser Asn
                115                 120                 125

        aac aag aga gca cca tac tgg acc aac aca gaa aag atg gaa aag cgg     432
        Asn Lys Arg Ala Pro Tyr Trp Thr Asn Thr Glu Lys Met Glu Lys Arg
                130                 135                 140

        ctc cat gct gtg cct gcg gcc aac act gtc aag ttt cgc tgc cca gcc     480
```

```
Leu His Ala Val Pro Ala Ala Asn Thr Val Lys Phe Arg Cys Pro Ala
145             150             155             160

ggg ggg aac cca atg cca acc atg cgg tgg ctg aaa aac ggg aag gag       528
Gly Gly Asn Pro Met Pro Thr Met Arg Trp Leu Lys Asn Gly Lys Glu
                165             170             175

ttt aag cag gag cat cgc att gga ggc tac aag gta cga aac cag cac       576
Phe Lys Gln Glu His Arg Ile Gly Gly Tyr Lys Val Arg Asn Gln His
                180             185             190

tgg agc ctc att atg gaa agt gtg gtc cca tct gac aag gga aat tat       624
Trp Ser Leu Ile Met Glu Ser Val Val Pro Ser Asp Lys Gly Asn Tyr
            195             200             205

acc tgt gtg gtg gag aat gaa tac ggg tcc atc aat cac acg tac cac       672
Thr Cys Val Val Glu Asn Glu Tyr Gly Ser Ile Asn His Thr Tyr His
        210             215             220

ctg gat gtt gtg gag cga tcg cct cac cgg ccc atc ctc caa gcc gga       720
Leu Asp Val Val Glu Arg Ser Pro His Arg Pro Ile Leu Gln Ala Gly
225             230             235             240

ctg ccg gca aat gcc tcc aca gtg gtc gga gga gac gta gag ttt gtc       768
Leu Pro Ala Asn Ala Ser Thr Val Val Gly Gly Asp Val Glu Phe Val
                245             250             255

tgc aag gtt tac agt gat gcc cag ccc cac atc cag tgg atc aag cac       816
Cys Lys Val Tyr Ser Asp Ala Gln Pro His Ile Gln Trp Ile Lys His
                260             265             270

gtg gaa aag aac ggc agt aaa tac ggg ccc gac ggg ctg ccc tac ctc       864
Val Glu Lys Asn Gly Ser Lys Tyr Gly Pro Asp Gly Leu Pro Tyr Leu
            275             280             285

aag gtt ctc aag gcc gcc ggt gtt aac acc acg gac aaa gag att gag       912
Lys Val Leu Lys Ala Ala Gly Val Asn Thr Thr Asp Lys Glu Ile Glu
        290             295             300

gtt ctc tat att cgg aat gta act ttt gag gac gct ggg gaa tat acg       960
Val Leu Tyr Ile Arg Asn Val Thr Phe Glu Asp Ala Gly Glu Tyr Thr
305             310             315             320

tgc ttg gcg ggt aat tct att ggg ata tcc ttt cac tct gca tgg ttg      1008
Cys Leu Ala Gly Asn Ser Ile Gly Ile Ser Phe His Ser Ala Trp Leu
                325             330             335

aca gtt ctg cca gcg cct gga aga gaa aag gag att aca gct tcc cca      1056
Thr Val Leu Pro Ala Pro Gly Arg Glu Lys Glu Ile Thr Ala Ser Pro
                340             345             350

gac tac ctg tca gcg cta gag ccc aaa tct tgt gac aaa act cac aca      1104
Asp Tyr Leu Ser Ala Leu Glu Pro Lys Ser Cys Asp Lys Thr His Thr
            355             360             365

tgc cca ccg tgc cca gca cct gaa ctc ctg ggg gga ccg tca gtc ttc      1152
Cys Pro Pro Cys Pro Ala Pro Glu Leu Leu Gly Gly Pro Ser Val Phe
        370             375             380

ctc ttc ccc cca aaa ccc aag gac acc ctc atg atc tcc cgg acc cct      1200
Leu Phe Pro Pro Lys Pro Lys Asp Thr Leu Met Ile Ser Arg Thr Pro
```

```
      385                          390                          395                          400

      gag gtc aca tgc gtg gtg gtg gac gtg agc cac gaa gac cct gag gtc      1248
      Glu Val Thr Cys Val Val Val Asp Val Ser His Glu Asp Pro Glu Val
                      405                 410                 415

      aag ttc aac tgg tac gtg gac ggc gtg gag gtg cat aat gcc aag aca      1296
      Lys Phe Asn Trp Tyr Val Asp Gly Val Glu Val His Asn Ala Lys Thr
                      420                 425                 430

      aag ccg cgg gag gag cag tac aac agc acg tac cgt gtg gtc agc gtc      1344
      Lys Pro Arg Glu Glu Gln Tyr Asn Ser Thr Tyr Arg Val Val Ser Val
                      435                 440                 445

      ctc acc gtc ctg cac cag gac tgg ctg aat ggc aag gag tac aag tgc      1392
      Leu Thr Val Leu His Gln Asp Trp Leu Asn Gly Lys Glu Tyr Lys Cys
                      450                 455                 460

      aag gtc tcc aac aaa gcc ctc cca gcc ccc atc gag aaa acc atc tcc      1440
      Lys Val Ser Asn Lys Ala Leu Pro Ala Pro Ile Glu Lys Thr Ile Ser
      465                 470                 475                 480

      aaa gcc aaa ggg cag ccc cga gaa cca cag gtg tac acc ctg ccc cca      1488
      Lys Ala Lys Gly Gln Pro Arg Glu Pro Gln Val Tyr Thr Leu Pro Pro
                      485                 490                 495

      tcc cgg gat gag ctg acc aag aac cag gtc agc ctg acc tgc ctg gtc      1536
      Ser Arg Asp Glu Leu Thr Lys Asn Gln Val Ser Leu Thr Cys Leu Val
                      500                 505                 510

      aaa ggc ttc tat ccc agc gac atc gcc gtg gag tgg gag agc aat ggg      1584
      Lys Gly Phe Tyr Pro Ser Asp Ile Ala Val Glu Trp Glu Ser Asn Gly
                      515                 520                 525

      cag ccg gag aac aac tac aag acc acg cct ccc gtg ctg gac tcc gac      1632
      Gln Pro Glu Asn Asn Tyr Lys Thr Thr Pro Pro Val Leu Asp Ser Asp
                      530                 535                 540

      ggc tcc ttc ttc ctc tac agc aag ctc acc gtg gac aag agc agg tgg      1680
      Gly Ser Phe Phe Leu Tyr Ser Lys Leu Thr Val Asp Lys Ser Arg Trp
      545                 550                 555                 560

      cag cag ggg aac gtc ttc tca tgc tcc gtg atg cat gag gct ctg cac      1728
      Gln Gln Gly Asn Val Phe Ser Cys Ser Val Met His Glu Ala Leu His
                      565                 570                 575

      aac cac tac acg cag aag agc ctc tcc ctg tct ccg ggt aaa tga          1773
      Asn His Tyr Thr Gln Lys Ser Leu Ser Leu Ser Pro Gly Lys
                      580                 585                 590
```

<210> 48
<211> 590
<212> PRT
<213> Homo sapiens

<400> 48

Arg Pro Ser Phe Ser Leu Val Glu Asp Thr Thr Leu Glu Pro Glu Glu
1               5               10              15

```
Pro Pro Thr Lys Tyr Gln Ile Ser Gln Pro Glu Val Tyr Val Ala Ala
        20                25                30

Pro Gly Glu Ser Leu Glu Val Arg Cys Leu Leu Lys Asp Ala Ala Val
        35                40                45

Ile Ser Trp Thr Lys Asp Gly Val His Leu Gly Pro Asn Asn Arg Thr
        50                55                60

Val Leu Ile Gly Glu Tyr Leu Gln Ile Lys Gly Ala Thr Pro Arg Asp
65                70                75                80

Ser Gly Leu Tyr Ala Cys Thr Ala Ser Arg Thr Val Asp Ser Glu Thr
            85                90                95

Trp Tyr Phe Met Val Asn Val Thr Asp Ala Ile Ser Ser Gly Asp Asp
            100               105               110

Glu Asp Asp Thr Asp Gly Ala Glu Asp Phe Val Ser Glu Asn Ser Asn
        115               120               125

Asn Lys Arg Ala Pro Tyr Trp Thr Asn Thr Glu Lys Met Glu Lys Arg
        130               135               140

Leu His Ala Val Pro Ala Ala Asn Thr Val Lys Phe Arg Cys Pro Ala
145               150               155               160

Gly Gly Asn Pro Met Pro Thr Met Arg Trp Leu Lys Asn Gly Lys Glu
            165               170               175

Phe Lys Gln Glu His Arg Ile Gly Gly Tyr Lys Val Arg Asn Gln His
        180               185               190

Trp Ser Leu Ile Met Glu Ser Val Val Pro Ser Asp Lys Gly Asn Tyr
        195               200               205

Thr Cys Val Val Glu Asn Glu Tyr Gly Ser Ile Asn His Thr Tyr His
        210               215               220

Leu Asp Val Val Glu Arg Ser Pro His Arg Pro Ile Leu Gln Ala Gly
225               230               235               240

Leu Pro Ala Asn Ala Ser Thr Val Val Gly Gly Asp Val Glu Phe Val
            245               250               255
```

```
Cys Lys Val Tyr Ser Asp Ala Gln Pro His Ile Gln Trp Ile Lys His
        260                 265             270

Val Glu Lys Asn Gly Ser Lys Tyr Gly Pro Asp Gly Leu Pro Tyr Leu
        275                 280             285

Lys Val Leu Lys Ala Ala Gly Val Asn Thr Thr Asp Lys Glu Ile Glu
        290                 295             300

Val Leu Tyr Ile Arg Asn Val Thr Phe Glu Asp Ala Gly Glu Tyr Thr
305                 310             315                 320

Cys Leu Ala Gly Asn Ser Ile Gly Ile Ser Phe His Ser Ala Trp Leu
                325                 330             335

Thr Val Leu Pro Ala Pro Gly Arg Glu Lys Glu Ile Thr Ala Ser Pro
        340                 345             350

Asp Tyr Leu Ser Ala Leu Glu Pro Lys Ser Cys Asp Lys Thr His Thr
        355                 360             365

Cys Pro Pro Cys Pro Ala Pro Glu Leu Leu Gly Gly Pro Ser Val Phe
        370                 375             380

Leu Phe Pro Pro Lys Pro Lys Asp Thr Leu Met Ile Ser Arg Thr Pro
385                 390             395                 400

Glu Val Thr Cys Val Val Val Asp Val Ser His Glu Asp Pro Glu Val
                405                 410             415

Lys Phe Asn Trp Tyr Val Asp Gly Val Glu Val His Asn Ala Lys Thr
                420                 425             430

Lys Pro Arg Glu Glu Gln Tyr Asn Ser Thr Tyr Arg Val Val Ser Val
        435                 440             445

Leu Thr Val Leu His Gln Asp Trp Leu Asn Gly Lys Glu Tyr Lys Cys
        450                 455             460

Lys Val Ser Asn Lys Ala Leu Pro Ala Pro Ile Glu Lys Thr Ile Ser
465                 470                 475                 480

Lys Ala Lys Gly Gln Pro Arg Glu Pro Gln Val Tyr Thr Leu Pro Pro
                485                 490             495

Ser Arg Asp Glu Leu Thr Lys Asn Gln Val Ser Leu Thr Cys Leu Val
```

84

                      500                      505                      510

Lys Gly Phe Tyr Pro Ser Asp Ile Ala Val Glu Trp Glu Ser Asn Gly
        515                 520                 525

Gln Pro Glu Asn Asn Tyr Lys Thr Thr Pro Pro Val Leu Asp Ser Asp
        530                 535                 540

Gly Ser Phe Phe Leu Tyr Ser Lys Leu Thr Val Asp Lys Ser Arg Trp
545                 550                 555                 560

Gln Gln Gly Asn Val Phe Ser Cys Ser Val Met His Glu Ala Leu His
                565                 570                 575

Asn His Tyr Thr Gln Lys Ser Leu Ser Leu Ser Pro Gly Lys
        580                 585                 590

<210> 49
<211> 2403
<212> DNA
<213> Rattus sp.

<220>
<221> CDS
<222> (1)..(2403)

<400> 49

```
atg tgg ctg ctg ttg gct ttg ttg agc atc ttt cag gag aca cca gcc        48
Met Trp Leu Leu Leu Ala Leu Leu Ser Ile Phe Gln Glu Thr Pro Ala
1               5                   10                  15

ttc tcc ctt gag gcc tct gag gaa atg gaa cag gag ccc tgc cca gcc        96
Phe Ser Leu Glu Ala Ser Glu Glu Met Glu Gln Glu Pro Cys Pro Ala
                20                  25                  30

cca atc tcg gag cag caa gag cag gtg ttg act gtg gcc ctt ggg cag       144
Pro Ile Ser Glu Gln Gln Glu Gln Val Leu Thr Val Ala Leu Gly Gln
            35                  40                  45

cct gtg cgg cta tgc tgt ggc cgc act gag cgt ggt cgt cat tgg tac       192
Pro Val Arg Leu Cys Cys Gly Arg Thr Glu Arg Gly Arg His Trp Tyr
    50                  55                  60

aag gag ggc agc cgt tta gca tct gct ggg aga gta cgg ggc tgg aga       240
Lys Glu Gly Ser Arg Leu Ala Ser Ala Gly Arg Val Arg Gly Trp Arg
65                  70                  75                  80

ggc cgc ctg gag atc gcc agc ttc ctt cct gag gat gct ggc cgg tac       288
Gly Arg Leu Glu Ile Ala Ser Phe Leu Pro Glu Asp Ala Gly Arg Tyr
                85                  90                  95

ctc tgc ctg gcc cgt ggc tcc atg act gtc gta cac aat ctt act ttg       336
Leu Cys Leu Ala Arg Gly Ser Met Thr Val Val His Asn Leu Thr Leu
            100                 105                 110
```

86

```
att atg gat gac tcc tta ccc tcc atc aat aac gag gac ccc aag acc        384
Ile Met Asp Asp Ser Leu Pro Ser Ile Asn Asn Glu Asp Pro Lys Thr
        115                 120                 125

ctc agc agc tcc tcg agt ggg cac tcc tac ctg cag caa gca cct tac        432
Leu Ser Ser Ser Ser Ser Gly His Ser Tyr Leu Gln Gln Ala Pro Tyr
        130                 135                 140

tgg aca cac ccc caa cgc atg gag aag aaa ctg cac gcg gta cct gcc        480
Trp Thr His Pro Gln Arg Met Glu Lys Lys Leu His Ala Val Pro Ala
145                 150                 155                 160

ggg aac act gtc aaa ttc cgc tgt cca gct gca ggg aac ccc atg ccc        528
Gly Asn Thr Val Lys Phe Arg Cys Pro Ala Ala Gly Asn Pro Met Pro
                165                 170                 175

acc atc cac tgg ctc aag aac gga cag gcc ttc cac gga gag aat cgt        576
Thr Ile His Trp Leu Lys Asn Gly Gln Ala Phe His Gly Glu Asn Arg
                180                 185                 190

atc gga ggc att cgg ctg cgt cac caa cac tgg agc ctc gtg atg gag        624
Ile Gly Gly Ile Arg Leu Arg His Gln His Trp Ser Leu Val Met Glu
                195                 200                 205

agc gtg gtg ccc tca gac cgt ggc acg tac acg tgt ctt gtg gag aac        672
Ser Val Val Pro Ser Asp Arg Gly Thr Tyr Thr Cys Leu Val Glu Asn
        210                 215                 220

tct ctg ggt agc att cgc tac agc tat ctg ctg gat gtg ctg gag cgg        720
Ser Leu Gly Ser Ile Arg Tyr Ser Tyr Leu Leu Asp Val Leu Glu Arg
225                 230                 235                 240

tcc ccg cac cgg ccc atc ctg cag gcg gga ctc cca gcc aac acc acg        768
Ser Pro His Arg Pro Ile Leu Gln Ala Gly Leu Pro Ala Asn Thr Thr
                245                 250                 255

gct gtg gtt ggc agc aac gtg gag ctg ctg tgc aag gtg tac agt gac        816
Ala Val Val Gly Ser Asn Val Glu Leu Leu Cys Lys Val Tyr Ser Asp
                260                 265                 270

gcc cag ccg cac atc cag tgg ctg aag cac atc gtt atc aac ggc agc        864
Ala Gln Pro His Ile Gln Trp Leu Lys His Ile Val Ile Asn Gly Ser
                275                 280                 285

agt ttc ggc gct gat ggt ttc ccc tac gta caa gtc ctg aag aca aca        912
Ser Phe Gly Ala Asp Gly Phe Pro Tyr Val Gln Val Leu Lys Thr Thr
        290                 295                 300

gac atc aat agc tca gag gtg gag gtg ctg tat ctg agg aac gtg tcg        960
Asp Ile Asn Ser Ser Glu Val Glu Val Leu Tyr Leu Arg Asn Val Ser
305                 310                 315                 320

gct gag gat gca ggg gag tac acc tgc ctg gcg ggc aac tcc atc ggc       1008
Ala Glu Asp Ala Gly Glu Tyr Thr Cys Leu Ala Gly Asn Ser Ile Gly
                325                 330                 335

ctc tcc tac cag tca gcg tgg ctc aca gtg cta ccc gca gag gaa gaa       1056
Leu Ser Tyr Gln Ser Ala Trp Leu Thr Val Leu Pro Ala Glu Glu Glu
        340                 345                 350
```

```
gac ctc gcg tgg aca aca gca aca tcg gag gcc aga tat aca gat att      1104
Asp Leu Ala Trp Thr Thr Ala Thr Ser Glu Ala Arg Tyr Thr Asp Ile
        355             360             365

atc cta tat gta tct ggc tca ctg gct ttg gtt ttg ctc ctg ctg ctg      1152
Ile Leu Tyr Val Ser Gly Ser Leu Ala Leu Val Leu Leu Leu Leu Leu
        370             375             380

gcc ggg gtg tat cac cga caa gca atc cac ggc cac cac tct cga cag      1200
Ala Gly Val Tyr His Arg Gln Ala Ile His Gly His His Ser Arg Gln
385             390             395             400

cct gtc act gta cag aag ctg tcc cgg ttc cct ttg gcc cgg cag ttc      1248
Pro Val Thr Val Gln Lys Leu Ser Arg Phe Pro Leu Ala Arg Gln Phe
            405             410             415

tcc ttg gag tcg agg tcc tct ggc aag tca agt ttg tcc ctg gtg cga      1296
Ser Leu Glu Ser Arg Ser Ser Gly Lys Ser Ser Leu Ser Leu Val Arg
            420             425             430

ggt gtc cgg ctc tcc tcc agt ggc ccg ccc ttg ctc acg ggc ctt gtg      1344
Gly Val Arg Leu Ser Ser Ser Gly Pro Pro Leu Leu Thr Gly Leu Val
            435             440             445

agt cta gac cta cct ctc gat cca ctt tgg gag ttc ccc cgg gac agg      1392
Ser Leu Asp Leu Pro Leu Asp Pro Leu Trp Glu Phe Pro Arg Asp Arg
        450             455             460

ctg gtg ctc gga aag ccc ctg ggt gag ggc tgc ttt ggg caa gtg gtt      1440
Leu Val Leu Gly Lys Pro Leu Gly Glu Gly Cys Phe Gly Gln Val Val
465             470             475             480

cgt gca gaa gcc ctt ggc atg gat tcc tcc cgg cca gac caa acc agc      1488
Arg Ala Glu Ala Leu Gly Met Asp Ser Ser Arg Pro Asp Gln Thr Ser
            485             490             495

acc gtg gct gtg aag atg ctg aaa gac aat gcc tcc gac aag gat ttg      1536
Thr Val Ala Val Lys Met Leu Lys Asp Asn Ala Ser Asp Lys Asp Leu
        500             505             510

gca gac ctg atc tct gag atg gag atg atg aag cta atc gga aga cac      1584
Ala Asp Leu Ile Ser Glu Met Glu Met Met Lys Leu Ile Gly Arg His
        515             520             525

aag aac atc att aac ctg ctg ggt gtc tgc act cag gaa ggg ccc ctg      1632
Lys Asn Ile Ile Asn Leu Leu Gly Val Cys Thr Gln Glu Gly Pro Leu
        530             535             540

tat gtg att gtg gaa tat gcg gcc aag gga aac ctt cgg gaa ttc ctc      1680
Tyr Val Ile Val Glu Tyr Ala Ala Lys Gly Asn Leu Arg Glu Phe Leu
545             550             555             560

cgt gcc cgg cgt ccc cca ggc cct gat ctc agc cct gat ggg cct cgg      1728
Arg Ala Arg Arg Pro Pro Gly Pro Asp Leu Ser Pro Asp Gly Pro Arg
            565             570             575

agc agc gaa gga ccg ctc tcc ttc ccg gcc ctg gtc tcc tgt gcc tac      1776
Ser Ser Glu Gly Pro Leu Ser Phe Pro Ala Leu Val Ser Cys Ala Tyr
            580             585             590

cag gtg gcc cga ggc atg cag tat ctg gag tct cgg aag tgc atc cac      1824
```

```
        Gln Val Ala Arg Gly Met Gln Tyr Leu Glu Ser Arg Lys Cys Ile His
                595                 600                 605

        cgg gac ctg gct gcc cga aac gtg ctg gtg acc gag gat gac gtg atg    1872
        Arg Asp Leu Ala Ala Arg Asn Val Leu Val Thr Glu Asp Asp Val Met
                610                 615                 620

        aag atc gct gac ttt ggt ctg gcc cgt ggt gtc cac cac atc gac tac    1920
        Lys Ile Ala Asp Phe Gly Leu Ala Arg Gly Val His His Ile Asp Tyr
        625                 630                 635                 640

        tat aag aaa acc agc aat ggc cgc ctg cca gtc aag tgg atg gct cct    1968
        Tyr Lys Lys Thr Ser Asn Gly Arg Leu Pro Val Lys Trp Met Ala Pro
                        645                 650                 655

        gag gcg ttg ttt gac cgt gta tac aca cac cag agt gac gtg tgg tcc    2016
        Glu Ala Leu Phe Asp Arg Val Tyr Thr His Gln Ser Asp Val Trp Ser
                        660                 665                 670

        ttc ggg atc ctg ctg tgg gaa atc ttc acc ctc ggg ggc tcc cca tac    2064
        Phe Gly Ile Leu Leu Trp Glu Ile Phe Thr Leu Gly Gly Ser Pro Tyr
                        675                 680                 685

        ccc ggc atc cca gtg gag gag ctg ttc tca ctg ctg cga gag ggg cac    2112
        Pro Gly Ile Pro Val Glu Glu Leu Phe Ser Leu Leu Arg Glu Gly His
                690                 695                 700

        agg atg gag cgg ccc cca aac tgc ccc tca gag ctg tat ggg cta atg    2160
        Arg Met Glu Arg Pro Pro Asn Cys Pro Ser Glu Leu Tyr Gly Leu Met
        705                 710                 715                 720

        agg gag tgt tgg cac gca gct cct tct cag agg ccg act ttt aag cag    2208
        Arg Glu Cys Trp His Ala Ala Pro Ser Gln Arg Pro Thr Phe Lys Gln
                        725                 730                 735

        ctg gtg gaa gct ctg gac aag gtc ctg ctg gct gtc tct gaa gag tac    2256
        Leu Val Glu Ala Leu Asp Lys Val Leu Leu Ala Val Ser Glu Glu Tyr
                        740                 745                 750

        ctt gac ctc cgc ctg acc ttt gga ccc tat tcc ccc aac aat ggg gat    2304
        Leu Asp Leu Arg Leu Thr Phe Gly Pro Tyr Ser Pro Asn Asn Gly Asp
                        755                 760                 765

        gcc agc agc acg tgc tcc tcc agc gac tcg gtt ttc agc cac gac cct    2352
        Ala Ser Ser Thr Cys Ser Ser Ser Asp Ser Val Phe Ser His Asp Pro
                770                 775                 780

        ttg ccc ctc gag cca agc ccc ttc cca ttt cct gag gcg cag acc aca    2400
        Leu Pro Leu Glu Pro Ser Pro Phe Pro Phe Pro Glu Ala Gln Thr Thr
        785                 790                 795                 800

        tga                                                                2403
```

<210> 50
<211> 800
<212> PRT
<213> Rattus sp.

<400> 50

```
Met Trp Leu Leu Leu Ala Leu Leu Ser Ile Phe Gln Glu Thr Pro Ala
1               5                   10                  15

Phe Ser Leu Glu Ala Ser Glu Glu Met Glu Gln Glu Pro Cys Pro Ala
            20                  25                  30

Pro Ile Ser Glu Gln Gln Glu Gln Val Leu Thr Val Ala Leu Gly Gln
        35                  40                  45

Pro Val Arg Leu Cys Cys Gly Arg Thr Glu Arg Gly Arg His Trp Tyr
    50                  55                  60

Lys Glu Gly Ser Arg Leu Ala Ser Ala Gly Arg Val Arg Gly Trp Arg
65                  70                  75                  80

Gly Arg Leu Glu Ile Ala Ser Phe Leu Pro Glu Asp Ala Gly Arg Tyr
            85                  90                  95

Leu Cys Leu Ala Arg Gly Ser Met Thr Val Val His Asn Leu Thr Leu
            100                 105                 110

Ile Met Asp Asp Ser Leu Pro Ser Ile Asn Asn Glu Asp Pro Lys Thr
        115                 120                 125

Leu Ser Ser Ser Ser Ser Gly His Ser Tyr Leu Gln Gln Ala Pro Tyr
    130                 135                 140

Trp Thr His Pro Gln Arg Met Glu Lys Lys Leu His Ala Val Pro Ala
145                 150                 155                 160

Gly Asn Thr Val Lys Phe Arg Cys Pro Ala Ala Gly Asn Pro Met Pro
                165                 170                 175

Thr Ile His Trp Leu Lys Asn Gly Gln Ala Phe His Gly Glu Asn Arg
            180                 185                 190

Ile Gly Gly Ile Arg Leu Arg His Gln His Trp Ser Leu Val Met Glu
        195                 200                 205

Ser Val Val Pro Ser Asp Arg Gly Thr Tyr Thr Cys Leu Val Glu Asn
    210                 215                 220

Ser Leu Gly Ser Ile Arg Tyr Ser Tyr Leu Leu Asp Val Leu Glu Arg
225                 230                 235                 240

Ser Pro His Arg Pro Ile Leu Gln Ala Gly Leu Pro Ala Asn Thr Thr
```

245　　　　　　　　　　250　　　　　　　　　　255

Ala Val Val Gly Ser Asn Val Glu Leu Leu Cys Lys Val Tyr Ser Asp
　　　　　　　260　　　　　　　　　265　　　　　　　　　270

Ala Gln Pro His Ile Gln Trp Leu Lys His Ile Val Ile Asn Gly Ser
　　　　275　　　　　　　　　280　　　　　　　　　285

Ser Phe Gly Ala Asp Gly Phe Pro Tyr Val Gln Val Leu Lys Thr Thr
　　290　　　　　　　　　295　　　　　　　　　300

Asp Ile Asn Ser Ser Glu Val Glu Val Leu Tyr Leu Arg Asn Val Ser
305　　　　　　　　　310　　　　　　　　　315　　　　　　　　　320

Ala Glu Asp Ala Gly Glu Tyr Thr Cys Leu Ala Gly Asn Ser Ile Gly
　　　　　　　325　　　　　　　　　330　　　　　　　　　335

Leu Ser Tyr Gln Ser Ala Trp Leu Thr Val Leu Pro Ala Glu Glu Glu
　　　　　　　340　　　　　　　　　345　　　　　　　　　350

Asp Leu Ala Trp Thr Thr Ala Thr Ser Glu Ala Arg Tyr Thr Asp Ile
　　　　355　　　　　　　　　360　　　　　　　　　365

Ile Leu Tyr Val Ser Gly Ser Leu Ala Leu Val Leu Leu Leu Leu Leu
　　　　370　　　　　　　　　375　　　　　　　　　380

Ala Gly Val Tyr His Arg Gln Ala Ile His Gly His His Ser Arg Gln
385　　　　　　　　　390　　　　　　　　　395　　　　　　　　　400

Pro Val Thr Val Gln Lys Leu Ser Arg Phe Pro Leu Ala Arg Gln Phe
　　　　　　　405　　　　　　　　　410　　　　　　　　　415

Ser Leu Glu Ser Arg Ser Ser Gly Lys Ser Ser Leu Ser Leu Val Arg
　　　　　　　420　　　　　　　　　425　　　　　　　　　430

Gly Val Arg Leu Ser Ser Ser Gly Pro Pro Leu Leu Thr Gly Leu Val
　　　　　　　435　　　　　　　　　440　　　　　　　　　445

Ser Leu Asp Leu Pro Leu Asp Pro Leu Trp Glu Phe Pro Arg Asp Arg
　　　　450　　　　　　　　　455　　　　　　　　　460

Leu Val Leu Gly Lys Pro Leu Gly Glu Gly Cys Phe Gly Gln Val Val
465　　　　　　　　　470　　　　　　　　　475　　　　　　　　　480

Arg Ala Glu Ala Leu Gly Met Asp Ser Ser Arg Pro Asp Gln Thr Ser
　　　　　　　485　　　　　　　　　490　　　　　　　　　495

```
Thr Val Ala Val Lys Met Leu Lys Asp Asn Ala Ser Asp Lys Asp Leu
            500             505             510

Ala Asp Leu Ile Ser Glu Met Glu Met Met Lys Leu Ile Gly Arg His
            515             520             525

Lys Asn Ile Ile Asn Leu Leu Gly Val Cys Thr Gln Glu Gly Pro Leu
        530             535             540

Tyr Val Ile Val Glu Tyr Ala Ala Lys Gly Asn Leu Arg Glu Phe Leu
545             550             555             560

Arg Ala Arg Arg Pro Pro Gly Pro Asp Leu Ser Pro Asp Gly Pro Arg
                565             570             575

Ser Ser Glu Gly Pro Leu Ser Phe Pro Ala Leu Val Ser Cys Ala Tyr
            580             585             590

Gln Val Ala Arg Gly Met Gln Tyr Leu Glu Ser Arg Lys Cys Ile His
        595             600             605

Arg Asp Leu Ala Ala Arg Asn Val Leu Val Thr Glu Asp Asp Val Met
    610             615             620

Lys Ile Ala Asp Phe Gly Leu Ala Arg Gly Val His His Ile Asp Tyr
625             630             635             640

Tyr Lys Lys Thr Ser Asn Gly Arg Leu Pro Val Lys Trp Met Ala Pro
            645             650             655

Glu Ala Leu Phe Asp Arg Val Tyr Thr His Gln Ser Asp Val Trp Ser
            660             665             670

Phe Gly Ile Leu Leu Trp Glu Ile Phe Thr Leu Gly Gly Ser Pro Tyr
            675             680             685

Pro Gly Ile Pro Val Glu Glu Leu Phe Ser Leu Leu Arg Glu Gly His
        690             695             700

Arg Met Glu Arg Pro Pro Asn Cys Pro Ser Glu Leu Tyr Gly Leu Met
705             710             715             720

Arg Glu Cys Trp His Ala Ala Pro Ser Gln Arg Pro Thr Phe Lys Gln
                725             730             735
```

```
        Leu Val Glu Ala Leu Asp Lys Val Leu Leu Ala Val Ser Glu Glu Tyr
                740                     745             750

        Leu Asp Leu Arg Leu Thr Phe Gly Pro Tyr Ser Pro Asn Asn Gly Asp
                755                     760             765

        Ala Ser Ser Thr Cys Ser Ser Ser Asp Ser Val Phe Ser His Asp Pro
                770                     775             780

        Leu Pro Leu Glu Pro Ser Pro Phe Pro Phe Pro Glu Ala Gln Thr Thr
                785                     790             795             800
```

<210> 51
<211> 31
<212> DNA
<213> Mus sp.


<400> 51
cggccgccag gtacctggaa ttcggcttgg g 31


<210> 52
<211> 31
<212> DNA
<213> Mus sp.


<400> 52
ctagaaggca ctctagaggc tgatctcgag c 31


<210> 53
<211> 14
<212> DNA
<213> Mus sp.


<400> 53
cgagctctgt acaa 14


<210> 54
<211> 22
<212> DNA
<213> Mus sp.


<400> 54
ctagttgtac agagctcggt ac 22


<210> 55
<211> 32
<212> DNA
<213> Mus sp.


<400> 55
gcgccgtcct ggagctcctg ctgctgaggt gg 32


<210> 56
<211> 24
<212> DNA

<213> Mus sp.

<400> 56
ggcaactaga aggcacagtc gagg 24

<210> 57
<211> 27
<212> DNA
<213> Mus sp.

<400> 57
gtccctggga tcctcgagga agtggag 27

<210> 58
<211> 31
<212> DNA
<213> Mus sp.

<400> 58
gcgcctgccc gagctccagc ccggccagca g 31

<210> 59
<211> 19
<212> PRT
<213> Mus sp.

<400> 59

```
Leu Ala Leu Ala Val Leu Leu Leu Ala Gly Leu Glu Leu Leu Leu Leu
1               5                   10                  15

Arg Trp Gln
```

<210> 60
<211> 12
<212> PRT
<213> Mus sp.

<400> 60

```
Leu Ala Leu Ala Val Leu Leu Leu Leu Arg Trp Gln
1               5                   10
```

<210> 61
<211> 52
<212> DNA
<213> Mus sp.

<400> 61
ggccttggct gtgctcctgc tgctgaggtg gcagtttcct gcacactaca gg 52

<210> 62
<211> 52
<212> DNA
<213> Mus sp.

<400> 62

cctgtagtgt gcaggaaact gccacctcag cagcaggagc acagccaagg cc 52

<210> 63
<211> 23
<212> DNA
<213> Mus sp.

<400> 63

cgactggagc acgaggacac tga 23

<210> 64
<211> 21
<212> DNA
<213> Mus sp.

<400> 64

tatgcaaggc ttacaaccac a 21

<210> 65
<211> 24
<212> DNA
<213> Mus sp.

<400> 65

ctcattcctg ttgaagctct tgac 24

<210> 66
<211> 27
<212> DNA
<213> Mus sp.

<400> 66

acactcagca cgggacaaac tcttctc 27

<210> 67
<211> 27
<212> DNA
<213> Mus sp.

<400> 67

acactctgca ggagacagac tcttttc 27

<210> 68
<211> 1062
<212> DNA
<213> Homo sapiens

<220>
<221> CDS
<222> (1)..(1062)

<400> 68

```
ctg gag gcc tct gag gaa gtg gag ctt gag ccc tgc ctg gct ccc agc      48
Leu Glu Ala Ser Glu Glu Val Glu Leu Glu Pro Cys Leu Ala Pro Ser
1               5                   10              15

ctg gag cag caa gag cag gag ctg aca gta gcc ctt ggg cag cct gtg      96
Leu Glu Gln Gln Glu Gln Glu Leu Thr Val Ala Leu Gly Gln Pro Val
                20                  25                  30

cgg ctg tgc tgt ggg cgg gct gag cgt ggt ggc cac tgg tac aag gag     144
Arg Leu Cys Cys Gly Arg Ala Glu Arg Gly Gly His Trp Tyr Lys Glu
            35                  40                  45

ggc agt cgc ctg gca cct gct ggc cgt gta cgg ggc tgg agg ggc cgc     192
Gly Ser Arg Leu Ala Pro Ala Gly Arg Val Arg Gly Trp Arg Gly Arg
        50                  55                  60

cta gag att gcc agc ttc cta cct gag gat gct ggc cgc tac ctc tgc     240
Leu Glu Ile Ala Ser Phe Leu Pro Glu Asp Ala Gly Arg Tyr Leu Cys
65                  70                  75                  80

ctg gca cga ggc tcc atg atc gtc ctg cag aat ctc acc ttg att aca     288
Leu Ala Arg Gly Ser Met Ile Val Leu Gln Asn Leu Thr Leu Ile Thr
                85                  90                  95

ggt gac tcc ttg acc tcc agc aac gat gat gag gac ccc aag tcc cat     336
Gly Asp Ser Leu Thr Ser Ser Asn Asp Asp Glu Asp Pro Lys Ser His
                100                 105                 110

agg gac ctc tcg aat agg cac agt tac ccc cag caa gca ccc tac tgg     384
Arg Asp Leu Ser Asn Arg His Ser Tyr Pro Gln Gln Ala Pro Tyr Trp
            115                 120                 125

aca cac ccc cag cgc atg gag aag aaa ctg cat gca gta cct gcg ggg     432
Thr His Pro Gln Arg Met Glu Lys Lys Leu His Ala Val Pro Ala Gly
        130                 135                 140

aac acc gtc aag ttc cgc tgt cca gct gca ggc aac ccc acg ccc acc     480
Asn Thr Val Lys Phe Arg Cys Pro Ala Ala Gly Asn Pro Thr Pro Thr
145                 150                 155                 160

atc cgc tgg ctt aag gat gga cag gcc ttt cat ggg gag aac cgc att     528
Ile Arg Trp Leu Lys Asp Gly Gln Ala Phe His Gly Glu Asn Arg Ile
                165                 170                 175

gga ggc att cgg ctg cgc cat cag cac tgg agt ctc gtg atg gag agc     576
Gly Gly Ile Arg Leu Arg His Gln His Trp Ser Leu Val Met Glu Ser
            180                 185                 190

gtg gtg ccc tcg gac cgc ggc aca tac acc tgc ctg gta gag aac gct     624
Val Val Pro Ser Asp Arg Gly Thr Tyr Thr Cys Leu Val Glu Asn Ala
            195                 200                 205

gtg ggc agc atc cgt tat aac tac ctg cta gat gtg ctg gag cgg tcc     672
Val Gly Ser Ile Arg Tyr Asn Tyr Leu Leu Asp Val Leu Glu Arg Ser
            210                 215                 220
```

```
ccg cac cgg ccc atc ctg cag gcc ggg ctc ccg gcc aac acc aca gcc    720
Pro His Arg Pro Ile Leu Gln Ala Gly Leu Pro Ala Asn Thr Thr Ala
225                 230                 235                 240

gtg gtg ggc agc gac gtg gag ctg ctg tgc aag gtg tac agc gat gcc    768
Val Val Gly Ser Asp Val Glu Leu Leu Cys Lys Val Tyr Ser Asp Ala
                    245                 250                 255

cag ccc cac atc cag tgg ctg aag cac atc gtc atc aac ggc agc agc    816
Gln Pro His Ile Gln Trp Leu Lys His Ile Val Ile Asn Gly Ser Ser
            260                 265                 270

ttc gga gcc gac ggt ttc ccc tat gtg caa gtc cta aag act gca gac    864
Phe Gly Ala Asp Gly Phe Pro Tyr Val Gln Val Leu Lys Thr Ala Asp
            275                 280                 285

atc aat agc tca gag gtg gag gtc ctg tac ctg cgg aac gtg tca gcc    912
Ile Asn Ser Ser Glu Val Glu Val Leu Tyr Leu Arg Asn Val Ser Ala
        290                 295                 300

gag gac gca ggc gag tac acc tgc ctc gca ggc aat tcc atc ggc ctc    960
Glu Asp Ala Gly Glu Tyr Thr Cys Leu Ala Gly Asn Ser Ile Gly Leu
305                 310                 315                 320

tcc tac cag tct gcc tgg ctc acg gtg cca gag gag gac ccc aca tgg   1008
Ser Tyr Gln Ser Ala Trp Leu Thr Val Pro Glu Glu Asp Pro Thr Trp
                325                 330                 335

acc gca gca gcg ccc gag gcc gct agc tgg agc cac ccg cag ttc gaa   1056
Thr Ala Ala Ala Pro Glu Ala Ala Ser Trp Ser His Pro Gln Phe Glu
                340                 345                 350

aaa tga                                                            1062
Lys
```

<210> 69
<211> 353
<212> PRT
<213> Homo sapiens

<400> 69

```
    Leu Glu Ala Ser Glu Glu Val Glu Leu Glu Pro Cys Leu Ala Pro Ser
    1               5                   10                  15

    Leu Glu Gln Gln Glu Gln Glu Leu Thr Val Ala Leu Gly Gln Pro Val
                    20                  25                  30

    Arg Leu Cys Cys Gly Arg Ala Glu Arg Gly Gly His Trp Tyr Lys Glu
                35                  40                  45

    Gly Ser Arg Leu Ala Pro Ala Gly Arg Val Arg Gly Trp Arg Gly Arg
            50                  55                  60
```

Leu Glu Ile Ala Ser Phe Leu Pro Glu Asp Ala Gly Arg Tyr Leu Cys
65                  70              75                  80

Leu Ala Arg Gly Ser Met Ile Val Leu Gln Asn Leu Thr Leu Ile Thr
                85              90                  95

Gly Asp Ser Leu Thr Ser Ser Asn Asp Asp Glu Asp Pro Lys Ser His
            100             105             110

Arg Asp Leu Ser Asn Arg His Ser Tyr Pro Gln Gln Ala Pro Tyr Trp
        115             120             125

Thr His Pro Gln Arg Met Glu Lys Lys Leu His Ala Val Pro Ala Gly
    130             135             140

Asn Thr Val Lys Phe Arg Cys Pro Ala Ala Gly Asn Pro Thr Pro Thr
145             150             155             160

Ile Arg Trp Leu Lys Asp Gly Gln Ala Phe His Gly Glu Asn Arg Ile
            165             170             175

Gly Gly Ile Arg Leu Arg His Gln His Trp Ser Leu Val Met Glu Ser
        180             185             190

Val Val Pro Ser Asp Arg Gly Thr Tyr Thr Cys Leu Val Glu Asn Ala
    195             200             205

Val Gly Ser Ile Arg Tyr Asn Tyr Leu Leu Asp Val Leu Glu Arg Ser
    210             215             220

Pro His Arg Pro Ile Leu Gln Ala Gly Leu Pro Ala Asn Thr Thr Ala
225             230             235             240

Val Val Gly Ser Asp Val Glu Leu Leu Cys Lys Val Tyr Ser Asp Ala
            245             250             255

Gln Pro His Ile Gln Trp Leu Lys His Ile Val Ile Asn Gly Ser Ser
        260             265             270

Phe Gly Ala Asp Gly Phe Pro Tyr Val Gln Val Leu Lys Thr Ala Asp
    275             280             285

Ile Asn Ser Ser Glu Val Glu Val Leu Tyr Leu Arg Asn Val Ser Ala
    290             295             300

Glu Asp Ala Gly Glu Tyr Thr Cys Leu Ala Gly Asn Ser Ile Gly Leu

98

|  | 305 |  |  |  | 310 |  |  |  | 315 |  |  |  | 320 |

Ser Tyr Gln Ser Ala Trp Leu Thr Val Pro Glu Glu Asp Pro Thr Trp
            325             330             335

Thr Ala Ala Ala Pro Glu Ala Ala Ser Trp Ser His Pro Gln Phe Glu
            340             345             350

Lys

<210> 70
<211> 98
<212> PRT
<213> Homo sapiens

<400> 70

Gln Ala Pro Tyr Trp Thr His Pro Gln Arg Met Glu Lys Lys Leu His
1           5            10             15

Ala Val Pro Ala Gly Asn Thr Val Lys Phe Arg Cys Pro Ala Ala Gly
           20            25             30

Asn Pro Thr Pro Thr Ile Arg Trp Leu Lys Asp Gly Gln Ala Phe His
           35            40             45

Gly Glu Asn Arg Ile Gly Gly Ile Arg Leu Arg His Gln His Trp Ser
       50            55             60

Leu Val Met Glu Ser Val Val Pro Ser Asp Arg Gly Thr Tyr Thr Cys
65             70            75             80

Leu Val Glu Asn Ala Val Gly Ser Ile Arg Tyr Asn Tyr Leu Leu Asp
           85            90             95

Val Leu

<210> 71
<211> 648
<212> DNA
<213> Homo sapiens

<220>
<221> CDS
<222> (1)..(648)

<400> 71

```
cag agc gaa ttg act cag cca ccc tca gcg tct ggg acc ccc ggg cag    48
Gln Ser Glu Leu Thr Gln Pro Pro Ser Ala Ser Gly Thr Pro Gly Gln
1               5                   10                  15

agg gtc acc atc tct tgt tct gga ggc tac tcc aac atg gga agc aat    96
Arg Val Thr Ile Ser Cys Ser Gly Gly Tyr Ser Asn Met Gly Ser Asn
                20                  25                  30

tat gca cac tgg tac cag cag ctc cca gga acg gcc ccc aaa ctc ctc   144
Tyr Ala His Trp Tyr Gln Gln Leu Pro Gly Thr Ala Pro Lys Leu Leu
        35                  40                  45

atc tat aac aat aat cag cgg ccc tca ggg gtc cct gac cga ttc tct   192
Ile Tyr Asn Asn Asn Gln Arg Pro Ser Gly Val Pro Asp Arg Phe Ser
    50                  55                  60

ggc tcc aag tct ggc acc tca gcc tcc ctg gcc atc agt ggg ctc cgg   240
Gly Ser Lys Ser Gly Thr Ser Ala Ser Leu Ala Ile Ser Gly Leu Arg
65                  70                  75                  80

tcc gag gat gag gct gat tat tac tgt gca gca tgg gat gac agc ctg   288
Ser Glu Asp Glu Ala Asp Tyr Tyr Cys Ala Ala Trp Asp Asp Ser Leu
                85                  90                  95

agt ggt ccg gtg ttc ggc gga ggg acc aag ctg acc gtc cta ggt cag   336
Ser Gly Pro Val Phe Gly Gly Gly Thr Lys Leu Thr Val Leu Gly Gln
                100                 105                 110

ccc aag gct gcc ccc tcg gtc act ctg ttc ccg ccc tcc tct gag gag   384
Pro Lys Ala Ala Pro Ser Val Thr Leu Phe Pro Pro Ser Ser Glu Glu
        115                 120                 125

ctt caa gcc aac aag gcc aca ctg gtg tgt ctg atc agt gac ttc tac   432
Leu Gln Ala Asn Lys Ala Thr Leu Val Cys Leu Ile Ser Asp Phe Tyr
    130                 135                 140

ccg gga gct gtg aca gtg gcc tgg aag gca gat ggc agc ccc gtc aag   480
Pro Gly Ala Val Thr Val Ala Trp Lys Ala Asp Gly Ser Pro Val Lys
145                 150                 155                 160

gcg gga gtg gag acc acc aca ccc tcc aaa caa agc aac aac aag tac   528
Ala Gly Val Glu Thr Thr Thr Pro Ser Lys Gln Ser Asn Asn Lys Tyr
                165                 170                 175

gcg gcc agc agc tat ctg agc ctg acg cct gag cag tgg aag tcc cac   576
Ala Ala Ser Ser Tyr Leu Ser Leu Thr Pro Glu Gln Trp Lys Ser His
        180                 185                 190

aga agc tac agc tgc cag gtc acg cat gaa ggg agc acc gtg gag aag   624
Arg Ser Tyr Ser Cys Gln Val Thr His Glu Gly Ser Thr Val Glu Lys
    195                 200                 205

aca gtg gcc cct aca gaa tgt tca                                    648
Thr Val Ala Pro Thr Glu Cys Ser
    210                 215
```

<210> 72
<211> 216
<212> PRT
<213> Homo sapiens

<400> 72

```
Gln Ser Glu Leu Thr Gln Pro Pro Ser Ala Ser Gly Thr Pro Gly Gln
1               5               10              15

Arg Val Thr Ile Ser Cys Ser Gly Gly Tyr Ser Asn Met Gly Ser Asn
            20              25              30

Tyr Ala His Trp Tyr Gln Gln Leu Pro Gly Thr Ala Pro Lys Leu Leu
        35              40              45

Ile Tyr Asn Asn Asn Gln Arg Pro Ser Gly Val Pro Asp Arg Phe Ser
    50              55              60

Gly Ser Lys Ser Gly Thr Ser Ala Ser Leu Ala Ile Ser Gly Leu Arg
65              70              75              80

Ser Glu Asp Glu Ala Asp Tyr Tyr Cys Ala Ala Trp Asp Asp Ser Leu
            85              90              95

Ser Gly Pro Val Phe Gly Gly Gly Thr Lys Leu Thr Val Leu Gly Gln
        100             105             110

Pro Lys Ala Ala Pro Ser Val Thr Leu Phe Pro Pro Ser Ser Glu Glu
        115             120             125

Leu Gln Ala Asn Lys Ala Thr Leu Val Cys Leu Ile Ser Asp Phe Tyr
    130             135             140

Pro Gly Ala Val Thr Val Ala Trp Lys Ala Asp Gly Ser Pro Val Lys
145             150             155             160

Ala Gly Val Glu Thr Thr Thr Pro Ser Lys Gln Ser Asn Asn Lys Tyr
            165             170             175

Ala Ala Ser Ser Tyr Leu Ser Leu Thr Pro Glu Gln Trp Lys Ser His
        180             185             190

Arg Ser Tyr Ser Cys Gln Val Thr His Glu Gly Ser Thr Val Glu Lys
        195             200             205

Thr Val Ala Pro Thr Glu Cys Ser
    210             215
```

<210> 73
<211> 9
<212> PRT

<213> Homo sapiens

<400> 73

```
                              Ser Asn Met Gly Ser Asn Tyr Ala His
                              1                   5
```

<210> 74
<211> 5
<212> PRT
<213> Homo sapiens

<400> 74

```
                                 Tyr Asn Asn Asn Gln
                                 1                   5
```

<210> 75
<211> 8
<212> PRT
<213> Homo sapiens

<400> 75

```
                              Ala Trp Asp Asp Ser Leu Ser Gly
                              1                   5
```

<210> 76
<211> 411
<212> DNA
<213> Homo sapiens

<220>
<221> CDS
<222> (1) .. (411)

<400> 76

```
gaa gtt caa ttg tta gag tct ggt ggc ggt ctt gtt cag cct ggt ggt      48
Glu Val Gln Leu Leu Glu Ser Gly Gly Gly Leu Val Gln Pro Gly Gly
1               5                   10                  15

tct tta cgt ctt tct tgc gct gct tcc gga ttc act ttc tct cct tac      96
Ser Leu Arg Leu Ser Cys Ala Ala Ser Gly Phe Thr Phe Ser Pro Tyr
            20                  25                  30

tgg atg ggt tgg gtt cgc caa gct cct ggt aaa ggt ttg gag tgg gtt     144
Trp Met Gly Trp Val Arg Gln Ala Pro Gly Lys Gly Leu Glu Trp Val
            35                  40                  45

tct gtt atc tgg cct tct ggt ggc act act gat tat gct gac tcc gtt     192
Ser Val Ile Trp Pro Ser Gly Gly Thr Thr Asp Tyr Ala Asp Ser Val
        50                  55                  60

aaa ggt cgc ttc act atc tct aga gac aac tct aag aat act ctc tac     240
Lys Gly Arg Phe Thr Ile Ser Arg Asp Asn Ser Lys Asn Thr Leu Tyr
65                  70                  75                  80

ttg cag atg aac agc tta agg gct gag gac acg gcc gtg tat tac tgt     288
Leu Gln Met Asn Ser Leu Arg Ala Glu Asp Thr Ala Val Tyr Tyr Cys
                85                  90                  95

gcg aga gtc cat cca gct ggc cac aac gac cac tgg ggc cag gga acc     336
Ala Arg Val His Pro Ala Gly His Asn Asp His Trp Gly Gln Gly Thr
            100                 105                 110

ctg gtc acc gtc tca agc gcc tcc acc aag ggc cca tcg gtc ttc ccg     384
Leu Val Thr Val Ser Ser Ala Ser Thr Lys Gly Pro Ser Val Phe Pro
            115                 120                 125

cta gca ccc tcc tcc aag agc acc tct                                 411
Leu Ala Pro Ser Ser Lys Ser Thr Ser
            130                 135
```

<210> 77
<211> 137
<212> PRT
<213> Homo sapiens

<400> 77

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Pro Tyr
            20                  25                  30

Trp Met Gly Trp Val Arg Gln Ala Pro Gly Lys Gly Leu Glu Trp Val
        35                  40                  45

Ser Val Ile Trp Pro Ser Gly Gly Thr Thr Asp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
            85                  90                  95

Ala Arg Val His Pro Ala Gly His Asn Asp His Trp Gly Gln Gly Thr
            100                 105                 110

Leu Val Thr Val Ser Ser Ala Ser Thr Lys Gly Pro Ser Val Phe Pro
            115                 120                 125

Leu Ala Pro Ser Ser Lys Ser Thr Ser
        130                 135
```

<210> 78
<211> 9
<212> PRT
<213> Homo sapiens

<400> 78

```
Phe Thr Phe Ser Pro Tyr Trp Met Gly
1               5
```

<210> 79
<211> 10
<212> PRT
<213> Homo sapiens

<400> 79

```
Val Ile Trp Pro Ser Gly Gly Thr Thr Asp
1               5                   10
```

<210> 80
<211> 10

<212> PRT
<213> Homo sapiens

<400> 80

```
          Arg Val His Pro Ala Gly His Asn Asp His
          1               5                   10
```

<210> 81
<211> 645
<212> DNA
<213> Homo sapiens

<220>
<221> CDS
<222> (1)..(645)

<400> 81

```
caa gac atc cag atg acc cag tct cca tcc tcc ctg tct ttg tct cca      48
Gln Asp Ile Gln Met Thr Gln Ser Pro Ser Ser Leu Ser Leu Ser Pro
1               5                   10                  15

ggg gaa aga gcc acc ctc tcc tgc agg gcc agt cag agt att aac aga      96
Gly Glu Arg Ala Thr Leu Ser Cys Arg Ala Ser Gln Ser Ile Asn Arg
            20                  25                  30

aac tta ggc tgg tac cag cag aag cct ggc cag gct ccc agg ctc ctc     144
Asn Leu Gly Trp Tyr Gln Gln Lys Pro Gly Gln Ala Pro Arg Leu Leu
        35                  40                  45

atc tat ggt gcg tcc acc agg gcc act ggt atc cca gcc agg ttc act     192
Ile Tyr Gly Ala Ser Thr Arg Ala Thr Gly Ile Pro Ala Arg Phe Thr
    50                  55                  60

ggc agt ggg tct ggg aca gag ttc tct ctc acc atc agc agc ctg cag     240
```

```
Gly Ser Gly Ser Gly Thr Glu Phe Ser Leu Thr Ile Ser Ser Leu Gln
65                  70              75                  80

tct gaa gat tat gca att tat tat tgt cag cag tat gat aac tgg ccg        288
Ser Glu Asp Tyr Ala Ile Tyr Tyr Cys Gln Gln Tyr Asp Asn Trp Pro
                85                  90                  95

atc atc ttc ggc caa ggg aca cga ctg gag att aaa gga act gtg gct        336
Ile Ile Phe Gly Gln Gly Thr Arg Leu Glu Ile Lys Gly Thr Val Ala
                100                 105                 110

gca cca tct gtc ttc atc ttc ccg cca tct gat gag cag ttg aaa tct        384
Ala Pro Ser Val Phe Ile Phe Pro Pro Ser Asp Glu Gln Leu Lys Ser
            115                 120                 125

gga act gcc tct gtt gtg tgc ctg ctg aat aac ttc tat ccc aga gag        432
Gly Thr Ala Ser Val Val Cys Leu Leu Asn Asn Phe Tyr Pro Arg Glu
        130                 135                 140

gcc aaa gta cag tgg aag gtg gat aac gcc ctc caa tcg ggt aac tcc        480
Ala Lys Val Gln Trp Lys Val Asp Asn Ala Leu Gln Ser Gly Asn Ser
145                 150                 155                 160

cag gag agt gtc aca gag cag gac agc aag gac agc acc tac agc ctc        528
Gln Glu Ser Val Thr Glu Gln Asp Ser Lys Asp Ser Thr Tyr Ser Leu
                165                 170                 175

agc agc acc ctg acg ctg agc aaa gca gac tac gag aaa cac aaa gtc        576
Ser Ser Thr Leu Thr Leu Ser Lys Ala Asp Tyr Glu Lys His Lys Val
            180                 185                 190

tac gcc tgc gaa gtc acc cat cag ggc ctg agc tcg ccc gtc aca aag        624
Tyr Ala Cys Glu Val Thr His Gln Gly Leu Ser Ser Pro Val Thr Lys
        195                 200                 205

agc ttc aac agg gga gag tgt                                            645
Ser Phe Asn Arg Gly Glu Cys
        210                 215
```

<210> 82
<211> 215
<212> PRT
<213> Homo sapiens

<400> 82

```
Gln Asp Ile Gln Met Thr Gln Ser Pro Ser Ser Leu Ser Leu Ser Pro
1               5                   10                  15

Gly Glu Arg Ala Thr Leu Ser Cys Arg Ala Ser Gln Ser Ile Asn Arg
            20                  25                  30

Asn Leu Gly Trp Tyr Gln Gln Lys Pro Gly Gln Ala Pro Arg Leu Leu
                35                  40                  45

Ile Tyr Gly Ala Ser Thr Arg Ala Thr Gly Ile Pro Ala Arg Phe Thr
        50                  55                  60

Gly Ser Gly Ser Gly Thr Glu Phe Ser Leu Thr Ile Ser Ser Leu Gln
65                  70                  75                  80

Ser Glu Asp Tyr Ala Ile Tyr Tyr Cys Gln Gln Tyr Asp Asn Trp Pro
                85                  90                  95

Ile Ile Phe Gly Gln Gly Thr Arg Leu Glu Ile Lys Gly Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
            165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
            195                 200                 205

Ser Phe Asn Arg Gly Glu Cys
    210                 215
```

<210> 83
<211> 7
<212> PRT
<213> Homo sapiens

<400> 83

Ser Ile Asn Arg Asn Leu Gly
1               5

<210> 84
<211> 5
<212> PRT
<213> Homo sapiens

<400> 84

Tyr Gly Ala Ser Thr

1               5

<210> 85
<211> 6
<212> PRT
<213> Homo sapiens

<400> 85

Gln Tyr Asp Asn Trp Pro
1               5

<210> 86
<211> 411
<212> DNA
<213> Homo sapiens

<220>
<221> CDS
<222> (1)..(411)

<400> 86

```
gaa gtt caa ttg tta gag tct ggt ggc ggt ctt gtt cag cct ggt ggt      48
Glu Val Gln Leu Leu Glu Ser Gly Gly Gly Leu Val Gln Pro Gly Gly
1               5                   10                  15

tct tta cgt ctt tct tgc gct gct tcc gga ttc act ttc tct cgt tac      96
Ser Leu Arg Leu Ser Cys Ala Ala Ser Gly Phe Thr Phe Ser Arg Tyr
                20                  25                  30

ttt atg aag tgg gtt cgc caa gct cct ggt aaa ggt ttg gag tgg gtt     144
Phe Met Lys Trp Val Arg Gln Ala Pro Gly Lys Gly Leu Glu Trp Val
            35                  40                  45

tct tgg atc tct cct tct ggt ggc ctt act gag tat gct gac tcc gtt     192
Ser Trp Ile Ser Pro Ser Gly Gly Leu Thr Glu Tyr Ala Asp Ser Val
        50                  55                  60

aaa ggt cgc ttc act atc tct aga gac aac tct aag aat act ctc tac     240
Lys Gly Arg Phe Thr Ile Ser Arg Asp Asn Ser Lys Asn Thr Leu Tyr
65                  70                  75                  80

ttg cag atg aac agc tta agg gct gag gac acg gcc gtg tat tac tgt     288
Leu Gln Met Asn Ser Leu Arg Ala Glu Asp Thr Ala Val Tyr Tyr Cys
                85                  90                  95

gcc aga gcc tca tac ggt gcc tgg ctg gac tac tgg ggc cag ggc acc     336
Ala Arg Ala Ser Tyr Gly Ala Trp Leu Asp Tyr Trp Gly Gln Gly Thr
                100                 105                 110

ctg gtc acc gtc tca agc gcc tcc acc aag ggc cca tcg gtc ttc ccg     384
Leu Val Thr Val Ser Ser Ala Ser Thr Lys Gly Pro Ser Val Phe Pro
            115                 120                 125

cta gca ccc tcc tcc aag agc acc tct                                 411
Leu Ala Pro Ser Ser Lys Ser Thr Ser
            130                 135
```

<210> 87
<211> 137
<212> PRT
<213> Homo sapiens

<400> 87

```
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser Arg Tyr
            20              25              30

Phe Met Lys Trp Val Arg Gln Ala Pro Gly Lys Gly Leu Glu Trp Val
        35              40              45

Ser Trp Ile Ser Pro Ser Gly Gly Leu Thr Glu Tyr Ala Asp Ser Val
    50              55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
            85              90              95

Ala Arg Ala Ser Tyr Gly Ala Trp Leu Asp Tyr Trp Gly Gln Gly Thr
        100             105             110

Leu Val Thr Val Ser Ser Ala Ser Thr Lys Gly Pro Ser Val Phe Pro
        115             120             125

Leu Ala Pro Ser Ser Lys Ser Thr Ser
    130             135
```

<210> 88
<211> 9
<212> PRT
<213> Homo sapiens

<400> 88

```
Phe Thr Phe Ser Arg Tyr Phe Met Lys
1               5
```

<210> 89
<211> 10
<212> PRT
<213> Homo sapiens

<400> 89

```
Trp Ile Ser Pro Ser Gly Gly Leu Thr Glu
1               5               10
```

<210> 90
<211> 10

110

<212> PRT
<213> Homo sapiens

<400> 90

Arg Ala Ser Tyr Gly Ala Trp Leu Asp Tyr
1               5               10

<210> 91
<211> 651
<212> DNA
<213> Homo sapiens

<220>
<221> CDS
<222> (1)..(651)

<400> 91

```
cag agc gtc ttg act cag cca ccc tca gcg tct ggg acc ccc ggc cag      48
Gln Ser Val Leu Thr Gln Pro Pro Ser Ala Ser Gly Thr Pro Gly Gln
1               5                   10                  15

ggg gtc acc atc tct tgt tct gga agc agc tcc aac atc gga agt aat      96
Gly Val Thr Ile Ser Cys Ser Gly Ser Ser Ser Asn Ile Gly Ser Asn
                20                  25                  30

agt gtt aac tgg tac cag cag ctc cca gga gcg gcc ccc aaa ctc ctc     144
Ser Val Asn Trp Tyr Gln Gln Leu Pro Gly Ala Ala Pro Lys Leu Leu
                35                  40                  45

atc tat agt aac aat ctg cgg ccc tca ggg gtc cct gcc cga ttc tct     192
Ile Tyr Ser Asn Asn Leu Arg Pro Ser Gly Val Pro Ala Arg Phe Ser
        50                  55                  60

ggc tcc aag tct ggc acc tca gcc tcc ctg gcc atc agt ggc ctc cgg     240
Gly Ser Lys Ser Gly Thr Ser Ala Ser Leu Ala Ile Ser Gly Leu Arg
65                  70                  75                  80

tct gag gat gag gct gat tat tac tgt gca gcc tgg gat gac ggc ctg     288
Ser Glu Asp Glu Ala Asp Tyr Tyr Cys Ala Ala Trp Asp Asp Gly Leu
                85                  90                  95

gat gcc tat gtg gtg ttc ggc gga ggg acc aaa ctg acc gtc ctg ggt     336
Asp Ala Tyr Val Val Phe Gly Gly Gly Thr Lys Leu Thr Val Leu Gly
                100                 105                 110

cag ccc aag gcc aac ccc act gtc act ctg ttc ccg ccc tcc tct gag     384
Gln Pro Lys Ala Asn Pro Thr Val Thr Leu Phe Pro Pro Ser Ser Glu
            115                 120                 125

gag ctc caa gcc aac aag gcc aca cta gtg tgt ctg atc agt gac ttc     432
Glu Leu Gln Ala Asn Lys Ala Thr Leu Val Cys Leu Ile Ser Asp Phe
```

```
                130                      135                      140
```

```
tac ccg gga gct gtg aca gtg gcc tgg aag gca gat ggc agc ccc gtc    480
Tyr Pro Gly Ala Val Thr Val Ala Trp Lys Ala Asp Gly Ser Pro Val
145             150             155             160
```

```
aag gcg gga gtg gag acc acc aaa ccc tcc aaa cag agc aac aac aag    528
Lys Ala Gly Val Glu Thr Thr Lys Pro Ser Lys Gln Ser Asn Asn Lys
                165             170             175
```

```
tac gcg gcc agc agc tac ctg agc ctg acg ccc gag cag tgg aag tcc    576
Tyr Ala Ala Ser Ser Tyr Leu Ser Leu Thr Pro Glu Gln Trp Lys Ser
            180             185             190
```

```
cac aga agc tac agc tgc cag gtc acg cat gaa ggg agc acc gtg gag    624
His Arg Ser Tyr Ser Cys Gln Val Thr His Glu Gly Ser Thr Val Glu
            195             200             205
```

```
aag aca gtg gcc cct gca gaa tgc tct                                651
Lys Thr Val Ala Pro Ala Glu Cys Ser
            210             215
```

<210> 92
<211> 217
<212> PRT
<213> Homo sapiens

<400> 92

```
Gln Ser Val Leu Thr Gln Pro Pro Ser Ala Ser Gly Thr Pro Gly Gln
1               5               10              15
```

```
Gly Val Thr Ile Ser Cys Ser Gly Ser Ser Ser Asn Ile Gly Ser Asn
            20              25              30
```

```
Ser Val Asn Trp Tyr Gln Gln Leu Pro Gly Ala Ala Pro Lys Leu Leu
            35              40              45
```

```
Ile Tyr Ser Asn Asn Leu Arg Pro Ser Gly Val Pro Ala Arg Phe Ser
        50              55              60
```

```
Gly Ser Lys Ser Gly Thr Ser Ala Ser Leu Ala Ile Ser Gly Leu Arg
65              70              75              80
```

```
Ser Glu Asp Glu Ala Asp Tyr Tyr Cys Ala Ala Trp Asp Asp Gly Leu
            85              90              95
```

```
Asp Ala Tyr Val Val Phe Gly Gly Gly Thr Lys Leu Thr Val Leu Gly
            100             105             110
```

```
Gln Pro Lys Ala Asn Pro Thr Val Thr Leu Phe Pro Pro Ser Ser Glu
            115             120             125
```

```
Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Gly Ser Pro Val
145                 150                 155                 160

Lys Ala Gly Val Glu Thr Thr Lys Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
            195                 200                 205

Lys Thr Val Ala Pro Ala Glu Cys Ser
    210                 215
```

<210> 93
<211> 9
<212> PRT
<213> Homo sapiens

<400> 93

```
Ser Asn Ile Gly Ser Asn Ser Val Asn
1               5
```

<210> 94
<211> 5
<212> PRT
<213> Homo sapiens

<400> 94

```
Tyr Ser Asn Asn Leu
1               5
```

<210> 95
<211> 9
<212> PRT
<213> Homo sapiens

<400> 95

```
Ala Trp Asp Asp Gly Leu Asp Ala Tyr
1               5
```

<210> 96
<211> 414

<212> DNA
<213> Homo sapiens

<220>
<221> CDS
<222> (1)..(414)

<400> 96

| gaa | gtt | caa | ttg | tta | gag | tct | ggt | ggc | ggt | ctt | gtt | cag | cct | ggt | ggt | 48 |
| Glu | Val | Gln | Leu | Leu | Glu | Ser | Gly | Gly | Gly | Leu | Val | Gln | Pro | Gly | Gly | |
| 1 | | | | 5 | | | | | 10 | | | | | 15 | | |

| tct | tta | cgt | ctt | tct | tgc | gct | gct | tcc | gga | ttc | act | ttc | tct | act | tac | 96 |
| Ser | Leu | Arg | Leu | Ser | Cys | Ala | Ala | Ser | Gly | Phe | Thr | Phe | Ser | Thr | Tyr | |
| | | | 20 | | | | | 25 | | | | | 30 | | | |

| tgg | atg | gct | tgg | gtt | cgc | caa | gct | cct | ggt | aaa | ggt | ttg | gag | tgg | gtt | 144 |
| Trp | Met | Ala | Trp | Val | Arg | Gln | Ala | Pro | Gly | Lys | Gly | Leu | Glu | Trp | Val | |
| | | 35 | | | | | 40 | | | | | 45 | | | | |

| tct | gtt | atc | ggt | cct | tct | ggt | ggc | atg | act | gtt | tat | gct | gac | tcc | gtt | 192 |
| Ser | Val | Ile | Gly | Pro | Ser | Gly | Gly | Met | Thr | Val | Tyr | Ala | Asp | Ser | Val | |
| | | 50 | | | | | 55 | | | | | 60 | | | | |

| aaa | ggt | cgc | ttc | act | atc | tct | aga | gac | aac | tct | aag | aat | act | ctc | tac | 240 |
| Lys | Gly | Arg | Phe | Thr | Ile | Ser | Arg | Asp | Asn | Ser | Lys | Asn | Thr | Leu | Tyr | |
| 65 | | | | | 70 | | | | | 75 | | | | | 80 | |

| ttg | cag | atg | aac | agc | tta | agg | gct | gag | gac | acg | gcc | gtg | tat | tac | tgt | 288 |
| Leu | Gln | Met | Asn | Ser | Leu | Arg | Ala | Glu | Asp | Thr | Ala | Val | Tyr | Tyr | Cys | |
| | | | | 85 | | | | | 90 | | | | | 95 | | |

| gca | cgg | att | aat | agt | ggg | agc | tcc | cac | ttt | gac | tac | tgg | ggc | cag | gga | 336 |
| Ala | Arg | Ile | Asn | Ser | Gly | Ser | Ser | His | Phe | Asp | Tyr | Trp | Gly | Gln | Gly | |
| | | | 100 | | | | | 105 | | | | | 110 | | | |

| acc | ctg | gtc | acc | gtc | tca | agc | gcc | tcc | acc | aag | ggc | cca | tcg | gtc | ttc | 384 |
| Thr | Leu | Val | Thr | Val | Ser | Ser | Ala | Ser | Thr | Lys | Gly | Pro | Ser | Val | Phe | |
| | | 115 | | | | | 120 | | | | | 125 | | | | |

| ccg | cta | gca | ccc | tcc | tcc | aag | agc | acc | tct | | | | | | | 414 |
| Pro | Leu | Ala | Pro | Ser | Ser | Lys | Ser | Thr | Ser | | | | | | | |
| | | 130 | | | | | 135 | | | | | | | | | |

<210> 97
<211> 138
<212> PRT
<213> Homo sapiens

<400> 97

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Trp Met Ala Trp Val Arg Gln Ala Pro Gly Lys Gly Leu Glu Trp Val

        35                  40                  45

Ser Val Ile Gly Pro Ser Gly Gly Met Thr Val Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Asn Ser Gly Ser Ser His Phe Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser
        130                 135
```

<210> 98
<211> 9
<212> PRT
<213> Homo sapiens

<400> 98

```
Phe Thr Phe Ser Thr Tyr Trp Met Ala
1               5
```

<210> 99
<211> 10
<212> PRT
<213> Homo sapiens

<400> 99

```
Val Ile Gly Pro Ser Gly Gly Met Thr Val
1               5                   10
```

<210> 100
<211> 11
<212> PRT
<213> Homo sapiens

<400> 100

```
Arg Ile Asn Ser Gly Ser Ser His Phe Asp Tyr
1               5                   10
```

<210> 101
<211> 651
<212> DNA
<213> Homo sapiens

<220>
<221> CDS
<222> (1)..(651)

<400> 101

```
cag tac gaa ttg act cag cca ccc tca gcg tct ggg acc ccc ggg cag      48
Gln Tyr Glu Leu Thr Gln Pro Pro Ser Ala Ser Gly Thr Pro Gly Gln
1               5                   10                  15

agg gtc acc atc tct tgt tct gga agc agc tcc aac atc gga agc aat      96
Arg Val Thr Ile Ser Cys Ser Gly Ser Ser Ser Asn Ile Gly Ser Asn
                20                  25                  30

att gta cac tgg tac cag cag ctc cca gga acg gcc ccc aaa ctc ctc     144
Ile Val His Trp Tyr Gln Gln Leu Pro Gly Thr Ala Pro Lys Leu Leu
            35                  40                  45

ata tat ggt aat aat cag cgg ccc tca ggg gtc cct gac cga ttc tct     192
Ile Tyr Gly Asn Asn Gln Arg Pro Ser Gly Val Pro Asp Arg Phe Ser
        50                  55                  60

gtc tcc aag tct ggc tcc tca gcc tcc ctg gcc atc agt ggg ctc cag     240
Val Ser Lys Ser Gly Ser Ser Ala Ser Leu Ala Ile Ser Gly Leu Gln
65                  70                  75                  80

tct gag gat gag gct gat tat tac tgt gca tca tgg gat gac agc ctg     288
Ser Glu Asp Glu Ala Asp Tyr Tyr Cys Ala Ser Trp Asp Asp Ser Leu
                85                  90                  95

aaa gcc ttt tat gtc ttc gga act ggg acc aag gtc acc gtc cta ggt     336
Lys Ala Phe Tyr Val Phe Gly Thr Gly Thr Lys Val Thr Val Leu Gly
            100                 105                 110

cag ccc aag gcc aac ccc act gtc act ctg ttc ccg ccc tcc tct gag     384
Gln Pro Lys Ala Asn Pro Thr Val Thr Leu Phe Pro Pro Ser Ser Glu
        115                 120                 125

gag ctc caa gcc aac aag gcc aca cta gtg tgt ctg atc agt gac ttc     432
Glu Leu Gln Ala Asn Lys Ala Thr Leu Val Cys Leu Ile Ser Asp Phe
    130                 135                 140

tac ccg gga gct gtg aca gtg gcc tgg aag gca gat ggc agc ccc gtc     480
Tyr Pro Gly Ala Val Thr Val Ala Trp Lys Ala Asp Gly Ser Pro Val
145                 150                 155                 160

aag gcg gga gtg gag acc acc aaa ccc tcc aaa cag agc aac aac aag     528
Lys Ala Gly Val Glu Thr Thr Lys Pro Ser Lys Gln Ser Asn Asn Lys
                165                 170                 175

tac gcg gcc agc agc tac ctg agc ctg acg ccc gag cag tgg aag tcc     576
Tyr Ala Ala Ser Ser Tyr Leu Ser Leu Thr Pro Glu Gln Trp Lys Ser
            180                 185                 190

cac aga agc tac agc tgc cag gtc acg cat gaa ggg agc acc gtg gag     624
His Arg Ser Tyr Ser Cys Gln Val Thr His Glu Gly Ser Thr Val Glu
        195                 200                 205

aag aca gtg gcc cct aca gaa tgt tca                                  651
Lys Thr Val Ala Pro Thr Glu Cys Ser
        210                 215
```

<210> 102
<211> 217
<212> PRT

<213> Homo sapiens

<400> 102

Gln Tyr Glu Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ile Val His Trp Tyr Gln Gln Leu Pro Gly Thr Ala Pro Lys Leu Leu
            35                  40                  45

Ile Tyr Gly Asn Asn Gln Arg Pro Ser Gly Val Pro Asp Arg Phe Ser
        50                  55                  60

Val Ser Lys Ser Gly Ser Ser Ala Ser Leu Ala Ile Ser Gly Leu Gln
65                  70                  75                  80

Ser Glu Asp Glu Ala Asp Tyr Tyr Cys Ala Ser Trp Asp Asp Ser Leu
                85                  90                  95

Lys Ala Phe Tyr Val Phe Gly Thr Gly Thr Lys Val Thr Val Leu Gly
            100                 105                 110

Gln Pro Lys Ala Asn Pro Thr Val Thr Leu Phe Pro Pro Ser Ser Glu
            115                 120                 125

Glu Leu Gln Ala Asn Lys Ala Thr Leu Val Cys Leu Ile Ser Asp Phe
        130                 135                 140

Tyr Pro Gly Ala Val Thr Val Ala Trp Lys Ala Asp Gly Ser Pro Val
145                 150                 155                 160

Lys Ala Gly Val Glu Thr Thr Lys Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
            195                 200                 205

Lys Thr Val Ala Pro Thr Glu Cys Ser
        210                 215

<210> 103

<211> 9
<212> PRT
<213> Homo sapiens

<400> 103

```
            Ser Asn Ile Gly Ser Asn Ile Val His
            1               5
```

<210> 104
<211> 5
<212> PRT
<213> Homo sapiens

<400> 104

```
            Tyr Gly Asn Asn Gln
            1               5
```

<210> 105
<211> 9
<212> PRT
<213> Homo sapiens

<400> 105

```
            Ser Trp Asp Asp Ser Leu Lys Ala Phe
            1               5
```

<210> 106
<211> 420
<212> DNA
<213> Homo sapiens

<220>
<221> CDS
<222> (1)..(420)

<400> 106

```
gaa gtt caa ttg tta gag tct ggt ggc ggt ctt gtt cag cct ggt ggt     48
Glu Val Gln Leu Leu Glu Ser Gly Gly Gly Leu Val Gln Pro Gly Gly
1               5               10              15

tct tta cgt ctt tct tgc gct gct tcc gga ttc act ttc tct cat tac     96
Ser Leu Arg Leu Ser Cys Ala Ala Ser Gly Phe Thr Phe Ser His Tyr
                20              25              30

tgg atg ggt tgg gtt cgc caa gct cct ggt aaa ggt ttg gag tgg gtt    144
```

```
Trp Met Gly Trp Val Arg Gln Ala Pro Gly Lys Gly Leu Glu Trp Val
        35                  40                  45

tct gtt atc tct cct tct ggt ggc ctt act att tat gct gac tcc gtt    192
Ser Val Ile Ser Pro Ser Gly Gly Leu Thr Ile Tyr Ala Asp Ser Val
        50                  55                  60

aaa ggt cgc ttc act atc tct aga gac aac tct aag aat act ctc tac    240
Lys Gly Arg Phe Thr Ile Ser Arg Asp Asn Ser Lys Asn Thr Leu Tyr
65                  70                  75                  80

ttg cag atg aac agc tta agg gct gag gac acg gcc gtg tat tac tgt    288
Leu Gln Met Asn Ser Leu Arg Ala Glu Asp Thr Ala Val Tyr Tyr Cys
                85                  90                  95

gca cgg agc agt ggc tgg gct ctg gat gct ttt gat atc tgg ggc caa    336
Ala Arg Ser Ser Gly Trp Ala Leu Asp Ala Phe Asp Ile Trp Gly Gln
                100                 105                 110

ggg aca atg gtc acc gtc tca agc gcc tcc acc aag ggc cca tcg gtc    384
Gly Thr Met Val Thr Val Ser Ser Ala Ser Thr Lys Gly Pro Ser Val
            115                 120                 125

ttc ccg cta gca ccc tcc tcc aag agc acc tct ggg                    420
Phe Pro Leu Ala Pro Ser Ser Lys Ser Thr Ser Gly
            130                 135                 140
```

<210> 107
<211> 140
<212> PRT
<213> Homo sapiens

<400> 107

```
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser His Tyr
            20              25              30

Trp Met Gly Trp Val Arg Gln Ala Pro Gly Lys Gly Leu Glu Trp Val
        35              40              45

Ser Val Ile Ser Pro Ser Gly Gly Leu Thr Ile Tyr Ala Asp Ser Val
    50              55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
            85              90              95

Ala Arg Ser Ser Gly Trp Ala Leu Asp Ala Phe Asp Ile Trp Gly Gln
        100             105             110

Gly Thr Met Val Thr Val Ser Ser Ala Ser Thr Lys Gly Pro Ser Val
        115             120             125

Phe Pro Leu Ala Pro Ser Ser Lys Ser Thr Ser Gly
    130             135             140
```

```
<210> 108
<211> 9
<212> PRT
<213> Homo sapiens

<400> 108
```

```
Phe Thr Phe Ser His Tyr Trp Met Gly
1               5
```

```
<210> 109
<211> 10
<212> PRT
<213> Homo sapiens

<400> 109
```

```
Val Ile Ser Pro Ser Gly Gly Leu Thr Ile
1               5               10
```

```
<210> 110
<211> 12
<212> PRT
```

<213> Homo sapiens

<400> 110

```
              Arg Ser Ser Gly Trp Ala Leu Asp Ala Phe Asp Ile
              1               5                   10
```

<210> 111
<211> 1215
<212> DNA
<213> Homo sapiens

<220>
<221> CDS
<222> (1)..(1215)

<400> 111

```
atg cgg ctg ctg ctg gcc ctg ttg ggg gtc ctg ctg agt gtg cct ggg     48
Met Arg Leu Leu Leu Ala Leu Leu Gly Val Leu Leu Ser Val Pro Gly
1               5                   10                  15

cct cca gtc ttg tcc ctg gag gcc tct gag gaa gtg gag ctt gag ccc     96
Pro Pro Val Leu Ser Leu Glu Ala Ser Glu Glu Val Glu Leu Glu Pro
            20                  25                  30
```

```
tgc ctg gct ccc agc ctg gag cag caa gag cag gag ctg aca gta gcc      144
Cys Leu Ala Pro Ser Leu Glu Gln Gln Glu Gln Glu Leu Thr Val Ala
         35                  40                  45

ctt ggg cag cct gtg cgg ctg tgc tgt ggg cgg gct gag cgt ggt ggc      192
Leu Gly Gln Pro Val Arg Leu Cys Cys Gly Arg Ala Glu Arg Gly Gly
         50                  55                  60

cac tgg tac aag gag ggc agt cgc ctg gca cct gct ggc cgt gta cgg      240
His Trp Tyr Lys Glu Gly Ser Arg Leu Ala Pro Ala Gly Arg Val Arg
65                  70                  75                  80

ggc tgg agg ggc cgc cta gag att gcc agc ttc cta cct gag gat gct      288
Gly Trp Arg Gly Arg Leu Glu Ile Ala Ser Phe Leu Pro Glu Asp Ala
                 85                  90                  95

ggc cgc tac ctc tgc ctg gca cga ggc tcc atg atc gtc ctg cag aat      336
Gly Arg Tyr Leu Cys Leu Ala Arg Gly Ser Met Ile Val Leu Gln Asn
            100                 105                 110

ctc acc ttg att aca ggt gac tcc ttg acc tcc agc aac gat gat gag      384
Leu Thr Leu Ile Thr Gly Asp Ser Leu Thr Ser Ser Asn Asp Asp Glu
         115                 120                 125

gac ccc aag tcc cat agg gac ctc tcg aat agg cac agt tac ccc cag      432
Asp Pro Lys Ser His Arg Asp Leu Ser Asn Arg His Ser Tyr Pro Gln
         130                 135                 140

caa gca ccc tac tgg aca cac ccc cag cgc atg gag aag aaa ctg cat      480
Gln Ala Pro Tyr Trp Thr His Pro Gln Arg Met Glu Lys Lys Leu His
145                 150                 155                 160

gca gta cct gcg ggg aac acc gtc aag ttc cgc tgt cca gct gca ggc      528
Ala Val Pro Ala Gly Asn Thr Val Lys Phe Arg Cys Pro Ala Ala Gly
                 165                 170                 175

aac ccc acg aaa act cac aca tgc cca ccg tgc cca gca cct gaa ctc      576
Asn Pro Thr Lys Thr His Thr Cys Pro Pro Cys Pro Ala Pro Glu Leu
            180                 185                 190

ctg ggg gga ccg tca gtc ttc ctc ttc ccc cca aaa ccc aag gac acc      624
Leu Gly Gly Pro Ser Val Phe Leu Phe Pro Pro Lys Pro Lys Asp Thr
            195                 200                 205

ctc atg atc tcc cgg acc cct gag gtc aca tgc gtg gtg gtg gac gtg      672
Leu Met Ile Ser Arg Thr Pro Glu Val Thr Cys Val Val Val Asp Val
         210                 215                 220

agc cac gaa gac cct gag gtc aag ttc aac tgg tac gtg gac ggc gtg      720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc      768
Glu Val His Asn Ala Lys Thr Lys Pro Arg Glu Glu Gln Tyr Asn Ser
                 245                 250                 255

acg tac cgt gtg gtc agc gtc ctc acc gtc ctg cac cag gac tgg ctg      816
Thr Tyr Arg Val Val Ser Val Leu Thr Val Leu His Gln Asp Trp Leu
            260                 265                 270
```

```
aat ggc aag gag tac aag tgc aag gtc tcc aac aaa gcc ctc cca gcc      864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca      912
Pro Ile Glu Lys Thr Ile Ser Lys Ala Lys Gly Gln Pro Arg Glu Pro
        290                 295                 300

cag gtg tac acc ctg ccc cca tcc cgg gat gag ctg acc aag aac cag      960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc     1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg     1056
Val Glu Trp Glu Ser Asn Gly Gln Pro Glu Asn Asn Tyr Lys Thr Thr
                340                 345                 350

cct ccc gtg ctg gac tcc gac ggc tcc ttc ttc ctc tac agc aag ctc     1104
Pro Pro Val Leu Asp Ser Asp Gly Ser Phe Phe Leu Tyr Ser Lys Leu
                355                 360                 365

acc gtg gac aag agc agg tgg cag cag ggg aac gtc ttc tca tgc tcc     1152
Thr Val Asp Lys Ser Arg Trp Gln Gln Gly Asn Val Phe Ser Cys Ser
        370                 375                 380

gtg atg cat gag gct ctg cac aac cac tac acg cag aag agc ctc tcc     1200
Val Met His Glu Ala Leu His Asn His Tyr Thr Gln Lys Ser Leu Ser
385                 390                 395                 400

ctg tct ccg ggt tga                                                  1215
Leu Ser Pro Gly
```

<210> 112
<211> 404
<212> PRT
<213> Homo sapiens

<400> 112

```
Met Arg Leu Leu Leu Ala Leu Leu Gly Val Leu Leu Ser Val Pro Gly
1               5                   10                  15


Pro Pro Val Leu Ser Leu Glu Ala Ser Glu Glu Val Glu Leu Glu Pro
            20                  25                  30


Cys Leu Ala Pro Ser Leu Glu Gln Gln Glu Gln Glu Leu Thr Val Ala
        35                  40                  45


Leu Gly Gln Pro Val Arg Leu Cys Cys Gly Arg Ala Glu Arg Gly Gly
        50                  55                  60


His Trp Tyr Lys Glu Gly Ser Arg Leu Ala Pro Ala Gly Arg Val Arg
```

```
                65                      70                      75                      80

Gly Trp Arg Gly Arg Leu Glu Ile Ala Ser Phe Leu Pro Glu Asp Ala
                85                      90                      95

Gly Arg Tyr Leu Cys Leu Ala Arg Gly Ser Met Ile Val Leu Gln Asn
                100                     105                     110

Leu Thr Leu Ile Thr Gly Asp Ser Leu Thr Ser Ser Asn Asp Asp Glu
                115                     120                     125

Asp Pro Lys Ser His Arg Asp Leu Ser Asn Arg His Ser Tyr Pro Gln
                130                     135                     140

Gln Ala Pro Tyr Trp Thr His Pro Gln Arg Met Glu Lys Lys Leu His
145                     150                     155                     160

Ala Val Pro Ala Gly Asn Thr Val Lys Phe Arg Cys Pro Ala Ala Gly
                165                     170                     175

Asn Pro Thr Lys Thr His Thr Cys Pro Pro Cys Pro Ala Pro Glu Leu
                180                     185                     190

Leu Gly Gly Pro Ser Val Phe Leu Phe Pro Pro Lys Pro Lys Asp Thr
                195                     200                     205

Leu Met Ile Ser Arg Thr Pro Glu Val Thr Cys Val Val Val Asp Val
                210                     215                     220

Ser His Glu Asp Pro Glu Val Lys Phe Asn Trp Tyr Val Asp Gly Val
225                     230                     235                     240

Glu Val His Asn Ala Lys Thr Lys Pro Arg Glu Glu Gln Tyr Asn Ser
                245                     250                     255

Thr Tyr Arg Val Val Ser Val Leu Thr Val Leu His Gln Asp Trp Leu
                260                     265                     270

Asn Gly Lys Glu Tyr Lys Cys Lys Val Ser Asn Lys Ala Leu Pro Ala
                275                     280                     285

Pro Ile Glu Lys Thr Ile Ser Lys Ala Lys Gly Gln Pro Arg Glu Pro
                290                     295                     300

Gln Val Tyr Thr Leu Pro Pro Ser Arg Asp Glu Leu Thr Lys Asn Gln
305                     310                     315                     320
```

```
        Val Ser Leu Thr Cys Leu Val Lys Gly Phe Tyr Pro Ser Asp Ile Ala
                        325             330                 335


        Val Glu Trp Glu Ser Asn Gly Gln Pro Glu Asn Asn Tyr Lys Thr Thr
                    340                 345                 350


        Pro Pro Val Leu Asp Ser Asp Gly Ser Phe Phe Leu Tyr Ser Lys Leu
                    355                 360                 365


        Thr Val Asp Lys Ser Arg Trp Gln Gln Gly Asn Val Phe Ser Cys Ser
                370                 375                 380


        Val Met His Glu Ala Leu His Asn His Tyr Thr Gln Lys Ser Leu Ser
        385                 390                 395                 400


        Leu Ser Pro Gly
```

<210> 113
<211> 1068
<212> DNA
<213> Homo sapiens

<220>
<221> CDS
<222> (1)..(1068)

<400> 113

```
        atg cgg ctg ctg ctg gcc ctg ttg ggg gtc ctg ctg agt gtg cct ggg       48
        Met Arg Leu Leu Leu Ala Leu Leu Gly Val Leu Leu Ser Val Pro Gly
        1               5                   10                  15

        cct cca gtc ttg tcc ctg gag gcc tct gag gaa gtg gag ctt gag ccc       96
        Pro Pro Val Leu Ser Leu Glu Ala Ser Glu Glu Val Glu Leu Glu Pro
                    20                  25                  30

        caa gca ccc tac tgg aca cac ccc cag cgc atg gag aag aaa ctg cat      144
        Gln Ala Pro Tyr Trp Thr His Pro Gln Arg Met Glu Lys Lys Leu His
                35                  40                  45

        gca gta cct gcg ggg aac acc gtc aag ttc cgc tgt cca gct gca ggc      192
        Ala Val Pro Ala Gly Asn Thr Val Lys Phe Arg Cys Pro Ala Ala Gly
            50                  55                  60

        aac ccc acg ccc acc atc cgc tgg ctt aag gat gga cag gcc ttt cat      240
        Asn Pro Thr Pro Thr Ile Arg Trp Leu Lys Asp Gly Gln Ala Phe His
        65                  70                  75                  80

        ggg gag aac cgc att gga ggc att cgg ctg cgc cat cag cac tgg agt      288
        Gly Glu Asn Arg Ile Gly Gly Ile Arg Leu Arg His Gln His Trp Ser
                        85                  90                  95
```

```
ctc gtg atg gag agc gtg gtg ccc tcg gac cgc ggc aca tac acc tgc        336
Leu Val Met Glu Ser Val Val Pro Ser Asp Arg Gly Thr Tyr Thr Cys
            100             105             110

ctg gta gag aac gct gtg ggc agc atc cgt tat aac tac ctg cta gat        384
Leu Val Glu Asn Ala Val Gly Ser Ile Arg Tyr Asn Tyr Leu Leu Asp
            115             120             125

gtg ctg aaa act cac aca tgc cca ccg tgc cca gca cct gaa ctc ctg        432
Val Leu Lys Thr His Thr Cys Pro Pro Cys Pro Ala Pro Glu Leu Leu
            130             135             140

ggg gga ccg tca gtc ttc ctc ttc ccc cca aaa ccc aag gac acc ctc        480
Gly Gly Pro Ser Val Phe Leu Phe Pro Pro Lys Pro Lys Asp Thr Leu
145             150             155             160

atg atc tcc cgg acc cct gag gtc aca tgc gtg gtg gtg gac gtg agc        528
Met Ile Ser Arg Thr Pro Glu Val Thr Cys Val Val Val Asp Val Ser
            165             170             175

cac gaa gac cct gag gtc aag ttc aac tgg tac gtg gac ggc gtg gag        576
His Glu Asp Pro Glu Val Lys Phe Asn Trp Tyr Val Asp Gly Val Glu
            180             185             190

gtg cat aat gcc aag aca aag ccg cgg gag gag cag tac aac agc acg        624
Val His Asn Ala Lys Thr Lys Pro Arg Glu Glu Gln Tyr Asn Ser Thr
            195             200             205

tac cgt gtg gtc agc gtc ctc acc gtc ctg cac cag gac tgg ctg aat        672
Tyr Arg Val Val Ser Val Leu Thr Val Leu His Gln Asp Trp Leu Asn
            210             215             220

ggc aag gag tac aag tgc aag gtc tcc aac aaa gcc ctc cca gcc ccc        720
Gly Lys Glu Tyr Lys Cys Lys Val Ser Asn Lys Ala Leu Pro Ala Pro
225             230             235             240

atc gag aaa acc atc tcc aaa gcc aaa ggg cag ccc cga gaa cca cag        768
Ile Glu Lys Thr Ile Ser Lys Ala Lys Gly Gln Pro Arg Glu Pro Gln
            245             250             255

gtg tac acc ctg ccc cca tcc cgg gat gag ctg acc aag aac cag gtc        816
Val Tyr Thr Leu Pro Pro Ser Arg Asp Glu Leu Thr Lys Asn Gln Val
            260             265             270

agc ctg acc tgc ctg gtc aaa ggc ttc tat ccc agc gac atc gcc gtg        864
Ser Leu Thr Cys Leu Val Lys Gly Phe Tyr Pro Ser Asp Ile Ala Val
            275             280             285

gag tgg gag agc aat ggg cag ccg gag aac aac tac aag acc acg cct        912
Glu Trp Glu Ser Asn Gly Gln Pro Glu Asn Asn Tyr Lys Thr Thr Pro
            290             295             300

ccc gtg ctg gac tcc gac ggc tcc ttc ttc ctc tac agc aag ctc acc        960
Pro Val Leu Asp Ser Asp Gly Ser Phe Phe Leu Tyr Ser Lys Leu Thr
305             310             315             320

gtg gac aag agc agg tgg cag cag ggg aac gtc ttc tca tgc tcc gtg      1008
Val Asp Lys Ser Arg Trp Gln Gln Gly Asn Val Phe Ser Cys Ser Val
            325             330             335

atg cat gag gct ctg cac aac cac tac acg cag aag agc ctc tcc ctg      1056
```

EP 2 315 781 B1

```
Met His Glu Ala Leu His Asn His Tyr Thr Gln Lys Ser Leu Ser Leu
            340                     345                     350


tct ccg ggt tga                                                      1068
Ser Pro Gly
            355
```

<210> 114
<211> 355
<212> PRT
<213> Homo sapiens

<400> 114

**128**

Met Arg Leu Leu Leu Ala Leu Leu Gly Val Leu Leu Ser Val Pro Gly
1            5                 10              15

Pro Pro Val Leu Ser Leu Glu Ala Ser Glu Glu Val Glu Leu Glu Pro
         20              25              30

Gln Ala Pro Tyr Trp Thr His Pro Gln Arg Met Glu Lys Lys Leu His
         35              40              45

Ala Val Pro Ala Gly Asn Thr Val Lys Phe Arg Cys Pro Ala Ala Gly
    50              55              60

Asn Pro Thr Pro Thr Ile Arg Trp Leu Lys Asp Gly Gln Ala Phe His
65              70              75              80

Gly Glu Asn Arg Ile Gly Gly Ile Arg Leu Arg His Gln His Trp Ser
             85              90              95

Leu Val Met Glu Ser Val Val Pro Ser Asp Arg Gly Thr Tyr Thr Cys
        100             105             110

Leu Val Glu Asn Ala Val Gly Ser Ile Arg Tyr Asn Tyr Leu Leu Asp
        115             120             125

Val Leu Lys Thr His Thr Cys Pro Pro Cys Pro Ala Pro Glu Leu Leu
    130             135             140

Gly Gly Pro Ser Val Phe Leu Phe Pro Pro Lys Pro Lys Asp Thr Leu
145             150             155             160

Met Ile Ser Arg Thr Pro Glu Val Thr Cys Val Val Val Asp Val Ser
             165             170             175

His Glu Asp Pro Glu Val Lys Phe Asn Trp Tyr Val Asp Gly Val Glu
             180             185             190

```
Val His Asn Ala Lys Thr Lys Pro Arg Glu Glu Gln Tyr Asn Ser Thr
        195                 200                 205

Tyr Arg Val Val Ser Val Leu Thr Val Leu His Gln Asp Trp Leu Asn
        210                 215                 220

Gly Lys Glu Tyr Lys Cys Lys Val Ser Asn Lys Ala Leu Pro Ala Pro
225                 230                 235                 240

Ile Glu Lys Thr Ile Ser Lys Ala Lys Gly Gln Pro Arg Glu Pro Gln
                245                 250                 255

Val Tyr Thr Leu Pro Pro Ser Arg Asp Glu Leu Thr Lys Asn Gln Val
            260                 265                 270

Ser Leu Thr Cys Leu Val Lys Gly Phe Tyr Pro Ser Asp Ile Ala Val
        275                 280                 285

Glu Trp Glu Ser Asn Gly Gln Pro Glu Asn Asn Tyr Lys Thr Thr Pro
    290                 295                 300

Pro Val Leu Asp Ser Asp Gly Ser Phe Phe Leu Tyr Ser Lys Leu Thr
305                 310                 315                 320

Val Asp Lys Ser Arg Trp Gln Gln Gly Asn Val Phe Ser Cys Ser Val
            325                 330                 335

Met His Glu Ala Leu His Asn His Tyr Thr Gln Lys Ser Leu Ser Leu
        340                 345                 350

Ser Pro Gly
        355
```

<210> 115
<211> 1188
<212> DNA
<213> Homo sapiens

<220>
<221> CDS
<222> (1)..(1188)

<400> 115

```
atg cgg ctg ctg ctg gcc ctg ttg ggg gtc ctg ctg agt gtg cct ggg      48
Met Arg Leu Leu Leu Ala Leu Leu Gly Val Leu Leu Ser Val Pro Gly
1               5                   10                  15
```

```
cct cca gtc ttg tcc ctg gag gcc tct gag gaa gtg gag ctt gag ccc        96
Pro Pro Val Leu Ser Leu Glu Ala Ser Glu Glu Val Glu Leu Glu Pro
            20                  25                  30

aac gct gtg ggc agc atc cgt tat aac tac ctg cta gat gtg ctg gag       144
Asn Ala Val Gly Ser Ile Arg Tyr Asn Tyr Leu Leu Asp Val Leu Glu
            35                  40                  45

cgg tcc ccg cac cgg ccc atc ctg cag gcc ggg ctc ccg gcc aac acc       192
Arg Ser Pro His Arg Pro Ile Leu Gln Ala Gly Leu Pro Ala Asn Thr
            50                  55                  60

aca gcc gtg gtg ggc agc gac gtg gag ctg ctg tgc aag gtg tac agc       240
Thr Ala Val Val Gly Ser Asp Val Glu Leu Leu Cys Lys Val Tyr Ser
65                  70                  75                  80

gat gcc cag ccc cac atc cag tgg ctg aag cac atc gtc atc aac ggc       288
Asp Ala Gln Pro His Ile Gln Trp Leu Lys His Ile Val Ile Asn Gly
                85                  90                  95

agc agc ttc gga gcc gac ggt ttc ccc tat gtg caa gtc cta aag act       336
Ser Ser Phe Gly Ala Asp Gly Phe Pro Tyr Val Gln Val Leu Lys Thr
            100                 105                 110

gca gac atc aat agc tca gag gtg gag gtc ctg tac ctg cgg aac gtg       384
Ala Asp Ile Asn Ser Ser Glu Val Glu Val Leu Tyr Leu Arg Asn Val
            115                 120                 125

tca gcc gag gac gca ggc gag tac acc tgc ctc gca ggc aat tcc atc       432
Ser Ala Glu Asp Ala Gly Glu Tyr Thr Cys Leu Ala Gly Asn Ser Ile
            130                 135                 140

ggc ctc tcc tac cag tct gcc tgg ctc acg gtg ctg cca gag gag gac       480
Gly Leu Ser Tyr Gln Ser Ala Trp Leu Thr Val Leu Pro Glu Glu Asp
145                 150                 155                 160

ccc aca tgg acc gca gca gcg ccc gag gcc aaa act cac aca tgc cca       528
Pro Thr Trp Thr Ala Ala Ala Pro Glu Ala Lys Thr His Thr Cys Pro
                165                 170                 175

ccg tgc cca gca cct gaa ctc ctg ggg gga ccg tca gtc ttc ctc ttc       576
Pro Cys Pro Ala Pro Glu Leu Leu Gly Gly Pro Ser Val Phe Leu Phe
            180                 185                 190

ccc cca aaa ccc aag gac acc ctc atg atc tcc cgg acc cct gag gtc       624
Pro Pro Lys Pro Lys Asp Thr Leu Met Ile Ser Arg Thr Pro Glu Val
            195                 200                 205

aca tgc gtg gtg gtg gac gtg agc cac gaa gac cct gag gtc aag ttc       672
Thr Cys Val Val Val Asp Val Ser His Glu Asp Pro Glu Val Lys Phe
            210                 215                 220

aac tgg tac gtg gac ggc gtg gag gtg cat aat gcc aag aca aag ccg       720
Asn Trp Tyr Val Asp Gly Val Glu Val His Asn Ala Lys Thr Lys Pro
225                 230                 235                 240

cgg gag gag cag tac aac agc acg tac cgt gtg gtc agc gtc ctc acc       768
Arg Glu Glu Gln Tyr Asn Ser Thr Tyr Arg Val Val Ser Val Leu Thr
                245                 250                 255

gtc ctg cac cag gac tgg ctg aat ggc aag gag tac aag tgc aag gtc       816
```

131

```
Val Leu His Gln Asp Trp Leu Asn Gly Lys Glu Tyr Lys Cys Lys Val
            260                 265                 270

tcc aac aaa gcc ctc cca gcc ccc atc gag aaa acc atc tcc aaa gcc      864
Ser Asn Lys Ala Leu Pro Ala Pro Ile Glu Lys Thr Ile Ser Lys Ala
            275                 280                 285

aaa ggg cag ccc cga gaa cca cag gtg tac acc ctg ccc cca tcc cgg      912
Lys Gly Gln Pro Arg Glu Pro Gln Val Tyr Thr Leu Pro Pro Ser Arg
        290                 295                 300

gat gag ctg acc aag aac cag gtc agc ctg acc tgc ctg gtc aaa ggc      960
Asp Glu Leu Thr Lys Asn Gln Val Ser Leu Thr Cys Leu Val Lys Gly
305                 310                 315                 320

ttc tat ccc agc gac atc gcc gtg gag tgg gag agc aat ggg cag ccg     1008
Phe Tyr Pro Ser Asp Ile Ala Val Glu Trp Glu Ser Asn Gly Gln Pro
                325                 330                 335

gag aac aac tac aag acc acg cct ccc gtg ctg gac tcc gac ggc tcc     1056
Glu Asn Asn Tyr Lys Thr Thr Pro Pro Val Leu Asp Ser Asp Gly Ser
            340                 345                 350

ttc ttc ctc tac agc aag ctc acc gtg gac aag agc agg tgg cag cag     1104
Phe Phe Leu Tyr Ser Lys Leu Thr Val Asp Lys Ser Arg Trp Gln Gln
        355                 360                 365

ggg aac gtc ttc tca tgc tcc gtg atg cat gag gct ctg cac aac cac     1152
Gly Asn Val Phe Ser Cys Ser Val Met His Glu Ala Leu His Asn His
        370                 375                 380

tac acg cag aag agc ctc tcc ctg tct ccg ggt tga                     1188
Tyr Thr Gln Lys Ser Leu Ser Leu Ser Pro Gly
385                 390                 395
```

<210> 116
<211> 395
<212> PRT
<213> Homo sapiens

<400> 116

```
Met Arg Leu Leu Leu Ala Leu Leu Gly Val Leu Leu Ser Val Pro Gly
1               5                   10              15

Pro Pro Val Leu Ser Leu Glu Ala Ser Glu Glu Val Glu Leu Glu Pro
            20                  25                  30

Asn Ala Val Gly Ser Ile Arg Tyr Asn Tyr Leu Leu Asp Val Leu Glu
            35                  40                  45

Arg Ser Pro His Arg Pro Ile Leu Gln Ala Gly Leu Pro Ala Asn Thr
    50                  55                  60

Thr Ala Val Val Gly Ser Asp Val Glu Leu Leu Cys Lys Val Tyr Ser
65                  70                  75                  80
```

```
Asp Ala Gln Pro His Ile Gln Trp Leu Lys His Ile Val Ile Asn Gly
                85               90               95

Ser Ser Phe Gly Ala Asp Gly Phe Pro Tyr Val Gln Val Leu Lys Thr
            100             105             110

Ala Asp Ile Asn Ser Ser Glu Val Glu Val Leu Tyr Leu Arg Asn Val
        115             120             125

Ser Ala Glu Asp Ala Gly Glu Tyr Thr Cys Leu Ala Gly Asn Ser Ile
    130             135             140

Gly Leu Ser Tyr Gln Ser Ala Trp Leu Thr Val Leu Pro Glu Glu Asp
145             150             155             160

Pro Thr Trp Thr Ala Ala Ala Pro Glu Ala Lys Thr His Thr Cys Pro
            165             170             175

Pro Cys Pro Ala Pro Glu Leu Leu Gly Gly Pro Ser Val Phe Leu Phe
        180             185             190

Pro Pro Lys Pro Lys Asp Thr Leu Met Ile Ser Arg Thr Pro Glu Val
    195             200             205

Thr Cys Val Val Val Asp Val Ser His Glu Asp Pro Glu Val Lys Phe
    210             215             220

Asn Trp Tyr Val Asp Gly Val Glu Val His Asn Ala Lys Thr Lys Pro
225             230             235             240

Arg Glu Glu Gln Tyr Asn Ser Thr Tyr Arg Val Val Ser Val Leu Thr
            245             250             255

Val Leu His Gln Asp Trp Leu Asn Gly Lys Glu Tyr Lys Cys Lys Val
            260             265             270

Ser Asn Lys Ala Leu Pro Ala Pro Ile Glu Lys Thr Ile Ser Lys Ala
    275             280             285

Lys Gly Gln Pro Arg Glu Pro Gln Val Tyr Thr Leu Pro Pro Ser Arg
    290             295             300

Asp Glu Leu Thr Lys Asn Gln Val Ser Leu Thr Cys Leu Val Lys Gly
305             310             315             320
```

```
Phe Tyr Pro Ser Asp Ile Ala Val Glu Trp Glu Ser Asn Gly Gln Pro
                325                 330                 335

Glu Asn Asn Tyr Lys Thr Thr Pro Pro Val Leu Asp Ser Asp Gly Ser
                340                 345                 350

Phe Phe Leu Tyr Ser Lys Leu Thr Val Asp Lys Ser Arg Trp Gln Gln
            355                 360                 365

Gly Asn Val Phe Ser Cys Ser Val Met His Glu Ala Leu His Asn His
            370                 375                 380

Tyr Thr Gln Lys Ser Leu Ser Leu Ser Pro Gly
385                 390                 395
```

<210> 117
<211> 330
<212> PRT
<213> Homo sapiens

<400> 117

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
                100                 105                 110

Pro Ala Pro Glu Leu Leu Gly Gly Pro Ser Val Phe Leu Phe Pro Pro
            115                 120                 125

Lys Pro Lys Asp Thr Leu Met Ile Ser Arg Thr Pro Glu Val Thr Cys
```

135

```
              130                     135                     140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
            195                 200                 205

Lys Ala Leu Pro Ala Pro Ile Glu Lys Thr Ile Ser Lys Ala Lys Gly
        210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
            325                 330
```